# EUROPEAN PATENT APPLICATION

(11) **EP 3 275 877 A1**
(43) Date of publication of application: **31.01.2018**
(21) Application number: 16181694.7
(22) Date of filing: 28.07.2016
(51) Int. Cl.: C07D 401/00, A01N 43/40, A01P 13/00

(54) **HERBICIDAL PYRIDINE COMPOUNDS**

(71) Applicant: BASF SE, 67056 Ludwigshafen am Rhein (DE)
(72) Inventor: VOGT, Florian, 67056 Ludwigshafen (DE); WITSCHEL, Matthias, 67056 Ludwigshafen (DE); SEISER, Tobias, 67056 Ludwigshafen (DE); LOPEZ CARRILLO, Veronica, 67056 Ludwigshafen (DE); SEITZ, Thomas, 67056 Ludwigshafen (DE); KRAEMER, Gerd, 67117 Limburgerhof (DE); NEWTON, Trevor William, 67117 Limburgerhof (DE); TRESCH, Stefan, 67056 Ludwigshafen (DE); SCHACHTSCHABEL, Doreen, 67117 Limburgerhof (DE); KREUZ, Klaus, 67117 Limburgerhof (DE)
(74) Representative: BASF IP Association

(57) **Abstract**

The present invention relates to the pyridine compounds of formula (I), 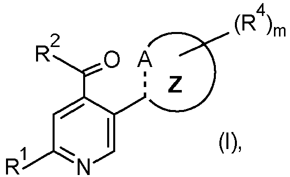 or their agriculturally acceptable salts or derivatives as herbicides, wherein the variables are defined according to the description, use of pyridine compounds of formula (I) as herbicides, compositions comprising them and their use as herbicides, i.e. for controlling harmful plants, and also a method for controlling unwanted vegetation which comprises allowing a herbicidal effective amount of at least one pyridine compounds of the formula (I) to act on plants, their seed and/or their habitat.

## Description

The present invention relates to pyridine compounds of the general formula (I) defined below and to their use as herbicides. Moreover, the invention relates to compositions for crop protection and to a method for controlling unwanted vegetation.

In agriculture, there is a constant demand to develop novel active ingredients, which complement or outperform present methods of treatment regarding activity, selectivity and environmental safety.

These and further objects are achieved by pyridine compounds of formula (I), defined below, and by their agriculturally suitable salts.

Accordingly, the present invention provides the pyridine compounds of formula (I) 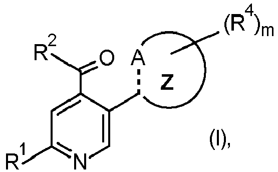 including their agriculturally acceptable salts or derivatives, provided the pyridine compounds of formula (I) have an acidic functionality,
wherein in formula (I) the variables have the following meanings:
the dotted line (------) is a single bond or a double bond;
- R¹: is C₁-C₆-alkyl, C₁-C₆-haloalkyl, hydroxy-C₁-C₆-alkyl, C₂-C₆-alkenyl, C₂-C₆-haloalkenyl, C₂-C₆-alkynyl, C₃-C₆-haloalkynyl, C₁-C₆-alkoxy-C₁-C₆-alkyl, C₁-C₆-alkoxy, C₃-C₆-alkenyloxy, C₃-C₆-haloalkenyloxy, C₃-C₆-alkynyloxy, C₃-C₆-haloalkynyloxy, C₁-C₆-haloalkoxy, C₃-C₆-cycloalkoxy, C₃-C₆-halocycloalkoxy, C₃-C₆-cycloalkenyloxy, C₃-C₆-halocycloalkenyloxy, C₁-C₆-alkylthio, C₁-C₆-haloalkylthio, (C₁-C₆-alkylamino, di(C₁-C₆-alkylamino, C₁-C₆-alkylsulfinyl, C₁-C₆-alkylsulfonyl, C₃-C₆-cycloalkyl, C₃-C₆-cycloalkenyl, C₃-C₆-halocycloalkyl, C₃-C₆-halocycloalkenyl, [1-(C₁-C₆-alkyl)]-C₃-C₆-cycloalkyl, [1-(C₂-C₆-alkenyl)]-C₃-C₆-cycloalkyl, [1-(C₂-C₆-alkynyl)]-C₃-C₆-cycloalkyl, [1-(C₁-C₆-haloalkyl)]-C₃-C₆-cycloalkyl, [1-(C₂-C₆-haloalkenyl)]-C₃-C₆-cycloalkyl, [1-(C₃-C₆-haloalkynyl)]-C₃-C₆-cycloalkyl, C₃-C₆-cycloalkylC₁-C₆-alkyl, C₃-C₆-cycloalkyl-C₁-C₆-haloalkyl, C₃-C₆-cycloalkyl-C₁-C₆-alkoxy, C₃-C₆-cycloalkyl-C₁-C₆-haloalkoxy, phenyl, 5- or 6-membered heteroaryl, or 3- to 6-membered heterocyclyl;
wherein the cyclic groups of R¹ are unsubstituted or substituted by R^{a};
- R²: is H, halogen, C₁-C₆-alkyl, C₁-C₆-haloalkyl, C₁-C₆-alkylcarbonyl-C₁-C₆-alkyl, C₁-C₆-alkoxycarbonyl-C₁-C₆-alkyl, C₁-C₆-haloalkylcarbonyl-C₁-C₆-alkyl, C₁-C₆-haloalkoxycarbonyl-C₁-C₆-alkyl, C₁-C₆-alkylcarbonyl-C₁-C₆-haloalkyl, C₁-C₆-alkoxycarbonyl-C₁-C₆-haloalkyl, C₁-C₆-haloalkylcarbonyl-C₁-C₆-haloalkyl, C₁-C₆-haloalkoxycarbonyl-C₁-C₆-haloalkyl, OH, C₁-C₆-alkoxy, C₁-C₆-alkoxy-C₁-C₆-alkoxy, C₁-C₆-haloalkoxy-C₁-C₆-alkoxy, C₁-C₆-alkoxy-C₁-C₆-haloalkoxy, C₁-C₆-haloalkoxy-C₁-C₆-haloalkoxy, C₁-C₆-alkoxy-C₁-C₆-alkoxy-C₁-C₆-alkoxy, C₁-C₆-haloalkoxy, C₁-C₆-cyanoalkoxy, C₁-C₆-hydroxyalkoxy, C₃-C₆-alkenyloxy, C₃-C₆-alkenyloxy-C₁-C₆-alkoxy, C₃-C₆-haloalkenyloxy-C₁-C₆-haloalkoxy, C₃-C₆-alkenyloxy- C₁-C₆-haloalkoxy, C₃-C₆-haloalkenyloxy, C₃-C₆-alkynyloxy, C₃-C₆-haloalkynyloxy, C₃-C₆-alkynyloxy-C₁-C₆-alkoxy, C₃-C₆-haloalkynyloxy-C₁-C₆-haloalkoxy, C₃-C₆-alkynyloxy- C₁-C₆-haloalkoxy, C₃-C₆-alkynyloxy-C₃-C₆-alkenyloxy, C₃-C₆-haloalkynyloxy-C₃-C₆-alkenyloxy, C₃-C₆-alkynyloxy-C₃-C₆-haloalkenyloxy, C₃-C₆-haloalkynyloxy-C₃-C₆-haloalkenyloxy, C₃-C₆-alkynyloxy-C₃-C₆-alkynyloxy, C₃-C₆-haloalkynyloxy-C₃-C₆-alkynyloxy, C₃-C₆-alkynyloxy-C₃-C₆-haloalkynyloxy, C₃-C₆-haloalkynyloxy-C₃-C₆-haloalkynyloxy, (C₁-C₆-alkyl)carbonyl-C₁-C₆-alkoxy, (C₁-C₆-haloalkyl)carbonyl-C₁-C₆-haloalkoxy, (C₁-C₆-haloalkyl)carbonyl-C₁-C₆-alkoxy, (C₁-C₆-alkyl)carbonyl-C₁-C₆-haloalkoxy, (C₁-C₆-alkoxy)carbonyl-C₁-C₆-alkoxy, (C₁-C₆-haloalkoxy)carbonyl-C₁-C₆-alkoxy, (C₁-C₆-alkoxy)carbonyl-C₁-C₆-haloalkoxy, (C₁-C₆-haloalkoxy)carbonyl-C₁-C₆-haloalkoxy, (C₁-C₆-alkoxy-C₁-C₆-alkyl)carbonyl-C₁-C₆-alkoxy, (C₁-C₆-haloalkoxy-C₁-C₆-alkyl)carbonyl-C₁-C₆-alkoxy, (C₁-C₆-alkoxy-C₁-C₆-haloalkyl)carbonyl-C₁-C₆-alkoxy, (C₁-C₆-alkoxy-C₁-C₆-alkyl)carbonyl-C₁-C₆-haloalkoxy, (C₁-C₆-haloalkoxy-C₁-C₆-haloalkyl)carbonyl-C₁-C₆-alkoxy, (C₁-C₆-haloalkoxyC₁-C₆-alkyl)carbonyl-C₁-C₆-haloalkoxy, (C₁-C₆-alkoxy-C₁-C₆-haloalkyl)carbonyl-C₁-C₆-haloalkoxy, (C₁-C₆-haloalkoxy-C₁-C₆-haloalkyl)carbonyl-C₁-C₆-haloalkoxy, (C₁-C₆-alkylthio)carbonyl-C₁-C₆-alkoxy, (C₁-C₆-haloalkylthio)carbonyl-C₁-C₆-alkoxy, (C₁-C₆-alkylthio)carbonyl-C₁-C₆-haloalkoxy, (C₁-C₆-haloalkylthio)carbonyl-C₁-C₆-haloalkoxy, (C₁-C₆-alkylthio-C₁-C₆-alkyl)carbonyl-C₁-C₆-alkoxy, (C₁-C₆-haloalkylthio-C₁-C₆-alkyl)carbonyl-C₁-C₆-alkoxy, (C₁-C₆-alkylthio-C₁-C₆-haloalkyl)carbonyl-C₁-C₆-alkoxy, (C₁-C₆-alkylthio-C₁-C₆-alkyl)carbonyl-C₁-C₆-haloalkoxy, (C₁-C₆-haloalkylthio-C₁-C₆-haloalkyl)carbonyl-C₁-C₆-alkoxy, (C₁-C₆-haloalkylthio-C₁-C₆-alkyl)carbonyl-C₁-C₆-haloalkoxy, (C₁-C₆-alkylthio-C₁-C₆-haloalkyl)carbonyl-C₁-C₆-haloalkoxy, (C₁-C₆-haloalkylthio-C₁-C₆-haloalkyl)carbonyl-C₁-C₆-haloalkoxy, C₃-C₆-cycloalkoxy, C₃-C₆-halocycloalkoxy, (C₃-C₆-cycloalkyl)C₁-C₆-alkoxy, (C₃-C₆-halocycloalkyl)C₁-C₆-alkoxy, (C₃-C₆-cycloalkyl)C₁-C₆-haloalkoxy, aminocarbonyl- C₁-C₆-alkoxy, (C₃-C₆-halocycloalkyl)C₁-C₆-haloalkoxy, aminocarbonyl- C₁-C₆-haloalkoxy, N-(C₁-C₆-alkyl)-aminocarbonyl-C₁-C₆-alkoxy, N-(C₁-C₆-alkyl)-aminocarbonyl-C₁-C₆-haloalkoxy, N,N-di(C₁-C₆-alkyl)-aminocarbonyl-C₁-C₆-alkoxy, N,N-di(C₁-C₆-alkyl)-aminocarbonyl-C₁-C₆-haloalkoxy, O-N=C(di(phenyl), O-N=C(phenyl)(C₁-C₆-alkyl), O-N=C[di(C₁-C₆-alkyl)], (C₁-C₆-alkyl)₃-silyl-C₁-C₆-alkoxy, C₁-C₆-alkylthio, C₁-C₆-haloalkylthio, C₁-C₆-alkoxy-C₁-C₆-alkylthio, C₁-C₆-haloalkoxy-C₁-C₆-alkylthio, C₁-C₆-alkoxy-C₁-C₆-haloalkylthio, C₁-C₆-haloalkoxy-C₁-C₆-haloalkylthio, C₁-C₆-alkoxy-C₁-C₆-alkoxy-C₁-C₆-alkylthio, C₁-C₆-cyanoalkylthio, C₃-C₆-alkenylthio, C₃-C₆-haloalkenylthio, C₃-C₆-alkenyloxy-C₁-C₆-alkylthio, C₃-C₆-haloalkenyloxy-C₁-C₆-alkylthio, C₃-C₆-alkenyloxy-C₁-C₆-haloalkylthio, C₃-C₆-haloalkenyloxy-C₁-C₆-haloalkylthio, C₃-C₆-alkynylthio, C₃-C₆-haloalkynylthio, C₃-C₆-alkynyloxy-C₁-C₆-alkylthio, C₃-C₆-haloalkynyloxy-C₁-C₆-haloalkylthio, C₃-C₆-alkynyloxy-C₁-C₆-haloalkylthio, C₃-C₆-alkynyloxy- C₃-C₆-alkenylthio, C₃-C₆-haloalkynyloxy-C₃-C₆-alkenylthio, C₃-C₆-alkynyloxy-C₃-C₆-haloalkenylthio, C₃-C₆-haloalkynyloxy-C₃-C₆-haloalkenylthio, C₃-C₆-alkynyloxy-C₃-C₆-alkynylthio, C₃-C₆-haloalkynyloxy-C₃-C₆-alkynylthio, C₃-C₆-alkynyloxy-C₃-C₆-haloalkynylthio, C₃-C₆-haloalkynyloxy-C₃-C₆-haloalkynylthio, (C₁-C₆-alkyl)carbonyl-C₁-C₆-alkylthio, (C₁-C₆-haloalkyl)carbonyl-C₁-C₆-alkylthio, (C₁-C₆-alkyl)carbonyl-C₁-C₆-haloalkylthio, (C₁-C₆-haloalkyl)carbonyl-C₁-C₆-haloalkylthio, (C₁-C₆-alkoxy)carbonyl-C₁-C₆-alkylthio, (C₁-C₆-haloalkoxy)carbonyl-C₁-C₆-alkylthio, (C₁-C₆-alkoxy)carbonyl-C₁-C₆-haloalkylthio, (C₁-C₆-haloalkoxy)carbonyl-C₁-C₆-haloalkylthio, (C₁-C₆-alkoxy-C₁-C₆-alkyl)carbonyl-C₁-C₆-alkylthio, (C₁-C₆-haloalkoxy-C₁-C₆-alkyl)carbonyl-C₁-C₆-alkylthio, (C₁-C₆-alkoxy-C₁-C₆-haloalkyl)carbonyl-C₁-C₆-alkylthio, (C₁-C₆-alkoxy-C₁-C₆-alkyl)carbonyl-C₁-C₆-haloalkylthio, (C₁-C₆-haloalkoxy-C₁-C₆-haloalkyl)carbonyl-C₁-C₆-alkylthio, (C₁-C₆-haloalkoxy-C₁-C₆-alkyl)carbonyl-C₁-C₆-haloalkylthio, (C₁-C₆-alkoxy-C₁-C₆-haloalkyl)carbonyl-C₁-C₆-haloalkylthio, (C₁-C₆-haloalkoxy-C₁-C₆-haloalkyl)carbonyl-C₁-C₆-haloalkylthio, (C₁-C₆-alkylthio)carbonyl-C₁-C₆-alkylthio, (C₁-C₆-haloalkylthio)carbonyl-C₁-C₆-alkylthio, (C₁-C₆-alkylthio)carbonyl-C₁-C₆-haloalkylthio, (C₁-C₆-haloalkylthio)carbonyl-C₁-C₆-haloalkylthio, (C₁-C₆-alkylthio-C₁-C₆-alkyl)carbonyl-C₁-C₆-alkylthio, (C₁-C₆-haloalkylthio-C₁-C₆-alkyl)carbonyl-C₁-C₆-alkylthio, (C₁-C₆-alkylthio-C₁-C₆-haloalkyl)carbonyl-C₁-C₆-alkylthio, (C₁-C₆-alkylthio-C₁-C₆-alkyl)carbonyl-C₁-C₆-haloalkylthio, (C₁-C₆-haloalkylthio-C₁-C₆-haloalkyl)carbonyl-C₁-C₆-alkylthio, (C₁-C₆-haloalkylthio-C₁-C₆-alkyl)carbonyl-C₁-C₆-haloalkylthio, (C₁-C₆-alkylthio-C₁-C₆-haloalkyl)carbonyl-C₁-C₆-haloalkylthio, (C₁-C₆-haloalkylthio-C₁-C₆-haloalkyl)car-bonyl-C₁-C₆-haloalkylthio, C₃-C₆-cycloalkylthio, C₃-C₆-halocycloalkylthio, (C₃-C₆-cycloalkyl)C₁-C₆-alkylthio, (C₃-C₆-cycloalkyl)C₁-C₆-haloalkylthio, (C₃-C₆-halocycloalkyl)C₁-C₆-alkylthio, (C₃-C₆-halocycloalkyl)C₁-C₆-haloalkylthio, aminocarbonyl- C₁-C₆-alkylthio, aminocarbonyl- C₁-C₆-haloalkylthio, N-(C₁-C₆-alkyl)-aminocarbonyl-C₁-C₆-alkylthio, N-(C₁-C₆-haloalkyl)-aminocarbonyl-C₁-C₆-alkylthio, N-(C₁-C₆-alkyl)-aminocarbonyl-C₁-C₆-haloalkylthio, N-(C₁-C₆-haloalkyl)-aminocarbonyl-C₁-C₆-haloalkylthio, N,N-di(C₁-C₆-alkyl)-aminocarbonyl- C₁-C₆-alkylthio, N,N-di(C₁-C₆-haloalkyl)-aminocarbonyl-C₁-C₆-alkylthio, N,N-di(C₁-C₆-alkyl)-aminocarbonyl-C₁-C₆-haloalkylthio, N,N-di(C₁-C₆-haloalkyl)-aminocarbonyl-C₁-C₆-haloalkylthio, NH₂, (C₁-C₆-alkyl)amino, hydroxyamino, (C₁-C₆-alkoxy)amino, (C₃-C₆-cycloalkoxy)amino, (C₁-C₆-alkyl)sulfinylamino, (C₁-C₆-alkyl)sulfonylamino, (amino)sulfinylamino, [(C₁-C₆-alkyl)amino]sulfinylamino, (amino)sulfonylamino, [(C₁-C₆-alkyl)amino]sulfonylamino, [di(C₁-C₆-alkyl)amino]sulfonylamino, di(C₁-C₆-alkyl)amino, (hydroxy)(C₁-C₆-alkyl)amino, (hydroxy)(C₁-C₆-cycloalkyl)amino, (C₁-C₆-alkoxy)(C₁-C₆-alkyl)amino, (C₁-C₆-alkoxy)(C₃-C₆-cycloalkyl)amino, (C₃-C₆-cycloalkoxy)(C₁-C₆-alkyl)amino, (C₃-C₆-cycloalkoxy)(C₃-C₆-cycloalkyl)amino, [(C₁-C₆-alkyl)sulfinyl](C₁-C₆-alkyl)amino, [(C₁-C₆-alkyl)sulfonyl](C₁-C₆-alkyl)amino, [di(C₁-C₆-alkyl)amino]sulfinylamino, [di(C₁-C₆-alkyl)amino]sulfonylamino, phenyloxy, phenyl-C₁-C₆-alkoxy, phenylthio, phenyl-C₁-C₆-alkylthio, phenylamino, (C₁-C₆-alkyl)(phenyl)amino, C₁-C₆-alkylcarbonylamino, [(C₁-C₆-alkyl)carbonyl](C₁-C₆-alkyl)amino, C₁-C₆-haloalkylcarbonylamino, [(C₁-C₆-haloalkyl)carbonyl](C₁-C₆-alkyl)amino, C₃-C₆-cycloalkylcarbonylamino, [(C₃-C₆-cycloalkyl)carbonyl](C₁-C₆-alkyl)amino, phenylcarbonylamino, (phenylcarbonyl)(C₁-C₆-alkyl)amino, heterocyclylcarbonylamino, (heterocyclylcarbonyl)(C₁-C₆-alkyl)amino, heteroarylcarbonylamino, (heteroarylcarbonyl)(C₁-C₆-alkyl)amino, [(C₁-C₆-alkyl)carbonyl](C₁-C₆-alkoxy)amino, [(C₁-C₆-haloalkyl)carbonyl](C₁-C₆-alkoxy)amino, [(C₃-C₆-cycloalkyl)carbonyl](C₁-C₆-alkyloxy)amino, (phenylcarbonyl)(C₁-C₆-alkoxy)amino, (heterocyclylcarbonyl)(C₁-C₆-alkoxy)amino, (heteroarylcarbonyl)(C₁-C₆-alkoxy)amino, [(C₁-C₆-alkyl)carbonyl](C₂-C₆-alkenyl)amino, [(C₁-C₆-haloalkyl)carbonyl](C₂-C₆-alkenyl)amino, [(C₃-C₆-cycloalkyl)carbonyl](C₂-C₆-alkenyl)amino, (phenylcarbonyl)(C₂-C₆-alkenyl)amino, (heterocyclylcarbonyl)(C₂-C₆-alkenyl)amino, (heteroarylcarbonyl)(C₂-C₆-alkenyl)amino, [(C₁-C₆-alkyl)carbonyl](C₃-C₆-alkynyl)amino, [(C₁-C₆-haloalkyl)carbonyl](C₃-C₆-alkynyl)amino, [(C₃-C₆-cycloalkyl)carbonyl](C₃-C₆-alkynyl)amino, (phenylcarbonyl)(C₃-C₆-alkynyl)amino, (heterocyclylcarbonyl)(C₃-C₆-alkynyl)amino, (heteroarylcarbonyl)(C₃-C₆-alkynyl)amino, [(C₂-C₆-alkenyl)carbonyl]amino, [(C₂-C₆-alkenyl)carbonyl](C₁-C₆-alkyl)amino, [(C₂-C₆-alkenyl)carbonyl](C₁-C₆-alkoxy)amino, [(C₃-C₆-alkynyl)carbonyl]amino, [(C₃-C₆-alkynyl)carbonyl](C₁-C₆-alkyl)amino, [(C₃-C₆-alkynyl)carbonyl](C₁-C₆-alkoxy)amino, [di(C₁-C₆-alkyl)amino]carbonylaminocarbonyl, [di(C₁-C₆-alkyl)aminocarbonyl](C₁-C₆-alkyl)amino, [di(C₁-C₆-alkyl)aminocarbonyl](C₁-C₆-alkoxy)amino, (heteroaryl)oxy, heteroaryl-C₁-C₆-alkoxy, (heterocyclyl)oxy, or heterocyclyl-C₁-C₆-alkoxy;
wherein the cyclic groups of R² are unsubstituted or substituted by R^{a};
- Z: is a 9 or 10 membered bicyclic ring comprising A;
- A: is C*, CR³, NR^{3A}, N, O, or S;
- C*: is a bridge carbon of the bicyclic ring Z;
- R³: is halogen, CN, CHO, NO₂, C₁-C₆-alkyl, C₁-C₆-haloalkyl, C₁-C₆-alkylcarbonyl, C₂-C₆-alkenyl, C₂-C₆-haloalkenyl, C₂-C₆-alkenyl, C₂-C₆-haloalkenyl, C₁-C₆-alkoxy, C₁-C₆-haloalkoxy, C₃-C₆-alkenyloxy, C₃-C₆-haloalkenylo_{X}y, C₃-C₆-alkenyloxy, C₃-C₆-haloalkenyloxy, C₁-C₆-alkoxy-C₁-C₆-alkoxy, hydroxycarbonyl, C₁-C₆-alkoxycarbonyl, C₁-C₆-alkylthio, C₁-C₆-haloalkylthio, NH₂, (C₁-C₆-alkyl)amino, di(C₁-C₆-alkylamino, (C₁-C₆-alkyl)sulfinyl, (C₁-C₆-alkyl)sulfonyl, C₃-C₆-cycloalkyl, (C₃-C₆-cycloalkyl)oxy, or phenyl;
wherein the cyclic groups of R³ are unsubstituted or substituted by substituents R^{a};
- R^{3A}: is H, C₁-C₆-alkyl, C₁-C₆-haloalkyl, C₁-C₆-alkylcarbonyl, C₂-C₆-alkenyl, C₂-C₆-haloalkenyl, C₂-C₆-alkenyl, C₂-C₆-haloalkenyl, C₁-C₆-alkoxy, C₁-C₆-haloalkoxy, C₃-C₆-alkenyloxy, C₃-C₆-haloalkenyloxy, C₃-C₆-alkenyloxy, C₃-C₆-haloalkenyloxy, C₁-C₆-alkoxy-C₁-C₆-alkoxy, C₁-C₆-alkoxycarbonyl, C₁-C₆-alkylthio, C₁-C₆-haloalkylthio, NH₂, (C₁-C₆-alkyl)amino, di(C₁-C₆-alkyl)amino, (C₁-C₆-alkyl)sulfinyl, (C₁-C₆-alkyl)sulfonyl, C₃-C₆-cycloalkyl, (C₃-C₆-cycloalkyl)oxy, or phenyl;
wherein the cyclic groups of R^{3A} are unsubstituted or substituted by R^{a};
- R⁴: is halogen, CN, CHO, NO₂, C₁-C₆-alkyl, C₁-C₆-haloalkyl, C₁-C₆-alkylcarbonyl, C₂-C₆-alkenyl, C₂-C₆-haloalkenyl, C₂-C₆-alkenyl, C₂-C₆-haloalkenyl, C₁-C₆-alkoxy, C₁-C₆-haloalkoxy, C₃-C₆-alkenyloxy, C₃-C₆-haloalkenyloxy, C₃-C₆-alkenyloxy, C₃-C₆-haloalkenyloxy, C₁-C₆-alkoxy-C₁-C₆-alkoxy, hydroxycarbonyl, C₁-C₆-alkoxycarbonyl, C₁-C₆-alkylthio, C₁-C₆-haloalkylthio, NH₂, (C₁-C₆-alkyl)amino, di(C₁-C₆-alkylamino, (C₁-C₆-alkyl)sulfinyl, (C₁-C₆-alkyl)sulfonyl, C₃-C₆-cycloalkyl, (C₃-C₆-cycloalkyl)oxy, or phenyl;
wherein the cyclic groups of R⁴ are unsubstituted or substituted by R^{a};
- R^{a}: is halogen, CN, NO₂, C₁-C₆-alkyl, C₁-C₆-haloalkyl, C₁-C₆-alkoxy, or C₁-C₆-haloalkoxy;
- m: is 0, 1, 2, or 3.

The present invention also provides use of pyridine compounds of formula (I) as described herein including their agriculturally acceptable salts or derivatives, provided the pyridine compounds of formula (I) have an acidic functionality, as herbicide.

The pyridine compounds of formula (I) according to the invention can be prepared by standard processes of organic chemistry, for example by the following processes:

### Process A:

The pyridine compounds of formula (I) can be obtained by reacting pyridines of formula (II) with boronic acids/esters of formula (III):

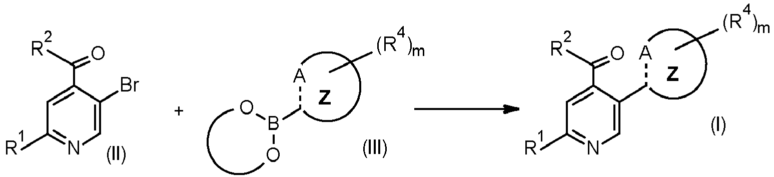

The reaction of the pyridine (II) with boronic acids/esters (III) is usually carried out from 0 °C to the boiling point of the reaction mixture, preferably from 15 °C to 110 °C, particularly preferably from 40 °C to 100 °C, in an inert organic solvent in the presence of a base and a catalyst.

The reaction may in principle be carried out in substance. However, preference is given to reacting the pyridines (II) with the boronic acids/esters (III) in an organic solvent with or without water as co-solvent.

Suitable in principle are all solvents which are capable of dissolving the pyridines (II) and the boronic acids (III) at least partly and preferably fully under the reaction conditions.

Examples of suitable solvents are aromatic hydrocarbons such as benzene, chlorobenzene, toluene, cresols, o-, m- and p-xylene, ethers such as diethyl ether, diisopropyl ether, tert.-butyl methylether (TBME), dioxane, anisole and tetrahydrofuran (THF), as well as dipolar aprotic solvents such as sulfolane, dimethylsulfoxide, N,N-dimethylformamide (DMF), N,N-dimethylacetamide (DMAC), 1,3-dimethyl-2-imidazolidinone (DMI), N,N'-dimethylpropylene urea (DMPU), dimethyl sulfoxide (DMSO) and 1-methyl-2 pyrrolidinone (NMP).

Preferred solvents are ethers such as diethyl ether, diisopropyl ether, tert.-butyl methylether (TBME), dioxane, anisole and tetrahydrofuran (THF) and dipolar aprotic solvents such as sulfolane, dimethylsulfoxide, N,N-dimethylformamide (DMF), N,N-dimethylacetamide (DMAC), 1,3-dimethyl-2-imidazolidinone (DMI), N,N'-dimethyl¬propylene urea (DMPU), dimethyl sulfoxide (DMSO) and 1-methyl-2 pyrroli-dinone (NMP).

More preferred solvents are ethers such as diethyl ether, diisopropyl ether, tert.-butyl methylether (TBME), dioxane, anisole and tetrahydrofuran (THF).

It is also possible to use mixtures of the solvents mentioned.

Examples of suitable metal-containing bases are inorganic compounds including metal-containing bases such as alkali metal and alkaline earth metal hydroxides, and other metal hydroxides, such as lithium hydroxide, sodium hydroxide, potassium hydroxide, magnesium hydroxide, calcium hydroxide and aluminum hydroxide; alkali metal and alkaline earth metal oxide, and other metal oxides, such as lithium oxide, sodium oxide, potassium oxide, magnesium oxide, calcium oxide and magnesium oxide, iron oxide, silver oxide; alkali metal and alkaline earth metal carbonates such as lithium carbonate, sodium carbonate, potassium carbonate, cesium carbonate magnesium carbonate, and calcium carbonate, as well as alkali metal hydrogen carbonates (bicarbonates) such as lithium hydrogen carbonate, sodium hydrogen carbonate, potassium hydrogen carbonate; alkali metal and alkaline earth metal phosphates such as potassium phosphate, calcium phosphate; alkali metal and alkaline earth metal acetates such as sodium acetate or potassium acetate.

Preferred bases are inorganic compounds such as alkali metal and alkaline earth metal hydroxides, and other metal hydroxides, such as lithium hydroxide, sodium hydroxide, potassium hydroxide, magnesium hydroxide, calcium hydroxide and aluminum hydroxide and alkali metal or alkaline earth metal carbonates such as lithium carbonate, sodium carbonate, potassium carbonate, cesium carbonate, magnesium carbonate, and calcium carbonate and alkaline earth metal phosphates such as potassium phosphate; ; alkali metal and alkaline earth metal acetates such as sodium acetate.

Especially preferred bases are inorganic compounds such as alkali metal and alkaline earth metal hydroxides, and other metal hydroxides, such as lithium hydroxide, sodium hydroxide, potassium hydroxide, magnesium hydroxide, calcium hydroxide and aluminum hydroxide and alkaline earth metal phosphates such as potassium phosphate.

The term base as used herein also includes mixtures of two or more, preferably two of the above compounds. Particular preference is given to the use of one base.

The bases are used preferably from 1 to 10 equivalents based on the pyridine (II), more preferably from 1.0 to 5.0 equivalents based on the pyridine (II), most preferably from 1.2 to 2.5 equivalents based on the pyridine (II).

It may be advantageous to add the base offset over a period of time.

The reaction of the pyridines (II) with the boronic acids/esters (III) is carried out in the presence of a catalyst. Examples of suitable catalysts include for example, palladium based catalysts like, for example, Palladium(II)acetate, tetrakis(triphenylphosphine)palladium(0), bis(tri-phenylphosphine)palladium(II)chloride or (1,1,-bis(diphenylphosphino)ferrocene)-dichloropalladium(II), and optionally suitable additives such as, for example, phosphines like, for example, P(o-tolyl)₃, tri-phenyl phosphine or BINAP (2,2'-Bis(diphenylphospino)-1,1'-binaphthyl).

The amount of catalyst is usually 0.01 to 20 mol % (0.0001 to 0.2 equivalents) based on the pyridine (II).

The end of the reaction can easily be determined by the skilled worker by means of routine methods.

The reaction mixtures are worked up in a customary manner, for example by mixing with water, separation of the phases and, if appropriate, chromatographic purification of the crude product.

Some of the intermediates and end products are obtained in the form of viscous oils, which can be purified or freed from volatile components under reduced pressure and at moderately elevated temperature.

If the intermediates and the end products are obtained as solid, purification can also be carried out by recrystallisation or digestion.

Pyridine compounds (I) wherein R² is OH can be easily obtained from a corresponding ester (I) in which R² equals an alkoxy group (e.g. R = Me) by methods known to a person skilled in the art.

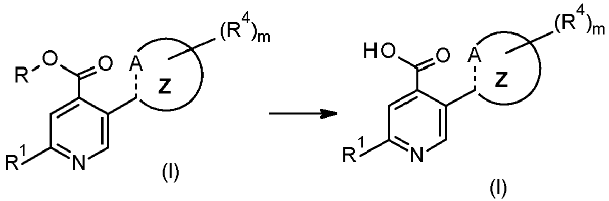

Alternatively pyridine compounds (I), wherein R² has any one of the above mentioned meanings except OH, can also be obtained by modifying pyridine compounds (I) wherein R² is OH by known methods (e.g. "oxy-substituents" except "OH" analogous to Arnab, P. et. al. Angew. Chem. Int. Ed. 2010, 49, 1492-1495; "thio-substituents" analogous to Silvestri, M. A. et. al. J. Med. Chem. 2004, 47, 3149-3162; "amino-substituents" analogous to Kuhn, B. et. al. J. Med. Chem. 2010, 53, 2601-2611).

The boronic acids or esters (III) required for the preparation of pyridine compounds of formula (I) are commercially available, known from literature or can easily prepared analogously to published procedures (e.g. Kamei et al. Tetrahedron Lett. 2014, 55, 4245 - 4247).

The pyridines (II) can easily prepared by the corresponding pyridines (IV) by modification of known methods.

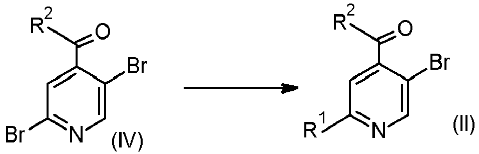

E.g. for R¹ = alkyl, haloalkyl, cycloalkyl, alkenyl, haloalkenyl, alkynyl, haloalkynyl, alkoxy-alkyl, cycloalkenyl, halocycloalkenyl, cycloalkyl-alkyl, phenyl, 5- or 6-membered heteroaryl, or 3- to 6-membered heterocyclyl cross-coupling reactions can be used (methods analog to WO 2006099972, to J. Med. Chem. 2000, 43, 4288-4312 or to Org. Lett. 2014,16, 5466-5469). For e.g. R¹ = alkoxy, haloalkoxy, alkenyloxy, haloalkenyloxy, alkynyloxy, haloalkynyloxy, cycloalkoxy, halocycloalkoxy, cycloalkenyloxy, halocycloalkenyloxy, alkylthio or (alkyl)amino S_{N}Ar substitution reactions can be used (methods analog to Chem. Eur. J. 2012, 18, 2498-2502, J. Med. Chem. 2012, 55, 10584-10600, WO2009099080 or to WO 2011100769).

The pyridine (IV) wherein R² is OH is commercially available.

To obtain the other pyridines (IV), wherein R² has any one of the above mentioned meanings except OH, the pyridines (IV) wherein R² is OH can easily be modified by known methods (e.g. "oxy-substituents" except "OH" analogous to Arnab, P. et. al. Angew. Chem. Int. Ed. 2010, 49, 1492-1495; "thio-substituents" analogous to Silvestri, M. A. et. al. J. Med. Chem. 2004, 47, 3149-3162; "amino-substituents" analogous to Kuhn, B. et. al. J. Med. Chem. 2010, 53, 2601-2611).

### Process B:

The pyridine compounds of formula (I) can in addition be obtained by reacting respective pyridine boronic acid esters of formula (V) with halides of formula (VI) in which X equals Cl, Br or I:

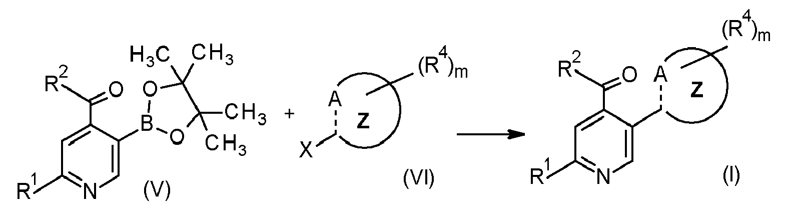

The reaction of the pyridine (V) with halides (VI) is usually carried out from 0 °C to the boiling point of the reaction mixture, preferably from 15 °C to 110 °C, particularly preferably from 40 °C to 100 °C, in an inert organic solvent in the presence of a base and a catalyst.

The reaction may in principle be carried out in substance. However, preference is given to reacting the pyridines (V) with the halides (VI) in an organic solvent with or without water as co-solvent.

Suitable in principle are all solvents which are capable of dissolving the pyridines (V) and the halides (VI) at least partly and preferably fully under the reaction conditions.

Examples of suitable solvents are aromatic hydrocarbons such as benzene, chlorobenzene, toluene, cresols, o-, m- and p-xylene, ethers such as diethyl ether, diisopropyl ether, tert.-butyl methylether (TBME), dioxane, anisole and tetrahydrofuran (THF), as well as dipolar aprotic solvents such as sulfolane, dimethylsulfoxide, N,N-dimethylformamide (DMF), N,N-dimethylacetamide (DMAC), 1,3-dimethyl-2-imidazolidinone (DMI), N,N'-dimethylpropylene urea (DMPU), dimethyl sulfoxide (DMSO) and 1-methyl-2 pyrrolidinone (NMP).

Preferred solvents are ethers such as diethyl ether, diisopropyl ether, tert.-butyl methylether (TBME), dioxane, anisole and tetrahydrofuran (THF) and dipolar aprotic solvents such as sulfolane, dimethylsulfoxide, N,N-dimethylformamide (DMF), N,N-dimethylacetamide (DMAC), 1,3-dimethyl-2-imidazolidinone (DMI), N,N'-dimethyl¬propylene urea (DMPU), dimethyl sulfoxide (DMSO) and 1-methyl-2 pyrroli-dinone (NMP).

More preferred solvents are ethers such as diethyl ether, diisopropyl ether, tert.-butyl methylether (TBME), dioxane, anisole and tetrahydrofuran (THF).

It is also possible to use mixtures of the solvents mentioned.

Examples of suitable metal-containing bases are inorganic compounds including metal-containing bases such as alkali metal and alkaline earth metal hydroxides, and other metal hydroxides, such as lithium hydroxide, sodium hydroxide, potassium hydroxide, magnesium hydroxide, calcium hydroxide and aluminum hydroxide; alkali metal and alkaline earth metal oxide, and other metal oxides, such as lithium oxide, sodium oxide, potassium oxide, magnesium oxide, calcium oxide and magnesium oxide, iron oxide, silver oxide; alkali metal and alkaline earth metal carbonates such as lithium carbonate, sodium carbonate, potassium carbonate, cesium carbonate magnesium carbonate, and calcium carbonate, as well as alkali metal hydrogen carbonates (bicarbonates) such as lithium hydrogen carbonate, sodium hydrogen carbonate, potassium hydrogen carbonate; alkali metal and alkaline earth metal phosphates such as potassium phosphate, calcium phosphate.

Preferred bases are inorganic compounds such as alkali metal and alkaline earth metal hydroxides, and other metal hydroxides, such as lithium hydroxide, sodium hydroxide, potassium hydroxide, magnesium hydroxide, calcium hydroxide and aluminum hydroxide and alkali metal or alkaline earth metal carbonates such as lithium carbonate, sodium carbonate, potassium carbonate, cesium carbonate, magnesium carbonate, and calcium carbonate and alkaline earth metal phosphates such as potassium phosphate.

Especially preferred bases are inorganic compounds such as alkali metal and alkaline earth metal hydroxides, and other metal hydroxides, such as lithium hydroxide, sodium hydroxide, potassium hydroxide, magnesium hydroxide, calcium hydroxide and aluminum hydroxide and alkaline earth metal phosphates such as potassium phosphate.

The term base as used herein also includes mixtures of two or more, preferably two of the above compounds. Particular preference is given to the use of one base.

The bases are used preferably from 1 to 10 equivalents based on the pyridine (V), more preferably from 1.0 to 5.0 equivalents based on the pyridine (V), most preferably from 1.2 to 2.5 equivalents based on the pyridine (V).

It may be advantageous to add the base offset over a period of time.

The reaction of the pyridines (V) with the halides (VI) is carried out in the presence of a catalyst. Examples of suitable catalysts include for example, palladium based catalysts like, for example, Palladium(II)acetate, tetrakis(triphenylphosphine)palladium(0), bis(tri-phenylphosphine)palladium(II)chloride or (1,1,-bis(diphenylphosphino)ferrocene)-dichloropalladium(II), and optionally suitable additives such as, for example, phosphines like, for example, P(o-tolyl)₃, triphenylphosphine or BINAP (2,2'-Bis(diphenylphospino)-1,1'-binaphthyl).

The amount of catalyst is usually 0.01 to 20 mol % (0.0001 to 0.2 equivalents) based on the pyridine (V).

The end of the reaction can easily be determined by the skilled worker by means of routine methods.

The reaction mixtures are worked up in a customary manner, for example by mixing with water, separation of the phases and, if appropriate, chromatographic purification of the crude product.

Some of the intermediates and end products are obtained in the form of viscous oils, which can be purified or freed from volatile components under reduced pressure and at moderately elevated temperature.

If the intermediates and the end products are obtained as solid, purification can also be carried out by recrystallization or digestion.

The halides (VI) required for the preparation of pyridine compounds of formula (I) are known from the literature or are commercially available.

The pyridines of formula (V) can be obtained by reacting the respective pyridines (II) with Bis(pinacolato)diboron VII.

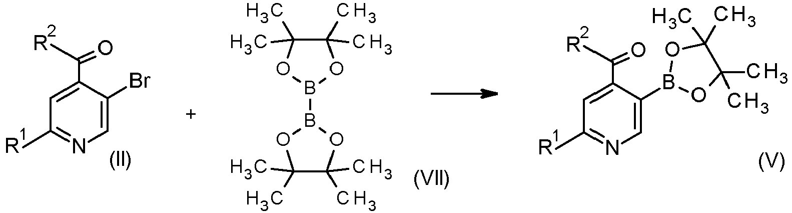

The reaction of the pyridine (II) with Bis(pinacolato)diboron VII is usually carried out from 0 °C to the boiling point of the reaction mixture, preferably from 15 °C to 110 °C, particularly preferably from 40 °C to 100 °C, in an inert organic solvent in the presence of a base and a catalyst.

The reaction may in principle be carried out in substance. However, preference is given to reacting the pyridines (II) with Bis(pinacolato)diboron VII in an organic solvent with or without water as co-solvent.

Suitable in principle are all solvents which are capable of dissolving the pyridines (II) and the Bis(pinacolato)diboron VII at least partly and preferably fully under the reaction conditions.

Examples of suitable solvents are aromatic hydrocarbons such as benzene, chlorobenzene, toluene, cresols, o-, m- and p-xylene, ethers such as diethyl ether, diisopropyl ether, tert.-butyl methylether (TBME), dioxane, anisole and tetrahydrofuran (THF), as well as dipolar aprotic solvents such as sulfolane, dimethylsulfoxide, N,N-dimethylformamide (DMF), N,N-dimethylacetamide (DMAC), 1,3-dimethyl-2-imidazolidinone (DMI), N,N'-dimethylpropylene urea (DMPU), dimethyl sulfoxide (DMSO) and 1-methyl-2 pyrrolidinone (NMP).

Preferred solvents are ethers such as diethyl ether, diisopropyl ether, tert.-butyl methylether (TBME), dioxane, anisole and tetrahydrofuran (THF) and dipolar aprotic solvents such as sulfolane, dimethylsulfoxide, N,N-dimethylformamide (DMF), N,N-dimethylacetamide (DMAC), 1,3-dimethyl-2-imidazolidinone (DMI), N,N'-dimethyl¬propylene urea (DMPU), dimethyl sulfoxide (DMSO) and 1-methyl-2 pyrroli-dinone (NMP).

More preferred solvents are ethers such as diethyl ether, diisopropyl ether, tert.-butyl methylether (TBME), dioxane, anisole and tetrahydrofuran (THF).

It is also possible to use mixtures of the solvents mentioned.

Examples of suitable metal-containing bases are inorganic compounds including metal-containing bases such as alkali metal and alkaline earth metal hydroxides, and other metal hydroxides, such as lithium hydroxide, sodium hydroxide, potassium hydroxide, magnesium hydroxide, calcium hydroxide and aluminum hydroxide; alkali metal and alkaline earth metal oxide, and other metal oxides, such as lithium oxide, sodium oxide, potassium oxide, magnesium oxide, calcium oxide and magnesium oxide, iron oxide, silver oxide; alkali metal and alkaline earth metal carbonates such as lithium carbonate, sodium carbonate, potassium carbonate, cesium carbonate magnesium carbonate, and calcium carbonate, as well as alkali metal hydrogen carbonates (bicarbonates) such as lithium hydrogen carbonate, sodium hydrogen carbonate, potassium hydrogen carbonate; alkali metal and alkaline earth metal phosphates such as potassium phosphate, calcium phosphate; alkali metal and alkaline earth metal acetates such as sodium acetate or potassium acetate.

Preferred bases are inorganic compounds such as alkali metal and alkaline earth metal hydroxides, and other metal hydroxides, such as lithium hydroxide, sodium hydroxide, potassium hydroxide, magnesium hydroxide, calcium hydroxide and aluminum hydroxide and alkali metal or alkaline earth metal carbonates such as lithium carbonate, sodium carbonate, potassium carbonate, cesium carbonate, magnesium carbonate, and calcium carbonate and alkaline earth metal phosphates such as potassium phosphate, and alkali metal acetates such as sodium acetate or potassium acetate.

Especially preferred bases are inorganic compounds such as alkali metal and alkaline earth metal hydroxides, and other metal hydroxides, such as lithium hydroxide, sodium hydroxide, potassium hydroxide, magnesium hydroxide, calcium hydroxide and aluminum hydroxide and alkaline earth metal phosphates such as potassium phosphate, and alkali metal acetates such as or potassium acetate.

The term base as used herein also includes mixtures of two or more, preferably two of the above compounds. Particular preference is given to the use of one base.

The bases are used preferably from 1 to 10 equivalents based on the pyridine (II), more preferably from 1.0 to 5.0 equivalents based on the pyridine (II), most preferably from 1.2 to 2.5 equivalents based on the pyridine (II).

It may be advantageous to add the base offset over a period of time.

The reaction of the pyridines (II) with Bis(pinacolato)diboron VI is carried out in the presence of a catalyst. Examples of suitable catalysts include for example, palladium based catalysts like, for example, Palladium(II)acetate, tetrakis(triphenylphosphine)palladium(0), bis(tri-phenylphosphine)palladium(II)chloride or (1,1,-bis(diphenylphosphino)ferrocene)-dichloropalladium(II), and optionally suitable additives such as, for example, phosphines like, for example, P(o-tolyl)₃, triphenylphosphine or BINAP (2,2'-Bis(diphenylphospino)-1,1'-binaphthyl).

The amount of catalyst is usually 0.01 to 20 mol % (0.0001 to 0.2 equivalents) based on the pyridine (II).

The end of the reaction can easily be determined by the skilled worker by means of routine methods.

The reaction mixtures are worked up in a customary manner, for example by mixing with water, separation of the phases and, if appropriate, chromatographic purification of the crude product.

Some of the intermediates and end products are obtained in the form of viscous oils, which can be purified or freed from volatile components under reduced pressure and at moderately elevated temperature.

If the intermediates and the end products are obtained as solid, purification can also be carried out by recrystallization or digestion.

### Process C:

The pyridine compounds of formula (I) can in addition be obtained by reacting non-halogenated pyridine compounds of formula (I) with halogenating agents NXS such as NCS, NBS or NIS.

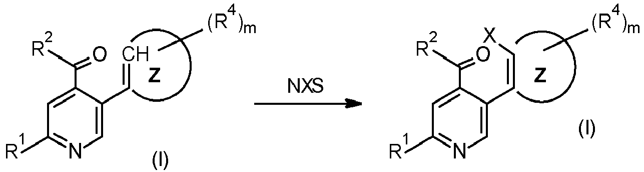

The halogenation is usually carried out from 0 °C to the boiling point of the reaction mixture, preferably from 15 °C to 110 °C, particularly preferably from 40 °C to 100 °C, in an inert organic solvent in the presence of a base and a catalyst.

Suitable in principle are all solvents which are capable of dissolving the pyridines (I) and the reactant NXS at least partly and preferably fully under the reaction conditions.

Examples of suitable solvents are ethers such as diethyl ether, diisopropyl ether, tert.-butyl methylether (TBME), dioxane, anisole and tetrahydrofuran (THF), chlorinated hydrocarbons such as dichloromethane, chloroform, carbon tetrachloride, and 1,2-dichloro ethane, as well as dipolar aprotic solvents such as sulfolane, dimethylsulfoxide, N,N-dimethylformamide (DMF), N,N-dimethylacetamide (DMAC), 1,3-dimethyl-2-imidazolidinone (DMI), N,N'-dimethylpropylene urea (DMPU), dimethyl sulfoxide (DMSO) and 1-methyl-2 pyrrolidinone (NMP).

Preferred solvents are dipolar aprotic solvents such as sulfolane, dimethylsulfoxide, N,N-dimethylformamide (DMF), N,N-dimethylacetamide (DMAC), 1,3-dimethyl-2-imidazolidinone (DMI), N,N'-dimethyl¬propylene urea (DMPU), dimethyl sulfoxide (DMSO) and 1-methyl-2 pyrroli-dinone (NMP).

More preferred solvents are dipolar aprotic solvents such as N,N-dimethylformamide (DMF) or N,N-dimethylacetamide (DMAC).

It is also possible to use mixtures of the solvents mentioned.

NXS is used preferably from 1 to 10 equivalents based on the pyridine (I), more preferably from 1.0 to 5.0 equivalents based on the pyridine (I), most preferably from 1.0 to 2.0 equivalents based on the pyridine (I).

It may be advantageous to add the agent offset over a period of time.

The end of the reaction can easily be determined by the skilled worker by means of routine methods.

The reaction mixtures are worked up in a customary manner, for example by mixing with water, separation of the phases and, if appropriate, chromatographic purification of the crude product.

Some of the intermediates and end products are obtained in the form of viscous oils, which can be purified or freed from volatile components under reduced pressure and at moderately elevated temperature.

If the intermediates and the end products are obtained as solid, purification can also be carried out by recrystallization or digestion.

The present invention also provides agrochemical compositions comprising at least one pyridine compounds of formula (I) and auxiliaries customary for formulating crop protection agents.

The present invention furthermore provides a method for controlling unwanted vegetation where a herbicidal effective amount of at least one pyridine compounds of formula (I) is allowed to act on plants, their seeds and/or their habitat. Application can be done before, during and/or after, preferably during and/or after, the emergence of the undesirable plants.

Further embodiments of the present invention are evident from the claims, the description and the examples. It is to be understood that the features mentioned above and still to be illustrated below of the subject matter of the invention can be applied not only in the combination given in each particular case but also in other combinations, without leaving the scope of the invention. As used herein, the terms "controlling" and "combating" are synonyms.

As used herein, the terms "undesirable vegetation" and "harmful plants" are synonyms.

If the pyridine compounds of formula (I) as described herein are capable of forming geometrical isomers, for example E/Z isomers, it is possible to use both, the pure isomers and mixtures thereof, in the compositions according to the invention.

If the pyridine compounds of formula (I) as described herein have one or more centres of chirality and, as a consequence, are present as enantiomers or diastereomers, it is possible to use both, the pure enantiomers and diastereomers and their mixtures, in the compositions according to the invention.

If the pyridine compounds of formula (I) as described herein have ionisable functional groups, preferably acidic groups, more preferably a carboxylic group or a sulphonic group, they can also be employed in the form of their agriculturally acceptable salts. Suitable are, in general, the salts of those cations and the acid addition salts of those acids whose cations and anions, respectively, have no adverse effect on the activity of the active compounds.

Preferred cations are the ions of the alkali metals, preferably of lithium, sodium and potassium, of the alkaline earth metals, preferably of calcium and magnesium, and of the transition metals, preferably of manganese, copper, zinc and iron, further ammonium and substituted ammonium in which one to four H atoms are replaced by C₁-C₄-alkyl, hydroxy-C₁-C₄-alkyl, C₁-C₄-alkoxy-C₁-C₄-alkyl, hydroxy-C₁-C₄-alkoxy-C₁-C₄-alkyl, phenyl or benzyl, preferably ammonium, methyl-ammonium, isopropylammonium, dimethylammonium, diisopropylammonium, trimethylammonium, heptylammonium, dodecylammonium, tetradecylammonium, tetramethylammonium, tetraethylammonium, tetrabutylammonium, 2-hydroxyethylammonium (olamine salt), 2-(2-hydroxyeth-1-oxy)eth-1-ylammonium (diglycolamine salt), di(2-hydroxyeth-1-yl)ammonium (diolamine salt), tris(2-hydroxy-ethyl)ammonium (trolamine salt), tris(2-hydroxypropyl)ammonium, benzyltrimethylammonium, benzyltriethylammonium, N,N,N-trimethylethanolammonium (choline salt), furthermore phosphonium ions, sulfonium ions, preferably tri(C₁-C₄-alkyl)sulfonium, such as trimethylsulfonium, and sulfoxonium ions, preferably tri(C₁-C₄-alkyl)sulfoxonium, and finally the salts of polybasic amines such as N,N-bis-(3-aminopropyl)methylamine and diethylenetriamine. Anions of useful acid addition salts are primarily chloride, bromide, fluoride, iodide, hydrogensulfate, methylsulfate, sulfate, dihydrogenphosphate, hydrogenphosphate, nitrate, bicarbonate, carbonate, hexafluorosilicate, hexafluorophosphate, benzoate and also the anions of C₁-C₄-alkanoic acids, preferably formate, acetate, propionate and butyrate.

Pyridine compounds of formula (I) as described herein having an acidic functionality, preferably a carboxylic group or a sulphonic group, can be employed, if applicable, in the form of the acid, in the form of an agriculturally suitable salt with the cations as defined above or else in the form of an agriculturally acceptable derivative, for example as amides, such as mono- and di-C₁-C₆-alkylamides or arylamides, as esters, for example as allyl esters, propargyl esters, C₁-C₁₀-alkyl esters, alkoxyalkyl esters, tefuryl ((tetrahydrofuran-2-yl)methyl) esters and also as thioesters, for example as C₁-C₁₀-alkylthio esters. Preferred mono- and di-C₁-C₆-alkylamides are the CH₃ and the dimethylamides. Preferred arylamides are, for example, the anilides and the 2-chloroanilides. Preferred alkyl esters are, for example, the methyl, ethyl, propyl, isopropyl, butyl, isobutyl, pentyl, mexyl (1-methylhexyl), meptyl (1-methylheptyl), heptyl, octyl or isooctyl (2-ethylhexyl) esters. Preferred C₁-C₄-alkoxy-C₁-C₄-alkyl esters are the straight-chain or branched C₁-C₄-alkoxy ethyl esters, for example the 2-methoxyethyl, 2-ethoxyethyl, 2-butoxyethyl (butotyl), 2-butoxypropyl or 3-butoxypropyl ester. An example of a straight-chain or branched C₁-C₁₀-alkylthio ester is the ethylthio ester.

The organic moieties mentioned in the definition of the variables R¹, R², A, Z, R³, R^{3A}, and R⁴, are - like the term halogen - collective terms for individual enumerations of the individual group members. The term halogen denotes in each case F, Cl, Br, or I. All hydrocarbon chains, for example all alkyl, alkenyl, alkynyl, alkoxy chains can be straight-chain or branched, the prefix Cₙ-Cₘ denoting in each case the possible number of carbon atoms in the group.

Examples of such meanings are:
- C₁-C₄-alkyl: for example CH₃, C₂H₅, n-propyl, CH(CH₃)₂, n-butyl, CH(CH₃)-C₂H₅, CH₂-CH(CH₃)₂, and C(CH₃)₃;
- C₁-C₆-alkyl: C₁-C₄-alkyl as mentioned above, and also, for example, n-pentyl, 1-methylbutyl, 2-methylbutyl, 3-methylbutyl, 2,2-dimethylpropyl, 1-ethylpropyl, n-hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl or 1-ethyl-2-methylpropyl, preferably methyl, ethyl, n-propyl, 1-methylethyl, n-butyl, 1,1-dimethylethyl, n-pentyl, or n-hexyl;
- C₁-C₄-haloalkyl: C₁-C₄-alkyl as mentioned above which is partially or fully substituted by fluorine, chlorine, bromine and/or iodine, for example, chloromethyl, dichloromethyl, trichloromethyl, fluoromethyl, difluoromethyl, trifluoromethyl, chlorofluoromethyl, dichlorofluoromethyl, chlorodifluoromethyl, bromomethyl, iodomethyl, 2-fluoroethyl, 2-chloroethyl, 2-bromoethyl, 2-iodoethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, pentafluoroethyl, 2-fluoropropyl, 3-fluoropropyl, 2,2-difluoropropyl, 2,3-difluoropropyl, 2-chloropropyl, 3-chloropropyl, 2,3-dichloropropyl, 2-bromopropyl, 3-bromopropyl, 3,3,3-trifluoropropyl, 3,3,3-trichloropropyl, 2,2,3,3,3-pentafluoropropyl, heptafluoro-propyl, 1-(fluoromethyl)-2-fluoroethyl, 1-(chloromethyl)-2-chloroethyl, 1-(bromomethyl)-2-bromo-ethyl, 4-fluorobutyl, 4-chlorobutyl, 4-bromobutyl, nonafluorobutyl, 1,1,2,2,-tetrafluoroethyl, and 1-trifluoromethyl-1,2,2,2-tetrafluoroethyl;
- C₁-C₆-haloalkyl: C₁-C₄-haloalkyl as mentioned above, and also, for example, 5-fluoropentyl, 5-chloropentyl, 5-bromopentyl, 5-iodopentyl, undecafluoropentyl, 6-fluorohexyl, 6-chlorohexyl, 6-bromohexyl, 6-iodohexyl, and dodecafluorohexyl;
- C₃-C₆-cycloalkyl: monocyclic saturated hydrocarbons having 3 to 6 ring members, such as cyclopropyl, cyclobutyl, cyclopentyl, and cyclohexyl;
- C₃-C₆-alkenyl: for example 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl, and 1-ethyl-2-methyl-2-propenyl;
- C₃-C₆-haloalkenyl: a C₃-C₆-alkenyl substituent as mentioned above which is partially or fully substituted by fluorine, chlorine, bromine and/or iodine, for example 2-chloroprop-2-en-1-yl, 3-chloroprop-2-en-1-yl, 2,3-dichloroprop-2-en-1-yl, 3,3-dichloroprop-2-en-1-yl, 2,3,3-trichloro-2-en-1-yl, 2,3-dichlorobut-2-en-1-yl, 2-bromoprop-2-en-1-yl, 3-bromoprop-2-en-1-yl, 2,3-dibromo-prop-2-en-1-yl, 3,3-dibromoprop-2-en-1-yl, 2,3,3-tribromo-2-en-1-yl, or 2,3-dibromobut-2-en-1-yl;
- C₃-C₆-alkynyl: for example 1-propynyl, 2-propynyl, 1-butynyl, 2-butynyl, 3-butynyl, 1-methyl-2-propynyl, 1-pentynyl, 2-pentynyl, 3-pentynyl, 4-pentynyl, 1-methyl-2-butynyl, 1-methyl-3-butynyl, 2-methyl-3-butynyl, 3-methyl-1-butynyl, 1,1-dimethyl-2-propynyl, 1-ethyl-2-propynyl, 1-hexynyl, 2-hexynyl, 3-hexynyl, 4-hexynyl, 5-hexynyl, 1-methyl-2-pentynyl, 1-methyl-3-pentynyl, 1-methyl-4-pentynyl, 2-methyl-3-pentynyl, 2-methyl-4-pentynyl, 3-methyl-1-pentynyl, 3-methyl-4-pentynyl, 4-methyl-1-pentynyl, 4-methyl-2-pentynyl, 1,1-dimethyl-2-butynyl, 1,1-dimethyl-3-butynyl, 1,2-dimethyl-3-butynyl, 2,2-dimethyl-3-butynyl, 3,3-dimethyl-1-butynyl, 1-ethyl-2-butynyl, 1-ethyl-3-butynyl, 2-ethyl-3-butynyl, and 1-ethyl-1-methyl-2-propynyl;
- C₂-C₆-alkynyl: C₃-C₆-alkynyl as mentioned above and also ethynyl;
- C₃-C₆-haloalkynyl: a C₃-C₆-alkynyl radical as mentioned above which is partially or fully substituted by F, Cl, Br and/or I, for example 1,1-difluoroprop-2-yn-1-yl, 3-chloroprop-2-yn-1-yl, 3-bromoprop-2-yn-1-yl, 3-iodoprop-2-yn-1-yl, 4-fluorobut-2-yn-1-yl, 4-chlorobut-2-yn-1-yl, 1,1-difluorobut-2-yn-1-yl, 4-iodobut-3-yn-1-yl, 5-fluoropent-3-yn-1-yl, 5-iodopent-4-yn-1-yl, 6-fluorohex-4-yn-1-yl, or 6-iodohex-5-yn-1-yl;
- C₁-C₄-alkoxy: for example methoxy, ethoxy, propoxy, 1-methylethoxy butoxy, 1-methylpropoxy, 2-methylpropoxy, and 1,1-dimethylethoxy;
- C₁-C₆-alkoxy: C₁-C₄-alkoxy as mentioned above, and also, for example, pentoxy, 1-methylbutoxy, 2-methylbutoxy, 3-methoxylbutoxy, 1,1-dimethylpropoxy, 1,2-dimethylpropoxy, 2,2-dimethylpropoxy, 1-ethylpropoxy, hexoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy, and 1-ethyl-2-methylpropoxy.
- C₁-C₄-haloalkoxy: a C₁-C₄-alkoxy radical as mentioned above which is partially or fully substituted by fluorine, chlorine, bromine and/or iodine, i.e., for example, fluoromethoxy, difluoromethoxy, trifluoromethoxy, chlorodifluoromethoxy, bromodifluoromethoxy, 2-fluoroethoxy, 2-chloroethoxy, 2-bromomethoxy, 2-iodoethoxy, 2,2-difluoroethoxy, 2,2,2-trifluoroethoxy, 2-chloro2-fluoroethoxy, 2-chloro-2,2-difluoroethoxy, 2,2-dichloro-2-fluoroethoxy, 2,2,2-trichloroethoxy, pentafluoroethoxy, 2-fluoropropoxy, 3-fluoropropoxy, 2-chloropropoxy, 3-chloropropoxy, 2-bromopropoxy, 3-bromopropoxy, 2,2-difluoropropoxy, 2,3-difluoropropoxy, 2,3-dichloropropoxy, 3,3,3-trifluoropropoxy, 3,3,3-trichloropropoxy, 2,2,3,3,3-pentafluoropropoxy, heptafluoropropoxy, 1-(fluoromethyl)-2-fluoroethoxy, 1-(chloromethyl)-2-chloroethoxy, 1-(bromomethyl)-2-bromoethoxy, 4-fluorobutoxy, 4-chlorobutoxy, 4-bromobutoxy, and nonafluorobutoxy;
- C₁-C₆-haloalkoxy: a C₁-C₄-haloalkoxy as mentioned above, and also, for example, 5-fluoropentoxy, 5-chloropentoxy, 5-bromopentoxy, 5-iodopentoxy, undecafluoropentoxy, 6-fluorohexoxy, 6-chlorohexoxy, 6-bromohexoxy, 6-iodohexoxy and dodecafluorohexoxy;
- C₁-C₄-alkylthio: for example methylthio, ethylthio, propylthio, 1-methylethylthio, butylthio, 1-methylpropylthio, 2-methylpropylthio, and 1,1-dimethylethylthio;
- C₁-C₆-alkylthio: C₁-C₄-alkylthio as mentioned above, and also, for example, pentylthio, 1-methylbutylthio, 2-methylbutylthio, 3-methylbutylthio, 2,2-dimethylpropylthio, 1-ethylpropylthio, hexylthio, 1,1-dimethylpropylthio, 1,2-dimethylpropylthio, 1-methylpentylthio, 2-methylpentylthio, 3-methylpentylthio, 4-methylpentylthio, 1,1-dimethylbutylthio, 1,2-dimethylbutylthio, 1,3-dimethylbutylthio, 2,2-dimethylbutylthio, 2,3-dimethylbutylthio, 3,3-dimethylbutylthio, 1-ethylbutylthio, 2-ethylbutylthio, 1,1,2-trimethylpropylthio, 1,2,2-trimethylpropylthio, 1-ethyl-1-methylpropylthio, and 1- ethyl-2-methylpropylthio;
- (C₁-C₄-alkyl)amino: for example methylamino, ethylamino, propylamino, 1-methylethylamino, butylamino, 1-methylpropylamino, 2-methylpropylamino, or 1,1-dimethylethylamino;
- (C₁-C₆-alkyl)amino: (C₁-C₄-alkylamino) as mentioned above, and also, for example, pentyl-amino, 1-methylbutylamino, 2-methylbutylamino, 3-methylbutylamino, 2,2-dimethylpropylamino, 1-ethylpropylamino, hexylamino, 1,1-dimethylpropylamino, 1,2-dimethylpropylamino, 1-methylpentylamino, 2-methylpentylamino, 3-methylpentylamino, 4-methylpentylamino, 1,1-dimethylbutylamino, 1,2-dimethylbutylamino, 1,3-dimethylbutylamino, 2,2-dimethylbutylamino, 2,3-dimethylbutyl-amino 3,3-dimethylbutylamino, 1-ethylbutylamino, 2-ethylbutylamino, 1,1,2-trimethylpropylamino, 1,2,2-trimethyl-propylamino, 1-ethyl-1-methylpropylamino, or 1-ethyl-2-methylpropylamino;
- di(C₁-C₄-alkyl)amino: for example N,N-dimethylamino, N,N-diethylamino, N,N-di(1-methyl-ethyl)amino, N,N-dipropylamino, N,N-dibutylamino, N,N-di(1-methylpropyl)amino, N,N-di(2-methyl-propyl)amino, N,N-di(1,1-dimethylethyl)amino, N-ethyl-N-methylamino, N-methyl-N-propyl-amino, N-methyl-N-(1-methylethyl)amino, N-butyl-N-methylamino, N-methyl-N-(1-methylpropyl)amino, N-methyl-N-(2-methylpropyl)amino, N-(1,1-dimethylethyl)-N-methylamino, N-ethyl-N-propylamino, N-ethyl-N-(1-methylethyl)amino, N-butyl-N-ethylamino, N-ethyl-N-(1-methylpropyl)-amino, N-ethyl-N-(2-methylpropyl)amino, N-ethyl-N-(1,1-dimethylethyl)amino, N-(1-methylethyl)-N-propylamino, N-butyl-N-propylamino, N-(1-methylpropyl)-N-propylamino, N-(2-methylpropyl)-N-propylamino, N-(1,1-dimethylethyl)-N-propylamino, N-butyl-N-(1-methylethyl)amino, N-(1-methyl-ethyl)-N-(1-methyl-propyl)amino, N-(1-methylethyl)-N-(2-methylpropyl)amino, N-(1,1-dimethylethyl)-N-(1-methylethyl)amino, N-butyl-N-(1-methylpropyl)amino, N-butyl-N-(2-methylpropyl)amino, N-butyl-N-(1,1-dimethylethyl)amino, N-(1-methylpropyl)-N-(2-methylpropyl)amino, N-(1,1-dimethyl-ethyl)-N-(1-methylpropyl)amino, or N-(1,1-dimethylethyl)-N-(2-methylpropyl)amino;
- di(C₁-C₆-alkyl)amino: di(C₁-C₄-alkyl)amino as mentioned above, and also, for example, N-methyl-N-pentylamino, N-methyl-N-(1-methylbutyl)amino, N-methyl-N-(2-methylbutyl)amino, N-methyl-N-(3-methylbutyl)amino, N-methyl-N-(2,2-dimethylpropyl)amino, N-methyl-N-(1-ethylpropyl)amino, N-methyl-N-hexylamino, N-methyl-N-(1,1-dimethylpropyl)amino, N-methyl-N-(1,2-dimethylpropyl)amino, N-methyl-N-(1-methylpentyl)amino, N-methyl-N-(2-methylpentyl)amino, N-methyl-N-(3-methylpentyl)amino, N-methyl-N-(4-methylpentyl)amino, N-methyl-N-(1,1-dimethylbutyl)amino, N-methyl-N-(1,2-dimethylbutyl)amino, N-methyl-N-(1,3-dimethylbutyl)amino, N-methyl-N-(2,2-dimethylbutyl)amino, N-methyl-N-(2,3-dimethylbutyl)amino, N-methyl-N-(3,3-dimethylbutyl)amino, N-methyl-N- (1-ethylbutyl)amino, N-methyl-N-(2-ethylbutyl)amino, N-methyl-N-(1,1,2-trimethylpropyl)amino, N-methyl-N- (1,2,2-trimethylpropyl)amino, N-methyl-N-(1-ethyl-1-methylpropyl)amino, N-methyl-N- (1-ethyl-2-methylpropyl)amino, N-ethyl-N-pentylamino, N-ethyl-N-(1-methylbutyl)amino, N-ethyl-N-(2-methylbutyl)amino, N-ethyl-N-(3-methylbutyl)amino, N-ethyl-N-(2,2-dimethylpropyl)amino, N-ethyl-N-(1-ethylpropyl)amino, N-ethyl-N-hexylamino, N-ethyl-N-(1,1-dimethylpropyl)amino, N-ethyl-N-(1,2-dimethylpropyl)amino, N-ethyl-N-(1-methylpentyl)amino, N-ethyl-N-(2-methylpentyl)amino, N-ethyl-N-(3-methylpentyl)amino, N-ethyl-N-(4-methylpentyl)amino, N-ethyl-N-(1,1-dimethylbutyl)amino, N-ethyl-N-(1,2-dimethylbutyl)amino, N-ethyl-N-(1,3-dimethylbutyl)amino, N-ethyl-N-(2,2-dimethylbutyl)amino, N-ethyl-N-(2,3-dimethylbutyl)amino, N-ethyl-N-(3,3-dimethylbutyl)amino, N-ethyl-N-(1-ethylbutyl)amino, N-ethyl-N-(2-ethylbutyl)amino, N-ethyl-N-(1,1,2-trimethylpropyl)amino, N-ethyl-N-(1,2,2-trimethylpropyl)amino, N-ethyl-N-(1-ethyl-1-methylpropyl)amino, N-ethyl-N-(1-ethyl-2-methylpropyl)amino, N-propyl-N-pentylamino, N-butyl-N-pentylamino, N,N-dipentylamino, N-propyl-N-hexylamino, N-butyl-N-hexylamino, N-pentyl-N-hexylamino, or N,N-dihexylamino;
- C₁-C₆-alkylsulfinyl (C₁-C₆-Alkyl-S(=O)-): for example methylsulfinyl, ethylsulfinyl, propylsulfinyl, 1-methylethylsulfinyl, butylsulfinyl, 1-methylpropylsulfinyl, 2-methylpropylsulfinyl, 1,1-dimethylethylsulfinyl, pentylsulfinyl, 1-methylbutylsulfinyl, 2-methylbutylsulfinyl, 3-methylbutylsulfinyl, 2,2-dimethylpropylsulfinyl, 1-ethylpropylsulfinyl, 1,1-dimethylpropylsulfinyl, 1,2-dimethylpropyl-sulfinyl, hexylsulfinyl, 1-methylpentylsulfinyl, 2-methylpentylsulfinyl, 3-methylpentylsulfinyl, 4-methylpentyl-sulfinyl, 1,1-dimethylbutylsulfinyl, 1,2-dimethylbutylsulfinyl, 1,3-dimethylbutyl-sulfinyl, 2,2-dimethylbutylsulfinyl, 2,3-dimethylbutylsulfinyl, 3,3-dimethylbutyl-sulfinyl, 1-ethylbutylsulfinyl, 2-ethylbutylsulfinyl, 1,1,2-trimethylpropylsulfinyl, 1,2,2-trimethylpropylsulfinyl, 1-ethyl-1-methylpropyl-sulfinyl, and 1-ethyl-2-methylpropylsulfinyl;
- C₁-C₆-alkylsulfonyl (C₁-C₆-alkyl-S(O)₂-): for example methylsulfonyl, ethylsulfonyl, propylsulfonyl, 1-methylethylsulfonyl, butylsulfonyl, 1-methylpropylsulfonyl, 2-methyl-propylsulfonyl, 1,1-dimethylethylsulfonyl, pentylsulfonyl, 1-methylbutylsulfonyl, 2-methylbutylsulfonyl, 3-methylbutylsulfonyl, 1,1-dimethylpropylsulfonyl, 1,2-dimethylpropylsulfonyl, 2,2-dimethylpropyl-sulfonyl, 1-ethylpropylsulfonyl, hexylsulfonyl, 1-methylpentylsulfonyl, 2-methylpentylsulfonyl, 3-methylpentylsulfonyl, 4-methylpentylsulfonyl, 1,1-dimethylbutylsulfonyl, 1,2-dimethylbutylsulfonyl, 1,3-dimethylbutylsulfonyl, 2,2-dimethylbutylsulfonyl, 2,3-dimethylbutylsulfonyl, 3,3-dimethylbutyl-sulfonyl, 1-ethylbutylsulfonyl, 2-ethylbutylsulfonyl, 1,1,2-trimethyl-propylsulfonyl, 1,2,2-trimethyl-propylsulfonyl, 1-ethyl-1-methylpropylsulfonyl, and 1-ethyl-2-methylpropylsulfonyl;
- C₃-C₆-cycloalkyl: a monocyclic saturated hydrocarbon having 3 to 6 ring members, such as cyclopropyl, cyclobutyl, cyclopentyl, and cyclohexyl;
- C₃-C₆-cycloalkenyl: 1-cyclopropenyl, 2-cyclopropenyl, 1-cyclobutenyl, 2-cyclobutenyl, 1-cyclopentenyl, 2-cyclopentenyl, 1,3-cyclopentadienyl, 1,4-cyclopentadienyl, 2,4-cyclopentadienyl, 1-cyclohexenyl, 2-cyclohexenyl, 3-cyclohexenyl, 1,3-cyclohexadienyl, 1,4-cyclohexadienyl, or 2,5-cyclohexadienyl;
- bicyclic ring: a 9- to 10-membered bicyclic ring: a partially or fully unsaturated 9- to 10-membered carbocyclic system wherein two partially or fully unsaturated carbocyclic rings are fused with each other through 2 ring members, and which in addition to carbon atoms and independent of their position in the ring can comprise as ring members 1 to 4 nitrogen atoms, or 1 or 2 oxygen atoms, or 1 or 2 oxygen atoms and 1 to 2 nitrogen atoms, or 1 or 3 sulfur atoms, or 1 to 4 nitrogen atoms and an oxygen atom, or one to three nitrogen atoms and a sulfur atom, or one sulfur and one oxygen atom, examples of such bicyclic ring are, 2,3-dihydrobenzothiophene, benzothiophene, 2,3-dihydrobenzofuran, benzofuran, 1,3-benzodioxole, 1,3-benzodithiole, 1,3-benzoxathiole, indole, indane, [1,3]dioxolo[4,5-c]pyridine, [1,3]dioxolo[4,5-b]pyridine, 2,3-dihydrofuro[2,3-c]pyridine, furo[2,3-c]pyridine, 2,3-dihydrofuro[2,3-b]pyridine, furo[2,3-b]pyridine, 2,3-dihydrofuro[3,2-c]pyridine, furo[3,2-c]pyridine, 2,3-dihydrofuro[3,2-b]pyridine, furo[3,2-b]pyridine, furo[3,2-d]pyrimidine, furo[2,3-d]pyrimidine, 6,7-dihydrofuro[3,2-d]pyrimidine, 5,6-dihydrofuro[2,3-d]pyrimidine, thieno[3,2-d]pyrimidine, thieno[2,3-d]pyrimidine, 6,7-dihydrothieno[3,2-d]pyrimidine, 5,6-dihydrothieno[2,3-d]pyrimidine, 2,3-dihydrothieno[2,3-c]pyridine, thieno[2,3-c]pyridine, 2,3-dihydrothieno[2,3-b]pyridine, thieno[2,3-b]pyridine, 2,3-dihydrothieno[3,2-c]pyridine, thieno[3,2-c]pyridine, 2,3-dihydrothieno[3,2-b]pyridine, thieno[3,2-b]pyridine, 1 H-pyrrolo[2,3-c]pyridine, 1 H-pyrrolo[2,3-b]pyridine, 1 H-pyrrolo[3,2-c]pyridine, 1H-pyrrolo[3,2-b]pyridine, 1H-imidazo[4,5-c]pyridine, 1H-imidazo[4,5-b]pyridine, 3H-imidazo[4,5-c]pyridine, 3H-imidazo[4,5-b]pyridine, 1 H-pyrazolo[3,4-b]pyridine, 1 H-pyrazolo[3,4-c]pyridine, 1H-pyrazolo[4,3-b]pyridine, 1 H-pyrazolo[4,3-c]pyridine, 1 H-indazole, benzimidazole, 1,2-benzoxazole, 1,3-benzoxazole, 1,3-benzothiazole, 1,2-benzothiazole, naphthalene, quinolone, isoquinoline, quinazoline, 1,3-benzoxathiole, [1,3]oxathiolo[4,5-b]pyridine, [1,3]oxathiolo[4,5-c]pyridine, [1,3]oxathiolo[5,4-c]pyridine, [1,3]oxathiolo[5,4-b]pyridine, 2,3-dihydro-1,4-benzodioxine, 2,3-dihydro-[1,4]dioxino[2,3-b]pyridine, 2,3-dihydro-[1,4]dioxino[2,3-c]pyridine;
- heterocyclyl: a 3- to 6-membered heterocyclyl: a saturated or partial unsaturated cycle having three to six ring members which comprises apart from carbon atoms one to four nitrogen atoms, or one or two oxygen atoms, or one or two sulfur atoms, or one to three nitrogen atoms and an oxygen atom, or one to three nitrogen atoms and a sulfur atom, or one sulfur and one oxygen atom, for example 3- or 4-membered heterocycles like 2-oxiranyl, 2-aziridinyl, 2-thiiranyl, 2-oxetanyl, 3-oxetanyl, 2-thietanyl, 3-thietanyl, 1-azetidinyl, 2-azetidinyl, 1-azetinyl, or 2-azetinyl;
   5-membered saturated heterocycles like2-tetrahydrofuranyl, 3-tetrahydrofuranyl, 2-tetrahydrothienyl, 3-tetrahydrothienyl, 1-pyrrolidinyl,2-pyrrolidinyl, 3-pyrrolidinyl, 3-isoxazolidinyl, 4-isoxazolidinyl, 5-isoxazolidinyl, 2-isothiazolidinyl, 3-isothiazolidinyl, 4-isothiazolidinyl, 5-isothiazolidinyl, 1-pyrazolidinyl, 3-pyrazolidinyl, 4-pyrazolidinyl, 5-pyrazolidinyl, 2-oxazolidinyl, 4-oxazolidinyl, 5-oxazolidinyl, 2-thiazolidinyl, 4-thiazolidinyl, 5-thiazolidinyl, 1-imidazolidinyl, 2-imidazolidinyl, 4-imidazolidinyl, 3-oxazolidinyl, 1,2,4-oxadiazolidin-3-yl, 1,2,4-oxadiazolidin-5-yl, 3-thiazolidinyl, 1,2,4-thiadiazolidin-3-yl, 1,2,4-thiadiazolidin-5-yl, 1,2,4-triazolidin-3-yl, 1,2,4-oxadiazolidin-2-yl, 1,2,4-oxadiazolidin-4-yl, 1,3,4-oxadiazolidin-2-yl, 1,2,4-thiadiazolidin-2-yl, 1,2,4-thiadiazolidin-4-yl, 1,3,4-thiadiazolidin-2-yl, 1,2,4-triazolidin-1-yl, or 1,3,4-triazolidin-2-yl;
   5-membered partial unsaturated heterocycles like 2,3-dihydrofur-2-yl, 2,3-dihydrofur-3-yl, 2,4-dihydrofur-2-yl, 2,4-dihydrofur-3-yl, dioxolan-2-yl, 1,3-dioxol-2-yl, 2,3-dihydrothien-2-yl, 2,3-dihydrothien-3-yl, 2,4-dihydrothien-2-yl, 2,4-dihydrothien-3-yl, 4,5-dihydropyrrol-1-yl, 4,5-dihydropyrrol-2-yl, 4,5-dihydropyrrol-3-yl, 2,5-dihydropyrrol-1-yl, 2,5-dihydropyrrol-2-yl, 2,5-dihydropyrrol-3-yl, 2,3-dihydroisoxazol-1-yl, 2,3-dihydroisoxazol-3-yl, 2,3-dihydroisoxazol-4-yl, 2,3-dihydroisoxazol-5-yl, 2,5-dihydroisoxazol-3-yl, 2,5-dihydroisoxazol-4-yl, 2,5-dihydroisoxazol-5-yl, 4,5-dihydroisoxazol-2-yl, 4,5-dihydroisoxazol-3-yl, 4,5-dihydroisoxazol-4-yl, 4,5-dihydroisoxazol-5-yl, 2,3-dihydroisothiazol-1-yl, 2,3-dihydroisothiazol-3-yl, 2,3-dihydroisothiazol-4-yl, 2,3-dihydroisothiazol-5-yl, 2,5-dihydroisothiazol-3-yl, 2,5-dihydroisothiazol-4-yl, 2,5-dihydroisothiazol-5-yl, 4,5-dihydroisothiazol-1-yl, 4,5-dihydroisothiazol-3-yl, 4,5-dihydroisothiazol-4-yl, 4,5-dihydroisothiazol-5-yl, 2,3-dihydropyrazol-1-yl, 2,3-dihydropyrazol-2-yl, 2,3-dihydropyrazol-3-yl, 2,3-dihydropyrazol-4-yl, 2,3-dihydropyrazol-5-yl, 3,4-dihydropyrazol-1-yl, 3,4-dihydropyrazol-3-yl, 3,4-dihydropyrazol-4-yl, 3,4-dihydropyrazol-5-yl, 4,5-dihydropyrazol-1-yl, 4,5-dihydropyrazol-3-yl, 4,5-dihydropyrazol-4-yl, 4,5-dihydropyrazol-5-yl, 2,3-dihydroimidazol-1-yl, 2,3-dihydroimidazol-2-yl, 2,3-dihydroimidazol-3-yl, 2,3-dihydroimidazol-4-yl, 2,3-dihydroimidazol-5-yl, 4,5-dihydroimidazol-1-yl, 4,5-dihydroimidazol-2-yl, 4,5-dihydroimidazol-4-yl, 4,5-dihydroimidazol-5-yl, 2,5-dihydroimidazol-1-yl, 2,5-dihydroimidazol-2-yl, 2,5-dihydroimidazol-4-yl, 2,5-dihydroimidazol-5-yl, 2,3-dihydrooxazol-2-yl, 2,3-dihydrooxazol-3-yl, 2,3-dihydrooxazol-4-yl, 2,3-dihydrooxazol-5-yl, 3,4-dihydrooxazol-2-yl, 3,4-dihydrooxazol-3-yl, 3,4-dihydrooxazol-4-yl, 3,4-dihydrooxazol-5-yl, 2,3-dihydrothiazol-2-yl, 2,3-dihydrothiazol-3-yl, 2,3-dihydrothiazol-4-yl, 2,3-dihydrothiazol-5-yl, 3,4-dihydrothiazol-2-yl, 3,4-dihydrothiazol-3-yl, 3,4-dihydrothiazol-4-yl, 3,4-dihydrothiazol-5-yl, 3,4-dihydrothiazol-2-yl, 3,4-dihydrothiazol-3-yl, or 3,4-dihydrothiazol-4-yl;
   6-membered saturated heterocycles like 1-piperidinyl, 2-piperidinyl, 3-piperidinyl, 4-piperidinyl, 1,3-dioxan-5-yl, 1,4-dioxanyl, 1,3-dithian-5-yl, 1,3-dithianyl, 1,3-oxathian-5-yl, 1,4-oxathianyl, 2-tetrahydropyranyl, 3-tetrahydopyranyl, 4-tetrahydropyranyl, 2-tetrahydrothiopyranyl, 3-tetrahydrothiopyranyl,4-tetrahydrothiopyranyl, 1-hexahydropyridazinyl, 3-hexahydropyridazinyl, 4-hexahydropyridazinyl, 1-hexahydropyrimidinyl, 2-hexahydropyrimidinyl, 4-hexahydropyrimidinyl, 5-hexahydropyrimidinyl, 1-piperazinyl, 2-piperazinyl, 1,3,5-hexahydrotriazin-1-yl, 1,3,5-hexahydrotriazin-2-yl, 1,2,4-hexahydrotriazin-1-yl, 1,2,4-hexahydrotriazin-3-yl, tetrahydro-1,3-oxazin-1-yl, tetrahydro-1,3-oxazin-2-yl, tetrahydro-1,3-oxazin-6-yl, 1-morpholinyl, or 2-morpholinyl, 3-morpholinyl;
   6-membered partial unsaturated heterocycles like 2H-pyran-2-yl, 2H-pyran-3-yl, 2H-pyran-4-yl, 2H-pyran-5-yl, 2H-pyran-6-yl, 2H-thiopyran-2-yl, 2H-thiopyran-3-yl, 2H-thiopyran-4-yl, 2H-thiopyran-5-yl, 2H-thiopyran-6-yl, or 5,6-dihydro-4H-1,3-oxazin-2-yl.
- heteroaryl: a 5- or 6-membered heteroaryl: monocyclic aromatic heteroaryl having 5 to 6 ring members which, in addition to carbon atoms and independent of their position in the ring, contains 1 to 4 nitrogen atoms, or 1 to 3 nitrogen atoms and an oxygen or sulfur atom, or an oxygen or a sulfur atom, for example 5-membered aromatic rings like furyl (for example 2-furyl, 3-furyl), thienyl (for example 2-thienyl, 3-thienyl), pyrrolyl (for example pyrrol-2-yl, pyrrol-3-yl), pyrazolyl (for example pyrazol-3-yl, pyrazol-4-yl), isoxazolyl (for example isoxazol-3-yl, isoxazol-4-yl, isoxazol-5-yl), isothiazolyl (for example isothiazol-3-yl, isothiazol-4-yl, isothiazol-5-yl), imidazolyl (for example imidazole-2-yl, imidazole-4-yl), oxazolyl (for example oxazol-2-yl, oxazol-4-yl, oxazol-5-yl), thiazolyl (for example thiazol-2-yl, thiazol-4-yl, thiazol-5-yl), oxadiazolyl (for example 1,2,3-oxadiazol-4-yl, 1,2,3-oxadiazol-5-yl, 1,2,4-oxadiazol-3-yl, 1,2,4-oxadiazol-5-yl, 1,3,4-oxadiazol-2-yl), thiadiazolyl (for example 1,2,3-thiadiazol-4-yl, 1,2,3-thiadiazol-5-yl, 1,2,4-thiadiazol-3-yl, 1,2,4-thiadiazol-5-yl, 1,3,4-thiadiazolyl-2-yl), triazolyl (for example 1,2,3-triazol-4-yl, 1,2,4-triazol-3-yl); 1-tetrazolyl; 6-membered aromatic rings like pyridyl (for example pyridine-2-yl, pyridine-3-yl, pyridine-4-yl), pyrazinyl (for example pyridazin-3-yl, pyridazin-4-yl), pyrimidinyl (for example pyrimidin-2-yl, pyrimidin-4-yl, pyrimidin-5-yl), pyrazin-2-yl, triazinyl (for example 1,3,5-triazin-2-yl, or 1,2,4-triazin-3-yl, 1,2,4-triazin-5-yl, 1,2,4-triazin-6-yl);

The term "substituted" if not specified otherwise refers to substituted by 1, 2 or maximum possible number of substituents. If substituents as defined in compounds of formula I are more than one then they are independently from each other are same or different if not mentioned otherwise.

The substitution R⁴ if present, can be present in any ring of Z.

The term "acidic functionality" if not specified otherwise refers to a functionality capable of donating a hydrogen (proton or hydrogen ion H⁺), such as a carboxylic group or a sulphonic group, or, alternatively, capable of forming a covalent bond with an electron pair.

The term "cyclic groups" comprises aliphatic cyclic groups such as cycloalkyl, cycloalkenyl and heterocyclyl and aromatic cyclic groups such as heteroaryl and phenyl.

The terms "compounds of formula (I)", "Pyridine compounds of formula (I)", "Compounds I" and "compounds of invention" are synonyms.

The preferred embodiments of the invention mentioned herein below have to be understood as being preferred either independently from each other or in combination with one another.

In general, pyridine compounds of formula (I) are suitable as herbicides.

According to a preferred embodiment of the invention preference is given pyridine compounds of formula (I), and their use as herbicides, wherein the variables, either independently of one another or in combination with one another, have the following meanings:
Preferred R¹ is C₁-C₆-alkyl, C₁-C₆-alkoxy, C₁-C₆-haloalkoxy, C₃-C₆-alkenyloxy, C₃-C₆-haloalkenyloxy C₃-C₆-alkynyloxy, C₃-C₆-haloalkynyloxy, C₁-C₆-alkylthio, C₃-C₆-cycloalkyl, wherein the cycloalkyl substituent is unsubstituted;
particularly preferred R¹ is C₁-C₆-alkyl, C₁-C₆-alkoxy, or C₃-C₆-cycloalkyl, wherein the cycloalkyl substituent is unsubstituted;
especially preferred R¹ is C₃-C₆-cycloalkyl, wherein the cycloalkyl substituent is unsubstituted;
also especially preferred R¹ is C₂H₅, i-C₃H₇, i-C₄H₉, OCH₃, c-C₃H₅, or c-C₄H₉;
more preferred R¹ is C₂H₅, OCH₃, or c-C₃H₅;
most preferred R¹ is c-C₃H₅.

Preferred R² is OH, C₁-C₆-alkoxy, C₁-C₆-haloalkoxy, C₃-C₆-alkenyloxy, C₃-C₆-haloalkenyloxy, C₃-C₆-alkynyloxy, C₄-C₆-haloalkynyloxy, (C₁-C₆-alkoxy)carbonyl-C₁-C₆-alkoxy, C₁-C₆-alkylthio, (C₁-C₆-alkoxy)carbonyl-C₁-C₆-alkylthio, NH₂, C₁-C₆-alkylcarbonylamino, C₁-C₆-haloalkylcarbonylamino, C₃-C₆-cycloalkylcarbonylamino, phenylcarbonylamino, heterocyclylcarbonylamino, heteroarylcarbonylamino, (C₁-C₆-alkyl)amino, (C₁-C₆-alkoxy)amino, (C₁-C₆-alkoxy)(C₁-C₆-alkyl)amino, hydroxy(C₁-C₆-alkyl)amino, hydroxyamino, (C₁-C₆-alkyl)sulfonylamino, [di(C₁-C₆-alkyl)amino]sulfonylamino, phenyloxy, phenyl-C₁-C₆-alkoxy, or phenyl-C₁-C₆-alkylthio, wherein the phenyl substituent is unsubstituted or substituted by R^{a};
particularly preferred R² is OH, C₁-C₆-alkoxy, C₁-C₆-haloalkoxy, C₃-C₆-alkynyloxy, C₁-C₆-alkylthio, phenyloxy, C₁-C₆-alkylcarbonylamino, C₁-C₆-haloalkylcarbonylamino, phenylcarbonylamino, heterocyclylcarbonylamino, heteroarylcarbonylamino, (C₁-C₆-alkoxy)amino, (C₁-C₆-alkoxy)(C₁-C₆-alkyl)amino, hydroxy(C₁-C₆-alkyl)amino, hydroxyamino, or phenyl-C₁-C₆-alkoxy, wherein the phenyl substituent is unsubstituted or substituted by R^{a};
also particularly preferred R² is OH, C₁-C₆-alkoxy, C₃-C₆-alkynyloxy, or C₁-C₆-haloalkoxy;
especially preferred C₁-C₆-alkoxy, C₃-C₆-alkynyloxy, or C₁-C₆-haloalkoxy;
also especially preferred R² is OH, C₁-C₆-alkoxy, or C₁-C₆-haloalkoxy;
more preferred R² is OH, C₃-C₆-alkynyloxy, or C₁-C₆-alkoxy,
most preferred R² is OH,
also most preferred R² is C₃-C₆-alkynyloxy,
also most preferred R² is C₁-C₆-alkoxy.
Preferred A is CR³, C*, NR^{3A}, N, S or O;
particularly preferred A is CR³, C*, NR^{3A}, or N;
also particularly preferred A is S or O;
especially preferred A is C*, CR³ or N;
most preferred A is CR³;
also most preferred A is C*;
also most preferred A is N.
Preferred Z is 9 or 10membered bicyclic ring;
particularly preferred Z is 9 membered bicyclic ring;
particularly preferred Z is 10 membered bicyclic ring;
more preferred Z is selected from below radicals A to O, 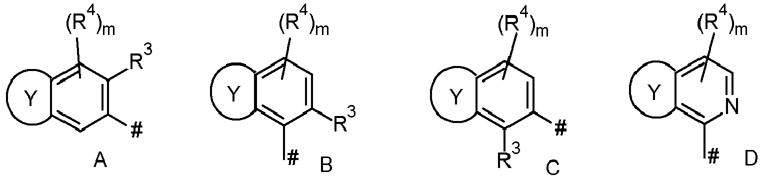 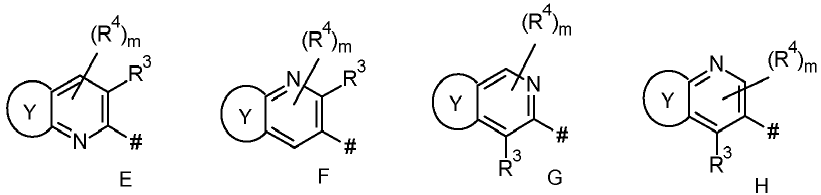 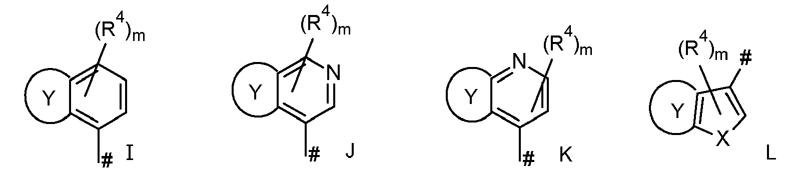 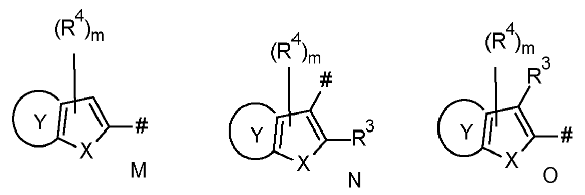 wherein
Y 5- or 6-membered partially or fully unsaturated carbocycle comprising 0, 1, 2, or 3 heteroatoms selected from O, N, and S;
- R³: is halogen, CN, C₁-C₆-alkyl, C₁-C₆-haloalkyl, or C₁-C₆-alkoxy;
- m: is 0, 1 or 2;
- R⁴: is halogen, CN, C₁-C₆-alkyl, C₁-C₆-haloalkyl, or C₁-C₆-alkoxy;
- X: is O, S, or NR^{3A};
- R^{3A}: is H, C₁-C₆-alkyl, C₁-C₆-haloalkyl, C₁-C₆-alkylcarbonyl, C₃-C₆-alkenyl, C₃-C₆-haloalkenyl, C₃-C₆-alkenyl, C₃-C₆-haloalkenyl, or C₃-C₆-cycloalkyl; and
# denotes the point of attachment to the pyridine ring.

Preferred Y is phenyl;
Also preferred Y is 5- or 6-membered partially or fully unsaturated carbocycle comprising 1, 2, or 3 heteroatoms selected from O, N, and S;
particularly preferred Y is phenyl;
also particularly preferred Y is 5-membered partially or fully unsaturated carbocycle comprising 1, or 2 heteroatoms selected from O, N, and S;
more preferred Y is 5-membered partially unsaturated carbocycle comprising 0, 1, or 2 heteroatoms selected from O, N, and S, for example 1,3-dithiolane, 1,3-oxathiolane, 1,3-dioxolane, 2,3-dihydrofuran, 2,3-dihydrothiophene, or 2,3-dihydro-1H-pyrrole ; more preferably heteroatoms are selected from O and S, for example 1,3-oxathiolane, 1,3-dioxolane, 2,3-dihydrofuran, or 2,3-dihydrothiophene; most preferred heteroatom in Y is O, for example 1,3-dioxolane, or 2,3-dihydrofuran; also most preferred heteroatom in Y is S, for example 2,3-dihydrothiophene;
also more preferred Y is 5-membered fully unsaturated carbocycle comprising 0, 1, or 2 heteroatoms selected from O, N, and S (furan, thiophene, 1H-pyrrole, 1,2-oxazole, 1,3-oxazole, 1,2-thiazole, 1,3-thiazole, imidazole, 1H-pyrazole); more preferably heteroatoms are selected from O and S; most preferred heteroatom is O (furan); also most preferred heteroatom is S (thiophene);
also particularly preferred Y is 6-membered partially or fully unsaturated carbocycle comprising 0, 1, or 2 heteroatoms selected from O, N, and S;
more preferred Y is 6-membered partially unsaturated carbocycle comprising 0, 1, or 2 heteroatoms selected from O, N, and S; more preferably heteroatoms are selected from O and S; also more preferably heteroatoms are selected from O and N; most preferred heteroatom is O;
also more preferred Y is 6-membered fully unsaturated carbocycle comprising 0, 1, or 2 heteroatoms N;
Preferred R³ is halogen, CN, NO₂, C₁-C₆-alkyl, C₁-C₆-haloalkyl, C₁-C₆-alkoxy, or C₃-C₆-cycloalkyl;
also preferred R³ is halogen, CN, C₁-C₆-alkyl, C₁-C₆-haloalkyl, or C₁-C₆-alkoxy;
particularly preferred R³ is halogen, CN, C₁-C₆-alkyl, or C₁-C₆-alkoxy;
especially preferred halogen, or CH₃;
also especially preferred R³ is halogen;
more preferred R³ is Cl, Br, or I;
most preferred R³ is Cl or Br.

Preferred R^{3A} is H, C₁-C₆-alkyl, C₁-C₆-haloalkyl, C₁-C₆-alkylcarbonyl, C₃-C₆-alkenyl, C₃-C₆-haloalkenyl, C₃-C₆-alkenyl, C₃-C₆-haloalkenyl, or C₃-C₆-cycloalkyl;
also preferred R^{3A} is H, C₁-C₆-alkyl, C₁-C₆-haloalkyl, or C₁-C₆-alkylcarbonyl;
particularly preferred R^{3A} is H, C₁-C₆-alkyl, or C₁-C₆-alkylcarbonyl;
especially preferred R^{3A} is H, or C₁-C₆-alkyl;
most preferred R^{3A} is H or CH₃.
Preferred R⁴ is halogen, CN, C₁-C₆-alkyl, C₁-C₆-haloalkyl, or C₁-C₆-alkoxy;
particularly preferred R⁴ is halogen, C₁-C₆-haloalkyl, or C₁-C₆-alkyl;
especially preferred R⁴ is halogen;
also especially preferred R⁴ is C₁-C₆-haloalkyl, or C₁-C₆-alkyl;
more preferred R⁴ is F, Cl, CHF₂, CF₃, CH₃, or C₂H₅;
most preferred R⁴ is F;
also most preferred R⁴ is CH₃;
also most preferred R⁴ is Cl;
also most preferred R⁴ is CF₃.

Preferred m is 0, 1, or 2;
more preferred m is 0 or 1;
most preferred m is 0.
also most preferred m is 1.

Also preferred is the pyridine compounds of formula (I), and their use as herbicide, wherein R¹ is preferably C₁-C₆-alkyl, C₁-C₆-alkoxy, or C₃-C₆-cycloalkyl, wherein the cycloalkyl substituent is unsubstituted;
particularly preferred R¹ is C₃-C₆-cycloalkyl, wherein the cycloalkyl substituent is unsubstituted;
R² is preferably OH, C₁-C₆-alkoxy, C₃-C₆-alkynyloxy, or C₁-C₆-haloalkoxy;
particularly preferred R² is C₁-C₆-alkoxy, C₃-C₆-alkynyloxy, or C₁-C₆-haloalkoxy also particularly preferred R² is OH or C₁-C₆-alkoxy,
more preferred R² is OH;
also more preferred R² is C₁-C₆-alkoxy;
A is preferably CR³, C*, N, O, S or NR^{3A};
particularly preferred A is CR³, C*, or N;
also particularly preferred A is CR³, C*, O or S;
Preferred Z is 9 or 10 membered bicyclic ring;
particularly preferred Z is 9 membered bicyclic ring;
particularly preferred Z is 10 membered bicyclic ring;
more preferred Z is selected from radicals A to O, as defined herein;
R³ is preferably halogen, CN, C₁-C₆-alkyl, C₁-C₆-haloalkyl, or C₁-C₆-alkoxy;
particularly preferred R³ is halogen or CH₃;
R^{3A} is preferably H or C₁-C₆-alkyl;
particularly preferred R^{3A} is H or CH₃;
m is preferably 0 or 1;
R⁴ is preferably halogen.

Also preferred is the pyridine compounds of formula (I.1) (corresponds to pyridine compounds of formula (I) wherein R² is OH), and their use as herbicide, 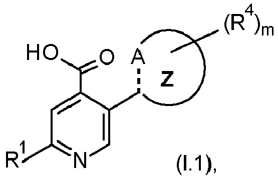 wherein the dotted line (------) is a single bond or a double bond;
R¹ is C₃-C₆-cycloalkyl, C₁-C₆-alkyl, or C₁-C₆-alkoxy;
A is CR³, C*, NR^{3A}, N, O or S;
R³ is halogen, C₁-C₆-alkyl, C₁-C₆-haloalkyl, or C₁-C₆-alkoxy;
R^{3A} is H or C₁-C₆-alkyl;
Z is radical selected from A to O;
m is 0 or 1;
R⁴ is F, CHF₂, CF₃, CH₃, or C₂H₅.

Also preferred is the pyridine compounds of formula (1.2) (corresponds to pyridine compounds of formula (I) wherein R² is OCH₃), and their use as herbicide, 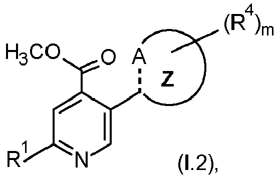 wherein the dotted line (------) is a single bond or a double bond;
R¹ is C₃-C₆-cycloalkyl, C₁-C₆-alkyl, or C₁-C₆-alkoxy;
A is CR³, C*, NR^{3A}, N, O or S;
R³ is halogen, C₁-C₆-alkyl, C₁-C₆-haloalkyl, or C₁-C₆-alkoxy;
Z is radical selected from A to O;
m is 0 or 1;
R⁴ is F, CHF₂, CH₃, CF₃, or C₂H₅.

Also preferred is the pyridine compounds of formula (1.3) (corresponds to pyridine compounds of formula (I) wherein R² is OC₂H₅), and their use as herbicide, 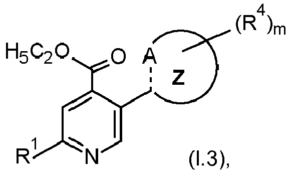 wherein the dotted line (------) is a single bond or a double bond;
R¹ is C₃-C₆-cycloalkyl, C₁-C₆-alkyl, or C₁-C₆-alkoxy;
A is CR³, C*, NR^{3A}, N, O or S;
R³ is halogen, C₁-C₆-alkyl, C₁-C₆-haloalkyl, or C₁-C₆-alkoxy;
Z is radical selected from A to O;
m is 0 or 1;
R⁴ is F, CHF₂, CH₃, CF₃, or C₂H₅.

Also preferred is the pyridine compounds of formula (1.4) (corresponds to pyridine compounds of formula (I) wherein R² is OCH₂C=CH), and their use as herbicide, 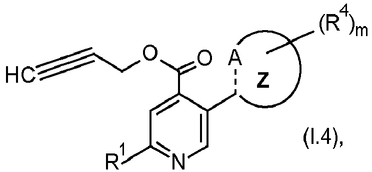 wherein the dotted line (------) is a single bond or a double bond;
R¹ is C₃-C₆-cycloalkyl, C₁-C₆-alkyl, or C₁-C₆-alkoxy;
A is CR³, C*, NR^{3A}, N, O or S;
R³ is halogen, C₁-C₆-alkyl, C₁-C₆-haloalkyl, or C₁-C₆-alkoxy;
Z is radical selected from A to O;
m is 0 or 1;
R⁴ is F, CHF₂, CH₃, CF₃, or C₂H₅.

Also preferred is the pyridine compounds of formula (1.5) (corresponds to pyridine compounds of formula (I) wherein R² is OCH₂CHF₂), and their use as herbicide, 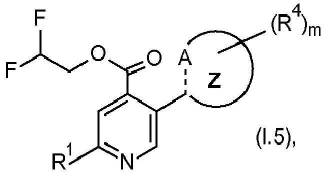 wherein the dotted line (------) is a single bond or a double bond;
R¹ is C₃-C₆-cycloalkyl, C₁-C₆-alkyl, or C₁-C₆-alkoxy;
A is CR³, C*, NR^{3A}, N, O or S;
R³ is halogen, C₁-C₆-alkyl, C₁-C₆-haloalkyl, or C₁-C₆-alkoxy;
Z is radical selected from A to O;
m is 0 or 1;
R⁴ is F, CHF₂, CH₃, CF₃, or C₂H₅.

Also preferred is the pyridine compounds of formula (1.6) (corresponds to pyridine compounds of formula (I) wherein R² is NHOCH₃), and their use as herbicide, 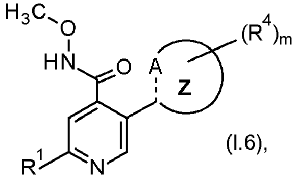 wherein the dotted line (------) is a single bond or a double bond;
R¹ is C₃-C₆-cycloalkyl, C₁-C₆-alkyl, or C₁-C₆-alkoxy;
A is CR³, C*, NR^{3A}, N, O or S;
R³ is halogen, C₁-C₆-alkyl, C₁-C₆-haloalkyl, or C₁-C₆-alkoxy;
Z is radical selected from A to O;
m is 0 or 1;
R⁴ is F, CHF₂, CH₃, CF₃, or C₂H₅.

Also preferred is the pyridine compounds of formula (1.7) (corresponds to pyridine compounds of formula (I) wherein R² is NHCOCH₃), and their use as herbicide, 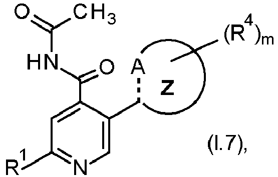 wherein the dotted line (------) is a single bond or a double bond;
R¹ is C₃-C₆-cycloalkyl, C₁-C₆-alkyl, or C₁-C₆-alkoxy;
A is CR³, C*, NR^{3A}, N, O or S;
R³ is halogen, C₁-C₆-alkyl, C₁-C₆-haloalkyl, or C₁-C₆-alkoxy;
Z is radical selected from A to O;
m is 0 or 1;
R⁴ is F, CHF₂, CH₃, CF₃, or C₂H₅.

Particular preference is given to the pyridine compounds of formula I which corresponds to compounds of formulae I.a to I.I, and their use as herbicide, wherein X, V and W independently are CH₂, CF₂, O, NR^{3A}, N or S.

Also particular preference is given to the pyridine compounds of formula I which corresponds to compounds of formulae I.m and I.n, and their use as herbicide, wherein X, and V independently are CH₂, CF₂, O, NR^{3A}, N or S, W independently is CH or N.
pyridine compounds

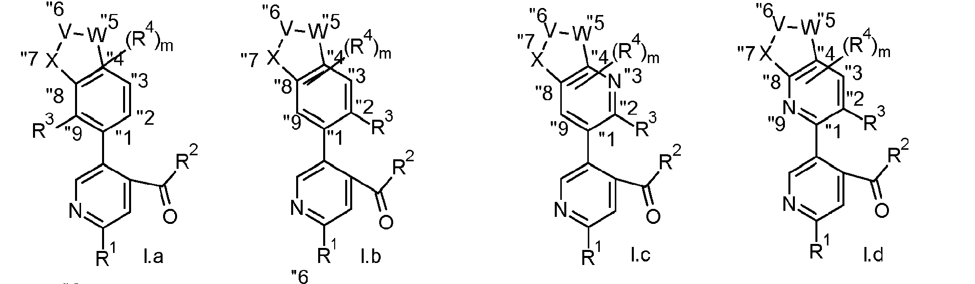

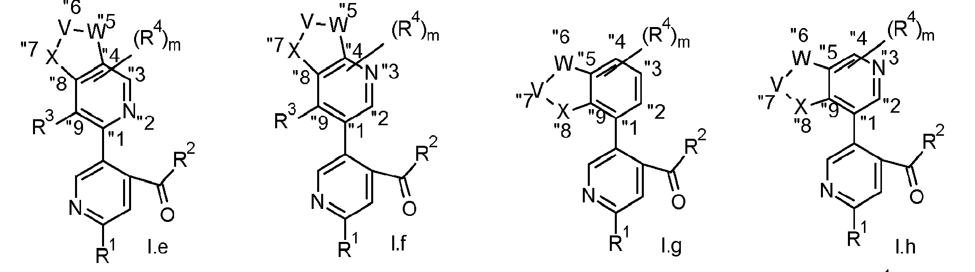

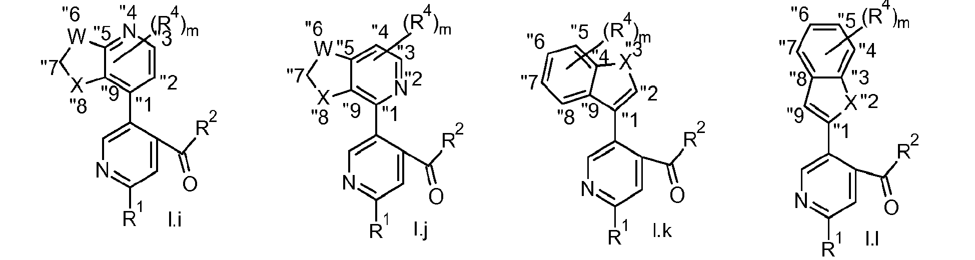

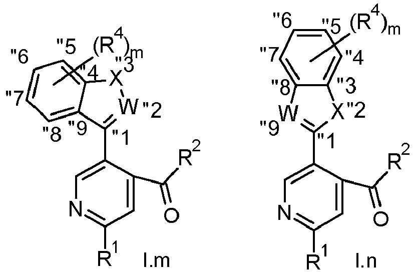

Most preferred compounds of formula I, and their use as herbicide, are the compounds of the formulae I.a to I.I wherein
R¹ is C₂H₅, c-C₃H₅, c-C₄H₇, or OCH₃;
R² is OH, OCH₃, OC₂H₅, OCH₂C≡CH, OCH₂CHF₂, NHOCH₃, or NHC(O)CH₃;
R³ is CH₃, OCH₃, Cl, Br, CHF₂, CF₃, F, or I;
X is CH₂, O, S, or NCH₃;
V is CH₂ or CF₂;
W is CH₂, O, N, or S;
m is 0 or 1;
R⁴ is F, CI or CF₃.

Also most preferred compounds of formula I, and their use as herbicide, are the compounds of the formulae I.m and I.n wherein
R¹ is C₂H₅, c-C₃H₅, c-C₄H₇, or OCH₃;
R² is OH, OCH₃, OC₂H₅, OCH₂C=CH, OCH₂CHF₂, NHOCH₃, or NHC(O)CH₃;
R³ is CH₃, OCH₃, Cl, Br, CHF₂, CF₃, F, or I;
X is CH₂, O, S, or NCH₃;
V is CH₂ or CF₂;
W is CH or N;
m is 0 or 1;
R⁴ is F, CI or CF₃.

Each of the groups mentioned for a substituent in the tables is furthermore per se, independently of the combination in which it is mentioned, a particularly preferred aspect of the substituent in question.

Particularly preferred compounds of formula I, and their use as herbicide, compounds of the invention are the compounds of the formulae that are compiled in the following Tables 1 to 56.
Table 1. Compounds of formula I.a, wherein m is 0, V is CH₂, X and W are O (= compound of formula 1.1), and the meaning for the combination of R¹, R², and R³ for each individual compound corresponds in each case to one line of Table A.
Table 2. Compounds of formula I.a, wherein m is 1, R⁴ is 2"- CI, V is CH₂, X and W are O (= compound of formula 1.2), and the meaning for the combination of R¹, R², and R³ for each individual compound corresponds in each case to one line of Table A.
Table 3. Compounds of formula I.b, wherein m is 0, V is CH₂, X and W are O (= compound of formula 1.3), and the meaning for the combination of R¹, R², and R³ for each individual compound corresponds in each case to one line of Table A.
Table 4. Compounds of formula I.b, wherein m is 1, R⁴ is 9"- CI, V is CH₂, X and W are O (= compound of formula 1.4), and the meaning for the combination of R¹, R², and R³ for each individual compound corresponds in each case to one line of Table A.
Table 5. Compounds of formula I.c, wherein m is 0, V is CH₂, X and W are O (= compound of formula 1.5), and the meaning for the combination of R¹, R², and R³ for each individual compound corresponds in each case to one line of Table A.
Table 6. Compounds of formula I.c, wherein m is 1, R⁴ is 9"- CI, V is CH₂, X and W are O (= compound of formula 1.6), and the meaning for the combination of R¹, R², and R³ for each individual compound corresponds in each case to one line of Table A.
Table 7. Compounds of formula I.d, wherein m is 0, V is CH₂, X and W are O (= compound of formula 1.7), and the meaning for the combination of R¹, R², and R³ for each individual compound corresponds in each case to one line of Table A.
Table 8. Compounds of formula I.d, wherein m is 1, R⁴ is 3"- CI, V is CH₂, X and W are O (= compound of formula 1.8), and the meaning for the combination of R¹, R², and R³ for each individual compound corresponds in each case to one line of Table A.
Table 9. Compounds of formula I.e, wherein m is 0, V is CH₂, X and W are O (= compound of formula 1.9), and the meaning for the combination of R¹, R², and R³ for each individual compound corresponds in each case to one line of Table A.
Table 10. Compounds of formula I.e, wherein m is 1, R⁴ is 3"- CI, V is CH₂, X and W are O (= compound of formula 1.10), and the meaning for the combination of R¹, R², and R³ for each individual compound corresponds in each case to one line of Table A.
Table 11. Compounds of formula I.f, wherein m is 0, V is CH₂, X and W are O (= compound of formula 1.11), and the meaning for the combination of R¹, R², and R³ for each individual compound corresponds in each case to one line of Table A.
Table 12. Compounds of formula I.f, wherein m is 1, R⁴ is 2"- CI, V is CH₂, X and W are O (= compound of formula 1.12), and the meaning for the combination of R¹, R², and R³ for each individual compound corresponds in each case to one line of Table A. 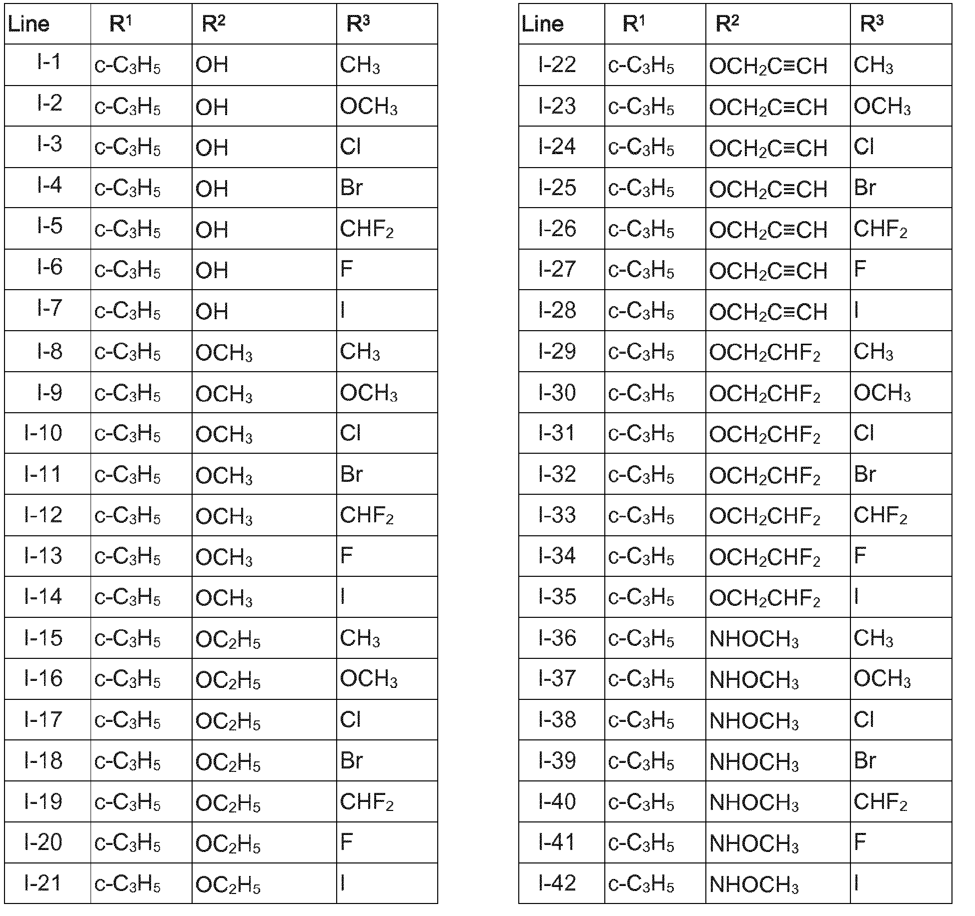 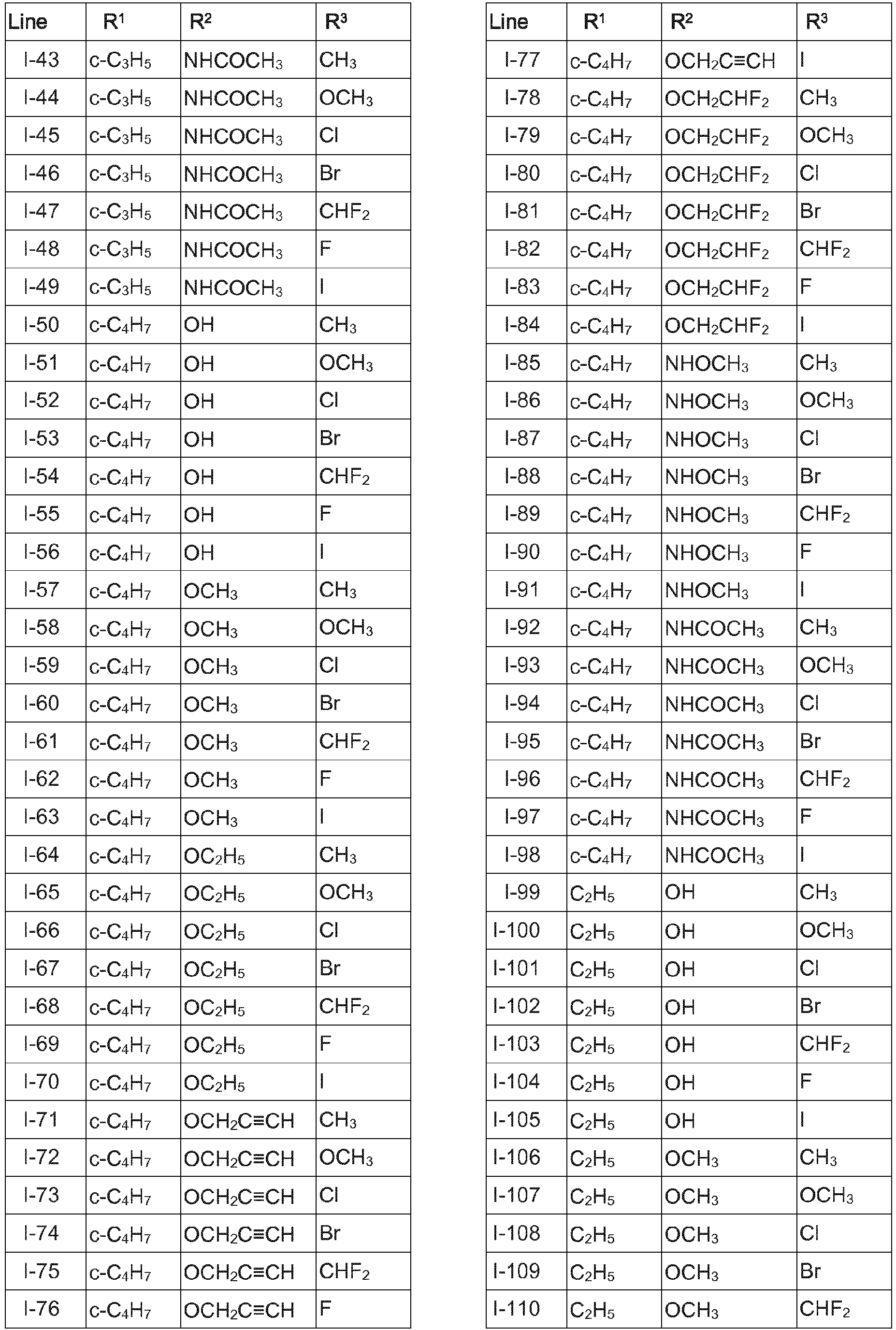 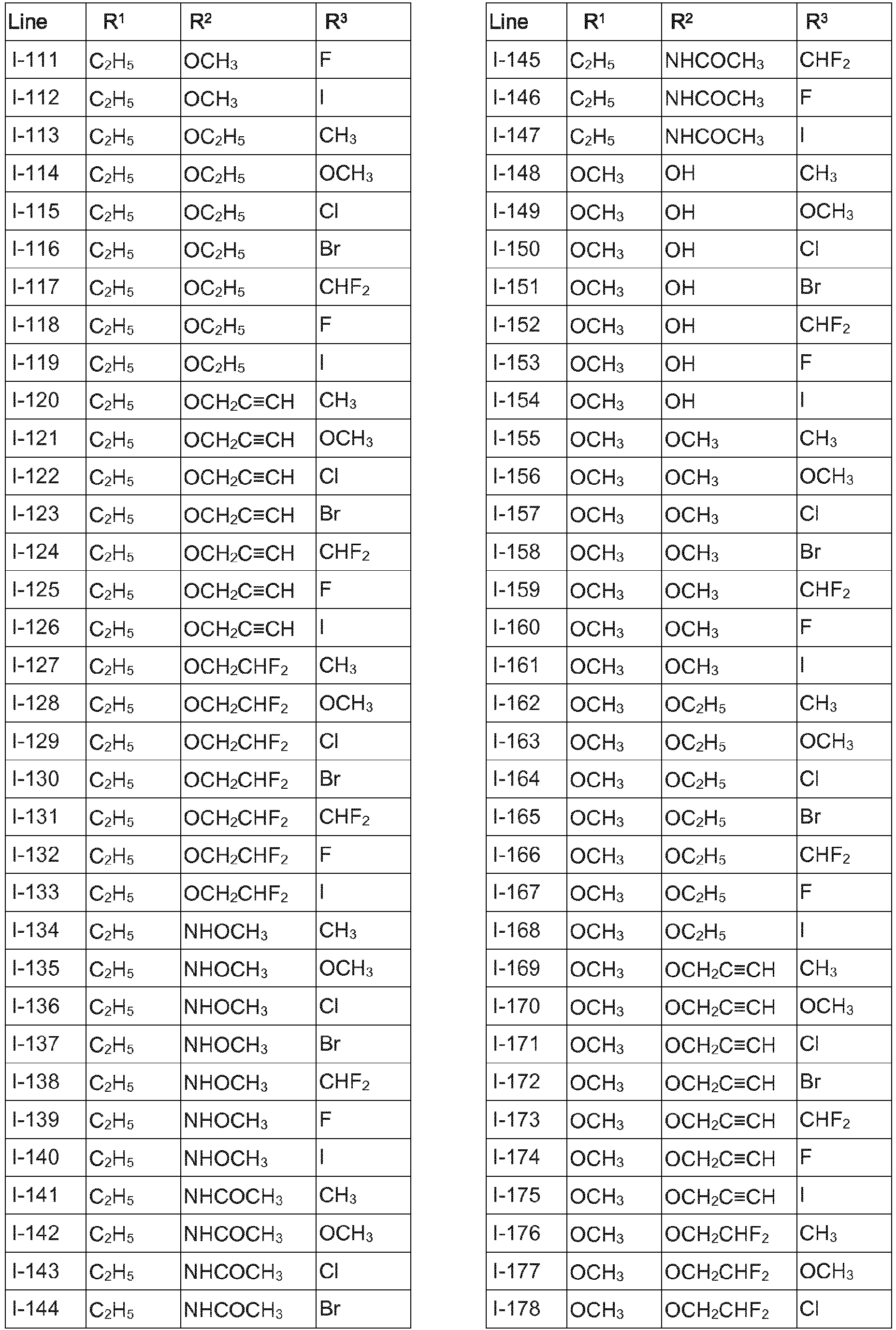 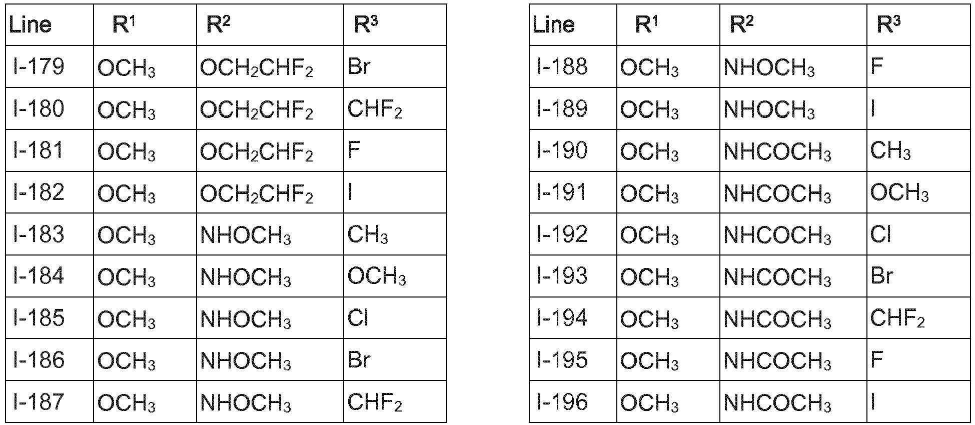
Table 13. Compounds of formula I.g, wherein m is 0, V is CF₂, V is CH₂, X and W are O (= compound of formula 1.13), and the meaning for the combination of R¹ and R² for each individual compound corresponds in each case to one line of Table A1.
Table 14. Compounds of formula I.g, wherein m is 1, R⁴ is 2"- CI, V is CF₂, X and W are O (= compound of formula 1.14), and the meaning for the combination of R¹ and R² for each individual compound corresponds in each case to one line of Table A1.
Table 15. Compounds of formula I.g, wherein m is 0, V is CH₂, X and W are O (= compound of formula 1.15), and the meaning for the combination of R¹ and R² for each individual compound corresponds in each case to one line of Table A1.
Table 16. Compounds of formula I.g, wherein m is 1, R⁴ is 2"- CI, V is CH₂, X and W are O (= compound of formula 1.16), and the meaning for the combination of R¹ and R² for each individual compound corresponds in each case to one line of Table A1.
Table 17. Compounds of formula I.g, wherein m is 0, V is CH₂, X is CH₂ and W is O (= compound of formula 1.17), and the meaning for the combination of R¹ and R² for each individual compound corresponds in each case to one line of Table A1.
Table 18. Compounds of formula I.g, wherein m is 1, R⁴ is 2"- CI, V is CH₂, X is CH₂ and W is O (= compound of formula 1.18), and the meaning for the combination of R¹ and R² for each individual compound corresponds in each case to one line of Table A1.
Table 19. Compounds of formula I.g, wherein m is 0, V, X, and W are CH₂ (= compound of formula 1.19), and the meaning for the combination of R¹ and R² for each individual compound corresponds in each case to one line of Table A1.
Table 20. Compounds of formula I.g, wherein m is 1, R⁴ is 2"- CI, V, X, and W are CH₂(= compound of formula 1.20), and the meaning for the combination of R¹ and R² for each individual compound corresponds in each case to one line of Table A1.
Table 21. Compounds of formula I.h, wherein m is 0, V is CF₂, X and W are O (= compound of formula 1.21), and the meaning for the combination of R¹ and R² for each individual compound corresponds in each case to one line of Table A1.
Table 22. Compounds of formula I.h, wherein m is 1, R⁴ is 2"- CI, V is CF₂, X and W are O (= compound of formula 1.22), and the meaning for the combination of R¹ and R² for each individual compound corresponds in each case to one line of Table A1.
Table 23. Compounds of formula I.h, wherein m is 0, V is CH₂, X and W are O (= compound of formula 1.23), and the meaning for the combination of R¹ and R² for each individual compound corresponds in each case to one line of Table A1.
Table 24. Compounds of formula I.h, wherein m is 1, R⁴ is 2"- CI, V is CH₂, X and W are O (= compound of formula 1.24), and the meaning for the combination of R¹ and R² for each individual compound corresponds in each case to one line of Table A1.
Table 25. Compounds of formula I.h, wherein m is 0, V is CH₂, X is CH₂ and W is O (= compound of formula 1.25), and the meaning for the combination of R¹ and R² for each individual compound corresponds in each case to one line of Table A1.
Table 26. Compounds of formula I.h, wherein m is 1, R⁴ is 2"- CI, V is CH₂, X is CH₂(= compound of formula 1.26), and W is O, and the meaning for the combination of R¹ and R² for each individual compound corresponds in each case to one line of Table A1.
Table 27. Compounds of formula I.h, wherein m is 0, V, X, and W are CH₂ (= compound of formula 1.27), and the meaning for the combination of R¹ and R² for each individual compound corresponds in each case to one line of Table A1.
Table 28. Compounds of formula I.h, wherein m is 1, R⁴ is 2"- CI, V, X, and W are CH₂(= compound of formula 1.28), and the meaning for the combination of R¹ and R² for each individual compound corresponds in each case to one line of Table A1.
Table 29. Compounds of formula I.i, wherein m is 0, X and W are O (= compound of formula 1.29), and the meaning for the combination of R¹ and R² for each individual compound corresponds in each case to one line of Table A1.
Table 30. Compounds of formula I.i, wherein m is 1, R⁴ is 2"- CI, X and W are O (= compound of formula 1.30), and the meaning for the combination of R¹, R², and R³ for each individual compound corresponds in each case to one line of Table A1.
Table 31. Compounds of formula I.j, wherein m is 0, X and W are O (= compound of formula 1.31), and the meaning for the combination of R¹ and R² for each individual compound corresponds in each case to one line of Table A1.
Table 32. Compounds of formula I.j, wherein m is 1, R⁴ is 3"- CI, X and W are O (= compound of formula 1.32), and the meaning for the combination of R¹ and R² for each individual compound corresponds in each case to one line of Table A1.
Table 33. Compounds of formula I.k, wherein m is 0, X is O (= compound of formula 1.33), and the meaning for the combination of R¹ and R² for each individual compound corresponds in each case to one line of Table A1.
Table 34. Compounds of formula I.k, wherein m is 1, X is O, R⁴ is 2"- CI, (= compound of formula 1.34) and the meaning for the combination of R¹ and R² for each individual compound corresponds in each case to one line of Table A1.
Table 35. Compounds of formula I.k, wherein m is 0, X is S (= compound of formula 1.35), and the meaning for the combination of R¹ and R² for each individual compound corresponds in each case to one line of Table A1.
Table 36. Compounds of formula I.k, wherein m is 1, X is S, R⁴ is 2"- CI, (= compound of formula 1.36) and the meaning for the combination of R¹ and R² for each individual compound corresponds in each case to one line of Table A1.
Table 37. Compounds of formula I.k, wherein m is 0, X is NCH₃, (= compound of formula 1.37) and the meaning for the combination of R¹ and R² for each individual compound corresponds in each case to one line of Table A1.
Table 38. Compounds of formula I.k, wherein m is 1, X is NCH₃, R⁴ is 2"- CI, (= compound of formula 1.38) and the meaning for the combination of R¹ and R² for each individual compound corresponds in each case to one line of Table A1.
Table 39. Compounds of formula I.l, wherein m is 0, X is O, (= compound of formula 1.39), and the meaning for the combination of R¹ and R² for each individual compound corresponds in each case to one line of Table A1.
Table 40. Compounds of formula I.I, wherein m is 1, X is O, R⁴ is 9"-Cl (= compound of formula 1.40), and the meaning for the combination of R¹ and R² for each individual compound corresponds in each case to one line of Table A1.
Table 41. Compounds of formula I.l, wherein m is 0, X is S (= compound of formula 1.41), and the meaning for the combination of R¹ and R² for each individual compound corresponds in each case to one line of Table A1.
Table 42. Compounds of formula I.l, wherein m is 1, X is S, R⁴ is 9"-Cl (= compound of formula 1.42), and the meaning for the combination of R¹ and R² for each individual compound corresponds in each case to one line of Table A1.
Table 43. Compounds of formula I.l, wherein m is 0, X is NCH₃ (= compound of formula 1.43), and the meaning for the combination of R¹ and R² for each individual compound corresponds in each case to one line of Table A1.
Table 44. Compounds of formula I.l, wherein m is 1, X is NCH₃, R⁴ is 9"-Cl (= compound of formula 1.44), and the meaning for the combination of R¹ and R² for each individual compound corresponds in each case to one line of Table A1.
Table 45. Compounds of formula I.m, wherein m is 0, X is O, W is N (= compound of formula 1.45), and the meaning for the combination of R¹ and R² for each individual compound corresponds in each case to one line of Table A1.
Table 46. Compounds of formula I.m, wherein m is 1, X is O, W is N, R⁴ is 8"-Cl (= compound of formula 1.46), and the meaning for the combination of R¹ and R² for each individual compound corresponds in each case to one line of Table A1.
Table 47. Compounds of formula I.m, wherein m is 0, X is S, W is N (= compound of formula 1.47), and the meaning for the combination of R¹ and R² for each individual compound corresponds in each case to one line of Table A1.
Table 48. Compounds of formula I.m, wherein m is 1, X is S, W is N, R⁴ is 8"-Cl (= compound of formula 1.48), and the meaning for the combination of R¹ and R² for each individual compound corresponds in each case to one line of Table A1.
Table 49. Compounds of formula I.m, wherein m is 0, X is NCH₃, W is N (= compound of formula 1.49), and the meaning for the combination of R¹ and R² for each individual compound corresponds in each case to one line of Table A1.
Table 50. Compounds of formula I.m, wherein m is 1, X is NCH₃, W is N, R⁴ is 8"-Cl (= compound of formula 1.50), and the meaning for the combination of R¹ and R² for each individual compound corresponds in each case to one line of Table A1.
Table 51. Compounds of formula I.n, wherein m is 0, X is O, W is N (= compound of formula 1.51), and the meaning for the combination of R¹ and R² for each individual compound corresponds in each case to one line of Table A1.
Table 52. Compounds of formula I.n, wherein m is 1, X is O, W is N, R⁴ is 7"-Cl (= compound of formula 1.52), and the meaning for the combination of R¹ and R² for each individual compound corresponds in each case to one line of Table A1.
Table 53. Compounds of formula I.n, wherein m is 0, X is S, W is N (= compound of formula 1.53), and the meaning for the combination of R¹ and R² for each individual compound corresponds in each case to one line of Table A1.
Table 54. Compounds of formula I.n, wherein m is 1, X is S, W is N, R⁴ is 7"-Cl (= compound of formula 1.54), and the meaning for the combination of R¹ and R² for each individual compound corresponds in each case to one line of Table A1.
Table 55. Compounds of formula I.n, wherein m is 0, X is NCH₃, W is N (= compound of formula 1.55), and the meaning for the combination of R¹ and R² for each individual compound corresponds in each case to one line of Table A1.
Table 56. Compounds of formula I.n, wherein m is 1, X is NCH₃, W is N, R⁴ is 7"-Cl (= compound of formula 1.56), and the meaning for the combination of R¹ and R² for each individual compound corresponds in each case to one line of Table A1.

**Table A1:**

| **Line** | **R¹** | **R²** |
|---|---|---|
| I-197 | c-C₃H₅ | OH |
| I-198 | c-C₃H₅ | OCH₃ |
| I-199 | c-C₃H₅ | OC₂H₅ |
| I-200 | c-C₃H₅ | OCH₂C≡CH |
| I-201 | c-C₃H₅ | OCH₂CHF₂ |
| I-202 | c-C₃H₅ | NHOCH₃ |
| I-203 | c-C₃H₅ | NHCOCH₃ |
| I-204 | c-C₄H₇ | OH |
| I-205 | c-C₄H₇ | OCH₃ |
| I-206 | c-C₄H₇ | OC₂H₅ |
| I-207 | c-C₄H₇ | OCH₂C≡CH |
| I-208 | c-C₄H₇ | OCH₂CHF₂ |
| I-209 | c-C₄H₇ | NHOCH₃ |
| I-210 | c-C₄H₇ | NHCOCH₃ |
| I-211 | C₂H₅ | OH |
| I-212 | C₂H₅ | OCH₃ |
| I-213 | C₂H₅ | OC₂H₅ |
| I-214 | C₂H₅ | OCH₂C≡CH |
| I-215 | C₂H₅ | OCH₂CHF₂ |
| I-216 | C₂H₅ | NHOCH₃ |
| I-217 | C₂H₅ | NHCOCH₃ |
| I-218 | OCH₃ | OH |
| I-219 | OCH₃ | OCH₃ |
| I-220 | OCH₃ | OC₂H₅ |
| I-221 | OCH₃ | OCH₂C≡CH |
| I-222 | OCH₃ | OCH₂CHF₂ |
| I-223 | OCH₃ | NHOCH₃ |
| I-224 | OCH₃ | NHCOCH₃ |

The specific number for each single compound is deductible as follows:
Compound 1.1.I-3 for example comprises the compound of formula 1.1 from Table 1 and line I-3 from Table A;
Compound 1.16.I-198 for example comprises the compound of formula 1.16 from Table 16 and line I-198 from Table A1.

To widen the spectrum of action and to achieve synergistic effects, the pyridine compounds of formula (I) may be mixed with a large number of representatives of other herbicidal or growth-regulating active ingredient groups and then applied concomitantly. Suitable components for mixtures are, for example, herbicides from the classes of the acetamides, amides, aryloxyphenoxypropionates, benzamides, benzofuran, benzoic acids, benzothiadiazinones, bipyridylium, carbamates, chloroacetamides, chlorocarboxylic acids, cyclohexanediones, dinitroanilines, dinitrophenol, diphenyl ether, glycines, imidazolinones, isoxazoles, isoxazolidinones, nitriles, N-phenylphthalimides, oxadiazoles, oxazolidinediones, oxyacetamides, phenoxycarboxylic acids, phenylcarbamates, phenylpyrazoles, phenylpyrazolines, phenylpyridazines, phosphinic acids, phosphoroamidates, phosphorodithioates, phthalamates, pyrazoles, pyridazinones, pyridines, pyridinecarboxylic acids, pyridinecarboxamides, pyrimidinediones, pyrimidinyl(thio)benzoates, quinolinecarboxylic acids, semicarbazones, sulfonylaminocarbonyltriazolinones, sulfonylureas, tetrazolinones, thiadiazoles, thiocarbamates, triazines, triazinones, triazoles, triazolinones, triazolocarboxamides, triazolopyrimidines, triketones, uracils, or ureas.

It may furthermore be beneficial to apply the pyridine compounds of formula (I) alone or in combination with other herbicides, or else in the form of a mixture with other crop protection agents, for example together with agents for controlling pests or phytopathogenic fungi or bacteria. Also of interest is the miscibility with mineral salt solutions, which are employed for treating nutritional and trace element deficiencies. Other additives such as non-phytotoxic oils and oil concentrates may also be added.

In one embodiment of the present invention the compositions according to the present invention comprise at least one pyridine compound of formula (I) (compound A) and at least one further active compound selected from herbicides B, preferably herbicides B of class b1) to b15), and safeners C (compound C).

In a preferred embodiment of the invention, the composition comprises as active compound A or component A at least one, preferably exactly one, compound A of formula (I.1) (corresponds to compound A of formula (I)), as defined herein;
In another preferred embodiment of the invention, the composition comprises as active compound A or component A at least one, preferably exactly one, compound A of formula (I.2) (corresponds to compound A of formula (I)), as defined herein;
In another preferred embodiment of the invention, the composition comprises as active compound A or component A at least one, preferably exactly one, compound A of formula (I.3) (corresponds to compound A of formula (I)), as defined herein;
In another preferred embodiment of the invention, the composition comprises as active compound A or component A at least one, preferably exactly one, compound A of formula (I.4) (corresponds to compound A of formula (I)), as defined herein;
In another preferred embodiment of the invention, the composition comprises as active compound A or component A at least one, preferably exactly one, compound A of formula (1.5) (corresponds to compound A of formula (I)), as defined herein;
In another preferred embodiment of the invention, the composition comprises as active compound A or component A at least one, preferably exactly one, compound A of formula (I.6) (corresponds to compound A of formula (I)), as defined herein;
In another preferred embodiment of the invention, the composition comprises as active compound A or component A at least one, preferably exactly one, compound A of formula (I.7) (corresponds to compound A of formula (I)), as defined herein;
Preferred compounds of the formula (I) which, as component A, are constituent of the composition according to the invention are the compounds I.1 to I.7, as defined above;
In another embodiment of the present invention the compositions according to the present invention comprise at least one pyridine compound of formula (I) and at least one further active compound B (herbicide B).

The further herbicidal compound B (component B) is preferably selected from the herbicides of class b1) to b15):
Mixing partners for the composition can be selected from below herbicides B as defined below:
   B) herbicides of class b1) to b15):
      b1) lipid biosynthesis inhibitors;
      b2) acetolactate synthase inhibitors (ALS inhibitors);
      b3) photosynthesis inhibitors;
      b4) protoporphyrinogen-IX oxidase inhibitors (PPO inhibitors);
      b5) bleacher herbicides;
      b6) enolpyruvyl shikimate 3-phosphate synthase inhibitors (EPSP inhibitors);
      b7) glutamine synthetase inhibitors;
      b8) 7,8-dihydropteroate synthase inhibitors (DHP inhibitors);
      b9) mitosis inhibitors;
      b10) inhibitors of the synthesis of very long chain fatty acids (VLCFA inhibitors);
      b11) cellulose biosynthesis inhibitors;
      b12) decoupler herbicides;
      b13) auxinic herbicides;
      b14) auxin transport inhibitors; and
      b15) other herbicides selected from the group consisting of bromobutide, chlorflurenol, chlorflurenol-methyl, cinmethylin, cumyluron, dalapon, dazomet, difenzoquat, difenzoquat-metilsulfate, dimethipin, DSMA, dymron, endothal and its salts, etobenzanid, flamprop, flamprop-isopropyl, flamprop-methyl, flamprop-M-isopropyl, flamprop-M-methyl, flurenol, flurenol-butyl, flurprimidol, fosamine, fosamine-ammonium, indanofan, indaziflam, maleic hydrazide, mefluidide, metam, methiozolin (CAS 403640-27-7), methyl azide, methyl bromide, methyl-dymron, methyl iodide, MSMA, oleic acid, oxaziclomefone, pelargonic acid, pyributicarb, quinoclamine, triaziflam, tridiphane and 6-chloro-3-(2-cyclopropyl-6-methylphenoxy)-4-pyridazinol (CAS 499223-49-3) and its salts and esters;
         including their agriculturally acceptable salts or derivatives;

In one embodiment of the invention, the compositions contain at least one inhibitor of the lipid biosynthesis (herbicide b1). These compounds inhibit lipid biosynthesis. Inhibition of the lipid biosynthesis can be affected either through inhibition of acetylCoA carboxylase (hereinafter-termed ACCase herbicides) or through a different mode of action (hereinafter termed non-ACCase herbicides). The ACCase herbicides belong to the group A of the HRAC classification system whereas the non-ACCase herbicides belong to the group N of the HRAC classification.

In another embodiment of the invention, the compositions contain at least one ALS inhibitor (herbicide b2). The herbicidal activity of these compounds is based on the inhibition of acetolactate synthase and thus on the inhibition of the branched chain amino acid biosynthesis. These inhibitors belong to the group B of the HRAC classification system.

In another embodiment of the invention, the compositions contain at least one inhibitor of photosynthesis (herbicide b3). The herbicidal activity of these compounds is based either on the inhibition of the photosystem II in plants (so-called PSII inhibitors, groups C1, C2 and C3 of HRAC classification) or on diverting the electron transfer in photosystem I in plants (so-called PSI inhibitors, group D of HRAC classification) and thus on an inhibition of photosynthesis. Amongst these, PSII inhibitors are preferred.

In another embodiment of the invention, the compositions contain at least one inhibitor of protoporphyrinogen-IX-oxidase (herbicide b4). The herbicidal activity of these compounds is based on the inhibition of the protoporphyrinogen-IX-oxidase. These inhibitors belong to the group E of the HRAC classification system.

In another embodiment of the invention, the compositions contain at least one bleacher-herbicide (herbicide b5). The herbicidal activity of these compounds is based on the inhibition of the carotenoid biosynthesis. These include compounds which inhibit carotenoid biosynthesis by inhibition of phytoene desaturase (so-called PDS inhibitors, group F1 of HRAC classification), compounds that inhibit the 4-hydroxyphenylpyruvate-dioxygenase (HPPD inhibitors, group F2 of HRAC classification), compounds that inhibit DOX synthase (group F4 of HRAC class) and compounds which inhibit carotenoid biosynthesis by an unknown mode of action (bleacher - unknown target, group F3 of HRAC classification).

In another embodiment of the invention, the compositions contain at least one EPSP synthase inhibitor (herbicide b6). The herbicidal activity of these compounds is based on the inhibition of enolpyruvyl shikimate 3-phosphate synthase, and thus on the inhibition of the amino acid biosynthesis in plants. These inhibitors belong to the group G of the HRAC classification system.

In another embodiment of the invention, the compositions contain at least one glutamine synthetase inhibitor (herbicide b7). The herbicidal activity of these compounds is based on the inhibition of glutamine synthetase, and thus on the inhibition of the aminoacid biosynthesis in plants. These inhibitors belong to the group H of the HRAC classification system.

In another embodiment of the invention, the compositions contain at least one DHP synthase inhibitor (herbicide b8). The herbicidal activity of these compounds is based on the inhibition of 7,8-dihydropteroate synthase. These inhibitors belong to the group I of the HRAC classification system.

In another embodiment of the invention, the compositions contain at least one mitosis inhibitor (herbicide b9). The herbicidal activity of these compounds is based on the disturbance or inhibition of microtubule formation or organization, and thus on the inhibition of mitosis. These inhibitors belong to the groups K1 and K2 of the HRAC classification system. Among these, compounds of the group K1, in particular dinitroanilines, are preferred.

In another embodiment of the invention, the compositions contain at least one VLCFA inhibitor (herbicide b10). The herbicidal activity of these compounds is based on the inhibition of the synthesis of very long chain fatty acids and thus on the disturbance or inhibition of cell division in plants. These inhibitors belong to the group K3 of the HRAC classification system.

In another embodiment of the invention, the compositions contain at least one cellulose biosynthesis inhibitor (herbicide b11). The herbicidal activity of these compounds is based on the inhibition of the biosynthesis of cellulose and thus on the inhibition of the synthesis of cell walls in plants. These inhibitors belong to the group L of the HRAC classification system.

In another embodiment of the invention, the compositions contain at least one decoupler herbicide (herbicide b12). The herbicidal activity of these compounds is based on the disruption of the cell membrane. These inhibitors belong to the group M of the HRAC classification system.

In another embodiment of the invention, the compositions contain at least one auxinic herbicide (herbicide b13). These include compounds that mimic auxins, i.e. plant hormones, and affect the growth of the plants. These compounds belong to the group O of the HRAC classification system.

In another embodiment of the invention, the compositions contain at least one auxin transport inhibitor (herbicide b14). The herbicidal activity of these compounds is based on the inhibition of the auxin transport in plants. These compounds belong to the group P of the HRAC classification system.

As to the given mechanisms of action and classification of the active substances, see e.g. "HRAC, Classification of Herbicides According to Mode of Action", http://www.plantprotection.org/hrac/MOA.html).

Preference is given to those compositions according to the present invention comprising at least one herbicide B selected from herbicides of class b1, b2, b3, b4, b5, b6, b9, b10, b13, and b14.

Specific preference is given to those compositions according to the present invention which comprise at least one herbicide B selected from the herbicides of class b1, b2, b4, b5, b9, b10, b13, and b14.

Particular preference is given to those compositions according to the present invention which comprise at least one herbicide B selected from the herbicides of class b1, b2, b4, b5, b9, b10, and b13

Examples of herbicides B which can be used in combination with the compound of formula (I) according to the present invention are:
b1) from the group of the lipid biosynthesis inhibitors:
   ACC-herbicides such as alloxydim, alloxydim-sodium, butroxydim, clethodim, clodinafop, clodinafop-propargyl, cycloxydim, cyhalofop, cyhalofop-butyl, diclofop, diclofop-methyl, fenoxaprop, fenoxaprop-ethyl, fenoxaprop-P, fenoxaprop-P-ethyl, fluazifop, fluazifop-butyl, fluazifop-P, fluazifop-P-butyl, haloxyfop, haloxyfop-methyl, haloxyfop-P, haloxyfop-P-methyl, metamifop, pinoxaden, profoxydim, propaquizafop, quizalofop, quizalofop-ethyl, quizalofop-tefuryl, quizalofop-P, quizalofop-P-ethyl, quizalofop-P-tefuryl, sethoxydim, tepraloxydim, tralkoxydim,
   4-(4'-Chloro-4-cyclopropyl-2'-fluoro[1,1'-biphenyl]-3-yl)-5-hydroxy-2,2,6,6-tetramethyl-2H-pyran-3(6H)-one (CAS 1312337-72-6); 4-(2',4'-Dichloro-4-cyclopropyl[1,1'-biphenyl]-3-yl)-5-hydroxy-2,2,6,6-tetramethyl-2H-pyran-3(6H)-one (CAS 1312337-45-3); 4-(4'-Chloro-4-ethyl-2'-fluoro[1,1'-biphenyl]-3-yl)-5-hydroxy-2,2,6,6-tetramethyl-2H-pyran-3(6H)-one (CAS 1033757-93-5); 4-(2',4'-Dichloro-4-ethyl[1,1'-biphenyl]-3-yl)-2,2,6,6-tetramethyl-2H-pyran-3,5(4H,6H)-dione (CAS 1312340-84-3); 5-(Acetyloxy)-4-(4'-chloro-4-cyclopropyl-2'-fluoro[1,1'-biphenyl]-3-yl)-3,6-dihydro-2,2,6,6-tetramethyl-2H-pyran-3-one (CAS 1312337-48-6); 5-(Acetyloxy)-4-(2',4'-dichloro-4-cyclopropyl- [1,1'-biphenyl]-3-yl)-3,6-dihydro-2,2,6,6-tetramethyl-2H-pyran-3-one; 5-(Acetyloxy)-4-(4'-chloro-4-ethyl-2'-fluoro[1,1'-biphenyl]-3-yl)-3,6-dihydro-2,2,6,6-tetramethyl-2H-pyran-3-one (CAS 1312340-82-1); 5-(Acetyloxy)-4-(2',4'-dichloro-4-ethyl[1,1'-biphenyl]-3-yl)-3,6-dihydro-2,2,6,6-tetramethyl-2H-pyran-3-one (CAS 1033760-55-2); 4-(4'-Chloro-4-cyclopropyl-2'-fluoro[1,1'-biphenyl]-3-yl)-5,6-dihydro-2,2,6,6-tetramethyl-5-oxo-2H-pyran-3-yl carbonic acid methyl ester (CAS 1312337-51-1); 4-(2',4'-Dichloro -4-cyclopropyl- [1,1'-biphenyl]-3-yl)-5,6-dihydro-2,2,6,6-tetramethyl-5-oxo-2H-pyran-3-yl carbonic acid methyl ester; 4-(4'-Chloro-4-ethyl-2'-fluoro[1,1'-biphenyl]-3-yl)-5,6-dihydro-2,2,6,6-tetramethyl-5-oxo-2H-pyran-3-yl carbonic acid methyl ester (CAS 1312340-83-2); 4-(2',4'-Dichloro-4-ethyl[1,1'-biphenyl]-3-yl)-5,6-dihydro-2,2,6,6-tetramethyl-5-oxo-2H-pyran-3-yl carbonic acid methyl ester (CAS 1033760-58-5); and non ACC herbicides such as benfuresate, butylate, cycloate, dalapon, dimepiperate, EPTC, esprocarb, ethofumesate, flupropanate, molinate, orbencarb, pebulate, prosulfocarb, TCA, thiobencarb, tio-carbazil, triallate and vernolate;
b2) from the group of the ALS inhibitors:
   sulfonylureas such as amidosulfuron, azimsulfuron, bensulfuron, bensulfuron-methyl, chlorimuron, chlorimuron-ethyl, chlorsulfuron, cinosulfuron, cyclosulfamuron, ethametsulfuron, ethametsulfuron-methyl, ethoxysulfuron, flazasulfuron, flucetosulfuron, flupyrsulfuron, flupyrsulfuron-methyl-sodium, foramsulfuron, halosulfuron, halosulfuron-methyl, imazosulfuron, iodosulfuron, iodosulfuron-methyl-sodium, iofensulfuron, iofensulfuron-sodium, mesosulfuron, metazosulfuron, metsulfuron, metsulfuron-methyl, nicosulfuron, orthosulfamuron, oxasulfuron, primisulfuron, primisulfuron-methyl, propyrisulfuron, prosulfuron, pyrazosulfuron, pyrazosulfuron-ethyl, rimsulfuron, sulfometuron, sulfometuron-methyl, sulfosulfuron, thifensulfuron, thifensulfuron-methyl, triasulfuron, tribenuron, tribenuron-methyl, trifloxysulfuron, triflusulfuron, triflusulfuron-methyl and tritosulfuron,
   imidazolinones such as imazamethabenz, imazamethabenz-methyl, imazamox, imazapic, imazapyr, imazaquin and imazethapyr, triazolopyrimidine herbicides and sulfonanilides such as cloransulam, cloransulam-methyl, diclosulam, flumetsulam, florasulam, metosulam, penoxsulam, pyrimisulfan and pyroxsulam,
   pyrimidinylbenzoates such as bispyribac, bispyribac-sodium, pyribenzoxim, pyriftalid, pyrimino-bac, pyriminobac-methyl, pyrithiobac, pyrithiobac-sodium, 4-[[[2-[(4,6-dimethoxy-2-pyrimidinyl)oxy]phenyl]methyl]amino]-benzoic acid-1-methylethyl ester (CAS 420138-41-6), 4-[[[2-[(4,6-dimethoxy-2-pyrimidinyl)oxy]phenyl]methyl]amino]-benzoic acid propyl ester (CAS 420138-40-5), N-(4-bromophenyl)-2-[(4,6-dimethoxy-2-pyrimidinyl)oxy]benzenemethanamine (CAS 420138-01-8),
   sulfonylaminocarbonyl-triazolinone herbicides such as flucarbazone, flucarbazone-sodium, propoxycarbazone, propoxycarbazone-sodium, thiencarbazone and thiencarbazone-methyl; and triafamone;
   among these, a preferred embodiment of the invention relates to those compositions comprising at least one imidazolinone herbicide;
b3) from the group of the photosynthesis inhibitors:
   amicarbazone, inhibitors of the photosystem II, e.g. triazine herbicides, including of chlorotriazine, triazinones, triazindiones, methylthiotriazines and pyridazinones such as ametryn, atrazine, chloridazone, cyanazine, desmetryn, dimethametryn,hexazinone, metribuzin, prometon, prometryn, propazine, simazine, simetryn, terbumeton, terbuthylazin, terbutryn and trietazin, aryl urea such as chlorobromuron, chlorotoluron, chloroxuron, dimefuron, diuron, fluometuron, isoproturon, isouron, linuron, metamitron, methabenzthiazuron, metobenzuron, metoxuron, monolinuron, neburon, siduron, tebuthiuron and thiadiazuron, phenyl carbamates such as desmedipham, karbutilat, phenmedipham, phenmedipham-ethyl, nitrile herbicides such as bromofenoxim, bromoxynil and its salts and esters, ioxynil and its salts and esters, uraciles such as bromacil, lenacil and terbacil, and bentazon and bentazon-sodium, pyridate, pyridafol, pentanochlor and propanil and inhibitors of the photosystem I such as diquat, diquat-dibromide, paraquat, paraquat-dichloride and paraquat-dimetilsulfate. Among these, a preferred embodiment of the invention relates to those compositions comprising at least one aryl urea herbicide. Among these, likewise a preferred embodiment of the invention relates to those compositions comprising at least one triazine herbicide. Among these, likewise a preferred embodiment of the invention relates to those compositions comprising at least one nitrile herbicide;
b4) from the group of the protoporphyrinogen-IX oxidase inhibitors:
   acifluorfen, acifluorfen-sodium, azafenidin, bencarbazone, benzfendizone, bifenox, butafenacil, carfentrazone, carfentrazone-ethyl, chlomethoxyfen, cinidon-ethyl, fluazolate, flufenpyr, flufenpyr-ethyl, flumiclorac, flumiclorac-pentyl, flumioxazin, fluoroglycofen, fluoroglycofen-ethyl, fluthiacet, fluthiacet-methyl, fomesafen, halosafen, lactofen, oxadiargyl, oxadiazon, oxyfluorfen, pentoxazone, profluazol, pyraclonil, pyraflufen, pyraflufen-ethyl, saflufenacil, sulfentrazone, thidiazimin, tiafenacil, trifludimoxazin, ethyl [3-[2-chloro-4-fluoro-5-(1-methyl-6-trifluoromethyl-2,4-dioxo-1,2,3,4-tetrahydropyrimidin-3-yl)phenoxy]-2-pyridyloxy]acetate (CAS 353292-31-6; S-3100), N-ethyl-3-(2,6-dichloro-4-trifluoromethylphenoxy)-5-methyl-1H-pyrazole-1-carboxamide (CAS 452098-92-9), N-tetrahydrofurfuryl-3-(2,6-dichloro-4-trifluoromethylphenoxy)-5-methyl-1H-pyrazole-1-carboxamide (CAS 915396-43-9), N-ethyl-3-(2-chloro-6-fluoro-4-trifluoromethylphenoxy)-5-methyl-1 H-pyrazole-1-carboxamide (CAS 452099-05-7),N-tetrahydrofurfuryl-3-(2-chloro-6-fluoro-4-trifluoromethylphenoxy)-5-methyl-1 H-pyrazole-1-carboxamide (CAS 452100-03-7), 3-[7-fluoro-3-oxo-4-(prop-2-ynyl)-3,4-dihydro-2H-benzo[1,4]oxazin-6-yl]-1,5-dimethyl-6-thioxo-[1,3,5]triazinan-2,4-dione (CAS 451484-50-7) , 2-(2,2,7-trifluoro-3-oxo-4-prop-2-ynyl-3,4-dihydro-2H-benzo[1,4]oxazin-6-yl)-4,5,6,7-tetrahydro-isoindole-1,3-dione (CAS 1300118-96-0), 1-methyl-6-trifluoromethyl-3-(2,2,7-trifluoro-3-oxo-4-prop-2-ynyl-3,4-dihydro-2H-benzo[1,4]oxazin-6-yl)-1 H-pyrimidine-2,4-dione (CAS 1304113-05-0), methyl (E)-4-[2-chloro-5-[4-chloro-5-(difluoromethoxy)-1H-methyl-pyrazol-3-yl]-4-fluoro-phenoxy]-3-methoxy-but-2-enoate (CAS 948893-00-3), and 3-[7-chloro-5-fluoro-2-(trifluoromethyl)-1H-benzimidazol-4-yl]-1-methyl-6-(trifluoromethyl)-1 H-pyrimidine-2,4-dione (CAS 212754-02-4) ;
b5) from the group of the bleacher herbicides:
   PDS inhibitors: beflubutamid, diflufenican, fluridone, flurochloridone, flurtamone, norflurazon, picolinafen, and 4-(3-trifluoromethylphenoxy)-2-(4-trifluoromethylphenyl)pyrimidine (CAS 180608-33-7), HPPD inhibitors: benzobicyclon, benzofenap, bicyclopyrone, clomazone, fenquinotrione, isoxaflutole, mesotrione, oxotrione (CAS 1486617-21-3), pyrasulfotole, pyrazolynate, pyrazoxyfen, sulcotrione, tefuryltrione, tembotrione, tolpyralate, topramezone , bleacher, unknown target: aclonifen, amitrole flumeturon and 2-chloro-3-methylsulfanyl-N-(1-methyltetrazol-5-yl)-4-(trifluoromethyl)benzamide (CAS 1361139-71-0);
b6) from the group of the EPSP synthase inhibitors:
   glyphosate, glyphosate-isopropylammonium, glyposate-potassium and glyphosate-trimesium (sulfosate);
b7) from the group of the glutamine synthase inhibitors:
   bilanaphos (bialaphos), bilanaphos-sodium, glufosinate, glufosinate-P and glufosinate-ammonium;
b8) from the group of the DHP synthase inhibitors:
   asulam;
b9) from the group of the mitosis inhibitors:
   compounds of group K1: dinitroanilines such as benfluralin, butralin, dinitramine, ethalfluralin, fluchloralin, oryzalin, pendimethalin, prodiamine and trifluralin, phosphoramidates such as amiprophos, amiprophos-methyl, and butamiphos, benzoic acid herbicides such as chlorthal, chlorthal-dimethyl, pyridines such as dithiopyr and thiazopyr, benzamides such as propyzamide and tebutam; compounds of group K2: carbetamide, chlorpropham, flamprop, flamprop-isopropyl, flamprop-methyl, flamprop-M-isopropyl, flamprop-M-methyl and propham ; among these, compounds of group K1, in particular dinitroanilines are preferred;
b10) from the group of the VLCFA inhibitors:
   chloroacetamides such as acetochlor, alachlor, amidochlor, butachlor, dimethachlor, dimethenamid, dimethenamid-P, metazachlor, metolachlor, metolachlor-S, pethoxamid, pretilachlor, propachlor, propisochlor and thenylchlor, oxyacetanilides such as flufenacet and mefenacet, acetanilides such as diphenamid, naproanilide, napropamide and napropamide-M, tetrazolinones such fentrazamide, and other herbicides such as anilofos, cafenstrole, fenoxasulfone, ipfencarbazone, piperophos, pyroxasulfone and isoxazoline compounds of the formulae II.1, II.2, II.3, II.4, II.5, II.6, II.7, II.8 and II.9 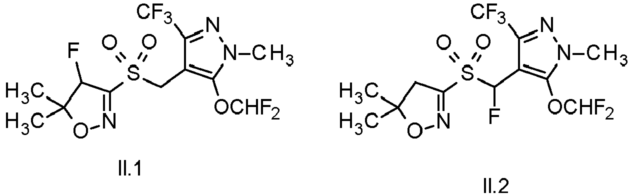 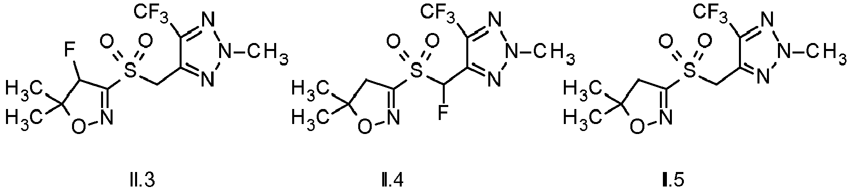 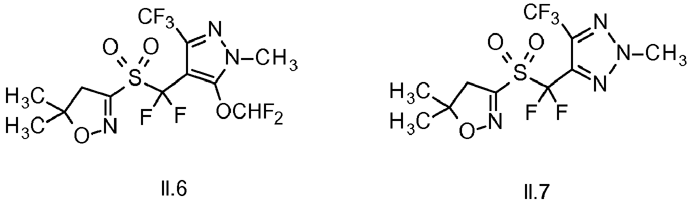 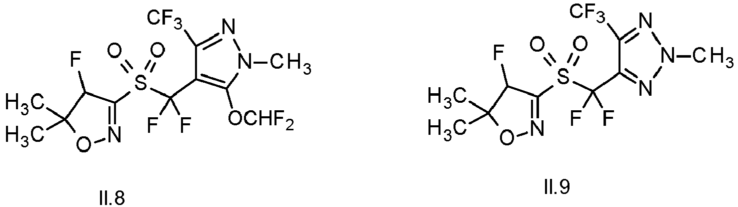
   the isoxazoline compounds of the formula (II) are known in the art, e.g. from WO 2006/024820, WO 2006/037945, WO 2007/071900 and WO 2007/096576;
   among the VLCFA inhibitors, preference is given to chloroacetamides and oxyacetamides;
b11) from the group of the cellulose biosynthesis inhibitors:
   chlorthiamid, dichlobenil, flupoxam, indaziflam, isoxaben, triaziflam and 1-cyclohexyl-5-pentafluorphenyloxy-1⁴-[1,2,4,6]thiatriazin-3-ylamine (CAS 175899-01-1);
b12) from the group of the decoupler herbicides:
   dinoseb, dinoterb and DNOC and its salts;
b13) from the group of the auxinic herbicides:
   2,4-D and its salts and esters such as clacyfos, 2,4-DB and its salts and esters, aminocyclopyrachlor and its salts and esters, aminopyralid and its salts such as aminopyralid-dimethylammonium, aminopyralid-tris(2-hydroxypropyl)ammonium and its esters, benazolin, benazolin-ethyl, chloramben and its salts and esters, clomeprop, clopyralid and its salts and esters, dicamba and its salts and esters, dichlorprop and its salts and esters, dichlorprop-P and its salts and esters, flopyrauxifen, fluroxypyr, fluroxypyr-butometyl, fluroxypyr-meptyl, halauxifen and its salts and esters (CAS 943832-60-8); MCPA and its salts and esters, MCPA-thioethyl, MCPB and its salts and esters, mecoprop and its salts and esters, mecoprop-P and its salts and esters, picloram and its salts and esters, quinclorac, quinmerac, TBA (2,3,6) and its salts and esters, triclopyr and its salts and esters, florpyrauxifen, florpyrauxifen-benzyl (CAS 1390661-72-9) and 4-amino-3-chloro-5-fluoro-6-(7-fluoro-1 H-indol-6-yl)picolinic acid (CAS 1629965-65-6) ;
b14) from the group of the auxin transport inhibitors: diflufenzopyr, diflufenzopyr-sodium, naptalam and naptalam-sodium;
b15) from the group of the other herbicides: bromobutide, chlorflurenol, chlorflurenol-methyl, cinmethylin, cumyluron, cyclopyrimorate (CAS 499223-49-3) and its salts and esters, dalapon, dazomet, difenzoquat, difenzoquat-metilsulfate, dimethipin, DSMA, dymron, endothal and its salts, etobenzanid, flurenol, flurenol-butyl, flurprimidol, fosamine, fosamine-ammonium, indanofan, maleic hydrazide, mefluidide, metam, methiozolin (CAS 403640-27-7), methyl azide, methyl bromide, methyl-dymron, methyl iodide, MSMA, oleic acid, oxaziclomefone, pelargonic acid, pyributicarb, quinoclamine and tridiphane.

Preferred herbicides B that can be used in combination with the pyridine compounds of the formula (I) according to the present invention are:
b1) from the group of the lipid biosynthesis inhibitors:
   clethodim, clodinafop-propargyl, cycloxydim, cyhalofop-butyl, diclofop-methyl, fenoxaprop-P-ethyl, fluazifop-P-butyl, haloxyfop-P-methyl, metamifop, pinoxaden, profoxydim, propaquizafop, quizalofop-P-ethyl, quizalofop-P-tefuryl, sethoxydim, tepraloxydim, tralkoxydim, 4-(4'-Chloro-4-cyclopropyl-2'-fluoro[1,1'-biphenyl]-3-yl)-5-hydroxy-2,2,6,6-tetramethyl-2H-pyran-3(6H)-one (CAS 1312337-72-6); 4-(2',4'-Dichloro-4-cyclopropyl[1,1'-biphenyl]-3-yl)-5-hydroxy-2,2,6,6-tetramethyl-2H-pyran-3(6H)-one (CAS 1312337-45-3); 4-(4'-Chloro-4-ethyl-2'-fluoro[1,1'-biphenyl]-3-yl)-5-hydroxy-2,2,6,6-tetramethyl-2H-pyran-3(6H)-one (CAS 1033757-93-5); 4-(2',4'-Dichloro-4-ethyl[1,1'-biphenyl]-3-yl)-2,2,6,6-tetramethyl-2H-pyran-3,5(4H,6H)-dione (CAS 1312340-84-3);
   5-(Acetyloxy)-4-(4'-chloro-4-cyclopropyl-2'-fluoro[1,1'-biphenyl]-3-yl)-3,6-dihydro-2,2,6,6-tetramethyl-2H-pyran-3-one (CAS 1312337-48-6); 5-(Acetyloxy)-4-(2',4'-dichloro-4-cyclopropyl-[1,1'-biphenyl]-3-yl)-3,6-dihydro-2,2,6,6-tetramethyl-2H-pyran-3-one; 5-(Acetyloxy)-4-(4'-chloro-4-ethyl-2'-fluoro[1,1'-biphenyl]-3-yl)-3,6-dihydro-2,2,6,6-tetramethyl-2H-pyran-3-one (CAS 1312340-82-1); 5-(Acetyloxy)-4-(2',4'-dichloro-4-ethyl[1,1'-biphenyl]-3-yl)-3,6-dihydro-2,2,6,6-tetramethyl-2H-pyran-3-one (CAS 1033760-55-2); 4-(4'-Chloro-4-cyclopropyl-2'-fluoro[1,1'-biphenyl]-3-yl)-5,6-dihydro-2,2,6,6-tetramethyl-5-oxo-2H-pyran-3-yl carbonic acid methyl ester (CAS 1312337-51-1); 4-(2',4'-Dichloro -4-cyclopropyl- [1,1'-biphenyl]-3-yl)-5,6-dihydro-2,2,6,6-tetramethyl-5-oxo-2H-pyran-3-yl carbonic acid methyl ester; 4-(4'-Chloro-4-ethyl-2'-fluoro[1,1'-biphenyl]-3-yl)-5,6-dihydro-2,2,6,6-tetramethyl-5-oxo-2H-pyran-3-yl carbonic acid methyl ester (CAS 1312340-83-2); 4-(2',4'-Dichloro-4-ethyl[1,1'-biphenyl]-3-yl)-5,6-dihydro-2,2,6,6-tetramethyl-5-oxo-2H-pyran-3-yl carbonic acid methyl ester (CAS 1033760-58-5); benfuresate, dimepiperate, EPTC, esprocarb, ethofumesate, molinate, orbencarb, prosulfocarb, thiobencarb and triallate;
b2) from the group of the ALS inhibitors:
   amidosulfuron, azimsulfuron, bensulfuron-methyl, bispyribac-sodium, chlorimuron-ethyl, chlorsulfuron, cloransulam-methyl, cyclosulfamuron, diclosulam, ethametsulfuron-methyl, ethoxysulfuron, flazasulfuron, florasulam, flucarbazone-sodium, flucetosulfuron, flumetsulam, flupyrsulfuron-methyl-sodium, foramsulfuron, halosulfuron-methyl, imazamethabenz-methyl, imazamox, imazapic, imazapyr, imazaquin, imazethapyr, imazosulfuron, iodosulfuron, iodosulfuron-methyl-sodium, iofensulfuron, iofensulfuron-sodium, mesosulfuron, metazosulfuron, metosulam, metsulfuron-methyl, nicosulfuron, orthosulfamuron, oxasulfuron, penoxsulam, primisulfuron-methyl, propoxycarbazon-sodium, propyrisulfuron, prosulfuron, pyrazosulfuron-ethyl, pyribenzoxim, pyrimisulfan, pyriftalid, pyriminobac-methyl, pyrithiobac-sodium, pyroxsulam, rimsulfuron, sulfometuron-methyl, sulfosulfuron, thiencarbazone-methyl, thifensulfuron-methyl, triasulfuron, tribenuron-methyl, trifloxysulfuron, triflusulfuron-methyl, tritosulfuron and triafamone;
b3) from the group of the photosynthesis inhibitors:
   ametryn, amicarbazone, atrazine, bentazone, bentazone-sodium, bromoxynil and its salts and esters, chloridazone, chlorotoluron, cyanazine, desmedipham, diquat-dibromide, diuron, fluometuron, hexazinone, ioxynil and its salts and esters, isoproturon, lenacil, linuron, metamitron, methabenzthiazuron, metribuzin, paraquat, paraquat-dichloride, phenmedipham, propanil, pyridate, simazine, terbutryn, terbuthylazine and thidiazuron;
b4) from the group of the protoporphyrinogen-IX oxidase inhibitors:
   acifluorfen-sodium, bencarbazone, benzfendizone, butafenacil, carfentrazone-ethyl, cinidon-ethyl, flufenpyr-ethyl, flumiclorac-pentyl, flumioxazin, fluoroglycofen-ethyl, fomesafen, lactofen, oxadiargyl, oxadiazon, oxyfluorfen, pentoxazone, pyraflufen, pyraflufen-ethyl, saflufenacil, sulfentrazone, tiafenacil, trifludimoxazin, ethyl [3-[2-chloro-4-fluoro-5-(1-methyl-6-trifluoromethyl-2,4-dioxo-1,2,3,4-tetrahydropyrimidin-3-yl)phenoxy]-2-pyridyloxy]acetate (CAS 353292-31-6; S-3100), N-ethyl-3-(2,6-dichloro-4-trifluoromethylphenoxy)-5-methyl-1 H-pyrazole-1-carboxamide (CAS 452098-92-9), N-tetrahydrofurfuryl-3-(2,6-dichloro-4-trifluoromethylphenoxy)-5-methyl-1H-pyrazole-1-carboxamide (CAS 915396-43-9), N-ethyl-3-(2-chloro-6-fluoro-4-trifluoromethylphenoxy)-5-methyl-1 H-pyrazole-1-carboxamide (CAS 452099-05-7), N-tetrahydrofurfuryl-3-(2-chloro-6-fluoro-4-trifluoromethylphenoxy)-5-methyl-1H-pyrazole-1-carboxamide (CAS 452100-03-7), 3-[7-fluoro-3-oxo-4-(prop-2-ynyl)-3,4-dihydro-2H-benzo[1,4]oxazin-6-yl]-1,5-dimethyl-6-thioxo-[1,3,5]triazinan-2,4-dione (CAS 451484-50-7) , 2-(2,2,7-trifluoro-3-oxo-4-prop-2-ynyl-3,4-dihydro-2H-benzo[1,4]oxazin-6-yl)-4,5,6,7-tetrahydro-isoindole-1,3-dione (CAS 1300118-96-0) ;1-methyl-6-trifluoromethyl-3-(2,2,7-trifluoro-3-oxo-4-prop-2-ynyl-3,4-dihydro-2H-benzo[1,4]oxazin-6-yl)-1H-pyrimidine-2,4-dione (CAS 1304113-05-0), and 3-[7-chloro-5-fluoro-2-(trifluoromethyl)-1 H-benzimidazol-4-yl]-1-methyl-6-(trifluoromethyl)-1H-pyrimidine-2,4-dione (CAS 212754-02-4) ;
b5) from the group of the bleacher herbicides:
   aclonifen, amitrole, beflubutamid, benzobicyclon, bicyclopyrone, clomazone, diflufenican, fenquinotrione, flumeturon, flurochloridone, flurtamone, isoxaflutole, mesotrione, oxotrione (CAS 1486617-21-3), norflurazon, picolinafen, pyrasulfotole, pyrazolynate, sulcotrione, tefuryltrione, tembotrione, tolpyralate, topramezone, 4-(3-trifluoromethylphenoxy)-2-(4-trifluoromethylphenyl)-pyrimidine (CAS 180608-33-7) and 2-chloro-3-methylsulfanyl-N-(1-methyltetrazol-5-yl)-4-(trifluoromethyl)benzamide (CAS 1361139-71-0);
b6) from the group of the EPSP synthase inhibitors:
   glyphosate, glyphosate-isopropylammonium, glyphosate-potassium and glyphosate-trimesium (sulfosate);
b7) from the group of the glutamine synthase inhibitors:
   glufosinate, glufosinate-P, glufosinate-ammonium;
b8) from the group of the DHP synthase inhibitors: asulam;
b9) from the group of the mitosis inhibitors:
   benfluralin, dithiopyr, ethalfluralin, flamprop, flamprop-isopropyl, flamprop-methyl, flamprop-M-isopropyl, flamprop-M-methyl, oryzalin, pendimethalin, thiazopyr and trifluralin;
b10) from the group of the VLCFA inhibitors:
   acetochlor, alachlor, amidochlor, anilofos, butachlor, cafenstrole, dimethenamid, dimethenamid-P, fentrazamide, flufenacet, mefenacet, metazachlor, metolachlor, S-metolachlor, naproanilide, napropamide, napropamide-M, pretilachlor, fenoxasulfone, ipfencarbazone, pyroxasulfone thenylchlor and isoxazoline-compounds of the formulae II.1, II.2, II.3, II.4, II.5, II.6, II.7, II.8 and II.9 as mentioned above;
b11) from the group of the cellulose biosynthesis inhibitors: dichlobenil, flupoxam, indaziflam, isoxaben, triaziflam and 1-cyclohexyl-5-pentafluorphenyloxy-1⁴-[1,2,4,6]thiatriazin-3-ylamine (CAS 175899-01-1);
b12) from the group of the decoupler herbicides:
   dinoseb, dinoterb and DNOC and its salts;
b13) from the group of the auxinic herbicides:
   2,4-D and its salts and esters, aminocyclopyrachlor and its salts and esters, aminopyralid and its salts such as aminopyralid-dimethylammonium, aminopyralid-tris(2-hydroxypropyl)ammonium and its esters, clopyralid and its salts and esters, dicamba and its salts and esters, dichlorprop-P and its salts and esters, flopyrauxifen, fluroxypyr-meptyl, halauxifen and its salts and esters (CAS 943832-60-8), MCPA and its salts and esters, MCPB and its salts and esters, mecoprop-P and its salts and esters, picloram and its salts and esters, quinclorac, quinmerac, triclopyr and its salts and esters, florpyrauxifen , florpyrauxifen-benzyl (CAS 1390661-72-9) and 4-amino-3-chloro-5-fluoro-6-(7-fluoro-1 H-indol-6-yl)picolinic acid (CAS 1629965-65-6) ;
b14) from the group of the auxin transport inhibitors: diflufenzopyr and diflufenzopyr-sodium;
b15) from the group of the other herbicides: bromobutide, cinmethylin, cumyluron, cyclopyrimorate (CAS 499223-49-3) and its salts and esters, dalapon, difenzoquat, difenzoquat-metilsulfate, DSMA, dymron (= daimuron), indanofan, metam, methylbromide, MSMA, oxaziclomefone, pyributicarb and tridiphane.

Particularly preferred herbicides B that can be used in combination with the pyridine compounds of the formula (I) according to the present invention are:
b1) from the group of the lipid biosynthesis inhibitors: clodinafop-propargyl, cycloxydim, cyhalofop-butyl, fenoxaprop-P-ethyl, pinoxaden, profoxydim, tepraloxydim, tralkoxydim, 4-(4'-Chloro-4-cyclopropyl-2'-fluoro[1,1'-biphenyl]-3-yl)-5-hydroxy-2,2,6,6-tetramethyl-2H-pyran-3(6H)-one (CAS 1312337-72-6); 4-(2',4'-Dichloro-4-cyclopropyl[1,1'-biphenyl]-3-yl)-5-hydroxy-2,2,6,6-tetramethyl-2H-pyran-3(6H)-one (CAS 1312337-45-3); 4-(4'-Chloro-4-ethyl-2'-fluoro[1,1'-biphenyl]-3-yl)-5-hydroxy-2,2,6,6-tetramethyl-2H-pyran-3(6H)-one (CAS 1033757-93-5); 4-(2',4'-Dichloro-4-ethyl[1,1'-biphenyl]-3-yl)-2,2,6,6-tetramethyl-2H-pyran-3,5(4H,6H)-dione (CAS 1312340-84-3); 5-(Acetyloxy)-4-(4'-chloro-4-cyclopropyl-2'-fluoro[1,1'-biphenyl]-3-yl)-3,6-dihydro-2,2,6,6-tetramethyl-2H-pyran-3-one (CAS 1312337-48-6); 5-(Acetyloxy)-4-(2',4'-dichloro-4-cyclopropyl- [1,1'-biphenyl]-3-yl)-3,6-dihydro-2,2,6,6-tetramethyl-2H-pyran-3-one; 5-(Acetyloxy)-4-(4'-chloro-4-ethyl-2'-fluoro[1,1'-biphenyl]-3-yl)-3,6-dihydro-2,2,6,6-tetramethyl-2H-pyran-3-one (CAS 1312340-82-1); 5-(Acetyloxy)-4-(2',4'-dichloro-4-ethyl[1,1'-biphenyl]-3-yl)-3,6-dihydro-2,2,6,6-tetramethyl-2H-pyran-3-one (CAS 1033760-55-2); 4-(4'-Chloro-4-cyclopropyl-2'-fluoro[1,1'-biphenyl]-3-yl)-5,6-dihydro-2,2,6,6-tetramethyl-5-oxo-2H-pyran-3-yl carbonic acid methyl ester (CAS 1312337-51-1); 4-(2',4'-Dichloro -4-cyclopropyl- [1,1'-biphenyl]-3-yl)-5,6-dihydro-2,2,6,6-tetramethyl-5-oxo-2H-pyran-3-yl carbonic acid methyl ester; 4-(4'-Chloro-4-ethyl-2'-fluoro[1,1'-biphenyl]-3-yl)-5,6-dihydro-2,2,6,6-tetramethyl-5-oxo-2H-pyran-3-yl carbonic acid methyl ester (CAS 1312340-83-2); 4-(2',4'-Dichloro-4-ethyl[1,1'-biphenyl]-3-yl)-5,6-dihydro-2,2,6,6-tetramethyl-5-oxo-2H-pyran-3-yl carbonic acid methyl ester (CAS 1033760-58-5); esprocarb, prosulfocarb, thiobencarb and triallate;
b2) from the group of the ALS inhibitors: bensulfuron-methyl, bispyribac-sodium, cyclosulfamuron, diclosulam, flumetsulam, flupyrsulfuron-methyl-sodium, foramsulfuron, imazamox, imazapic, imazapyr, imazaquin, imazethapyr, imazosulfuron, iodosulfuron, iodosulfuron-methyl-sodium, iofensulfuron, iofensulfuron-sodium, mesosulfuron, metazosulfuron, nicosulfuron, penoxsulam, propoxycarbazon-sodium, propyrisulfuron, pyrazosulfuron-ethyl, pyroxsulam, rimsulfuron, sulfosulfuron, thiencarbazon-methyl, tritosulfuron and triafamone;
b3) from the group of the photosynthesis inhibitors: ametryn, atrazine, diuron, fluometuron, hexazinone, isoproturon, linuron, metribuzin, paraquat, paraquat-dichloride, propanil, terbutryn and terbuthylazine;
b4) from the group of the protoporphyrinogen-IX oxidase inhibitors: flumioxazin, oxyfluorfen, pyraflufen, pyraflufen-ethyl, saflufenacil, sulfentrazone, trifludimoxazin , ethyl [3-[2-chloro-4-fluoro-5-(1-methyl-6-trifluoromethyl-2,4-dioxo-1,2,3,4-tetrahydropyrimidin-3-yl)phenoxy]-2-pyridyloxy]acetate (CAS 353292-31-6; S-3100), 3-[7-fluoro-3-oxo-4-(prop-2-ynyl)-3,4-dihydro-2H-benzo[1,4]oxazin-6-yl]-1,5-dimethyl-6-thioxo-[1,3,5]triazinan-2,4-dione (CAS 451484-50-7), 2-(2,2,7-trifluoro-3-oxo-4-prop-2-ynyl-3,4-dihydro-2H-benzo[1,4]oxazin-6-yl)-4,5,6,7-tetrahydroisoindole-1,3-dione (CAS 1300118-96-0), and 1-methyl-6-trifluoromethyl-3-(2,2,7-trifluoro-3-oxo-4-prop-2-ynyl-3,4-dihydro-2H-benzo[1,4]oxazin-6-yl)-1H-pyrimidine-2,4-dione (CAS 1304113-05-0);
b5) from the group of the bleacher herbicides: amitrole, bicyclopyrone, clomazone, diflufenican, fenquinotrione, flumeturon, flurochloridone, isoxaflutole, mesotrione, oxotrione (CAS 1486617-21-3), picolinafen, sulcotrione, tefuryltrione, tembotrione, tolpyralate, topramezone and 2-chloro-3-methylsulfanyl-N-(1-methyltetrazol-5-yl)-4-(trifluoromethyl)benzamide (CAS 1361139-71-0) ;
b6) from the group of the EPSP synthase inhibitors: glyphosate, glyphosate-isopropylammonium and glyphosate-trimesium (sulfosate);
b7) from the group of the glutamine synthase inhibitors: glufosinate, glufosinate-P and glufosinate-ammonium;
b9) from the group of the mitosis inhibitors: pendimethalin and trifluralin;
b10) from the group of the VLCFA inhibitors: acetochlor, cafenstrole, dimethenamid-P, fentrazamide, flufenacet, mefenacet, metazachlor, metolachlor, S-metolachlor, fenoxasulfone, ipfencarbazone and pyroxasulfone; likewise, preference is given to isoxazoline compounds of the formulae II.1, II.2, II.3, II.4, II.5, II.6, II.7, II.8 and II.9 as mentioned above;
b11) from the group of the cellulose biosynthesis inhibitors: indaziflam, isoxaben and triaziflam;
b13) from the group of the auxinic herbicides: 2,4-D and its salts and esters such as clacyfos, and aminocyclopyrachlor and its salts and esters, aminopyralid and its salts and its esters, clopyralid and its salts and esters, dicamba and its salts and esters, flopyrauxifen, fluroxypyr-meptyl, halauxifen, halauxifen-methyl, quinclorac, quinmerac, florpyrauxifen , florpyrauxifen-benzyl (CAS 1390661-72-9) and 4-amino-3-chloro-5-fluoro-6-(7-fluoro-1 H-indol-6-yl)picolinic acid (CAS 1629965-65-6);
b14) from the group of the auxin transport inhibitors: diflufenzopyr and diflufenzopyr-sodium;
b15) from the group of the other herbicides: cinmethylin, dymon (= daimuron), indanofan, oxaziclomefone.

Particularly preferred herbicides B are the herbicides B as defined above; in particular the herbicides B.1 - B.201 listed below in table B:

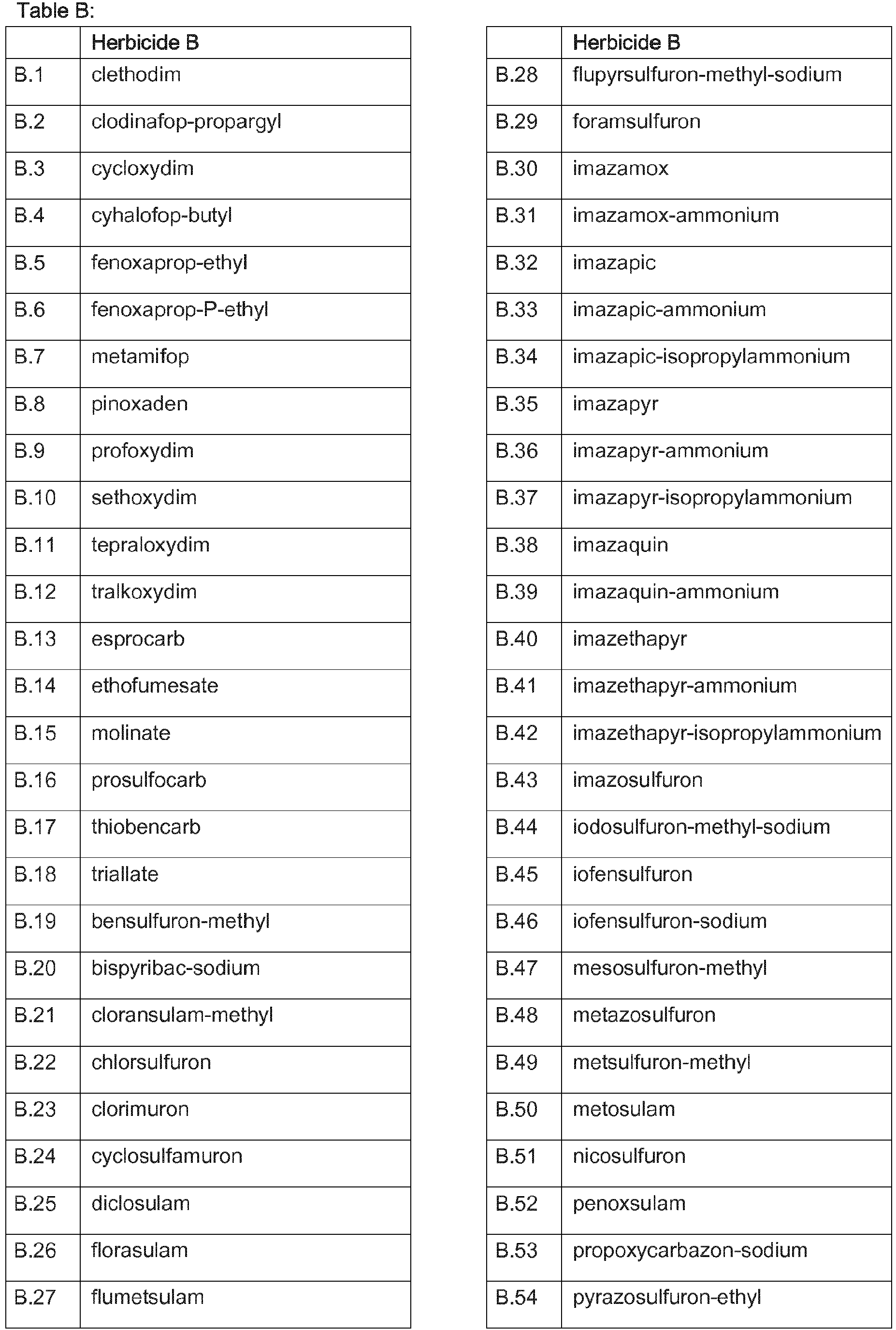

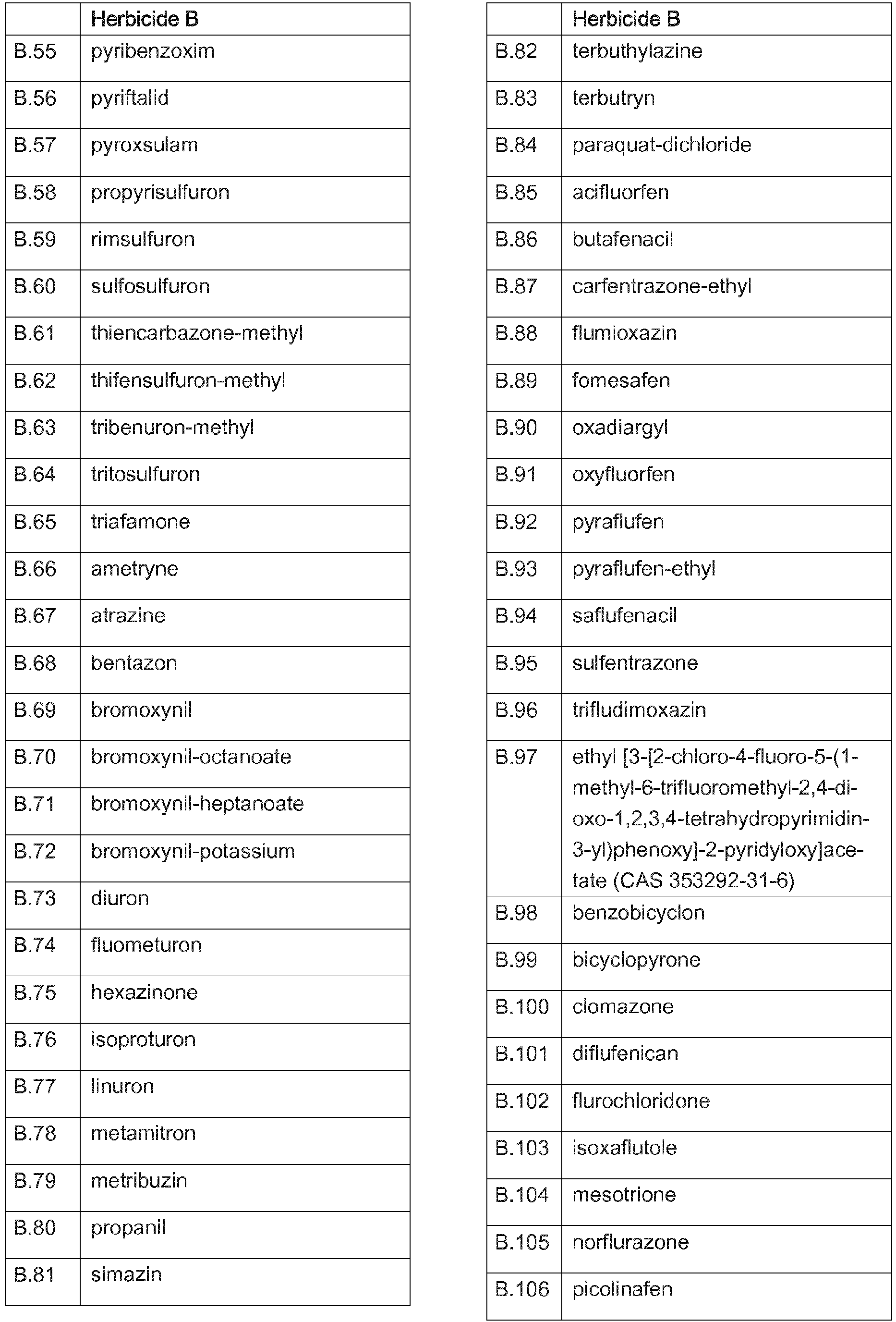

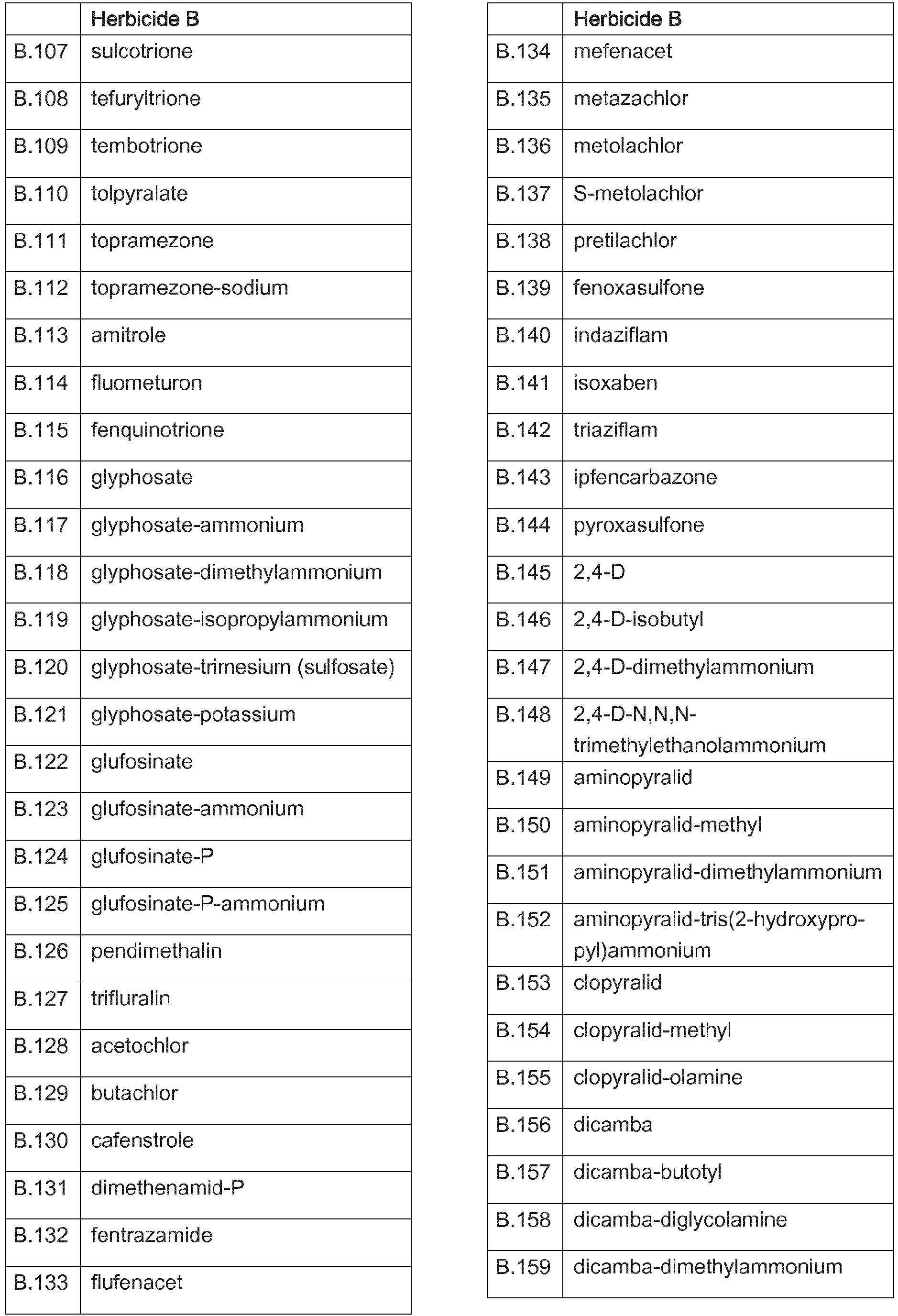

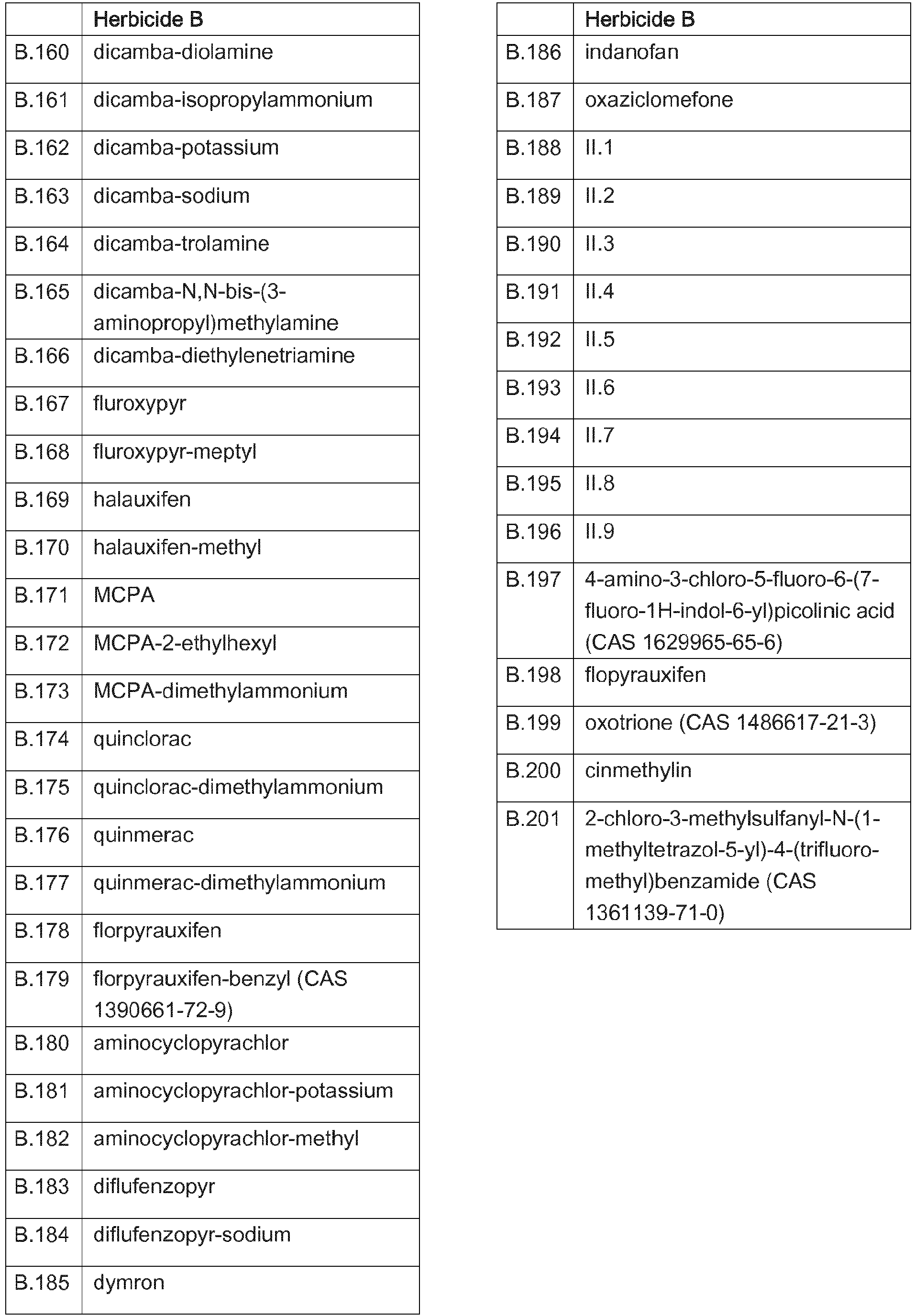

In another embodiment of the present invention the compositions according to the present invention comprise at least one pyridine compound of formula (I) and at least one safener C.

Safeners are chemical compounds which prevent or reduce damage on useful plants without having a major impact on the herbicidal action of the herbicidal active components of the present compositions towards unwanted plants. They can be applied either before sowings (e.g. on seed treatments, shoots or seedlings) or in the pre-emergence application or post-emergence application of the useful plant. The safeners and the pyridine compounds of formula (I) and/or the herbicides B can be applied simultaneously or in succession.

Suitable safeners are e.g. (quinolin-8-oxy)acetic acids, 1-phenyl-5-haloalkyl-1H-1,2,4-triazol-3-carboxylic acids, 1-phenyl-4,5-dihydro-5-alkyl-1 H-pyrazol-3,5-dicarboxylic acids, 4,5-dihydro-5,5-diaryl-3-isoxazol carboxylic acids, dichloroacetamides, alpha-oximinophenylacetonitriles, acetophenonoximes, 4,6-dihalo-2-phenylpyrimidines, N-[[4-(aminocarbonyl)phenyl]sulfonyl]-2-benzoic amides, 1,8-naphthalic anhydride, 2-halo-4-(haloalkyl)-5-thiazol carboxylic acids, phosphorthiolates and N-alkyl-O-phenylcarbamates and their agriculturally acceptable salts and their agriculturally acceptable derivatives such amides, esters, and thioesters, provided they have an acid group.

Examples of preferred safeners C are benoxacor, cloquintocet, cyometrinil, cyprosulfamide, dichlormid, dicyclonon, dietholate, fenchlorazole, fenclorim, flurazole, fluxofenim, furilazole, isoxadifen, mefenpyr, mephenate, naphthalic anhydride, oxabetrinil, 4-(dichloroacetyl)-1-oxa-4-azaspiro[4.5]decane (MON4660, CAS 71526-07-3), 2,2,5-trimethyl-3-(dichloroacetyl)-1,3-oxazolidine (R-29148, CAS 52836-31-4) and N-(2-Methoxybenzoyl)-4-[(methylaminocarbonyl)amino]benzenesulfonamide (CAS 129531-12-0).

Especially preferred safeners C are benoxacor, cloquintocet, cyprosulfamide, dichlormid, fenchlorazole, fenclorim, flurazole, fluxofenim, furilazole, isoxadifen, mefenpyr, naphthalic anhydride, oxabetrinil, 4-(dichloroacetyl)-1-oxa-4-azaspiro[4.5]decane (MON4660, CAS 71526-07-3), 2,2,5-trimethyl-3-(dichloroacetyl)-1,3-oxazolidine (R-29148, CAS 52836-31-4) and N-(2-Methoxybenzoyl)-4-[(methylaminocarbonyl)amino]benzenesulfonamide (CAS 129531-12-0).

Particularly preferred safeners C are benoxacor, cloquintocet, cyprosulfamide, dichlormid, fenchlorazole, fenclorim, furilazole, isoxadifen, mefenpyr, naphtalic anhydride, 4-(dichloroacetyl)-1-oxa-4-azaspiro[4.5]decane (MON4660, CAS 71526-07-3), 2,2,5-trimethyl-3-(dichloroacetyl)-1,3-oxazolidine (R-29148, CAS 52836-31-4) and N-(2-Methoxybenzoyl)-4-[(methylaminocarbonyl)amino]benzenesulfonamide (CAS 129531-12-0).

Particularly preferred safeners C, which, as component C, are constituent of the composition according to the invention are the safeners C as defined above; in particular the safeners C.1 - C.17 listed below in table C:

**Table C**

| | Safener C |
|---|---|
| C.1 | benoxacor |
| C.2 | cloquintocet |
| C.3 | cloquintocet-mexyl |
| C.4 | cyprosulfamide |
| C.5 | dichlormid |
| C.6 | fenchlorazole |
| C.7 | fenchlorazole-ethyl |
| C.8 | fenclorim |
| C.9 | furilazole |
| C.10 | isoxadifen |
| C.11 | isoxad ifen-ethyl |
| C.12 | mefenpyr |
| C.13 | mefenpyr-diethyl |
| C.14 | naphtalic acid anhydride |
| C.15 | 4-(dichloroacetyl)-1-oxa-4-azaspiro[4.5]decane (MON4660, CAS 71526-07-3) |
| C.16 | 2,2,5-trimethyl-3-(dichloroacetyl)-1,3-oxazolidine (R-29148, CAS 52836-31-4) |
| C.17 | N-(2-Methoxybenzoyl)-4-[(methylaminocarbonyl)amino]benzenesulfonamide (CAS 129531-12-0) |

The active compounds B of groups b1) to b15) and the active compounds C are known herbicides and safeners, see, for example, The Compendium of Pesticide Common Names (http://www.alanwood.net/pesticides/); Farm Chemicals Handbook 2000 volume 86, Meister Publishing Company, 2000; B. Hock, C. Fedtke, R. R. Schmidt, Herbizide [Herbicides], Georg Thieme Verlag, Stuttgart 1995; W. H. Ahrens, Herbicide Handbook, 7th edition, Weed Science Society of America, 1994; and K. K. Hatzios, Herbicide Handbook, Supplement for the 7th edition, Weed Science Society of America, 1998. 2,2,5-Trimethyl-3-(dichloroacetyl)-1,3-oxazolidine [CAS No. 52836-31-4] is also referred to as R-29148. 4-(Dichloroacetyl)-1-oxa-4-azaspiro[4.5]decane [CAS No. 71526-07-3] is also referred to as AD-67 and MON 4660.

The assignment of the active compounds to the respective mechanisms of action is based on current knowledge. If several mechanisms of action apply to one active compound, this substance was only assigned to one mechanism of action.

Active compounds B and C having a carboxyl group can be employed in the form of the acid, in the form of an agriculturally suitable salt as mentioned above or else in the form of an agriculturally acceptable derivative in the compositions according to the invention.

In the case of dicamba, suitable salts include those, where the counterion is an agriculturally acceptable cation. For example, suitable salts of dicamba are dicamba-sodium, dicamba-potassium, dicamba-methylammonium, dicamba-dimethylammonium, dicamba-isopropylammonium, dicamba-diglycolamine, dicamba-olamine, dicamba-diolamine, dicamba-trolamine, dicamba-N,N-bis-(3-aminopropyl)methylamine and dicamba-diethylenetriamine. Examples of a suitable ester are dicamba-methyl and dicamba-butotyl.

Suitable salts of 2,4-D are 2,4-D-ammonium, 2,4-D-dimethylammonium, 2,4-D-diethylammonium, 2,4-D-diethanolammonium (2,4-D-diolamine), 2,4-D-triethanolammonium, 2,4-D-isopropylammonium, 2,4-D-triisopropanolammonium, 2,4-D-heptylammonium, 2,4-D-dodecylammonium, 2,4-D-tetradecylammonium, 2,4-D-triethylammonium, 2,4-D-tris(2-hydroxypropyl)ammonium, 2,4-D-tris(isopropyl)ammonium, 2,4-D-trolamine, 2,4-D-lithium, 2,4-D-sodium. Examples of suitable esters of 2,4-D are 2,4-D-butotyl, 2,4-D-2-butoxypropyl, 2,4-D-3-butoxypropyl, 2,4-D-butyl, 2,4-D-ethyl, 2,4-D-ethylhexyl, 2,4-D-isobutyl, 2,4-D-isooctyl, 2,4-D-isopropyl, 2,4-D-meptyl, 2,4-D-methyl, 2,4-D-octyl, 2,4-D-pentyl, 2,4-D-propyl, 2,4-D-tefuryl and clacyfos.

Suitable salts of 2,4-DB are for example 2,4-DB-sodium, 2,4-DB-potassium and 2,4-DB-dimethylammonium. Suitable esters of 2,4-DB are for example 2,4-DB-butyl and 2,4-DB-isoctyl.

Suitable salts of dichlorprop are for example dichlorprop-sodium, dichlorprop-potassium and dichlorprop-dimethylammonium. Examples of suitable esters of dichlorprop are dichlorprop-butotyl and dichlorprop-isoctyl.

Suitable salts and esters of MCPA include MCPA-butotyl, MCPA-butyl, MCPA-dimethylammonium, MCPA-diolamine, MCPA-ethyl, MCPA-thioethyl, MCPA-2-ethylhexyl, MCPA-isobutyl, MCPA-isoctyl, MCPA-isopropyl, MCPA-isopropylammonium, MCPA-methyl, MCPA-olamine, MCPA-potassium, MCPA-sodium and MCPA-trolamine.

A suitable salt of MCPB is MCPB sodium. A suitable ester of MCPB is MCPB-ethyl.

Suitable salts of clopyralid are clopyralid-potassium, clopyralid-olamine and clopyralid-tris-(2-hydroxypropyl)ammonium. Example of suitable esters of clopyralid is clopyralid-methyl.

Examples of a suitable ester of fluroxypyr are fluroxypyr-meptyl and fluroxypyr-2-butoxy-1-methylethyl, wherein fluroxypyr-meptyl is preferred.

Suitable salts of picloram are picloram-dimethylammonium, picloram-potassium, picloramtriisopropanolammonium, picloram-triisopropylammonium and picloram-trolamine. A suitable ester of picloram is picloram-isoctyl.

A suitable salt of triclopyr is triclopyr-triethylammonium. Suitable esters of triclopyr are for example triclopyr-ethyl and triclopyr-butotyl.

Suitable salts and esters of chloramben include chloramben-ammonium, chloramben-diolamine, chloramben-methyl, chloramben-methylammonium and chloramben-sodium. Suitable salts and esters of 2,3,6-TBA include 2,3,6-TBA-dimethylammonium, 2,3,6-TBA-lithium, 2,3,6-TBA-potassium and 2,3,6-TBA-sodium.

Suitable salts and esters of aminopyralid include aminopyralid-potassium, aminopyralid-dimethylammonium, and aminopyralid-tris(2-hydroxypropyl)ammonium.

Suitable salts of glyphosate are for example glyphosate-ammonium, glyphosate-diammonium, glyphoste-dimethylammonium, glyphosate-isopropylammonium, glyphosate-potassium, glyphosate-sodium, glyphosate-trimesium as well as the ethanolamine and diethanolamine salts, preferably glyphosate-diammonium, glyphosate-isopropylammonium and glyphosate-trimesium (sulfosate).

A suitable salt of glufosinate is for example glufosinate-ammonium.

A suitable salt of glufosinate-P is for example glufosinate-P-ammonium.

Suitable salts and esters of bromoxynil are for example bromoxynil-butyrate, bromoxynil-heptanoate, bromoxynil-octanoate, bromoxynil-potassium and bromoxynil-sodium.

Suitable salts and esters of ioxonil are for example ioxonil-octanoate, ioxonil-potassium and ioxonil-sodium.

Suitable salts and esters of mecoprop include mecoprop-butotyl, mecoprop-dimethylammonium, mecoprop-diolamine, mecoprop-ethadyl, mecoprop-2-ethylhexyl, mecoprop-isoctyl, mecoprop-methyl, mecoprop-potassium, mecoprop-sodium and mecoprop-trolamine.

Suitable salts of mecoprop-P are for example mecoprop-P-butotyl, mecoprop-P-dimethylammonium, mecoprop-P-2-ethylhexyl, mecoprop-P-isobutyl, mecoprop-P-potassium and mecoprop-P-sodium.

A suitable salt of diflufenzopyr is for example diflufenzopyr-sodium.

A suitable salt of naptalam is for example naptalam-sodium.

Suitable salts and esters of aminocyclopyrachlor are for example aminocyclopyrachlor-dimethylammonium, aminocyclopyrachlor-methyl, aminocyclopyrachlor-triisopropanolammonium, aminocyclopyrachlor-sodium and aminocyclopyrachlor-potassium.

A suitable salt of quinclorac is for example quinclorac-dimethylammonium.

A suitable salt of quinmerac is for example quinmerac-dimethylammonium.

A suitable salt of imazamox is for example imazamox-ammonium.

Suitable salts of imazapic are for example imazapic-ammonium and imazapic-isopropylammonium.

Suitable salts of imazapyr are for example imazapyr-ammonium and imazapyr-isopropylammonium.

A suitable salt of imazaquin is for example imazaquin-ammonium.

Suitable salts of imazethapyr are for example imazethapyr-ammonium and imazethapyr-isopropylammonium.

A suitable salt of topramezone is for example topramezone-sodium.

According to a preferred embodiment of the invention, the composition comprises as herbicidal active compound B or component B at least one, preferably exactly one herbicide B.

According to another preferred embodiment of the invention, the composition comprises as herbicidal active compounds B or component B at least two, preferably exactly two herbicides B different from each other.

According to another preferred embodiment of the invention, the composition comprises as herbicidal active compounds B or component B at least three, preferably exactly three herbicides B different from each other.

According to another preferred embodiment of the invention, the composition comprises as safening component C or component C at least one, preferably exactly one safener C.

According to another preferred embodiment of the invention, the composition comprises as component B at least one, preferably exactly one herbicide B, and as component C at least one, preferably exactly one, safener C.

According to another preferred embodiment of the invention, the composition comprises at least two, preferably exactly two, herbicides B different from each other, and as component C at least one, preferably exactly one, safener C.

According to another preferred embodiment of the invention, the composition comprises at least three, preferably exactly three, herbicides B different from each other, and as component C at least one, preferably exactly one, safener C.

According to another preferred embodiment of the invention, the composition comprises as component A at least one, preferably exactly one pyridine compound of formula (I), preferably of formula (1.3), especially preferred the compound (1.3.I-3), (1.3.I-4), (1.3.I-10), (1.3.I-11), (1.3.I-17), (1.3.I-18), (1.3.I-24), (1.3.I-25), (1.3.I-31), (1.3.1-32), (1.3.1-38), (1.3.1-39), (1.3.1-45), or (1.3.1-46), and as component B at least one, preferably exactly one, herbicide B.

According to another preferred embodiment of the invention, the composition comprises as component A at least one, preferably exactly one pyridine compound of formula (I), preferably of formula (1.3), especially preferred the compound (1.3.I-3), (1.3.I-4), (1.3.I-10), (1.3.I-11), (1.3.I-17), (1.3.I-18), (1.3.I-24), (1.3.I-25), (1.3.I-31), (1.3.I-32), (1.3.I-38), (1.3.I-39), (1.3.I-45), or (1.3.I-46), and at least two, preferably exactly two, herbicides B different from each other. According to another preferred embodiment of the invention, the composition comprises as component A at least one, preferably exactly one pyridine compound of formula (I), preferably of formula (1.3), especially preferred the compound(1.3.I-3), (1.3.I-4), (1.3.I-10), (1.3.I-11), (1.3.I-17), (1.3.I-18), (1.3.I-24), (1.3.I-25), (1.3.I-31), (1.3.I-32), (1.3.I-38), (1.3.I-39), (1.3.I-45), or (1.3.I-46), and at least three, preferably exactly three, herbicides B different from each other. According to another preferred embodiment of the invention, the composition comprises as component A at least one, preferably exactly one pyridine compound of formula (I), preferably of formula (1.3), especially preferred the compound (1.3.1-3), (1.3.1-4), (1.3.1-10), (1.3.1-11), (1.3.1-17), (1.3.1-18), (1.3.1-24), (1.3.1-25), (1.3.I-31), (1.3.1-32), (1.3.1-38), (1.3.1-39), (1.3.1-45), or (1.3.1-46), and as component C at least one, preferably exactly one, safener C.

According to another preferred embodiment of the invention, the composition comprises as component A at least one, preferably exactly one pyridine compound of formula (I), preferably of formula (1.3), especially preferred the compound (1.3.1-3), (1.3.1-4), (1.3.1-10), (1.3.1-11), (1.3.1-17), (1.3.1-18), (1.3.1-24), (1.3.1-25), (1.3.I-31), (1.3.1-32), (1.3.1-38), (1.3.1-39), (1.3.1-45), or (1.3.1-46), as component B at least one, preferably exactly one, herbicide B, and as component C at least one, preferably exactly one safener C.

According to another preferred embodiment of the invention, the composition comprises as component A at least one, preferably exactly pyridine compound of formula (I), preferably of formula (1.3), especially preferred the compound (1.3.1-3), (1.3.1-4), (1.3.1-10), (1.3.1-11), (1.3.1-17), (1.3.1-18), (1.3.1-24), (1.3.1-25), (1.3.I-31), (1.3.1-32), (1.3.1-38), (1.3.1-39), (1.3.1-45), or (1.3.1-46), at least two, preferably exactly two herbicides B different from each other, and as component C at least one, preferably exactly one, safener C.

According to another preferred embodiment of the invention, the composition comprises as component A at least one, preferably exactly one pyridine compound of formula (I), preferably of formula (1.3), especially preferred the compound (1.3.1-3), (1.3.1-4), (1.3.1-10), (1.3.1-11), (1.3.1-17), (1.3.1-18), (1.3.1-24), (1.3.1-25), (1.3.I-31), (1.3.1-32), (1.3.1-38), (1.3.1-39), (1.3.1-45), or (1.3.1-46), at least three, preferably exactly three herbicides B different from each other, and as component C at least one, preferably exactly one, safener C.

According to another preferred embodiment of the invention, the composition comprises as component A at least one, preferably exactly one pyridine compound of formula (I), preferably of formula (1.15), especially preferred the compound (1.15.1-3), (1.15.1-4), (1.15.1-10), (1.15.1-11), (1.15.1-17), (1.15.1-18), (1.15.1-24), (1.15.1-25), (1.15.1-31), (1.15.1-32), (1.15.1-38), (1.15.1-39), (1.15.1-45), or (1.15.1-46), and as component B at least one, preferably exactly one, herbicide B. According to another preferred embodiment of the invention, the composition comprises as component A at least one, preferably exactly one pyridine compound of formula (I), preferably of formula (1.15), especially preferred the compound (1.15.I-3), (1.15.I-4), (1.15.I-10), (1.15.I-11), (1.15.I-17), (1.15.I-18), (1.15.I-24), (1.15.I-25), (1.15.I-31), (1.15.I-32), (1.15.I-38), (1.15.I-39), (1.15.I-45), or (1.15.I-46), and at least two, preferably exactly two, herbicides B different from each other.

According to another preferred embodiment of the invention, the composition comprises as component A at least one, preferably exactly one pyridine compound of formula (I), preferably of formula (1.15), especially preferred the compound(1.15.I-3), (1.15.I-4), (1.15.I-10), (1.15.I-11), (1.15.I-17), (1.15.I-18), (1.15.I-24), (1.15.I-25), (1.15.I-31), (1.15.I-32), (1.15.I-38), (1.15.I-39), (1.15.I-45), or (1.15.I-46), and at least three, preferably exactly three, herbicides B different from each other.

According to another preferred embodiment of the invention, the composition comprises as component A at least one, preferably exactly one pyridine compound of formula (I), preferably of formula (1.15), especially preferred the compound (1.15.I-3), (1.15.I-4), (1.15.I-10), (1.15.I-11), (1.15.I-17), (1.15.I-18), (1.15.I-24), (1.15.I-25), (1.15.I-31), (1.15.I-32), (1.15.I-38), (1.15.1-39), (1.15.1-45), or (1.15.1-46), and as component C at least one, preferably exactly one, safener C. According to another preferred embodiment of the invention, the composition comprises as component A at least one, preferably exactly one pyridine compound of formula (I), preferably of formula (1.15), especially preferred the compound (1.15.I-3), (1.15.I-4), (1.15.I-10), (1.15.I-11), (1.15.I-17), (1.15.I-18), (1.15.I-24), (1.15.I-25), (1.15.I-31), (1.15.I-32), (1.15.I-38), (1.15.I-39), (1.15.I-45), or (1.15.I-46), as component B at least one, preferably exactly one, herbicide B, and as component C at least one, preferably exactly one safener C.

According to another preferred embodiment of the invention, the composition comprises as component A at least one, preferably exactly pyridine compound of formula (I), preferably of formula (1.15), especially preferred the compound (1.15.I-3), (1.15.I-4), (1.15.I-10), (1.15.I-11), (1.15.I-17), (1.15.I-18), (1.15.I-24), (1.15.I-25), (1.15.I-31), (1.15.I-32), (1.15.I-38), (1.15.I-39), (1.15.I-45), or (1.15.I-46), at least two, preferably exactly two herbicides B different from each other, and as component C at least one, preferably exactly one, safener C.

According to another preferred embodiment of the invention, the composition comprises as component A at least one, preferably exactly one pyridine compound of formula (I), preferably of formula (1.15), especially preferred the compound (1.15.I-3), (1.15.I-4), (1.15.I-10), (1.15.I-11), (1.15.I-17), (1.15.I-18), (1.15.I-24), (1.15.I-25), (1.15.I-31), (1.15.I-32), (1.15.I-38), (1.15.I-39), (1.15.I-45), or (1.15.I-46), at least three, preferably exactly three herbicides B different from each other, and as component C at least one, preferably exactly one, safener C.

According to another preferred embodiment of the invention, the composition comprises as component A at least one, preferably exactly one pyridine compound of formula (I), preferably of formula (1.16), especially preferred the compound (1.16.I-3), (1.16.I-4), (1.16.I-10), (1.16.I-11), (1.16.I-17), (1.16.I-18), (1.16.I-24), (1.16.I-25), (1.16.I-31), (1.16.I-32), (1.16.I-38), (1.16.I-39), (1.16.I-45), or (1.16.I-46), and as component B at least one, preferably exactly one, herbicide B. According to another preferred embodiment of the invention, the composition comprises as component A at least one, preferably exactly one pyridine compound of formula (I), preferably of formula (1.16), especially preferred the compound (1.16.I-3), (1.16.I-4), (1.16.I-10), (1.16.I-11), (1.16.I-17), (1.16.I-18), (1.16.I-24), (1.16.I-25), (1.16.I-31), (1.16.I-32), (1.16.I-38), (1.16.I-39), (1.16.I-45), or (1.16.I-46), and at least two, preferably exactly two, herbicides B different from each other.

According to another preferred embodiment of the invention, the composition comprises as component A at least one, preferably exactly one pyridine compound of formula (I), preferably of formula (1.16), especially preferred the compound(1.16.I-3), (1.16.I-4), (1.16.I-10), (1.16.I-11), (1.16.I-17), (1.16.I-18), (1.16.I-24), (1.16.I-25), (1.16.I-31), (1.16.I-32), (1.16.I-38), (1.16.I-39), (1.16.I-45), or (1.16.I-46), and at least three, preferably exactly three, herbicides B different from each other.

According to another preferred embodiment of the invention, the composition comprises as component A at least one, preferably exactly one pyridine compound of formula (I), preferably of formula (1.16), especially preferred the compound (1.16.I-3), (1.16.I-4), (1.16.I-10), (1.16.I-11), (1.16.I-17), (1.16.I-18), (1.16.I-24), (1.16.I-25), (1.16.I-31), (1.16.I-32), (1.16.I-38), (1.16.I-39), (1.16.I-45), or (1.16.I-46), and as component C at least one, preferably exactly one, safener C.

According to another preferred embodiment of the invention, the composition comprises as component A at least one, preferably exactly one pyridine compound of formula (I), preferably of formula (1.16), especially preferred the compound (1.16.I-3), (1.16.I-4), (1.16.I-10), (1.16.I-11), (1.16.I-17), (1.16.I-18), (1.16.I-24), (1.16.I-25), (1.16.I-31), (1.16.I-32), (1.16.I-38), (1.16.I-39), (1.16.I-45), or (1.16.I-46), as component B at least one, preferably exactly one, herbicide B, and as component C at least one, preferably exactly one safener C.

According to another preferred embodiment of the invention, the composition comprises as component A at least one, preferably exactly pyridine compound of formula (I), preferably of formula (1.16), especially preferred the compound (1.16.I-3), (1.16.I-4), (1.16.I-10), (1.16.I-11), (1.16.I-17), (1.16.I-18), (1.16.I-24), (1.16.I-25), (1.16.I-31), (1.16.I-32), (1.16.I-38), (1.16.I-39), (1.16.I-45), or (1.16.I-46), at least two, preferably exactly two herbicides B different from each other, and as component C at least one, preferably exactly one, safener C.

According to another preferred embodiment of the invention, the composition comprises as component A at least one, preferably exactly one pyridine compound of formula (I), preferably of formula (1.16), especially preferred the compound (1.16.I-3), (1.16.I-4), (1.16.I-10), (1.16.I-11), (1.16.I-17), (1.16.I-18), (1.16.I-24), (1.16.I-25), (1.16.I-31), (1.16.I-32), (1.16.I-38), (1.16.I-39), (1.16.I-45), or (1.16.I-46), at least three, preferably exactly three herbicides B different from each other, and as component C at least one, preferably exactly one, safener C.

According to another preferred embodiment of the invention, the composition comprises, in addition to a pyridine compounds of formula (I), especially an active compound from the group consisting of (1.3.I-3), (1.3.I-4), (1.3.I-10), (1.3.I-11), (1.3.I-17), (1.3.I-18), (1.3.I-24), (1.3.I-25), (1.3.I-31), (1.3.I-32), (1.3.I-38), (1.3.I-39), (1.3.I-45), (1.3.I-46),(1.15.I-3), (1.15.I-4), (1.15.I-10), (1.15.I-11), (1.15.I-17), (1.15.I-18), (1.15.I-24), (1.15.I-25), (1.15.I-31), (1.15.I-32), (1.15.I-38), (1.15.I-39), (1.15.I-45), or (1.15.1-46), (1.16.I-3), (1.16.I-4), (1.16.I-10), (1.16.I-11), (1.16.I-17), (1.16.I-18), (1.16.I-24), (1.16.I-25), (1.16.I-31), (1.16.I-32), (1.16.I-38), (1.16.I-39), (1.16.I-45), and (1.16.I-46), at least one and especially exactly one herbicidally active compound from group b1), in particular selected from the group consisting of clethodim, clodinafop-propargyl, cycloxydim, cyhalofop-butyl, fenoxaprop-ethyl, fenoxaprop-P-ethyl, metamifop, pinoxaden, profoxydim, sethoxydim, tepraloxydim, tralkoxydim, esprocarb, ethofumesate, molinate, prosulfocarb, thiobencarb and triallate.

According to another preferred embodiment of the invention, the composition comprises, in addition to a pyridine compounds of formula (I), especially an active compound from the group consisting of (1.3.I-3), (1.3.I-4), (1.3.I-10), (1.3.I-11), (1.3.I-17), (1.3.I-18), (1.3.I-24), (1.3.I-25), (1.3.I-31), (1.3.I-32), (1.3.I-38), (1.3.I-39), (1.3.I-45), (1.3.I-46), (1.15.I-3), (1.15.I-4), (1.15.I-10), (1.15.I-11), (1.15.I-17), (1.15.I-18), (1.15.I-24), (1.15.I-25), (1.15.I-31), (1.15.I-32), (1.15.I-38), (1.15.I-39), (1.15.I-45), or (1.15.I-46), (1.16.I-3), (1.16.I-4), (1.16.I-10), (1.16.I-11), (1.16.I-17), (1.16.I-18), (1.16.I-24), (1.16.I-25), (1.16.I-31), (1.16.I-32), (1.16.I-38), (1.16.I-39), (1.16.I-45), and (1.16.1-46), at least one and especially exactly one herbicidally active compound from group b2), in particular selected from the group consisting of bensulfuron-methyl, bispyribac-sodium, cloransulam-methyl, chlorsulfuron, clorimuron, cyclosulfamuron, diclosulam, florasulam, flumetsulam, flupyrsulfuron-methyl-sodium, foramsulfuron, imazamox, imazamox-ammonium, imazapic, imazapic-ammonium, imazapic-isopropylammonium, imazapyr, imazapyr-ammonium, imazethapyr-isopropylammonium, imazaquin, imazaquin-ammonium, imazethapyr, imazethapyr-ammonium, imazethapyr-isopropylammonium, imazosulfuron, iodosulfuron-methyl-sodium, iofensulfuron, iofensulfuron-sodium, mesosulfuron-methyl, metazosulfuron, metsulfuron-methyl, metosulam, nicosulfuron, penoxsulam, propoxycarbazon-sodium, pyrazosulfuron-ethyl, pyribenzoxim, pyriftalid, pyroxsulam, propyrisulfuron, rimsulfuron, sulfosulfuron, thiencarbazonmethyl, thifensulfuron-methyl, tribenuron-methyl, tritosulfuron and triafamone.

According to another preferred embodiment of the invention, the composition comprises, in addition to a pyridine compounds of formula (I), especially an active compound from the group consisting (1.3.1-3), (1.3.1-4), (1.3.1-10), (1.3.1-11), (1.3.1-17), (1.3.1-18), (1.3.1-24), (1.3.1-25), (1.3.1-31), (1.3.1-32), (1.3.1-38), (1.3.1-39), (1.3.1-45), (1.3.1-46),(1.15.1-3), (1.15.1-4), (1.15.1-10), (1.15.1-11), (1.15.1-17), (1.15.1-18), (1.15.1-24), (1.15.1-25), (1.15.1-31), (1.15.1-32), (1.15.1-38), (1.15.1-39), (1.15.1-45), or (1.15.1-46),(1.16.1-3), (1.16.1-4), (1.16.1-10), (1.16.1-11), (1.16.1-17), (1.16.1-18), (1.16.1-24), (1.16.1-25), (1.16.1-31), (1.16.1-32), (1.16.1-38), (1.16.1-39), (1.16.1-45), and (1.16.1-46), at least one and especially exactly one herbicidally active compound from group b3), in particular selected from the group consisting of ametryn, atrazine, bentazon, bromoxynil, bromoxynil-octanoate, bromoxynil-heptanoate, bromoxynil-potassium, diuron, fluometuron, hexazinone, isoproturon, linuron, metamitron, metribuzin, paraquat-dichloride, propanil, simazin, terbutryn and terbuthylazine.

According to another preferred embodiment of the invention, the composition comprises, in addition to a pyridine compounds of formula (I), especially an active compound from the group consisting of (1.3.I-3), (1.3.I-4), (1.3.I-10), (1.3.I-11), (1.3.I-17), (1.3.I-18), (1.3.I-24), (1.3.I-25), (1.3.I-31), (1.3.I-32), (1.3.I-38), (1.3.I-39), (1.3.I-45), (1.3.I-46), (1.15.I-3), (1.15.I-4), (1.15.I-10), (1.15.I-11), (1.15.I-17), (1.15.I-18), (1.15.I-24), (1.15.I-25), (1.15.I-31), (1.15.I-32), (1.15.I-38), (1.15.I-39), (1.15.I-45), or (1.15.I-46), (1.16.I-3), (1.16.I-4), (1.16.I-10), (1.16.I-11), (1.16.I-17), (1.16.I-18), (1.16.I-24), (1.16.I-25), (1.16.I-31), (1.16.I-32), (1.16.I-38), (1.16.I-39), (1.16.I-45), and (1.16.I-46), at least one and especially exactly one herbicidally active compound from group b4), in particular selected from the group consisting of acifluorfen, butafencil, carfenetrazoneethyl, flumioxazin, fomesafen, oxadiargyl, oxyfluorfen, pyraflufen, pyraflufen-ethyl, saflufenacil, sulfentrazone, trifludimoxazin , ethyl [3-[2-chloro-4-fluoro-5-(1-methyl-6-trifluoromethyl-2,4-dioxo-1,2,3,4-tetrahydropyrimidin-3-yl)phenoxy]-2-pyridyloxy]acetate (CAS 353292-31-6; S-3100). According to another preferred embodiment of the invention, the composition comprises, in addition to a pyridine compounds of formula (I), especially an active compound from the group consisting of (1.3.I-3), (1.3.I-4), (1.3.I-10), (1.3.I-11), (1.3.I-17), (1.3.I-18), (1.3.I-24), (1.3.I-25), (1.3.I-31), (1.3.I-32), (1.3.I-38), (1.3.I-39), (1.3.I-45), (1.3.I-46),(1.15.I-3), (1.15.I-4), (1.15.I-10), (1.15.I-11), (1.15.I-17), (1.15.I-18), (1.15.I-24), (1.15.I-25), (1.15.I-31), (1.15.I-32), (1.15.I-38), (1.15.I-39), (1.15.I-45), or (1.15.I-46), (1.16.I-3), (1.16.I-4), (1.16.I-10), (1.16.I-11), (1.16.I-17), (1.16.I-18), (1.16.I-24), (1.16.I-25), (1.16.I-31), (1.16.I-32), (1.16.I-38), (1.16.I-39), (1.16.I-45), and (1.16.I-46), at least one and especially exactly one herbicidally active compound from group b5), in particular selected from the group consisting of amitrole, benzobicyclon, bicyclopyrone, clomazone, diflufenican, fenquintrone, fluometuron, flurochloridone, isoxaflutole, mesotrione, norflurazone, oxotrione (CAS 1486617-21-3), picolinafen, sulcotrione, tefuryltrione, tembotrione, tolpyralate, topramezone, topramezone-sodium and 2-chloro-3-methylsulfanyl-N-(1-methyltetrazol-5-yl)-4-(trifluoromethyl)benzamide (CAS 1361139-71-0) .

According to another preferred embodiment of the invention, the composition comprises, in addition to a pyridine compounds of formula (I), especially an active compound from the group consisting of (1.3.I-3), (1.3.I-4), (1.3.I-10), (1.3.I-11), (1.3.I-17), (1.3.I-18), (1.3.I-24), (1.3.I-25), (1.3.I-31), (1.3.I-32), (1.3.I-38), (1.3.I-39), (1.3.I-45), (1.3.I-46), (1.15.I-3), (1.15.I-4), (1.15.I-10), (1.15.I-11), (1.15.I-17), (1.15.I-18), (1.15.I-24), (1.15.I-25), (1.15.I-31), (1.15.I-32), (1.15.I-38), (1.15.I-39), (1.15.I-45), or (1.15.I-46), (1.16.I-3), (1.16.I-4), (1.16.I-10), (1.16.I-11), (1.16.I-17), (1.16.1-18), (1.16.1-24), (1.16.1-25), (1.16.1-31), (1.16.1-32), (1.16.1-38), (1.16.1-39), (1.16.1-45), and (1.16.1-46), at least one and especially exactly one herbicidally active compound from group b6), in particular selected from the group consisting of glyphosate, glyphosate-ammonium, glyphosate-dimethylammonium , glyphosate-isopropylammonium and glyphosate-trimesium (sulfosate) and glyphosate-potassium.

According to another preferred embodiment of the invention, the composition comprises, in addition to a pyridine compounds of formula (I), especially an active compound from the group consisting of (1.3.I-3), (1.3.I-4), (1.3.I-10), (1.3.I-11), (1.3.I-17), (1.3.I-18), (1.3.I-24), (1.3.I-25), (1.3.I-31), (1.3.I-32), (1.3.I-38), (1.3.I-39), (1.3.I-45), (1.3.I-46),(1.15.I-3), (1.15.I-4), (1.15.I-10), (1.15.I-11), (1.15.I-17), (1.15.I-18), (1.15.I-24), (1.15.I-25), (1.15.I-31), (1.15.I-32), (1.15.I-38), (1.15.I-39), (1.15.I-45), or (1.15.I-46), (1.16.I-3), (1.16.I-4), (1.16.I-10), (1.16.I-11), (1.16.I-17), (1.16.I-18), (1.16.I-24), (1.16.I-25), (1.16.I-31), (1.16.I-32), (1.16.I-38), (1.16.I-39), (1.16.I-45), and (1.16.I-46), at least one and especially exactly one herbicidally active compound from group b7), in particular selected from the group consisting of glufosinate, glufosinate-ammonium, glufosinate-P and glufosinate-P-ammonium.

According to another preferred embodiment of the invention, the composition comprises, in addition to a pyridine compounds of formula (I), especially an active compound from the group consisting of (1.3.I-3), (1.3.I-4), (1.3.I-10), (1.3.I-11), (1.3.I-17), (1.3.I-18), (1.3.I-24), (1.3.I-25), (1.3.I-31), (1.3.I-32), (1.3.I-38), (1.3.I-39), (1.3.I-45), (1.3.I-46), (1.15.I-3), (1.15.I-4), (1.15.I-10), (1.15.I-11), (1.15.I-17), (1.15.I-18), (1.15.I-24), (1.15.I-25), (1.15.I-31), (1.15.I-32), (1.15.I-38), (1.15.I-39), (1.15.I-45), or (1.15.I-46), (1.16.I-3), (1.16.I-4), (1.16.I-10), (1.16.I-11), (1.16.I-17), (1.16.I-18), (1.16.I-24), (1.16.I-25), (1.16.I-31), (1.16.I-32), (1.16.I-38), (1.16.I-39), (1.16.I-45), and (1.16.I-46), at least one and especially exactly one herbicidally active compound from group b9), in particular selected from the group consisting of pendimethalin and trifluralin.

According to another preferred embodiment of the invention, the composition comprises, in addition to a pyridine compounds of formula (I), especially an active compound from the group consisting of (1.3.I-3), (1.3.I-4), (1.3.I-10), (1.3.I-11), (1.3.I-17), (1.3.I-18), (1.3.I-24), (1.3.I-25), (1.3.I-31), (1.3.I-32), (1.3.I-38), (1.3.I-39), (1.3.I-45), (1.3.I-46), (1.15.I-3), (1.15.I-4), (1.15.I-10), (1.15.I-11), (1.15.I-17), (1.15.I-18), (1.15.I-24), (1.15.I-25), (1.15.I-31), (1.15.I-32), (1.15.I-38), (1.15.I-39), (1.15.I-45), or (1.15.I-46), (1.16.I-3), (1.16.I-4), (1.16.I-10), (1.16.I-11), (1.16.I-17), (1.16.I-18), (1.16.I-24), (1.16.I-25), (1.16.I-31), (1.16.I-32), (1.16.I-38), (1.16.I-39), (1.16.I-45), and (1.16.I-46), at least one and especially exactly one herbicidally active compound from group b10), in particular selected from the group consisting of acetochlor, butachlor, cafenstrole, dimethenamid-P, fentrazamide, flufenacet, mefenacet, metazachlor, metolachlor, S-metolachlor, fenoxasulfone, ipfencarbazone and pyroxasulfone. Likewise, preference is given to compositions comprising in addition to a pyridine compounds of formula (I), especially an active compound from the group consisting of (1.3.I-3), (1.3.I-4), (1.3.I-10), (1.3.I-11), (1.3.I-17), (1.3.I-18), (1.3.I-24), (1.3.I-25), (1.3.I-31), (1.3.I-32), (1.3.I-38), (1.3.I-39), (1.3.I-45), (1.3.I-46), (1.15.I-3), (1.15.I-4), (1.15.I-10), (1.15.I-11), (1.15.I-17), (1.15.I-18), (1.15.I-24), (1.15.I-25), (1.15.I-31), (1.15.I-32), (1.15.I-38), (1.15.I-39), (1.15.I-45), or (1.15.I-46), (1.16.I-3), (1.16.I-4), (1.16.I-10), (1.16.I-11), (1.16.I-17), (1.16.I-18), (1.16.I-24), (1.16.I-25), (1.16.I-31), (1.16.I-32), (1.16.I-38), (1.16.I-39), (1.16.I-45), and (1.16.I-46), at least one and especially exactly one herbicidally active compound from group b10), in particular selected from the group consisting of isoxazoline compounds of the formulae II.1, II.2, II.3, II.4, II.5, II.6, II.7, II.8 and II.9, as defined above. According to another preferred embodiment of the invention, the composition comprises, in addition to a pyridine compounds of formula (I), especially an active compound from the group consisting of (1.3.I-3), (1.3.I-4), (1.3.I-10), (1.3.I-11), (1.3.I-17), (1.3.I-18), (1.3.I-24), (1.3.I-25), (1.3.I-31), (1.3.I-32), (1.3.I-38), (1.3.I-39), (1.3.I-45), (1.3.I-46), (1.15.I-3), (1.15.I-4), (1.15.I-10), (1.15.I-11), (1.15.I-17), (1.15.I-18), (1.15.I-24), (1.15.I-25), (1.15.I-31), (1.15.I-32), (1.15.I-38), (1.15.I-39), (1.15.I-45), or (1.15.I-46), (1.16.I-3), (1.16.I-4), (1.16.I-10), (1.16.I-11), (1.16.I-17), (1.16.I-18), (1.16.I-24), (1.16.I-25), (1.16.I-31), (1.16.I-32), (1.16.I-38), (1.16.I-39), (1.16.I-45), and (1.16.I-46), at least one and especially exactly one herbicidally active compound from group b11), in particular indaziflam, isoxaben and triaziflam.

According to another preferred embodiment of the invention, the composition comprises, in addition to a pyridine compounds of formula (I), especially an active compound from the group consisting of (1.3.I-3), (1.3.I-4), (1.3.I-10), (1.3.I-11), (1.3.I-17), (1.3.I-18), (1.3.I-24), (1.3.I-25), (1.3.I-31), (1.3.I-32), (1.3.I-38), (1.3.I-39), (1.3.I-45), (1.3.I-46), (1.15.I-3), (1.15.I-4), (1.15.I-10), (1.15.I-11), (1.15.I-17), (1.15.I-18), (1.15.I-24), (1.15.I-25), (1.15.I-31), (1.15.I-32), (1.15.I-38), (1.15.I-39), (1.15.I-45), or (1.15.I-46), (1.16.I-3), (1.16.I-4), (1.16.I-10), (1.16.I-11), (1.16.I-17), (1.16.I-18), (1.16.I-24), (1.16.I-25), (1.16.I-31), (1.16.I-32), (1.16.I-38), (1.16.I-39), (1.16.I-45), and (1.16.I-46), at least one and especially exactly one herbicidally active compound from group b13), in particular selected from the group consisting of 2,4-D, 2,4-D-isobutyl, 2,4-D-dimethylammonium, 2,4-D-N,N,N-trimethylethanolammonium, aminocyclopyrachlor, aminocyclopyrachlor-potassium, aminocyclopyrachlor-methyl, aminopyralid, aminopyralid-methyl, aminopyralid-dimethylammonium, aminopyralid-tris(2-hydroxypropyl)ammonium, clopyralid, clopyralid-methyl, clopyralid-olamine, dicamba, dicamba-butotyl, dicamba-diglycolamine, dicamba-dimethylammonium, dicamba-diolamine, dicamba-isopropylammonium, dicamba-potassium, dicamba-sodium, dicamba-trolamine, dicamba-N,N-bis-(3-aminopropyl)methylamine, dicamba-diethylenetriamine, flopyrauxifen, fluroxypyr, fluroxypyr-meptyl, halauxifen, halauxifen-methyl, MCPA, MCPA-2-ethylhexyl, MCPA-dimethylammonium, quinclorac, quinclorac-dimethylammonium, quinmerac, quinmerac-dimethylammonium, florpyrauxifen , florpyrauxifen-benzyl (CAS 1390661-72-9), and 4-amino-3-chloro-5-fluoro-6-(7-fluoro-1 H-indol-6-yl)picolinic acid (CAS 1629965-65-6) . According to another preferred embodiment of the invention, the composition comprises, in addition to a pyridine compounds of formula (I), especially an active compound from the group consisting of (1.3.I-3), (1.3.I-4), (1.3.I-10), (1.3.I-11), (1.3.I-17), (1.3.I-18), (1.3.I-24), (1.3.I-25), (1.3.I-31), (1.3.I-32), (1.3.I-38), (1.3.I-39), (1.3.I-45), (1.3.I-46), (1.15.I-3), (1.15.I-4), (1.15.I-10), (1.15.I-11), (1.15.I-17), (1.15.I-18), (1.15.I-24), (1.15.I-25), (1.15.I-31), (1.15.I-32), (1.15.I-38), (1.15.I-39), (1.15.I-45), or (1.15.I-46), (1.16.I-3), (1.16.I-4), (1.16.I-10), (1.16.I-11), (1.16.I-17), (1.16.I-18), (1.16.I-24), (1.16.I-25), (1.16.I-31), (1.16.I-32), (1.16.I-38), (1.16.I-39), (1.16.I-45), and (1.16.I-46), at least one and especially exactly one herbicidally active compound from group b14), in particular selected from the group consisting of diflufenzopyr, diflufenzopyr-sodium, dymron, indanofan and diflufenzopyr-sodium.

According to another preferred embodiment of the invention, the composition comprises, in addition to a pyridine compounds of formula (I), especially an active compound from the group consisting of (1.3.I-3), (1.3.I-4), (1.3.I-10), (1.3.I-11), (1.3.I-17), (1.3.I-18), (1.3.I-24), (1.3.I-25), (1.3.I-31), (1.3.I-32), (1.3.I-38), (1.3.I-39), (1.3.I-45), (1.3.I-46), (1.15.I-3), (1.15.I-4), (1.15.I-10), (1.15.I-11), (1.15.I-17), (1.15.I-18), (1.15.I-24), (1.15.I-25), (1.15.I-31), (1.15.I-32), (1.15.I-38), (1.15.I-39), (1.15.I-45), or (1.15.I-46), (1.16.I-3), (1.16.I-4), (1.16.I-10), (1.16.I-11), (1.16.I-17), (1.16.I-18), (1.16.I-24), (1.16.I-25), (1.16.I-31), (1.16.I-32), (1.16.I-38), (1.16.I-39), (1.16.I-45), and (1.16.1-46), at least one and especially exactly one herbicidally active compound from group b15), in particular selected from the group consisting of cinmethylin, dymron (= daimuron), indanofan and oxaziclomefone.

According to another preferred embodiment of the invention, the composition comprises, in addition to a pyridine compounds of formula (I), especially an active compound from the group consisting of (1.3.I-3), (1.3.I-4), (1.3.I-10), (1.3.I-11), (1.3.I-17), (1.3.I-18), (1.3.I-24), (1.3.I-25), (1.3.I-31), (1.3.I-32), (1.3.I-38), (1.3.I-39), (1.3.I-45), (1.3.I-46), (1.15.I-3), (1.15.I-4), (1.15.I-10), (1.15.I-11), (1.15.I-17), (1.15.I-18), (1.15.I-24), (1.15.I-25), (1.15.I-31), (1.15.I-32), (1.15.I-38), (1.15.I-39), (1.15.I-45), or (1.15.I-46), (1.16.I-3), (1.16.I-4), (1.16.I-10), (1.16.I-11), (1.16.I-17), (1.16.I-18), (1.16.I-24), (1.16.I-25), (1.16.I-31), (1.16.I-32), (1.16.I-38), (1.16.I-39), (1.16.1-45), and (1.16.I-46), at least one and especially exactly one safener C, in particular selected from the group consisting of benoxacor, cloquintocet, cyprosulfamide, dichlormid, fenchlorazole, fenclorim, furilazole, isoxadifen, mefenpyr, 4-(dichloroacetyl)-1-oxa-4-azaspiro[4.5]decane (MON4660, CAS 71526-07-3) and 2,2,5-trimethyl-3-(dichloroacetyl)-1,3-oxazolidine (R-29148, CAS 52836-31-4).

Here and below, the term "binary compositions" includes compositions comprising one or more, for example 1, 2 or 3, active compounds of the formula (I) and either one or more, for example 1, 2 or 3, herbicides B or one or more safeners C.

Correspondingly, the term "ternary compositions" includes compositions comprising one or more, for example 1, 2 or 3, active compounds of the formula (I), one or more, for example 1, 2 or 3, herbicides B and one or more, for example 1, 2 or 3, safeners C.

In binary compositions comprising at least one pyridine compound of formula (I) as component A and at least one herbicide B, the weight ratio of the active compounds A:B is generally in the range of from 1:1000 to 1000:1, preferably in the range of from 1:500 to 500:1, in particular in the range of from 1:250 to 250:1 and particularly preferably in the range of from 1:75 to 75:1.

In binary compositions comprising at least one pyridine compound of formula (I) as component A and at least one safener C, the weight ratio of the active compounds A:C is generally in the range of from 1:1000 to 1000:1, preferably in the range of from 1:500 to 500:1, in particular in the range of from 1:250 to 250:1 and particularly preferably in the range of from 1:75 to 75:1.

In ternary compositions comprising at least one pyridine compound of formula (I) as component A, at least one herbicide B and at least one safener C, the relative proportions by weight of the components A:B are generally in the range of from 1:1000 to 1000:1, preferably in the range of from 1:500 to 500:1, in particular in the range of from 1:250 to 250:1 and particularly preferably in the range of from 1:75 to 75:1, the weight ratio of the components A:C is generally in the range of from 1:1000 to 1000:1, preferably in the range of from 1:500 to 500:1, in particular in the range of from 1:250 to 250:1 and particularly preferably in the range of from 1:75 to 75:1, and the weight ratio of the components B:C is generally in the range of from 1:1000 to 1000:1, preferably in the range of from 1:500 to 500:1, in particular in the range of from 1:250 to 250:1 and particularly preferably in the range of from 1:75 to 75:1. The weight ratio of components A + B to component C is preferably in the range of from 1:500 to 500:1, in particular in the range of from 1:250 to 250:1 and particularly preferably in the range of from 1:75 to 75:1.

The weight ratios of the individual components in the preferred mixtures mentioned below are within the limits given above, in particular within the preferred limits.

Particularly preferred are the compositions mentioned below comprising the pyridine compounds of formula I as defined and the substance(s) as defined in the respective row of table T; especially preferred comprising as only herbicidal active compounds the pyridine compounds of formula I as defined and the substance(s) as defined in the respective row of table T; most preferably comprising as only active compounds the pyridine compounds of formula I as defined and the substance(s) as defined in the respective row of table T.

Particularly preferred are compositions 1.1 to 1.3635, comprising the compound (1.3.1-3) and the substance(s) as defined in the respective row of table T:

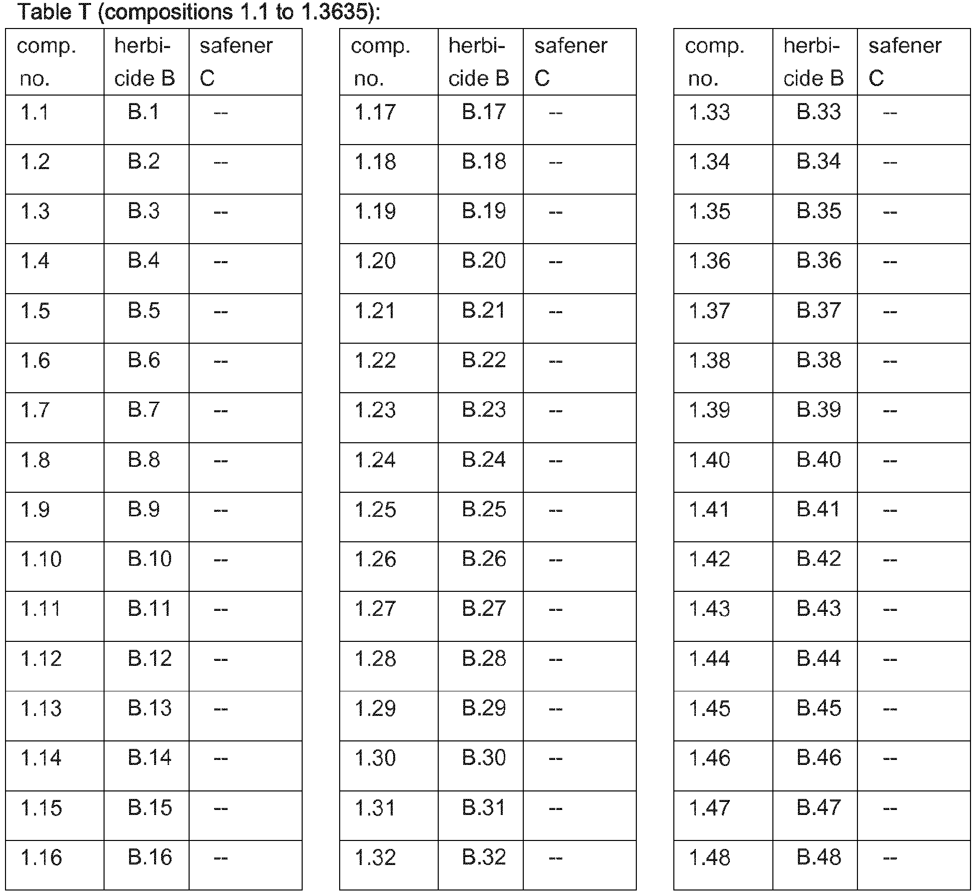

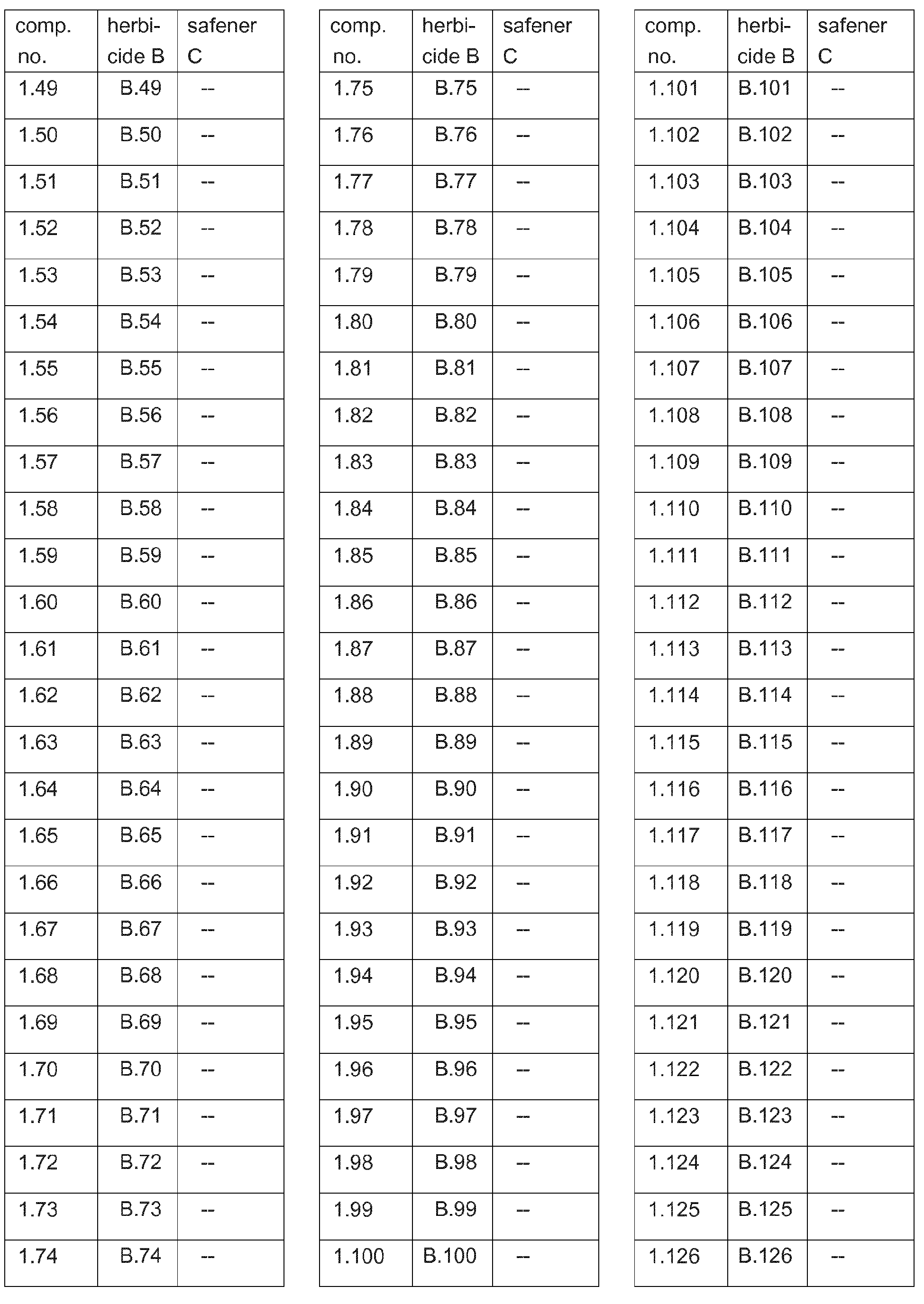

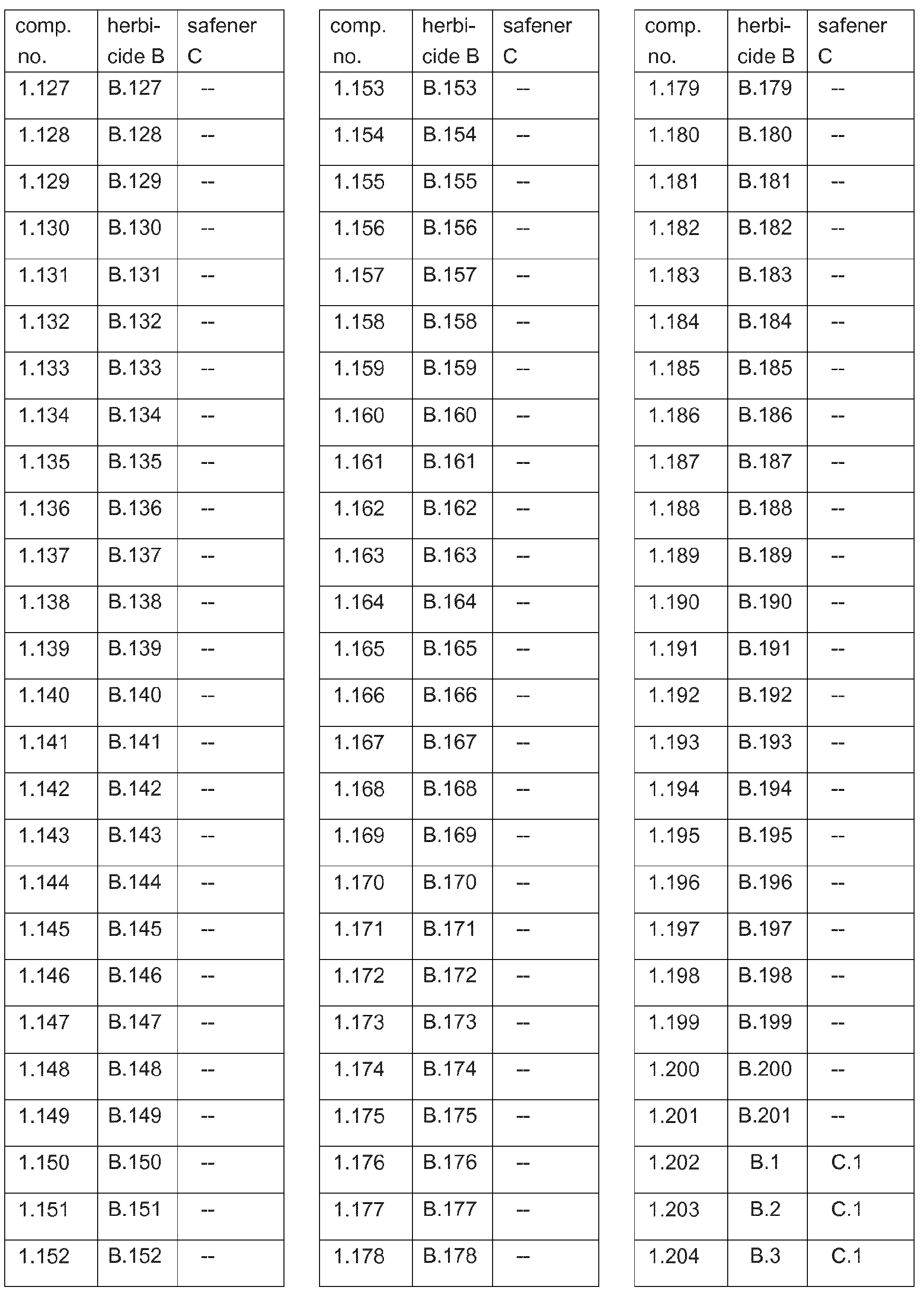

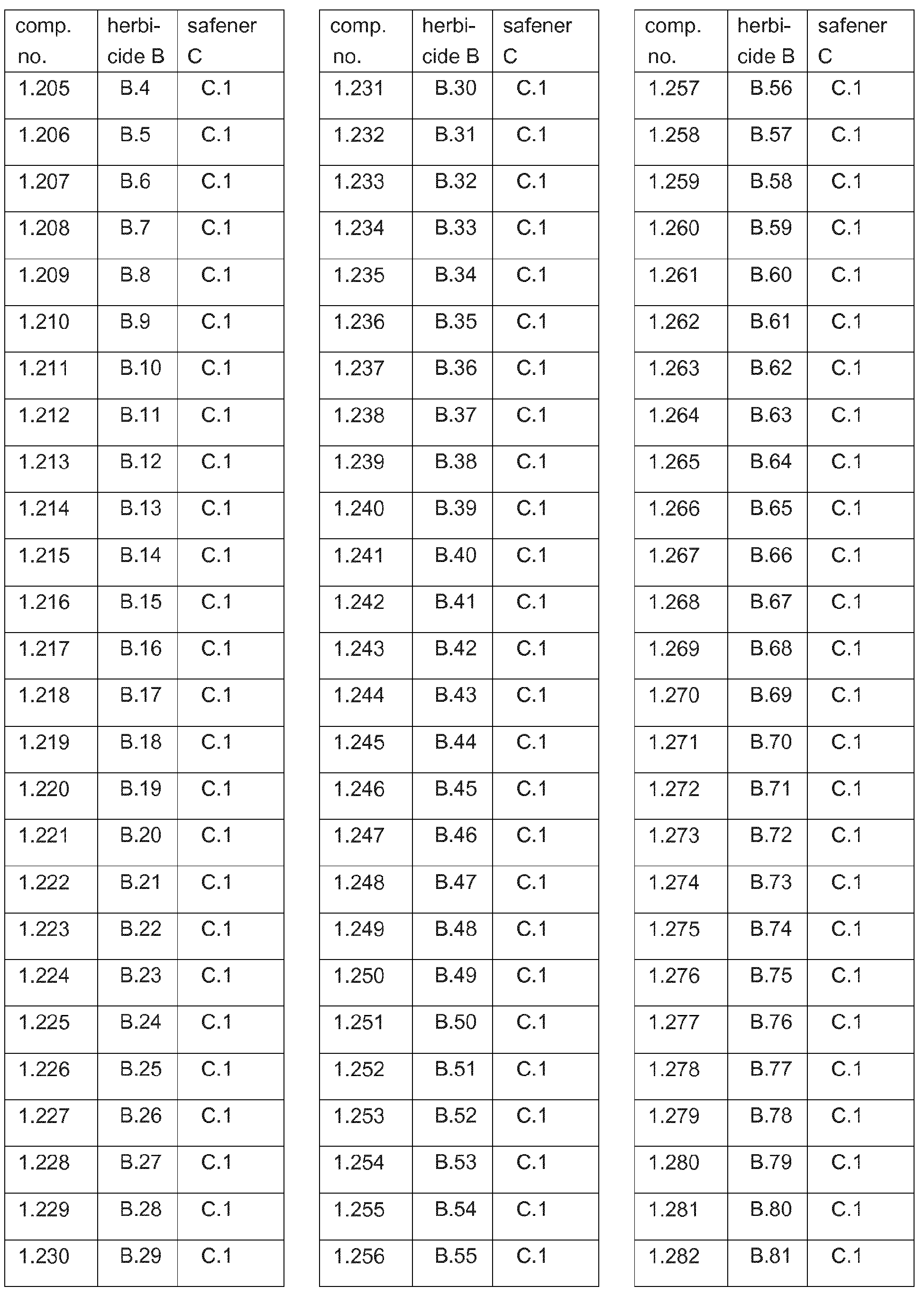

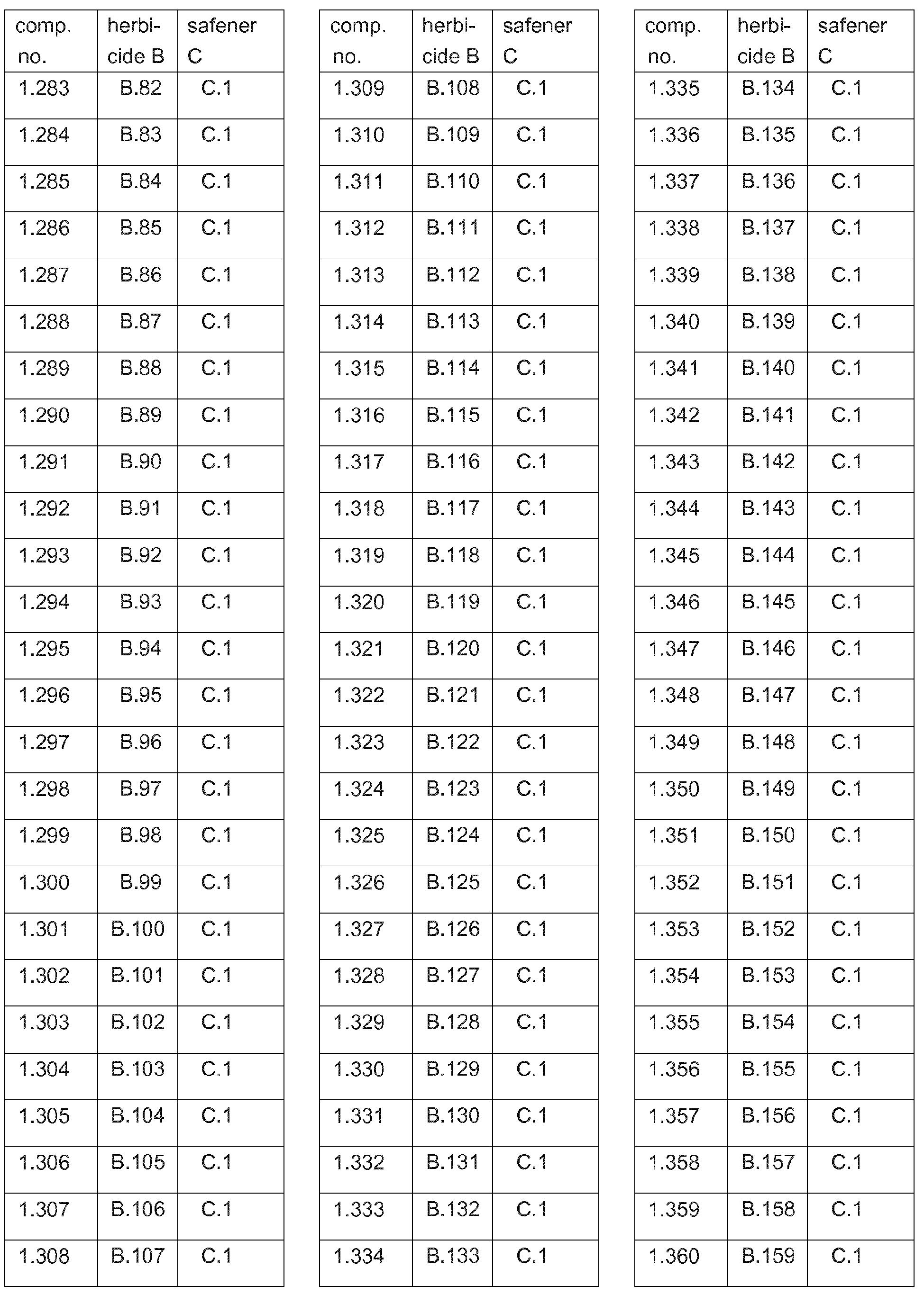

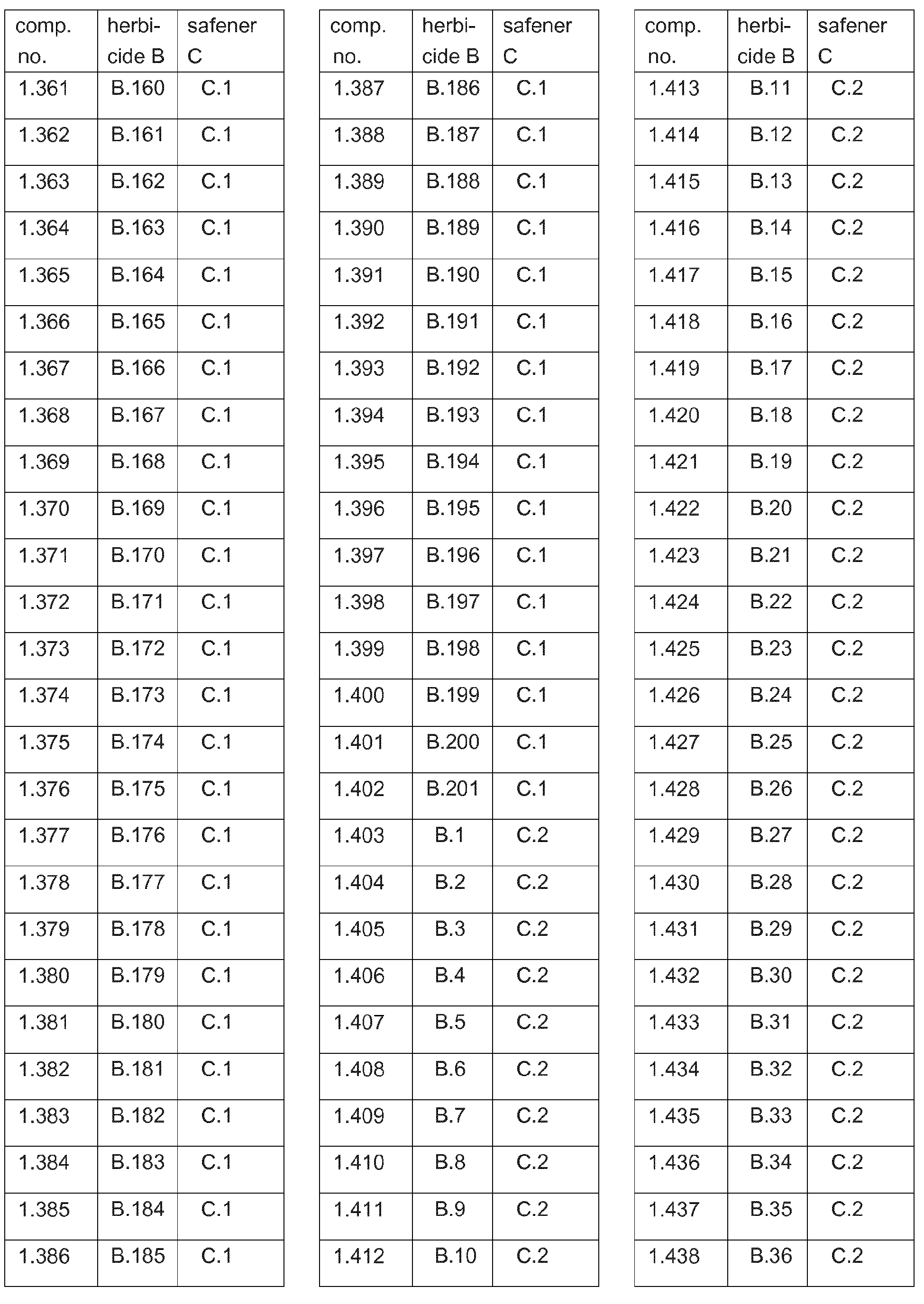

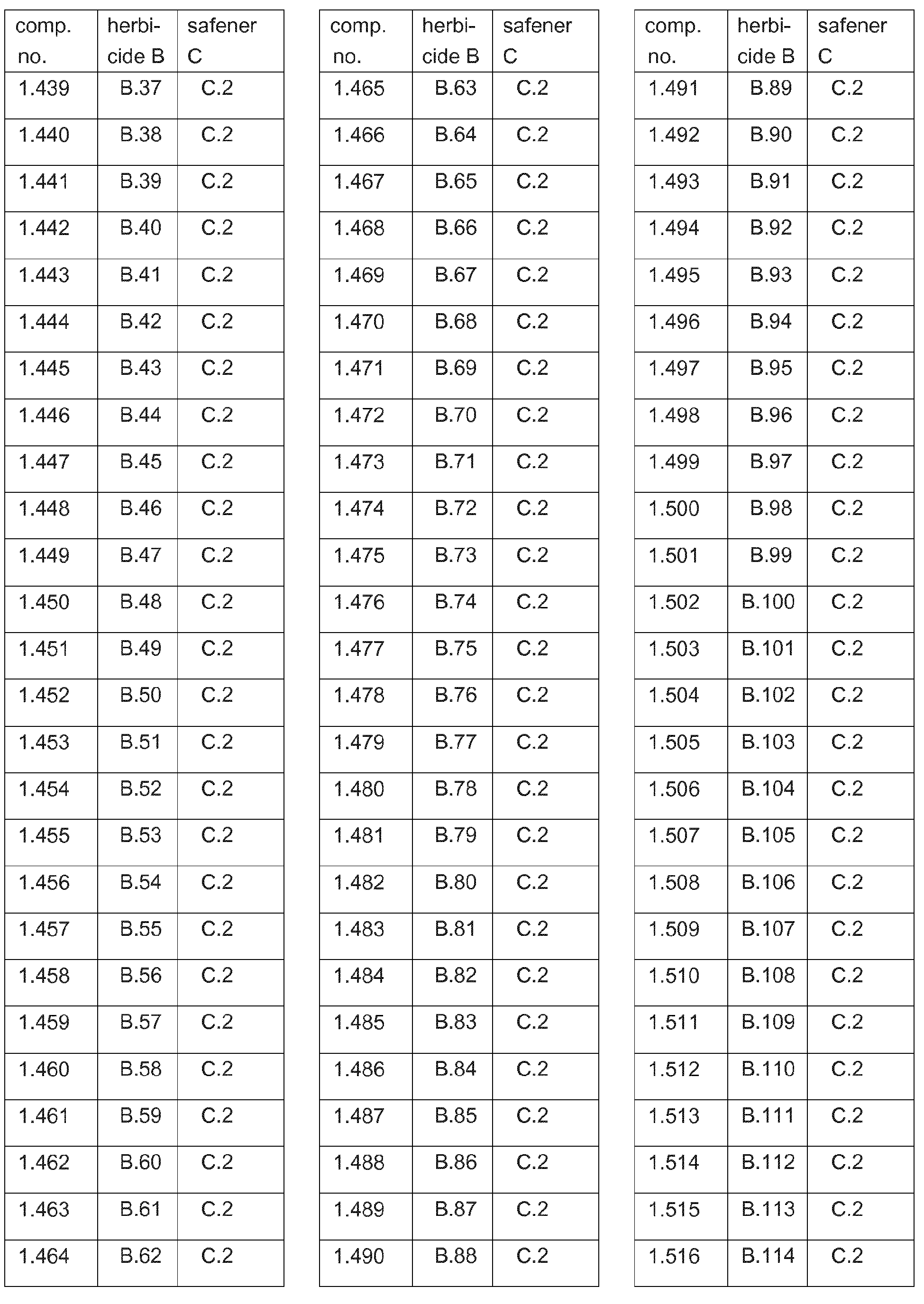

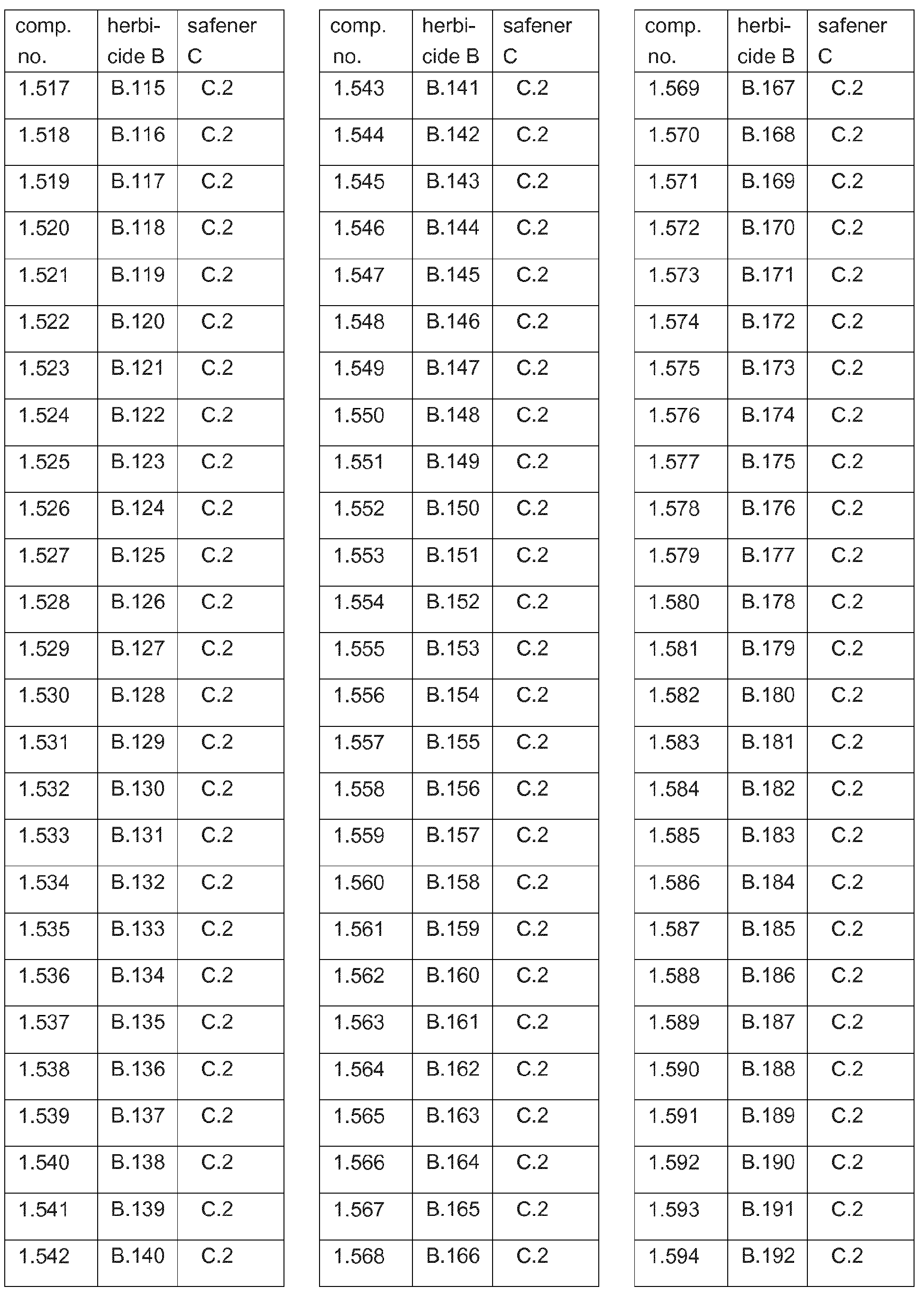

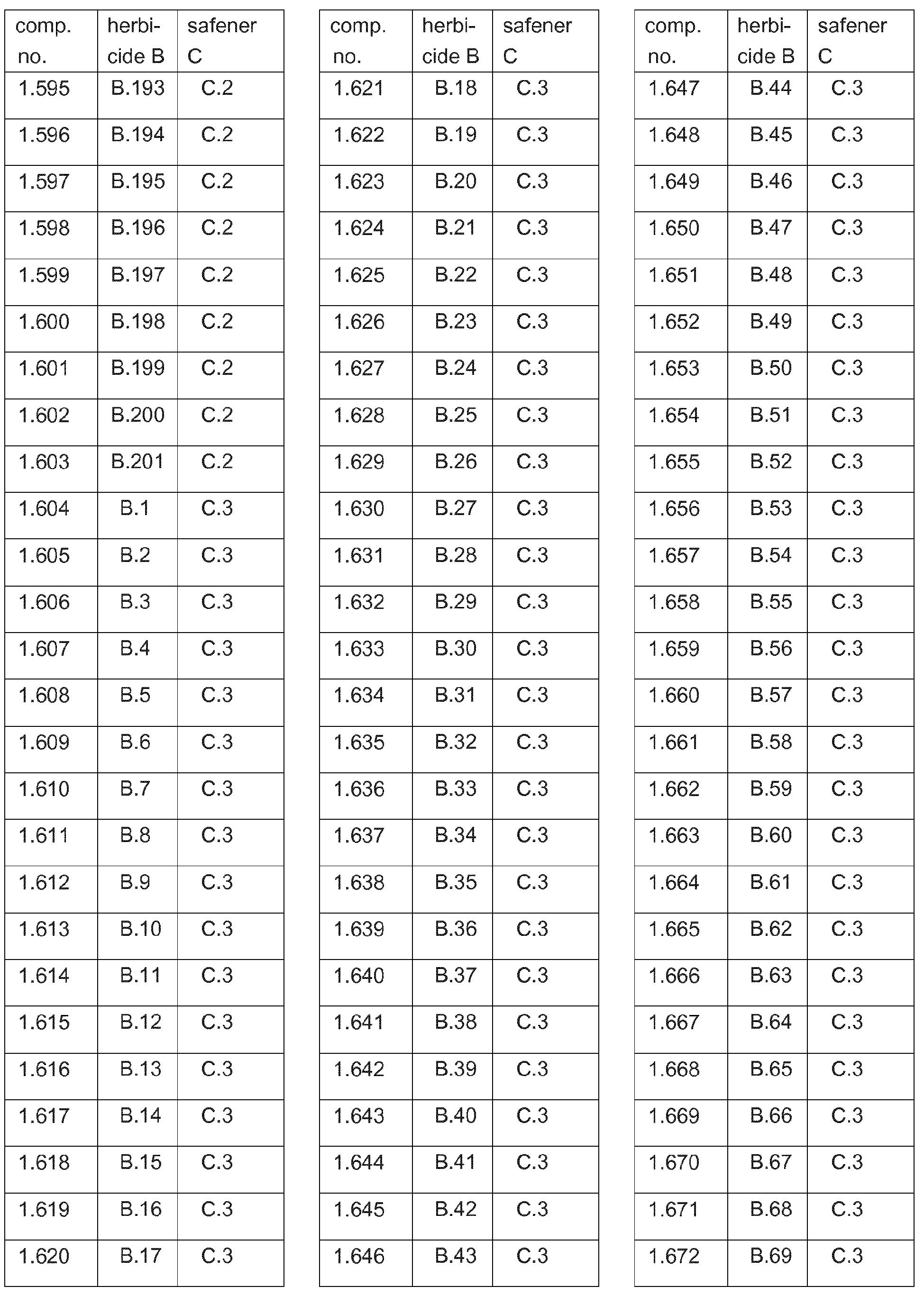

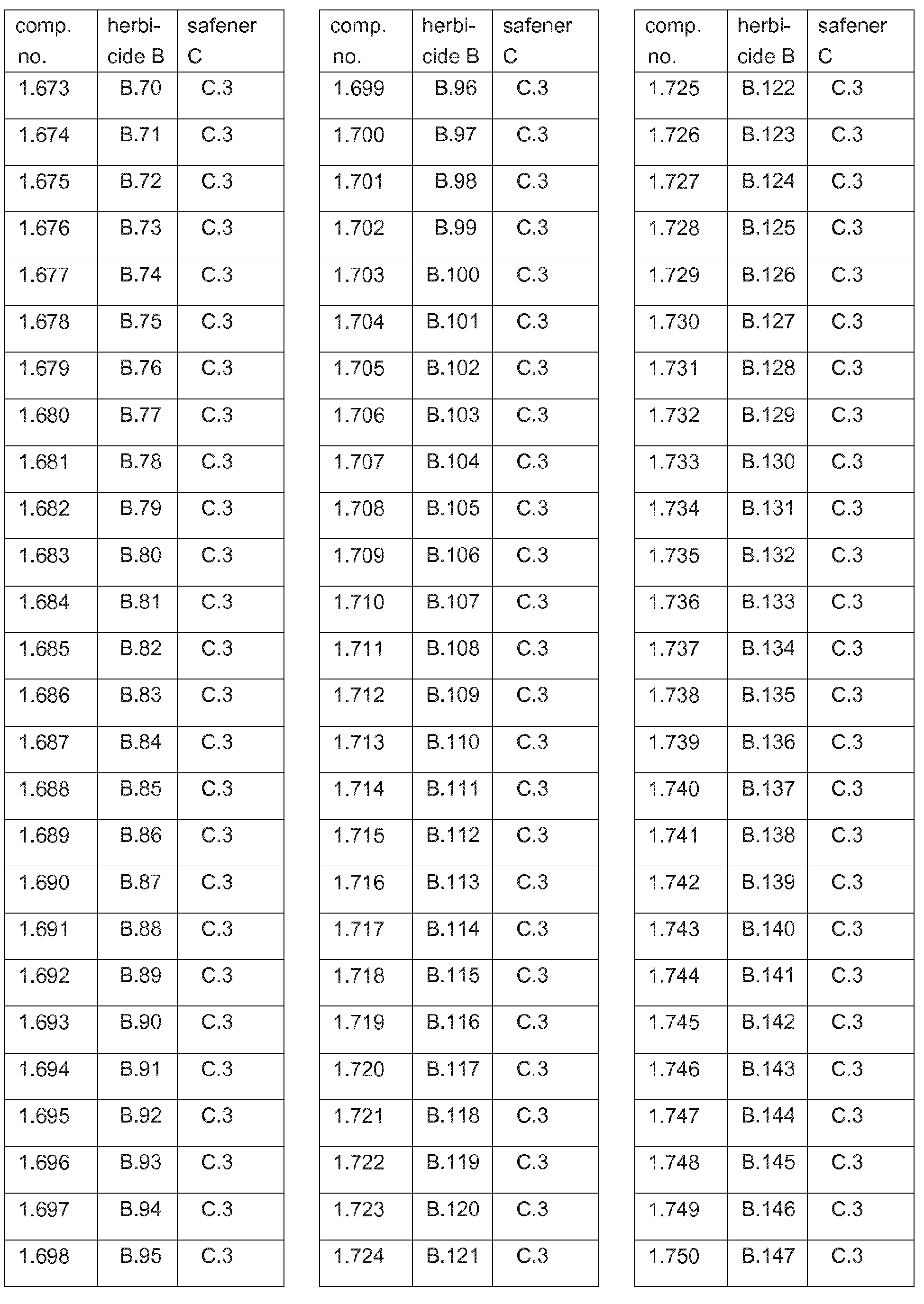

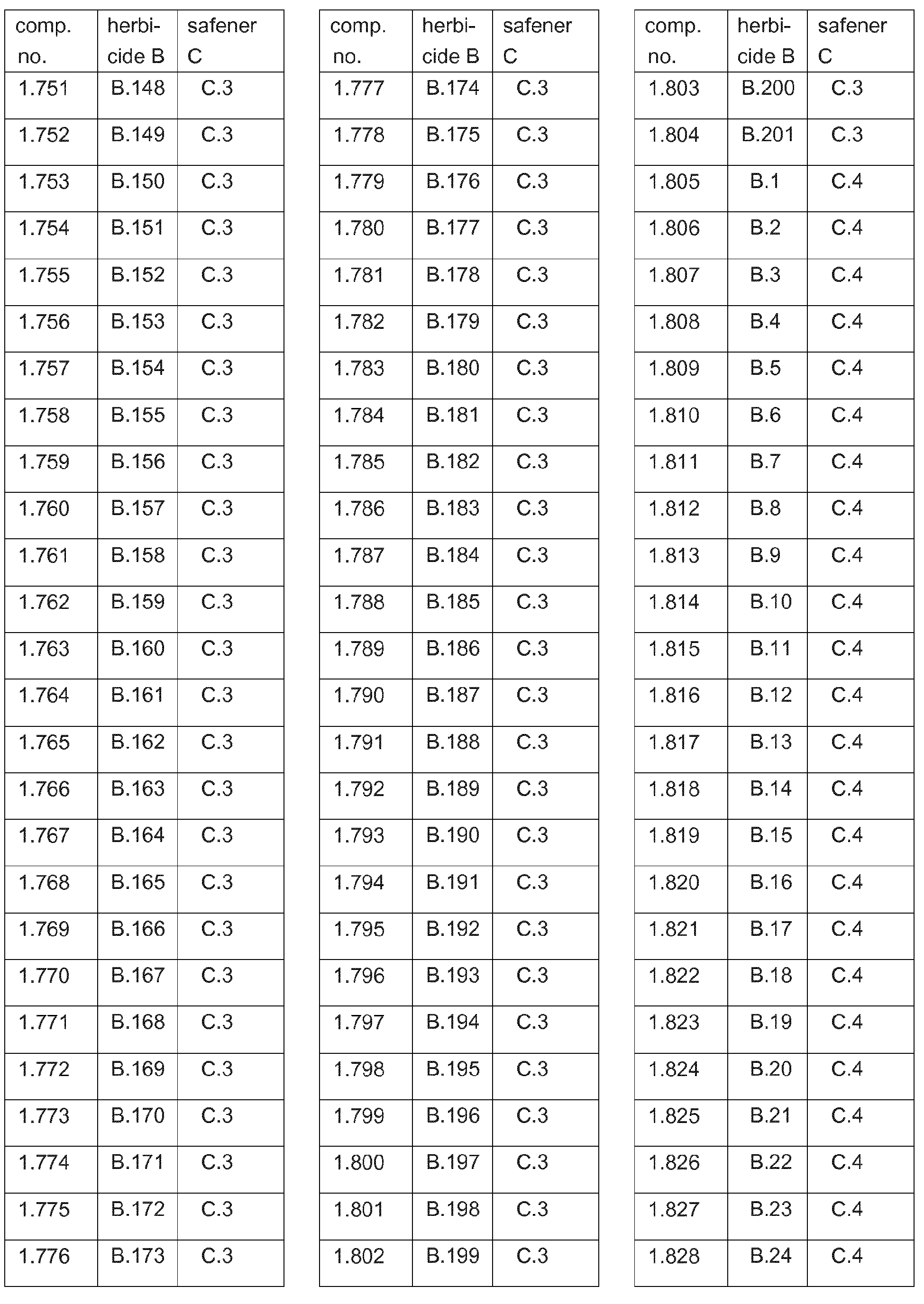

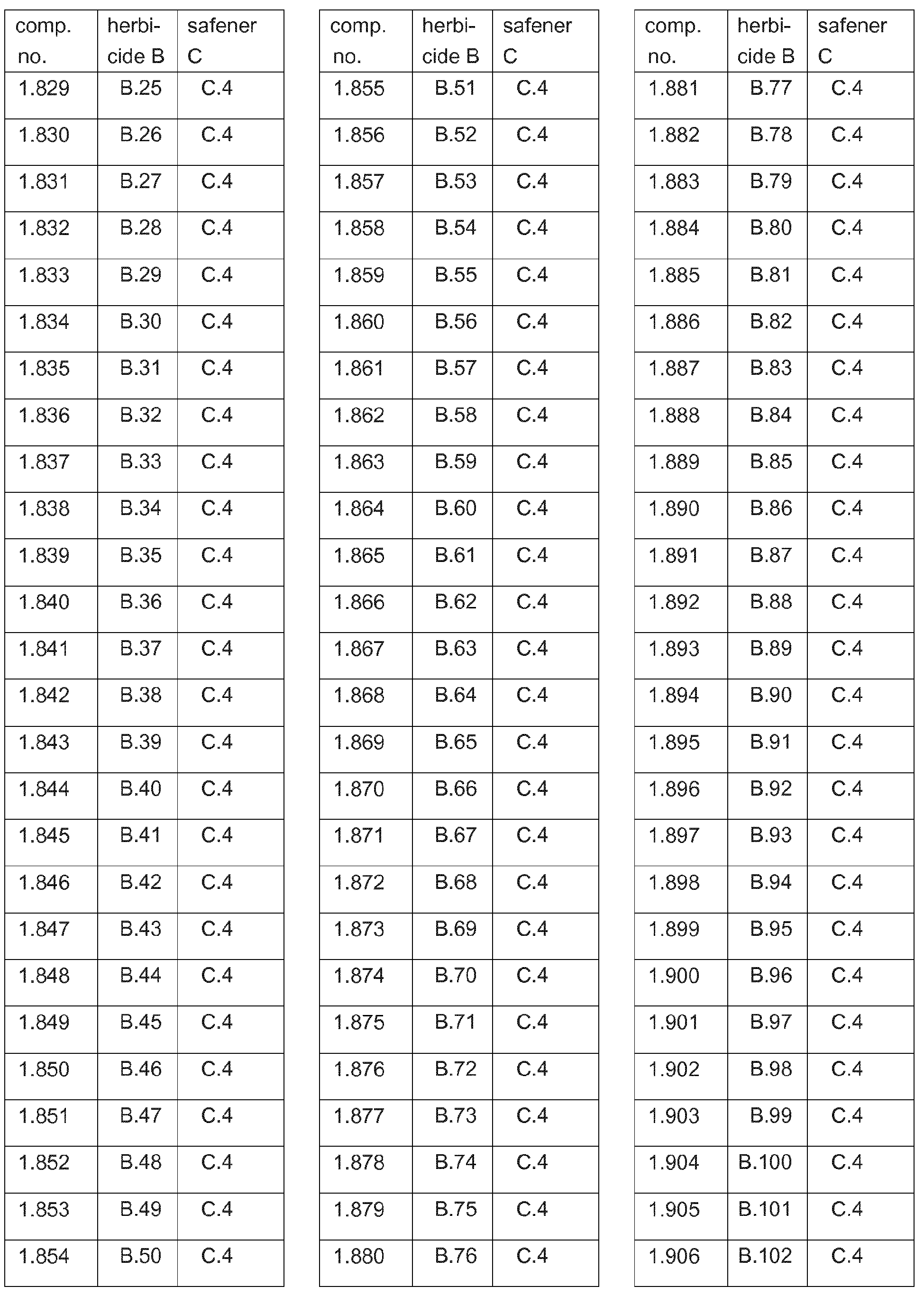

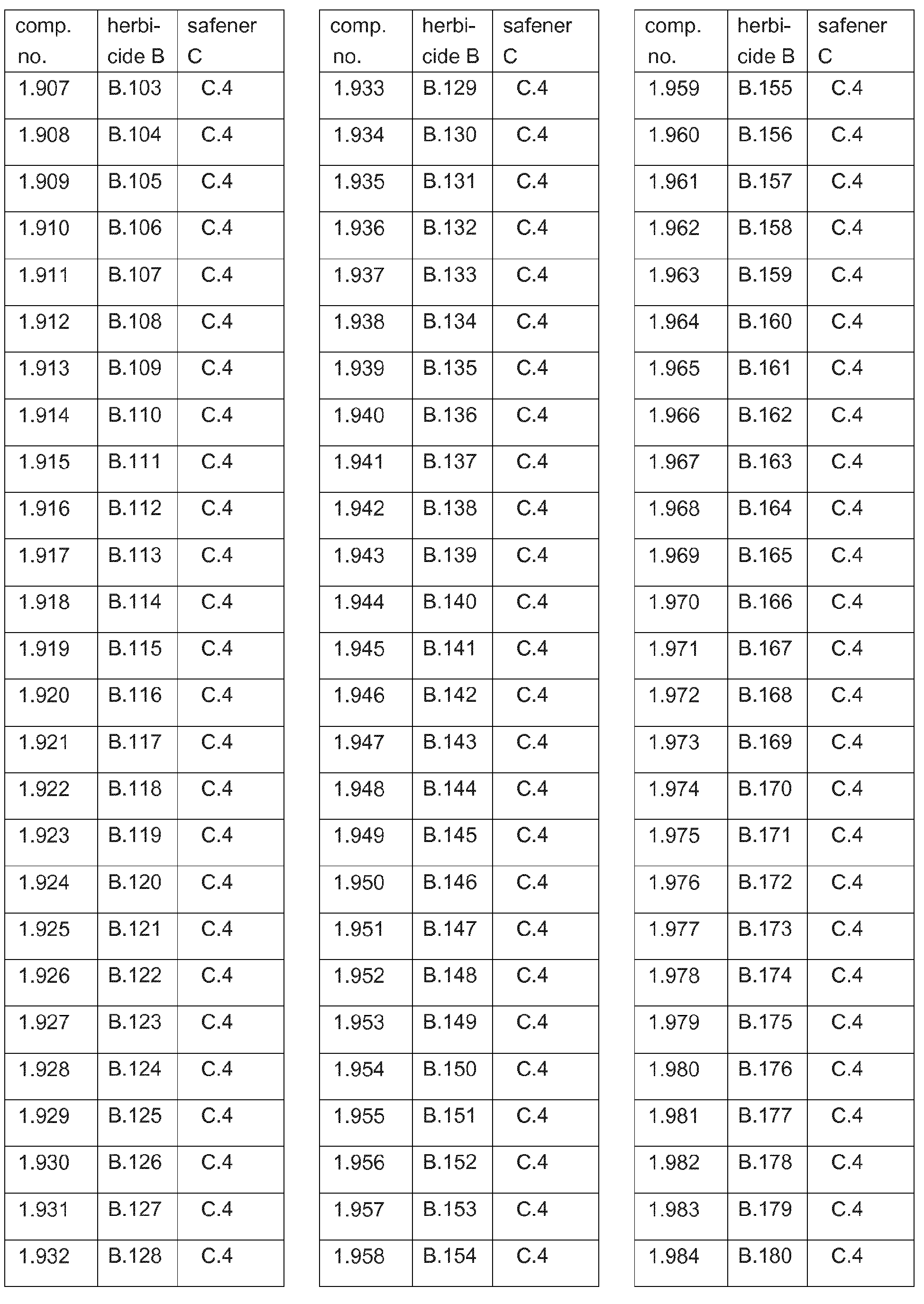

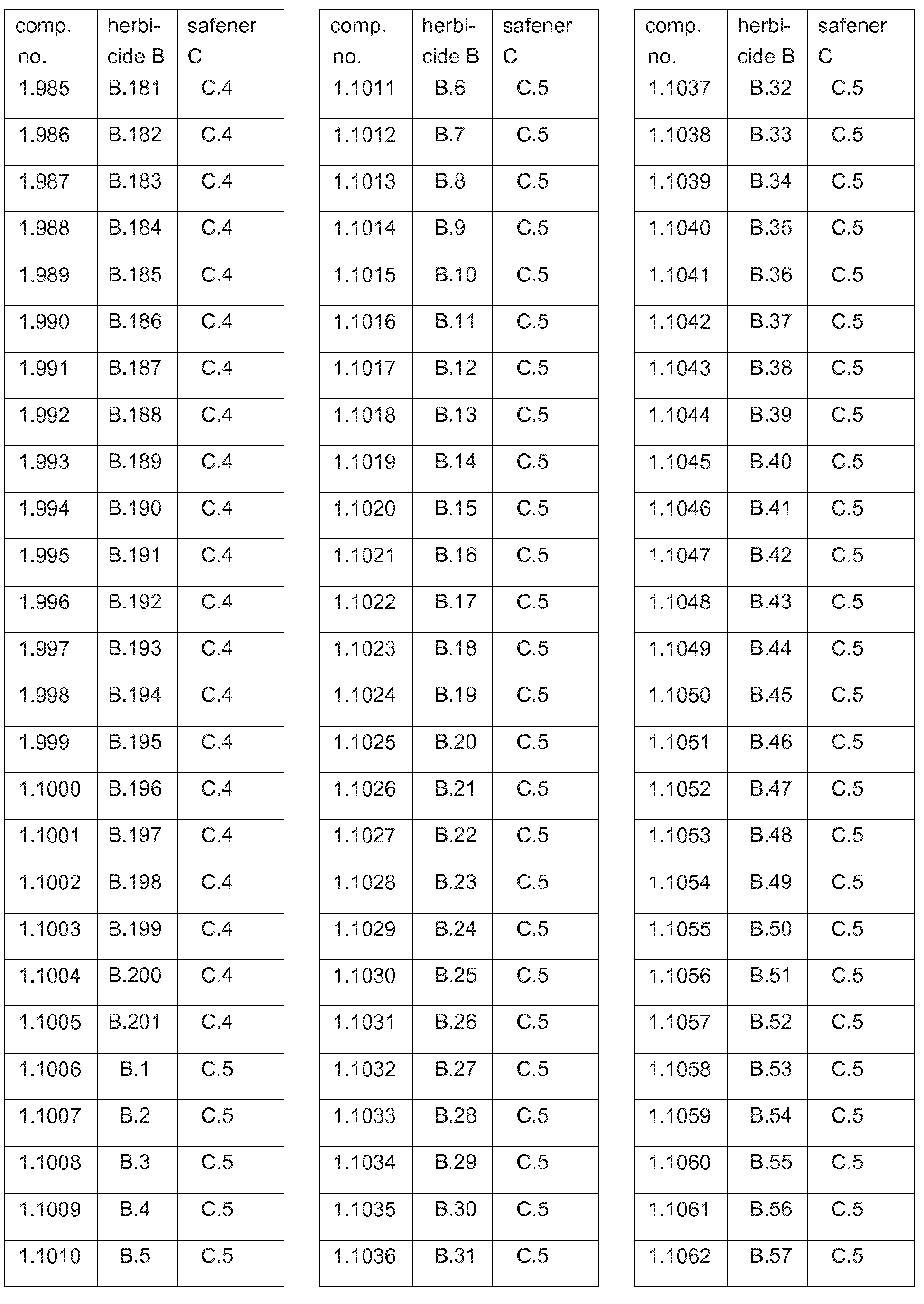

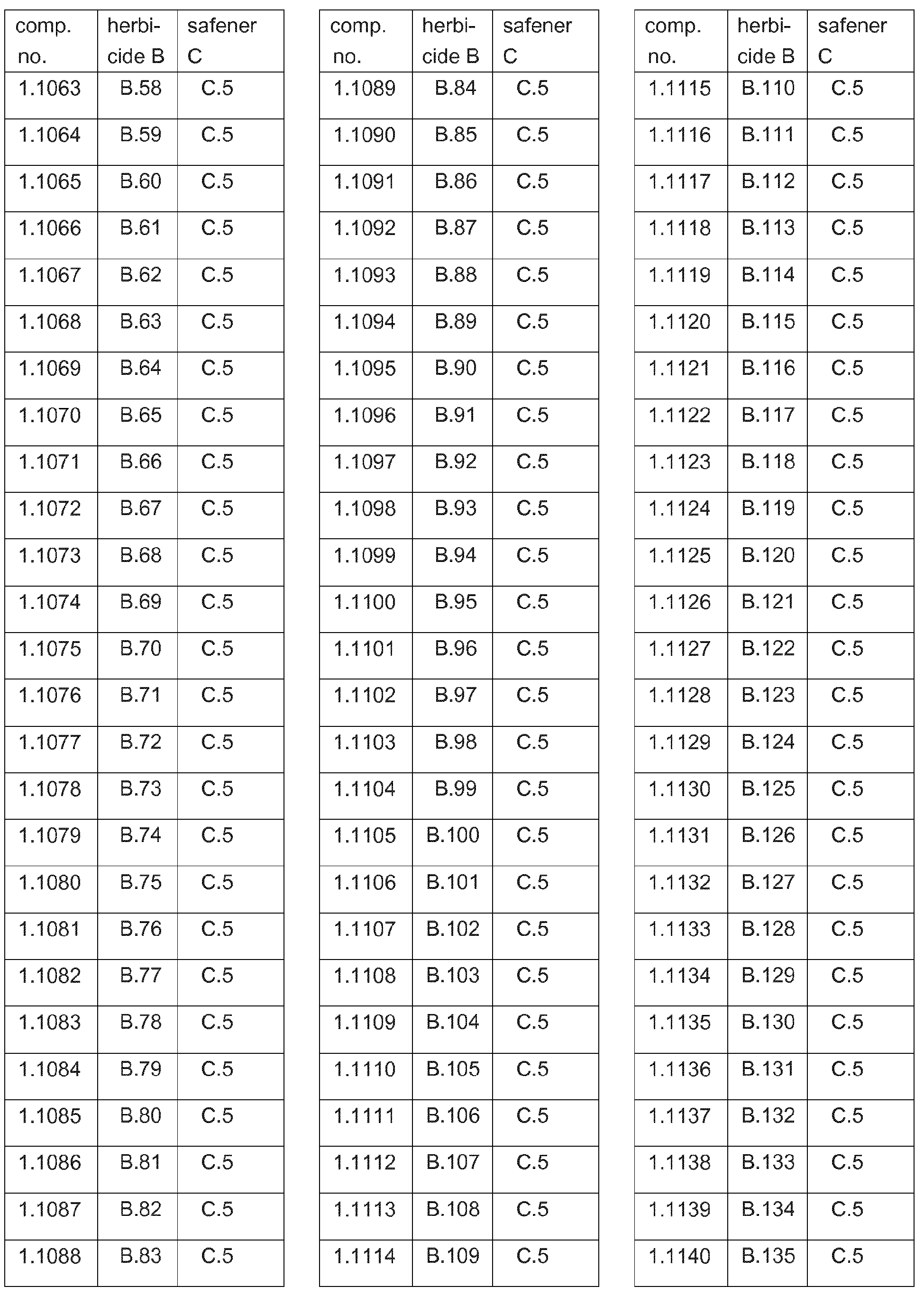

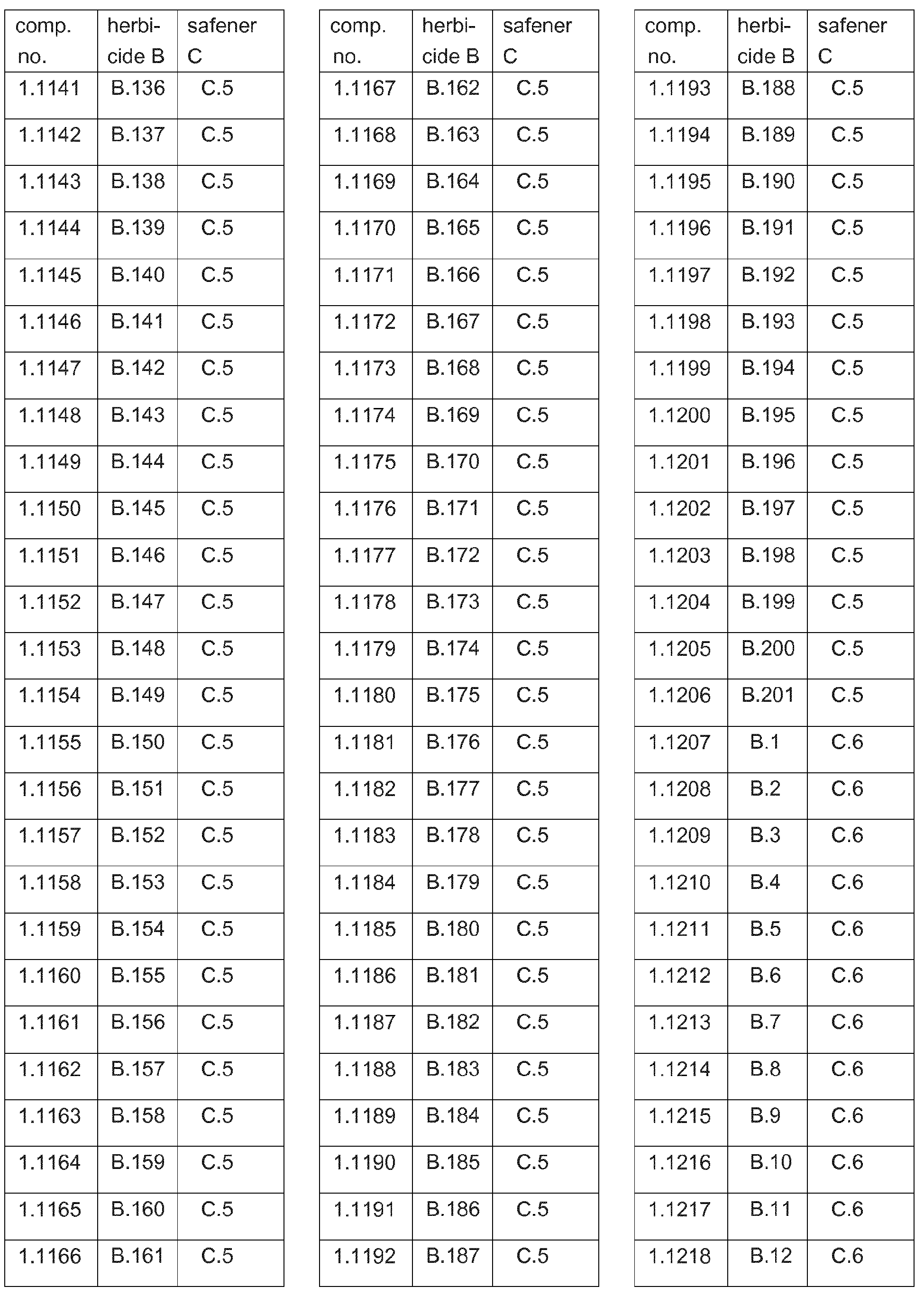

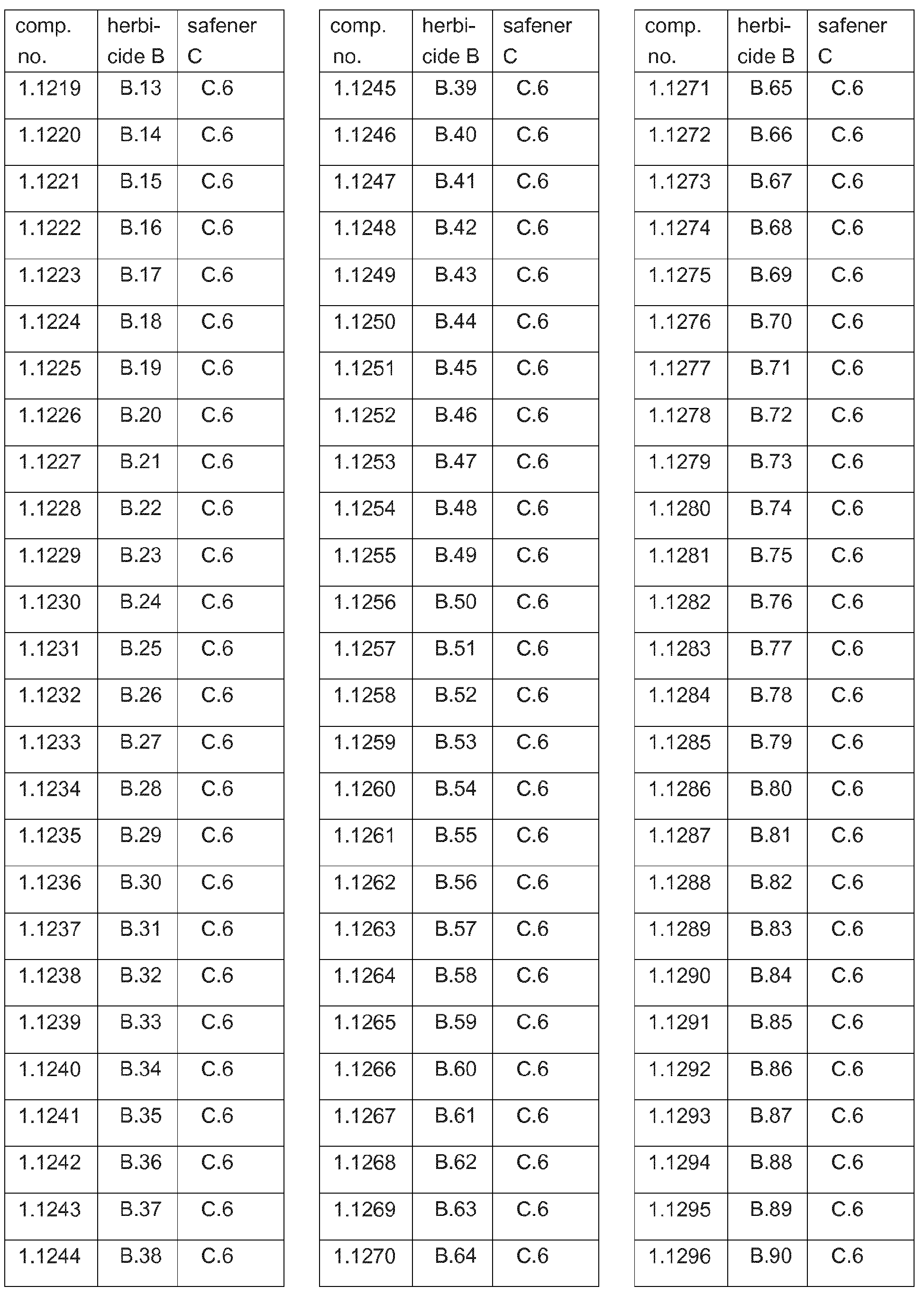

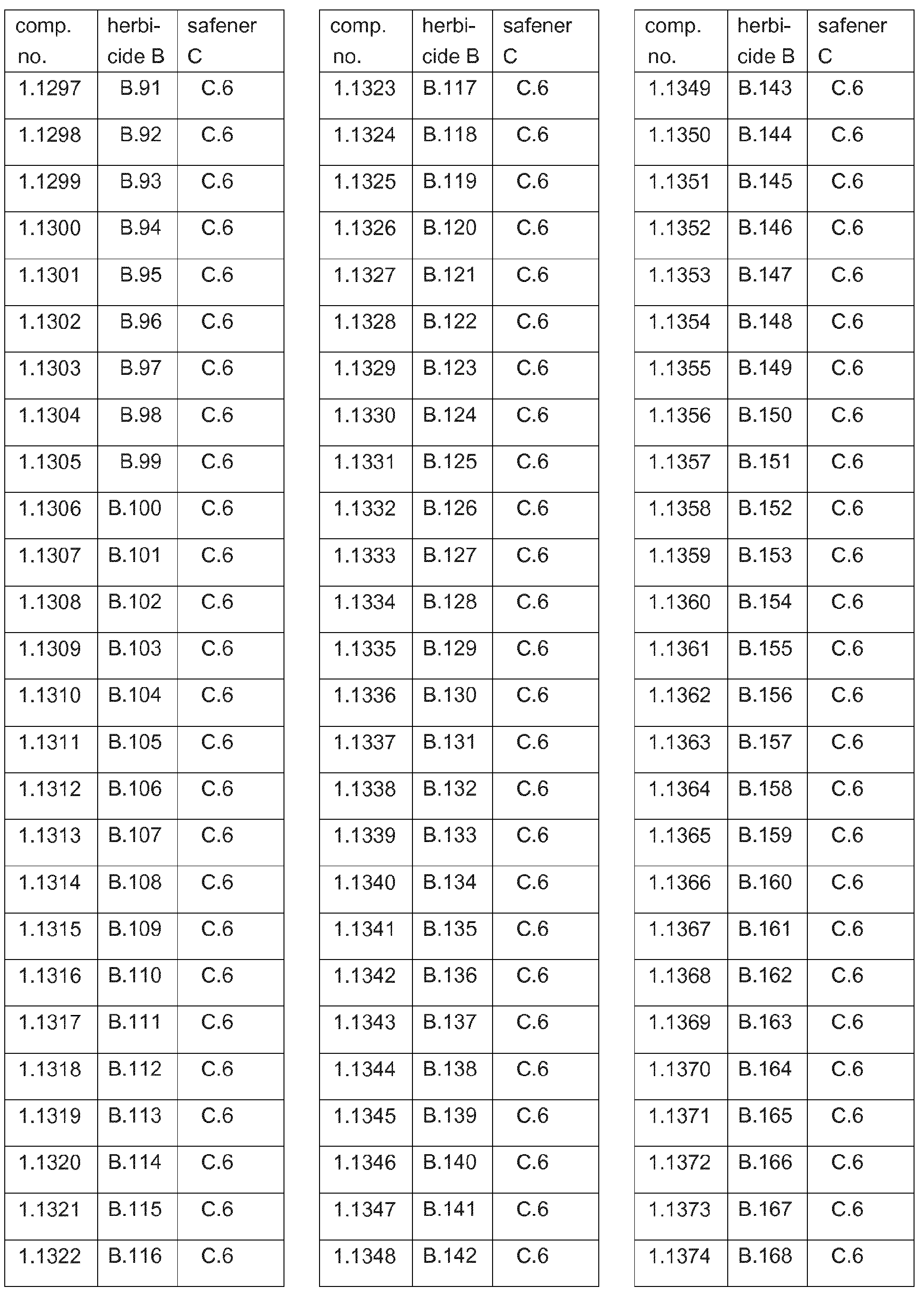

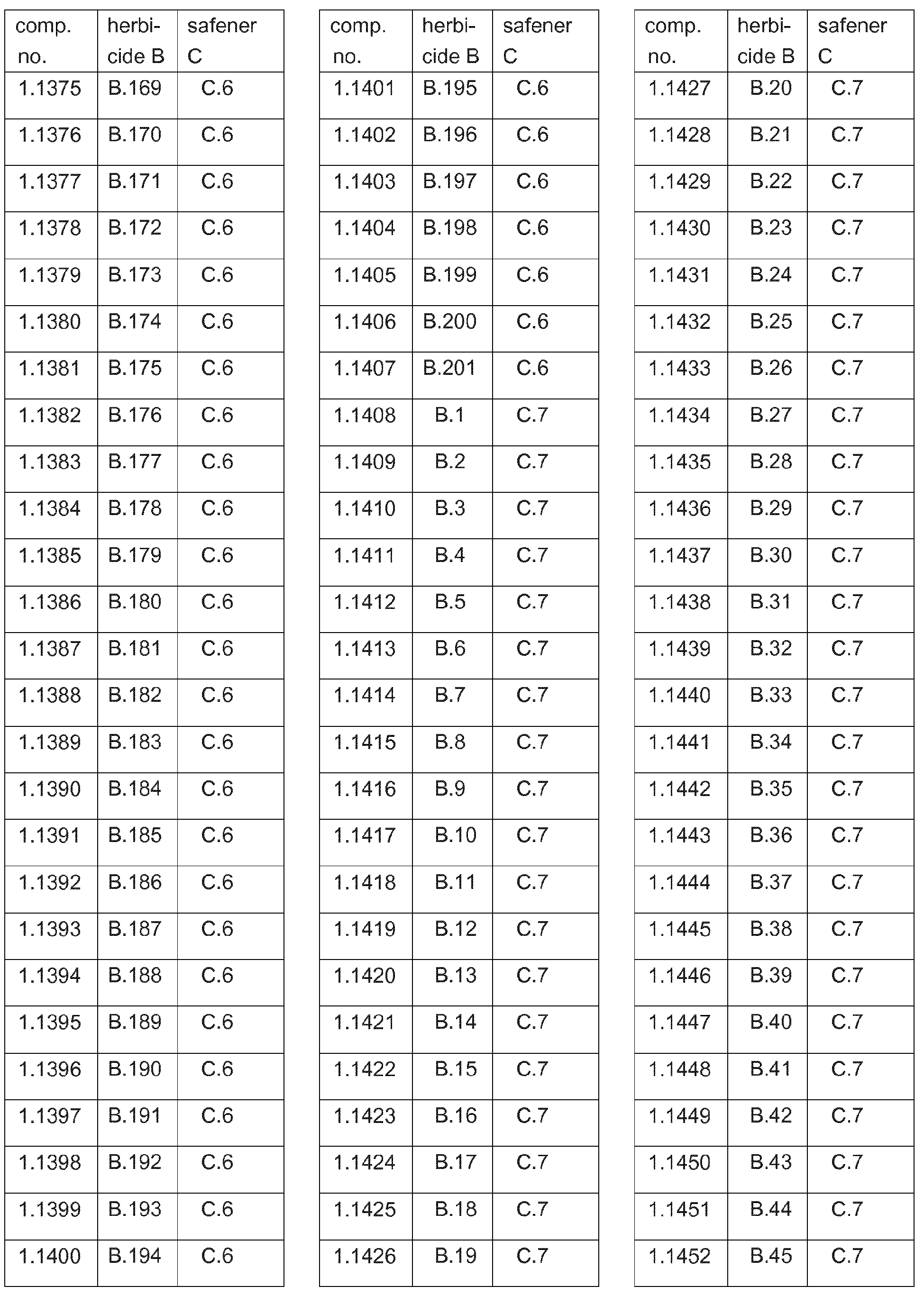

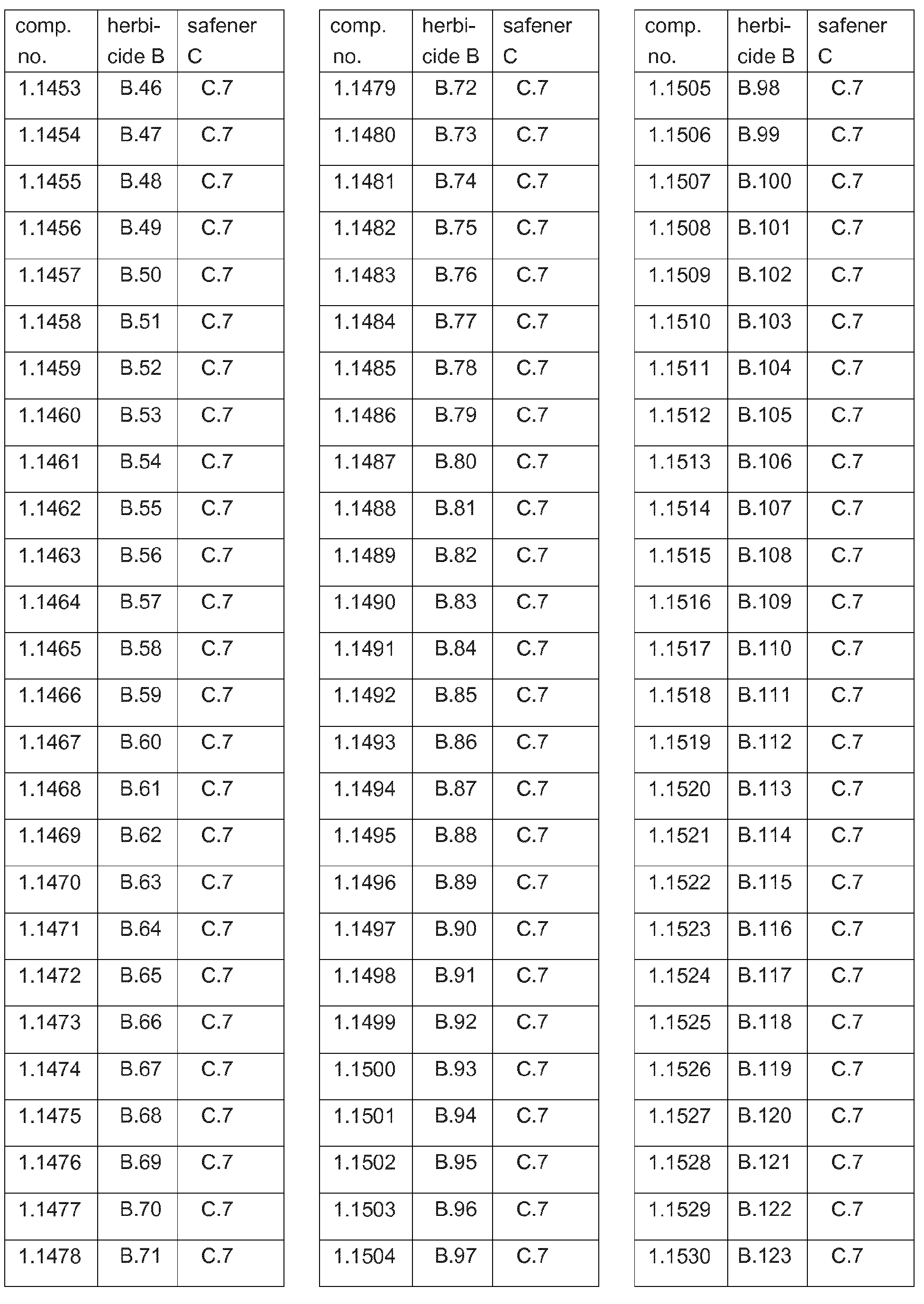

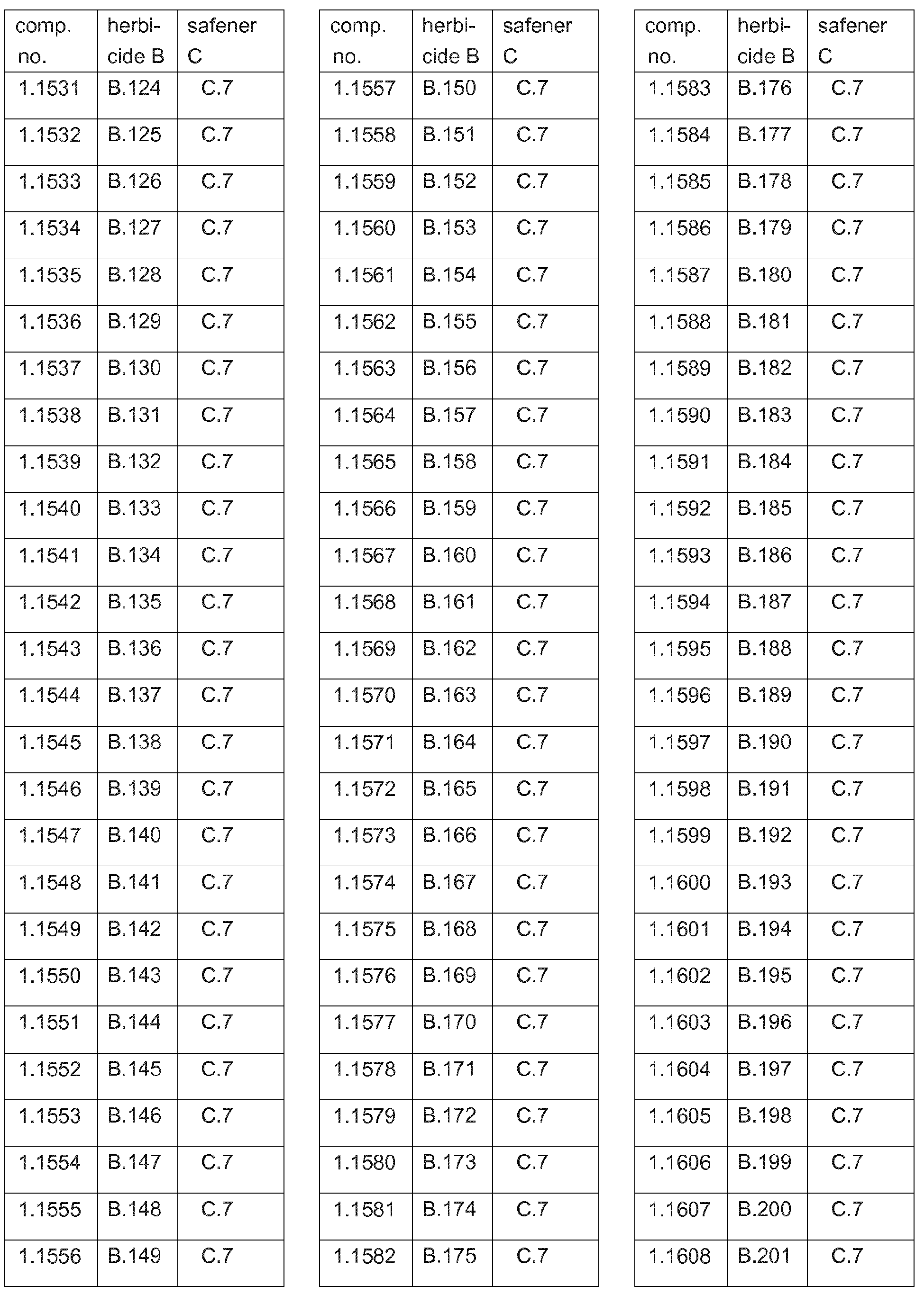

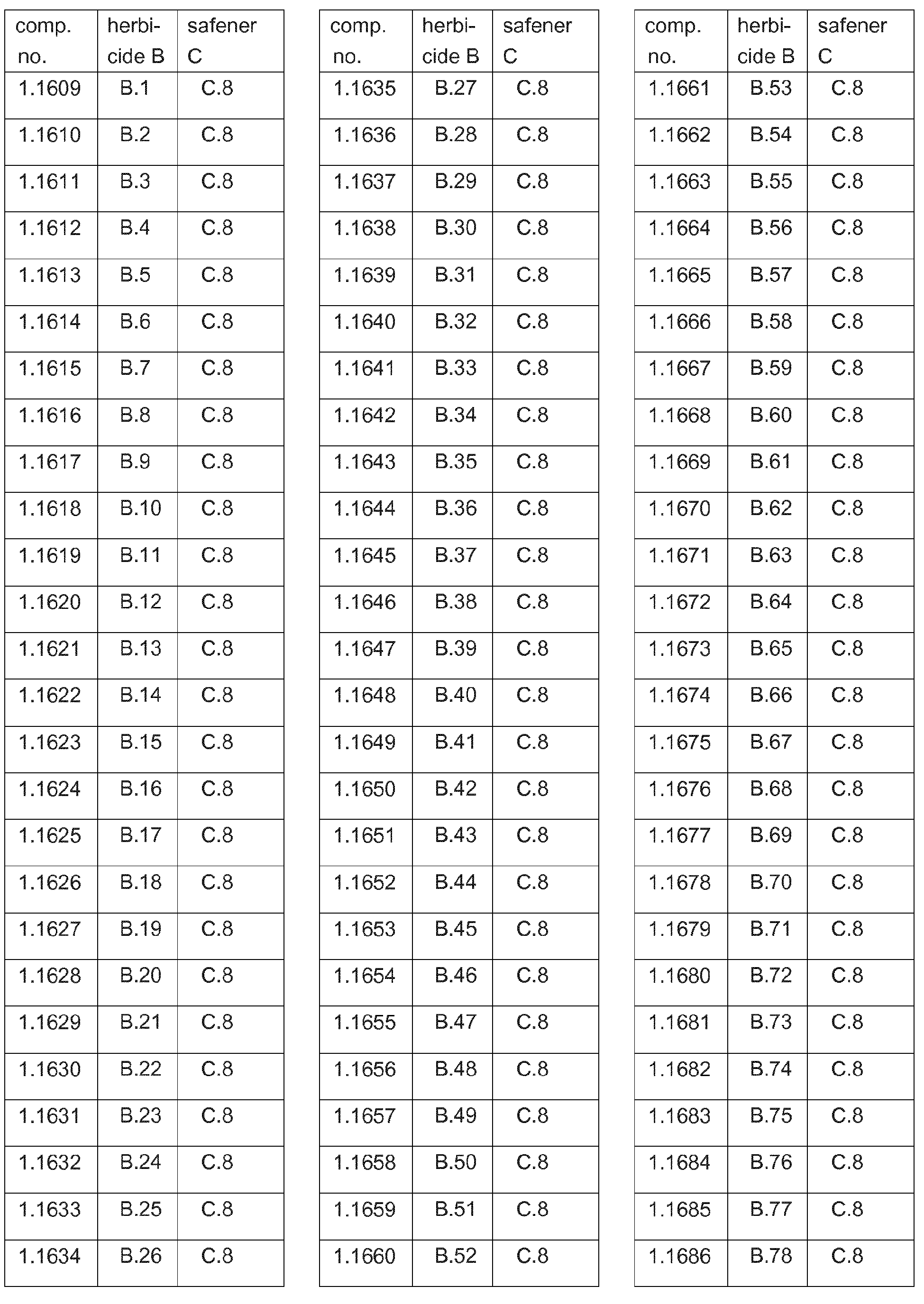

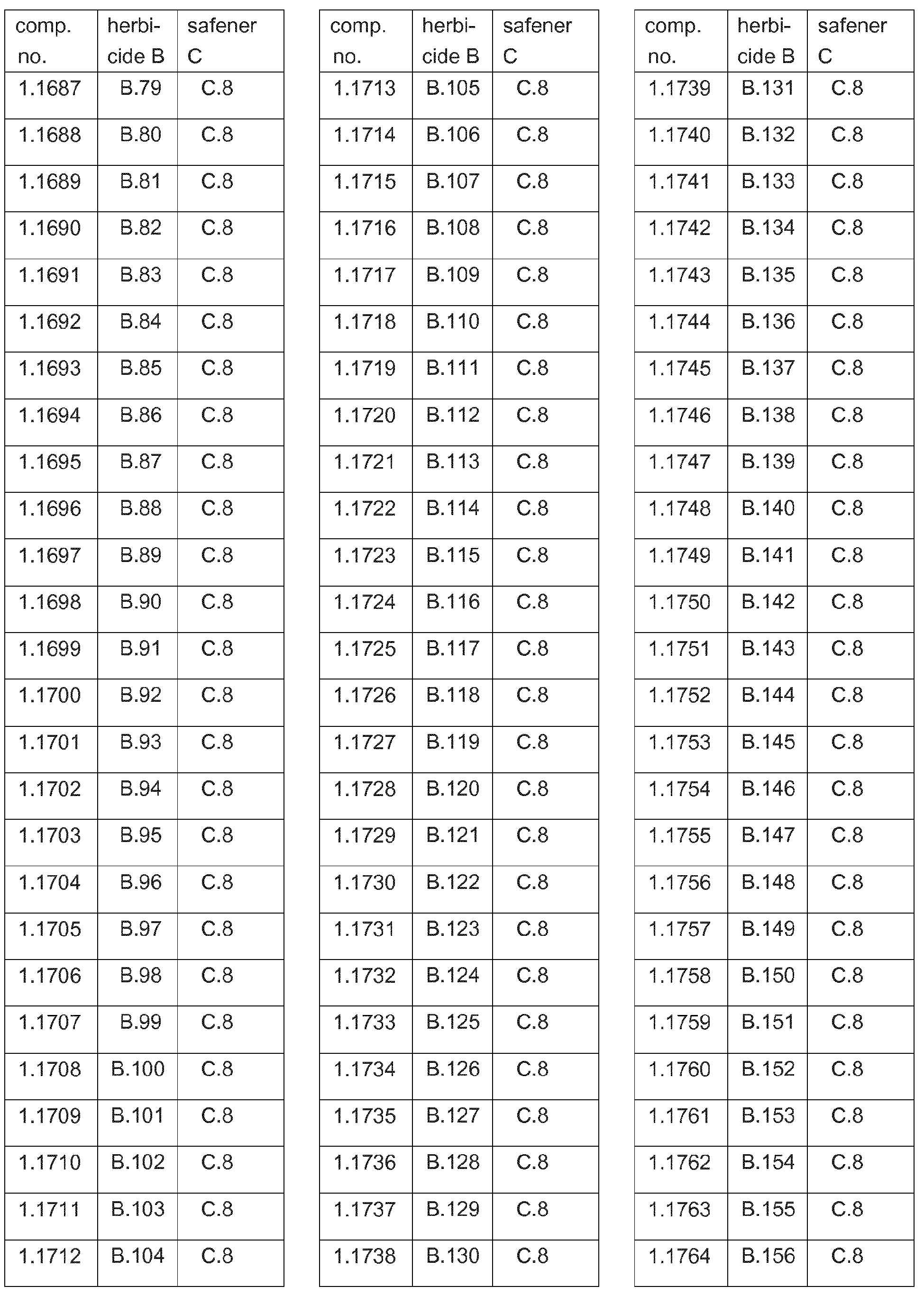

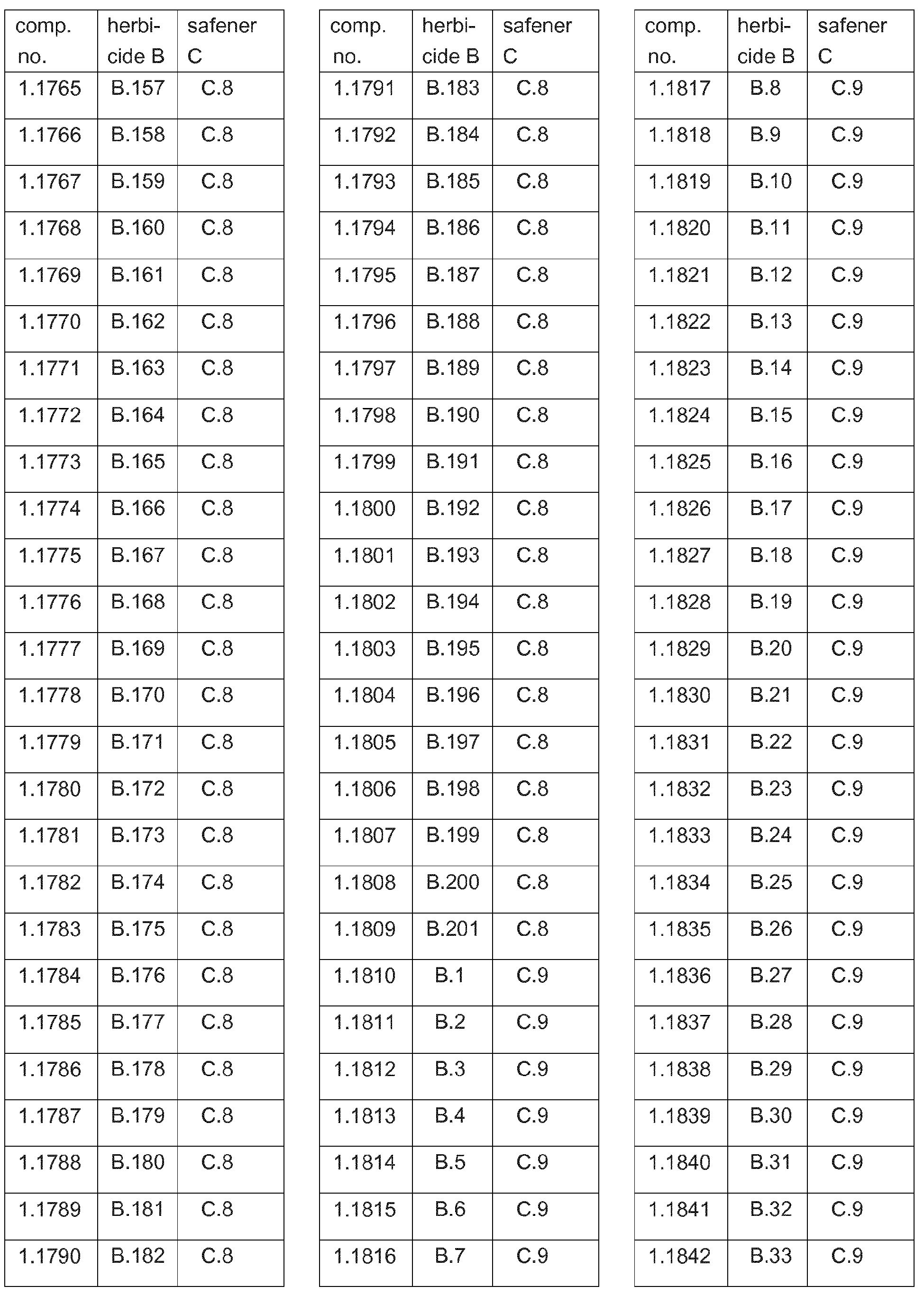

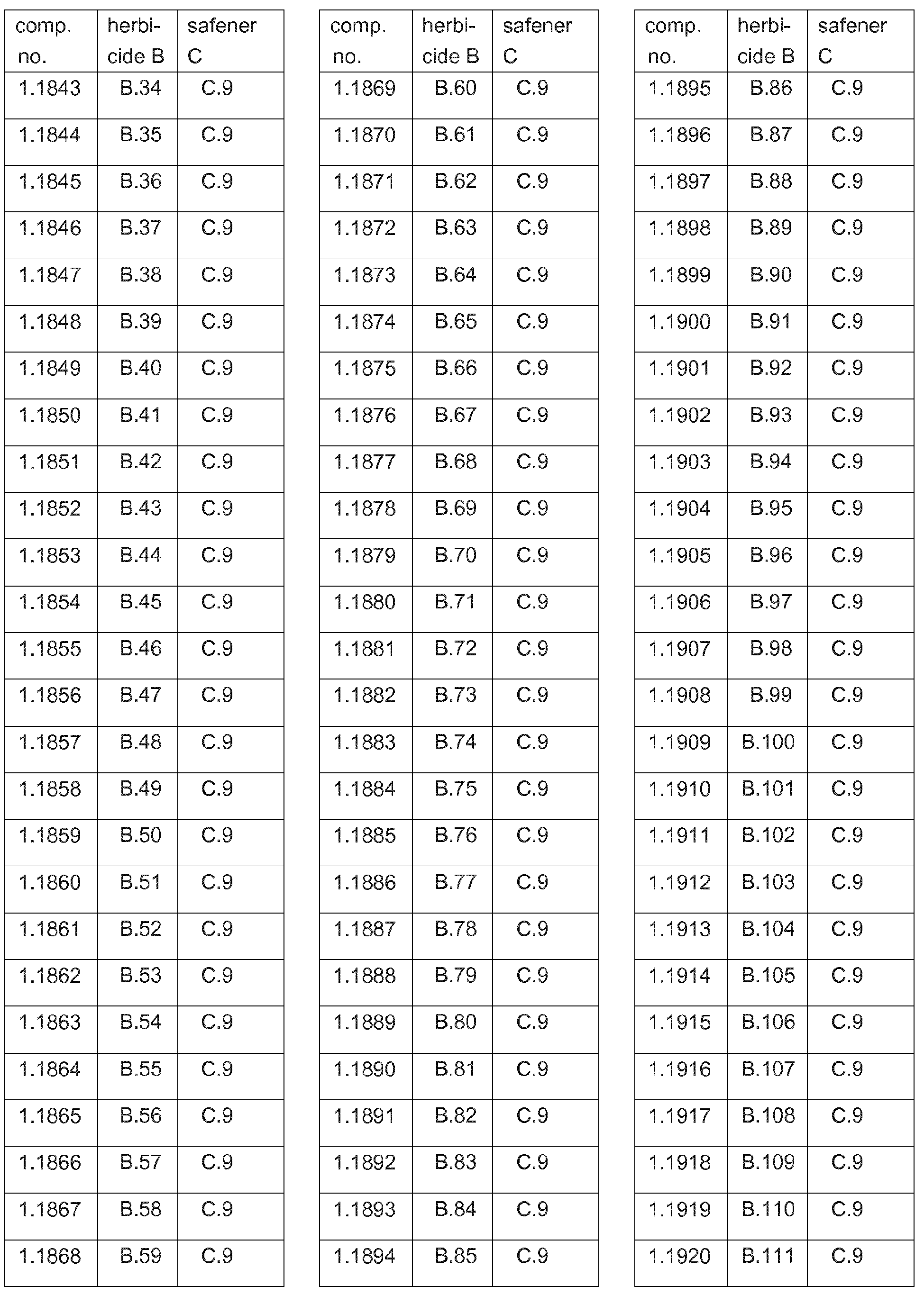

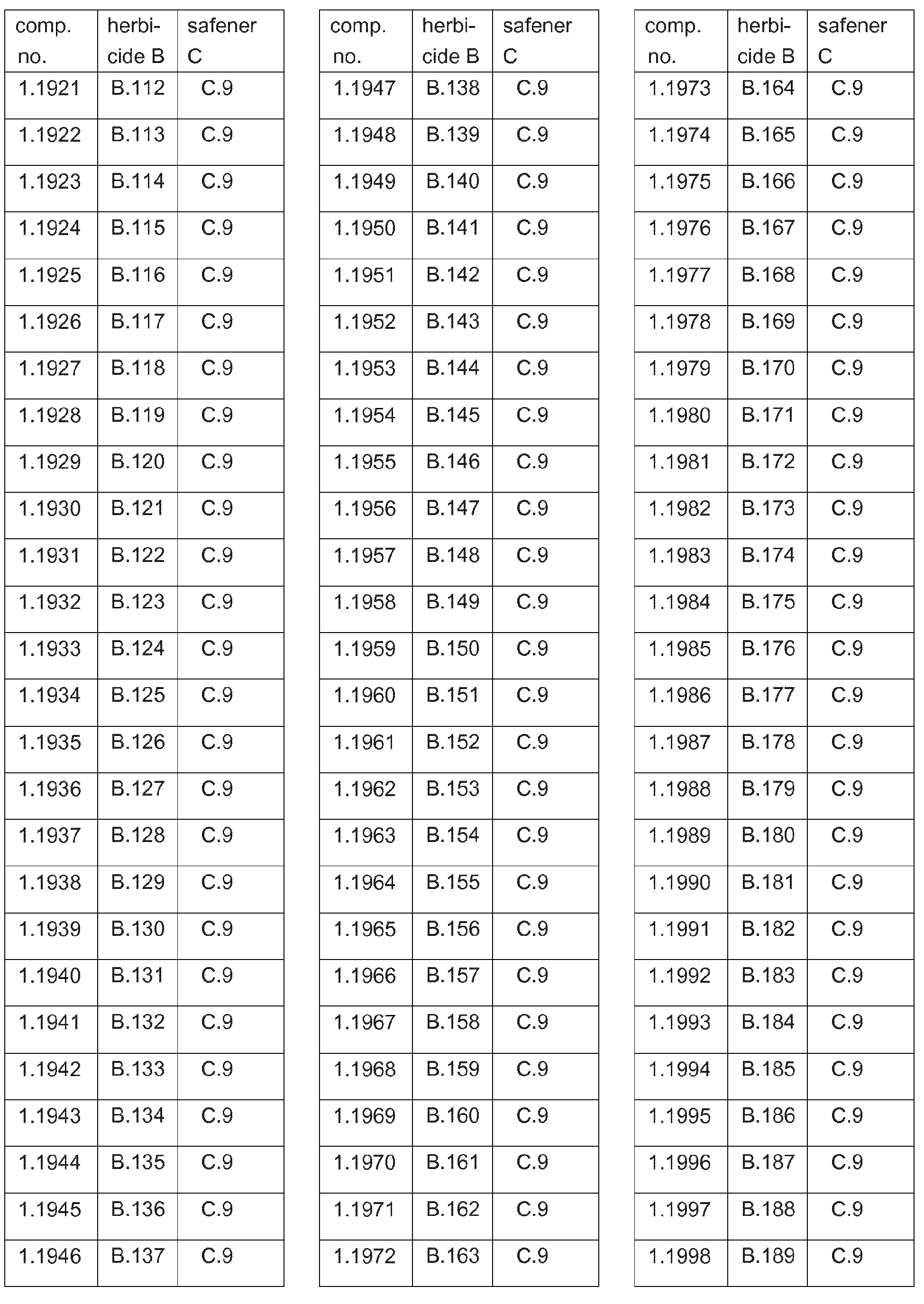

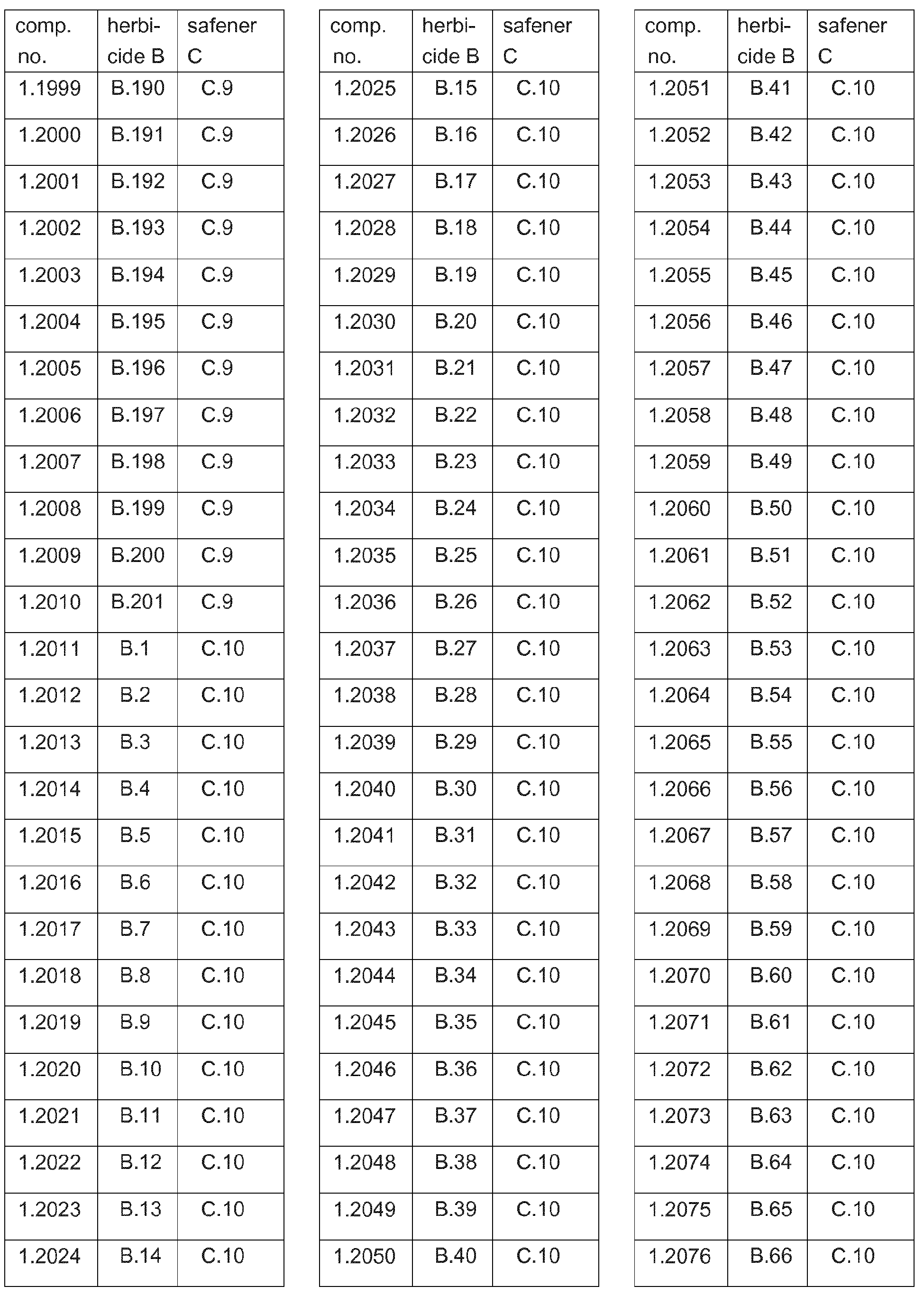

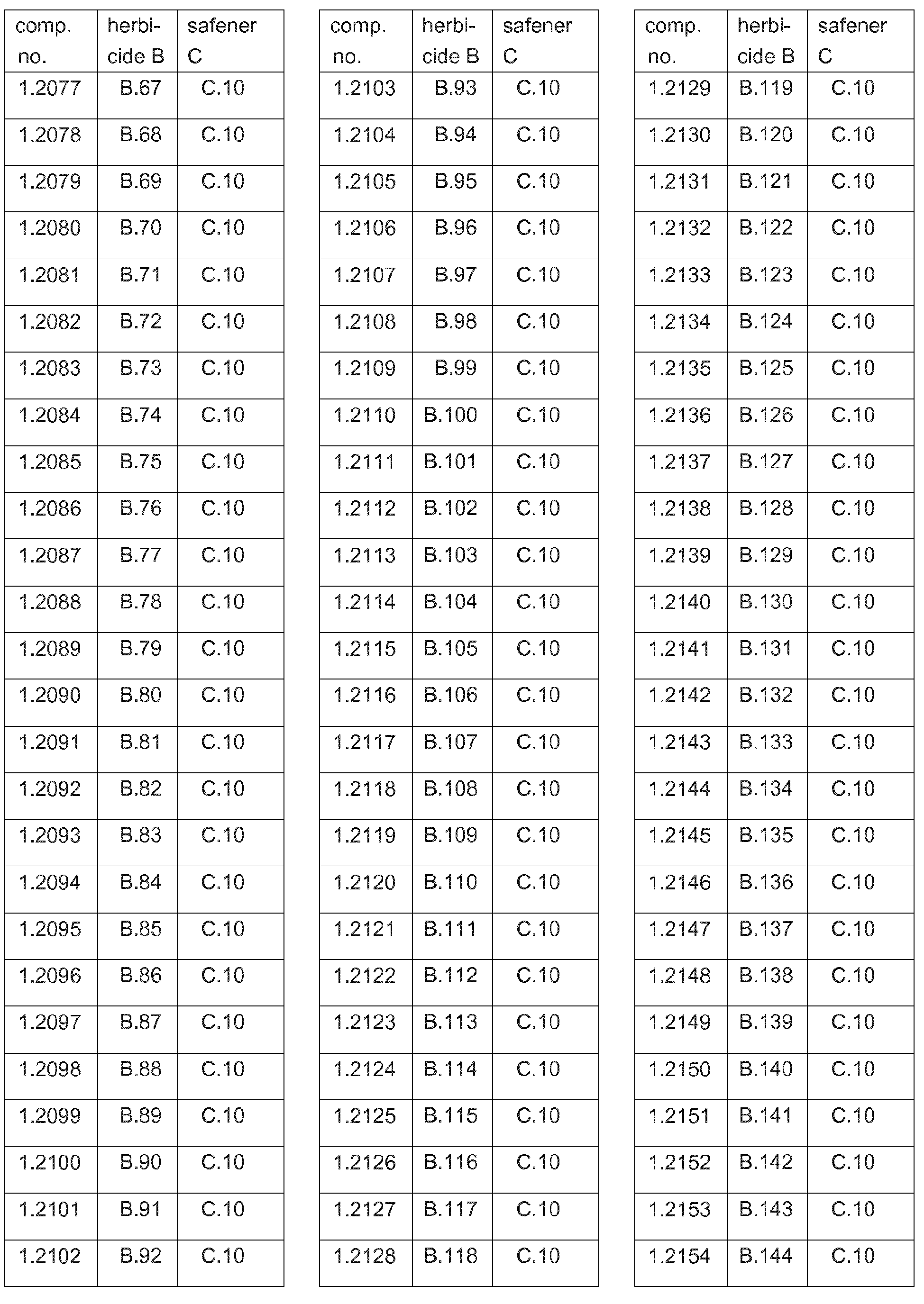

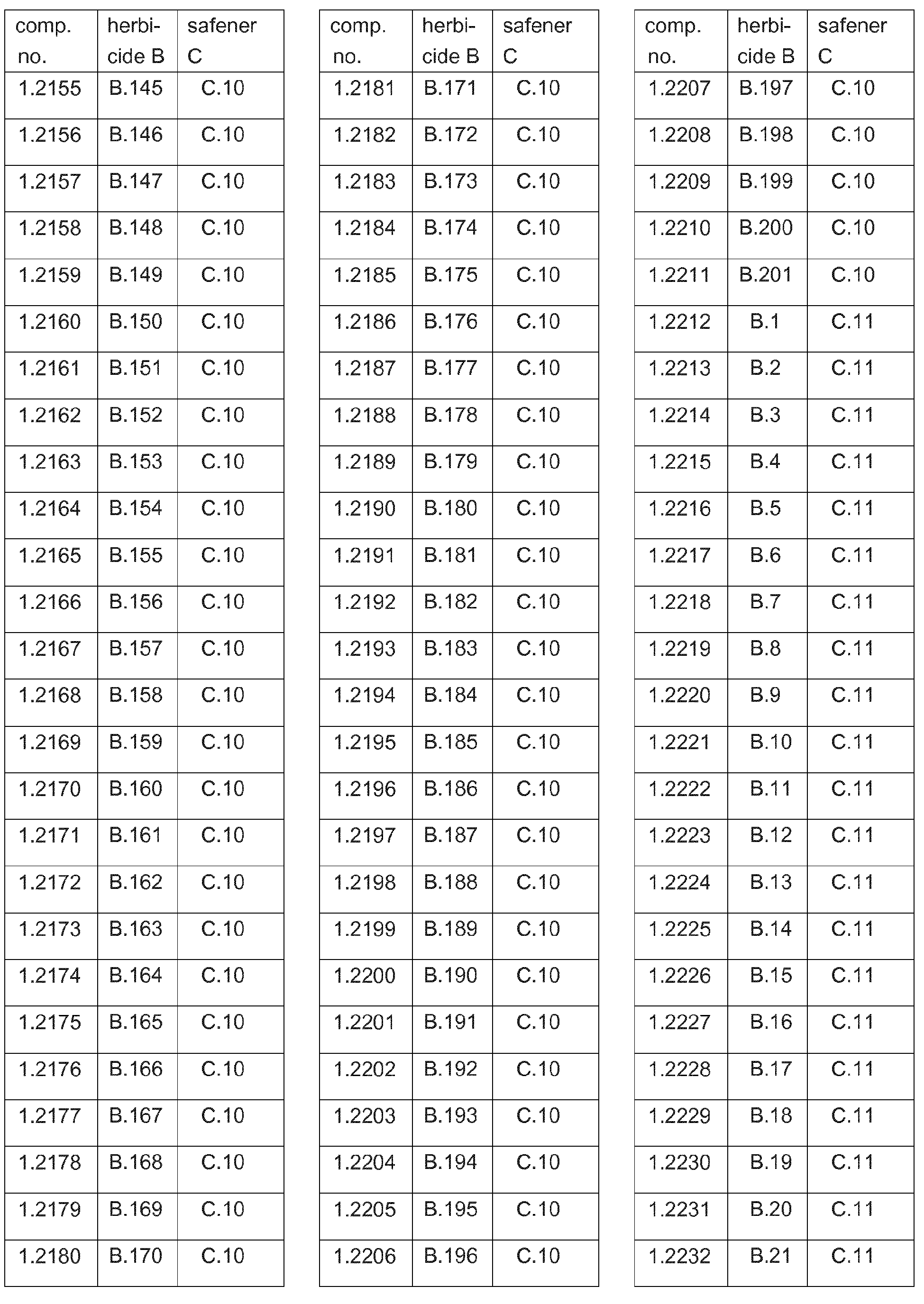

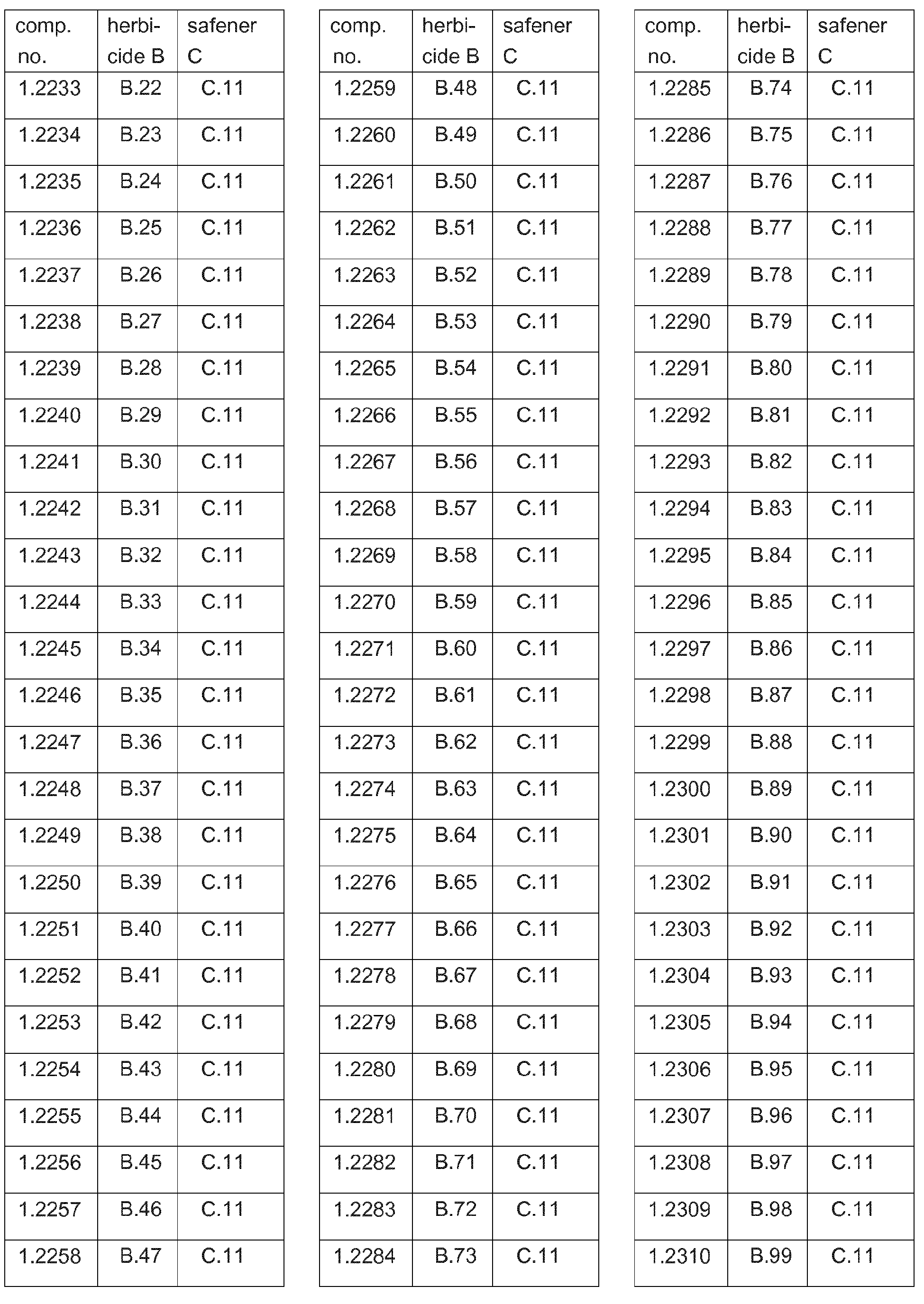

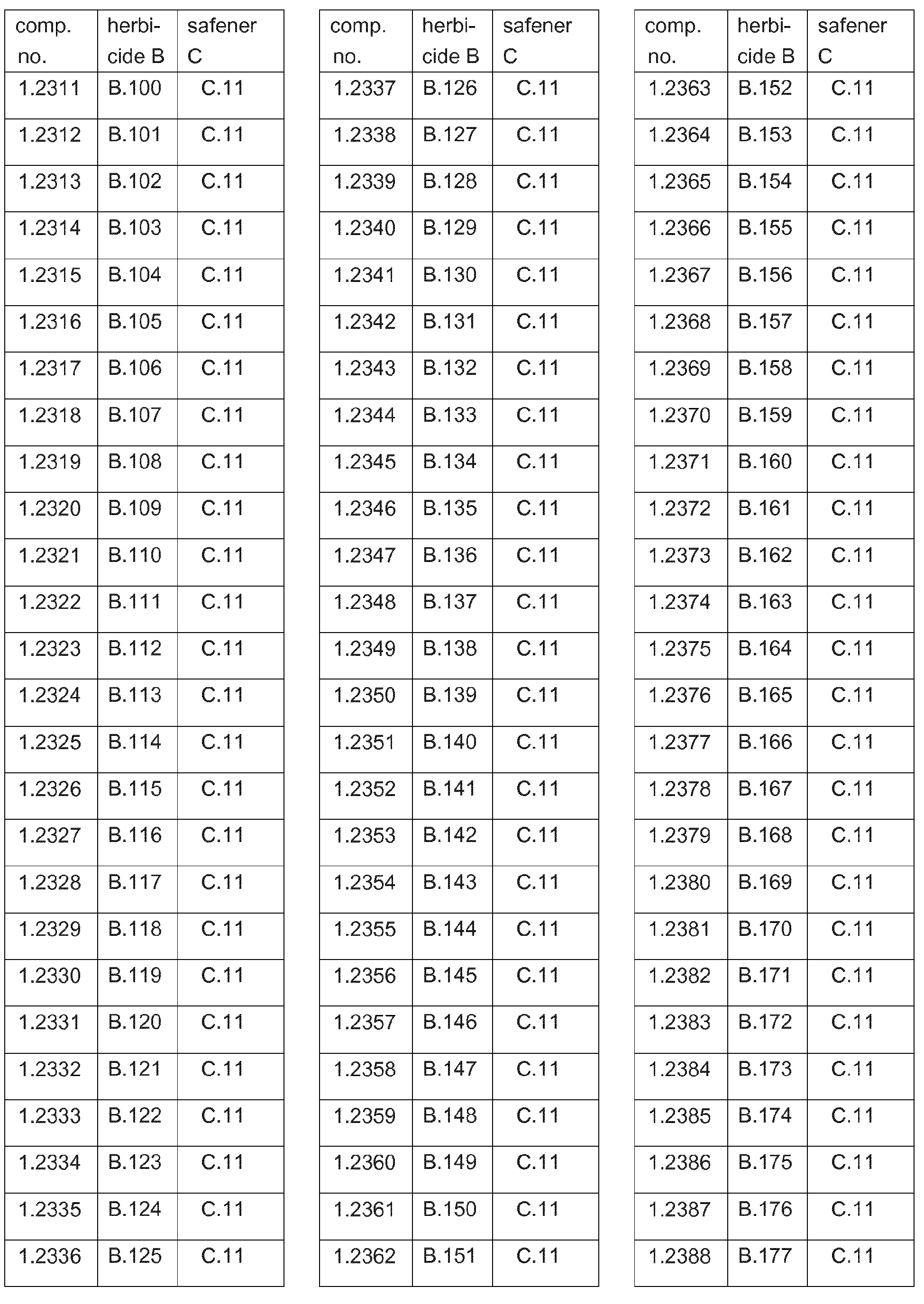

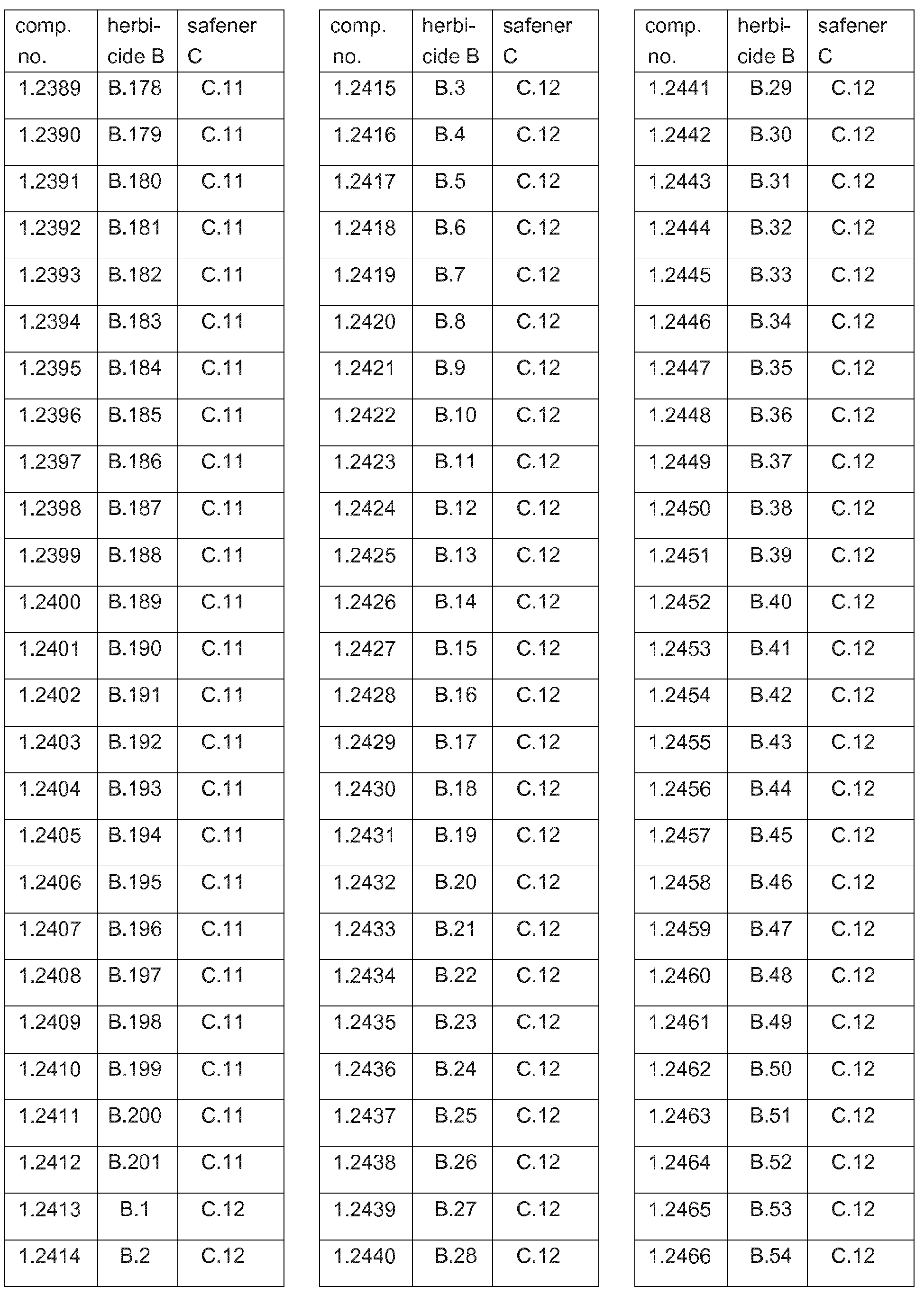

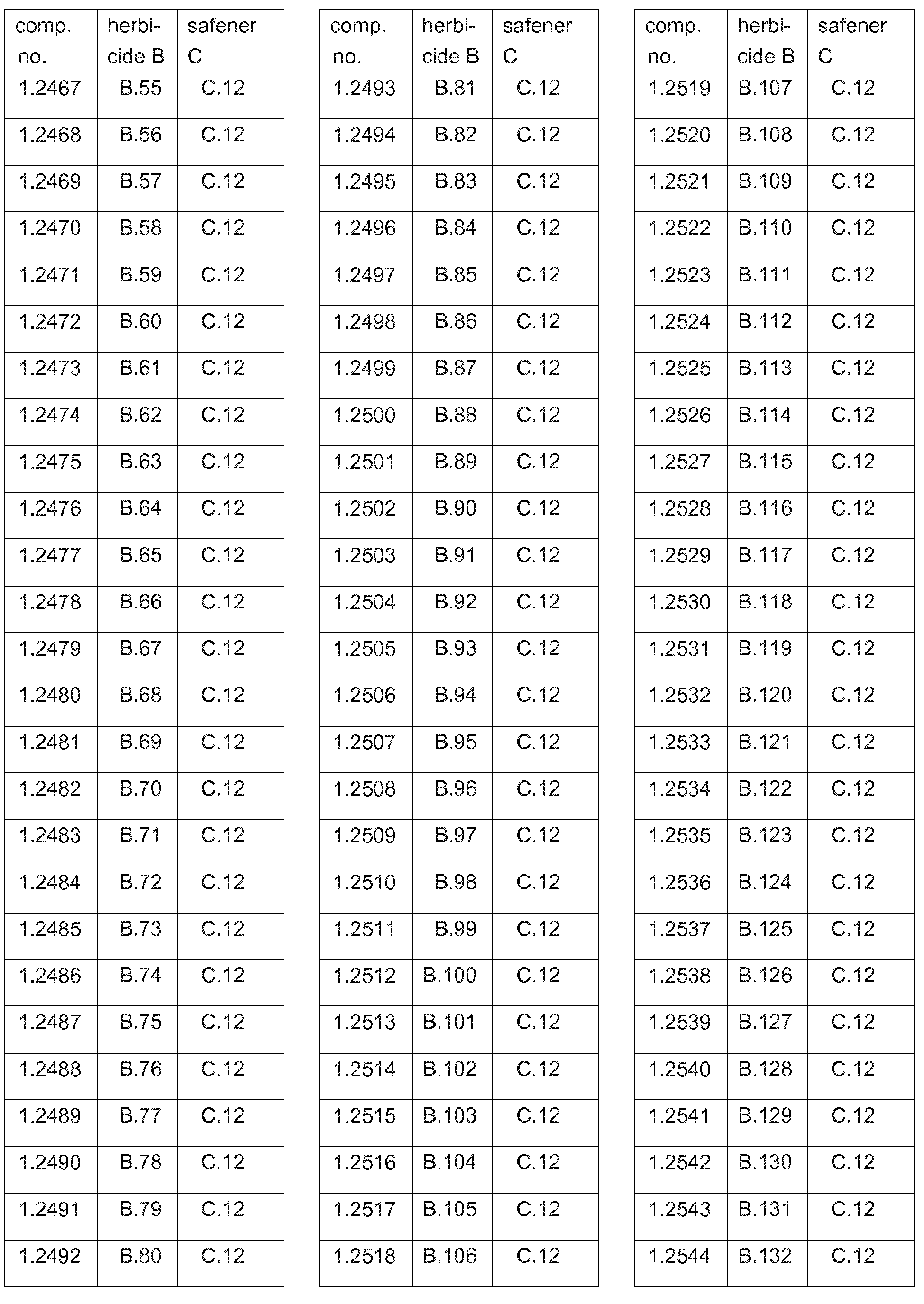

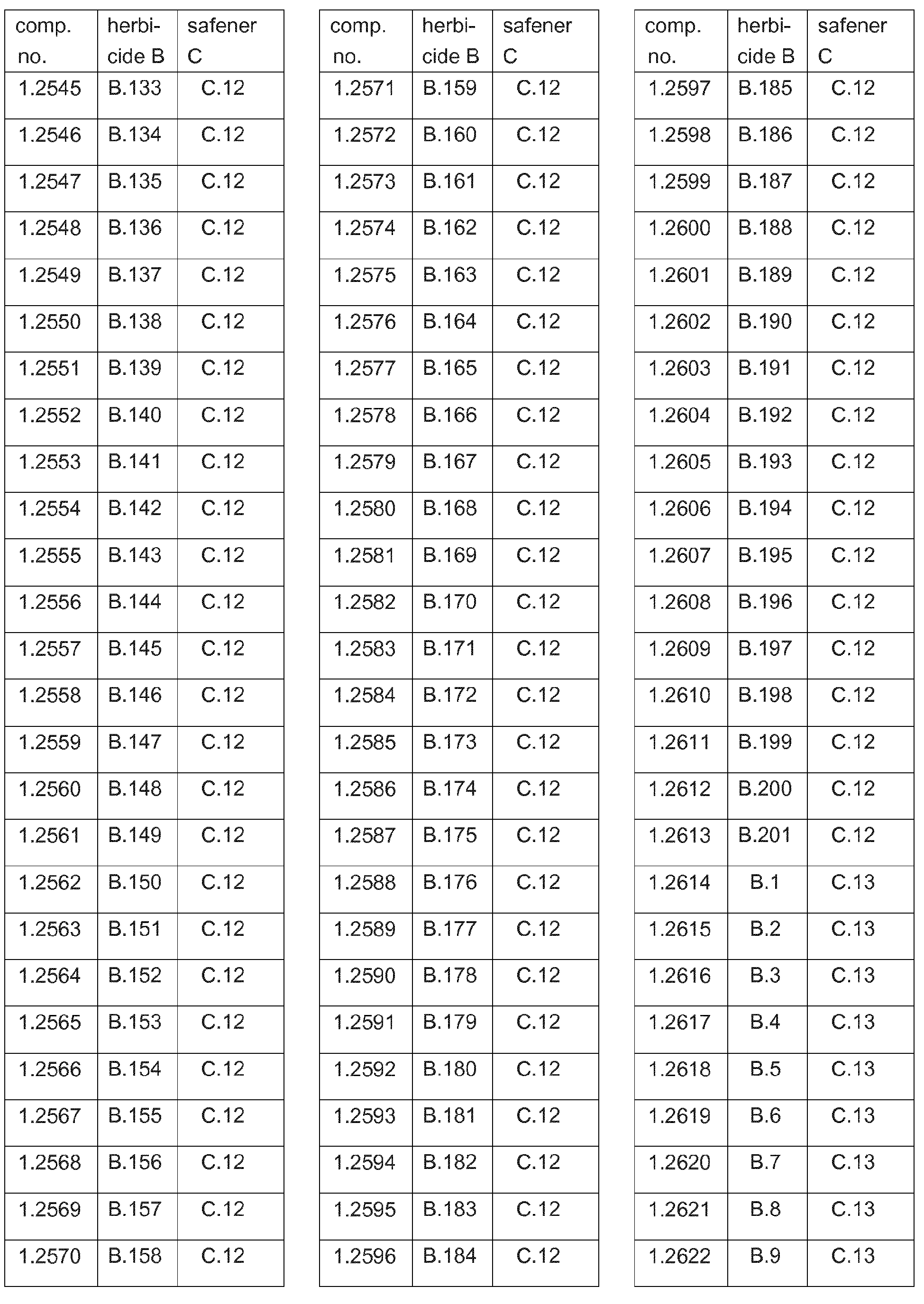

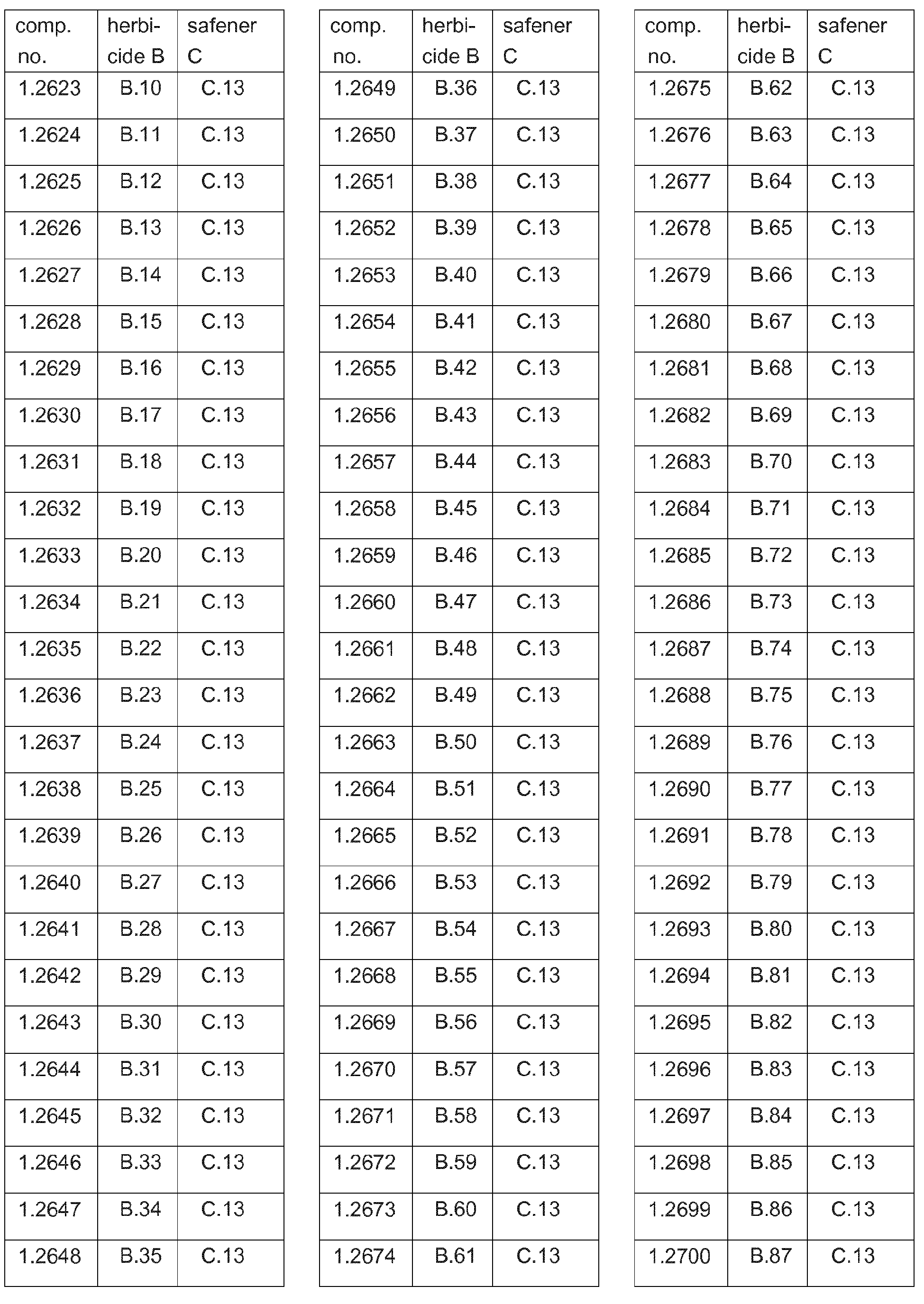

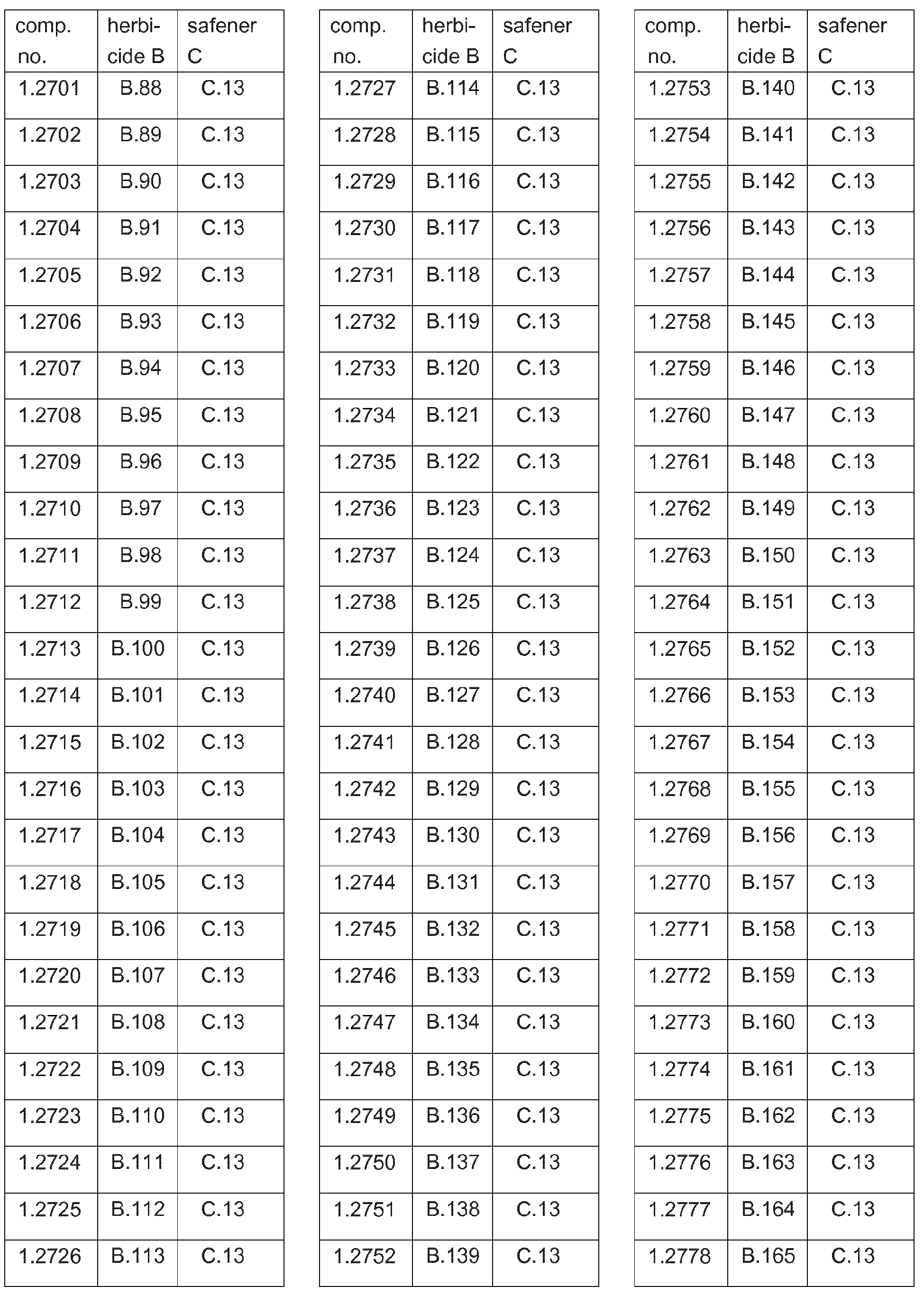

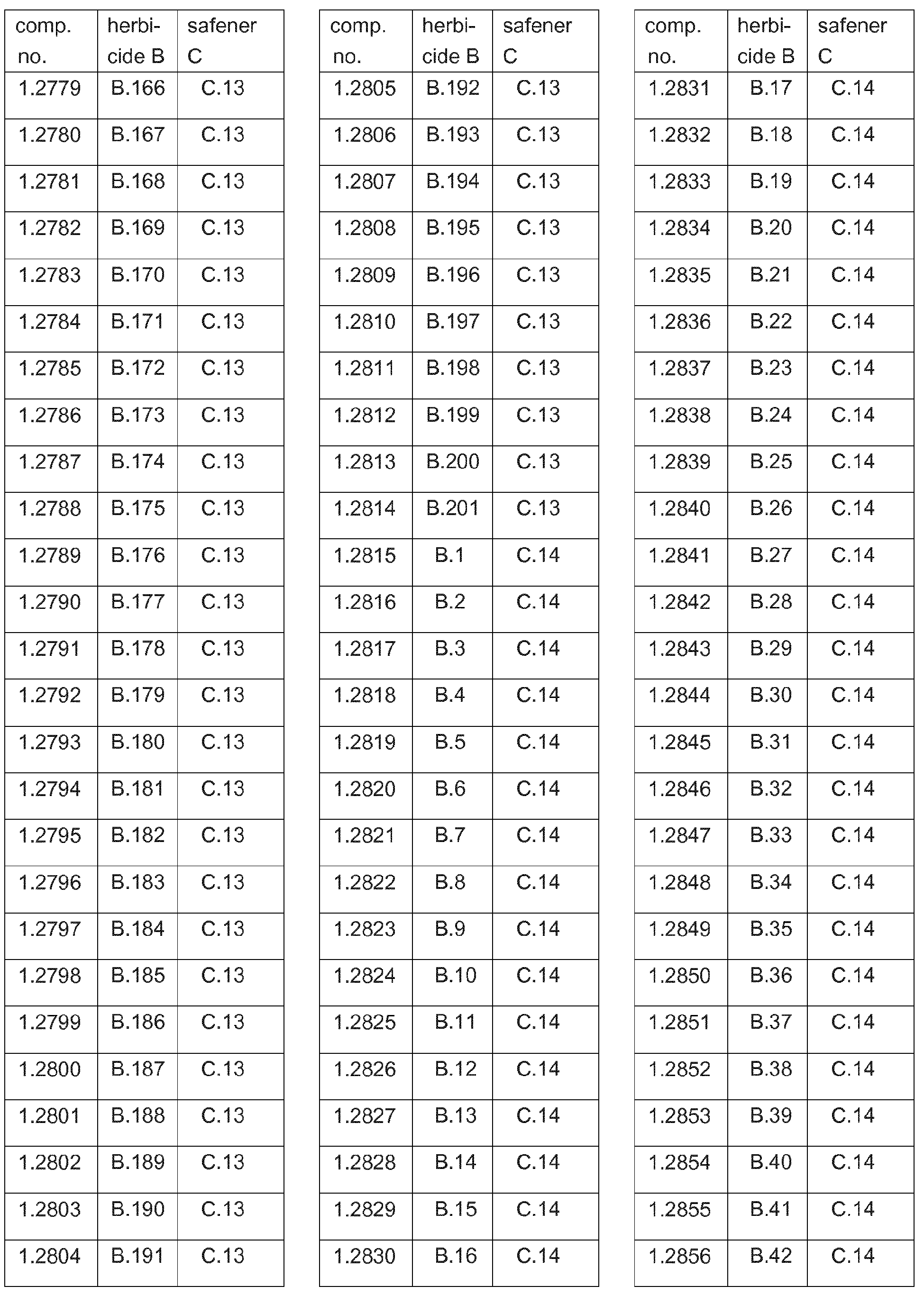

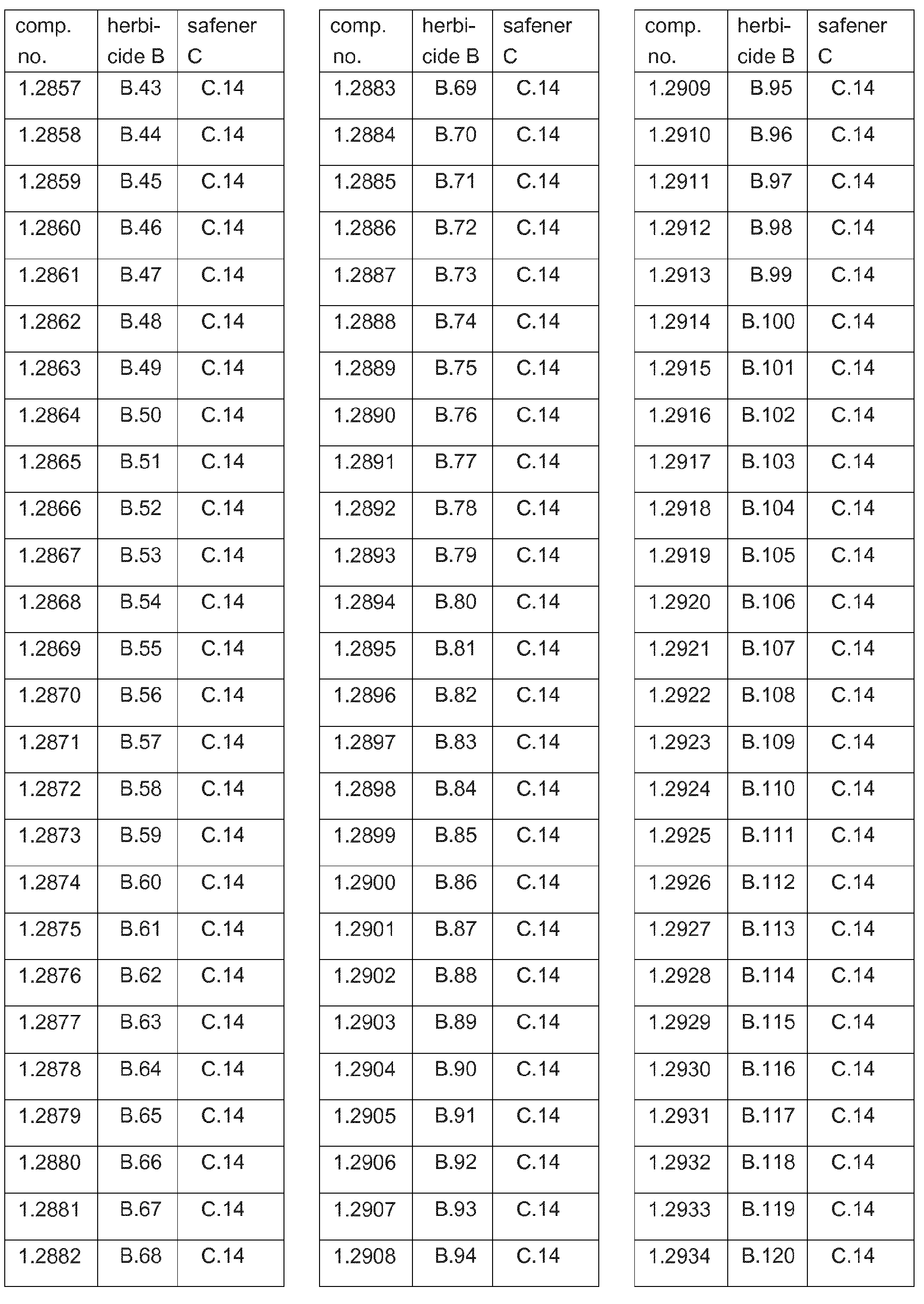

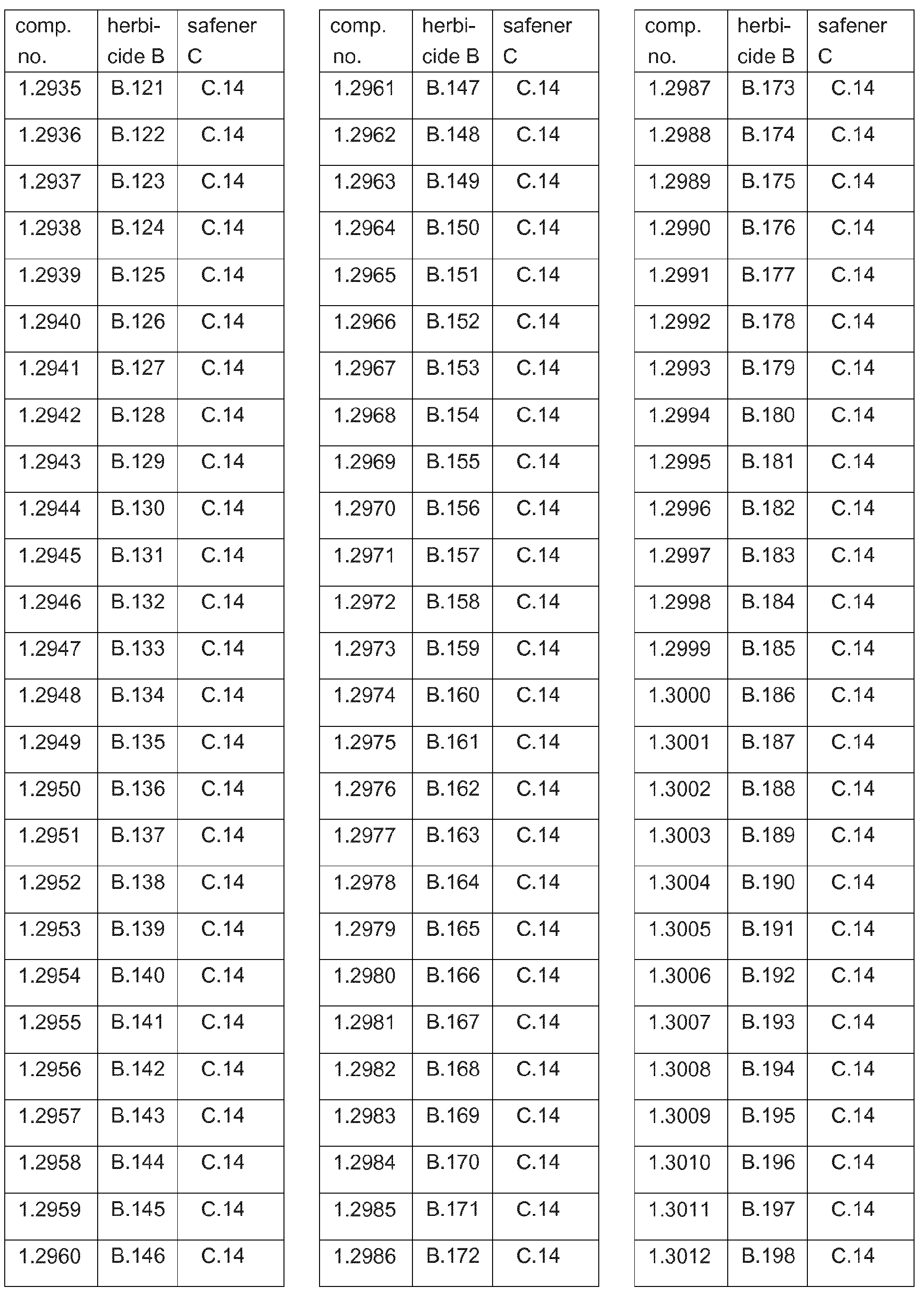

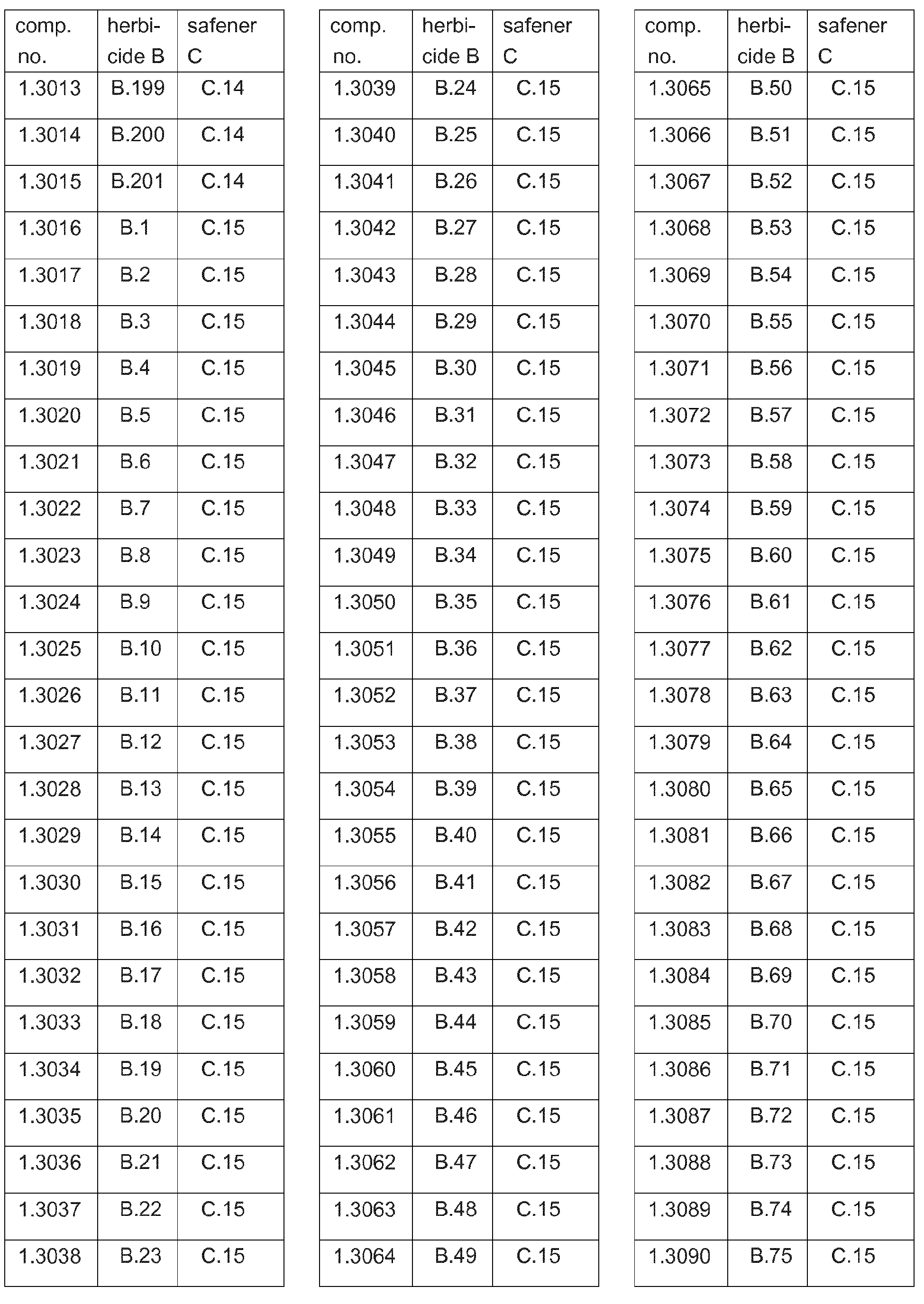

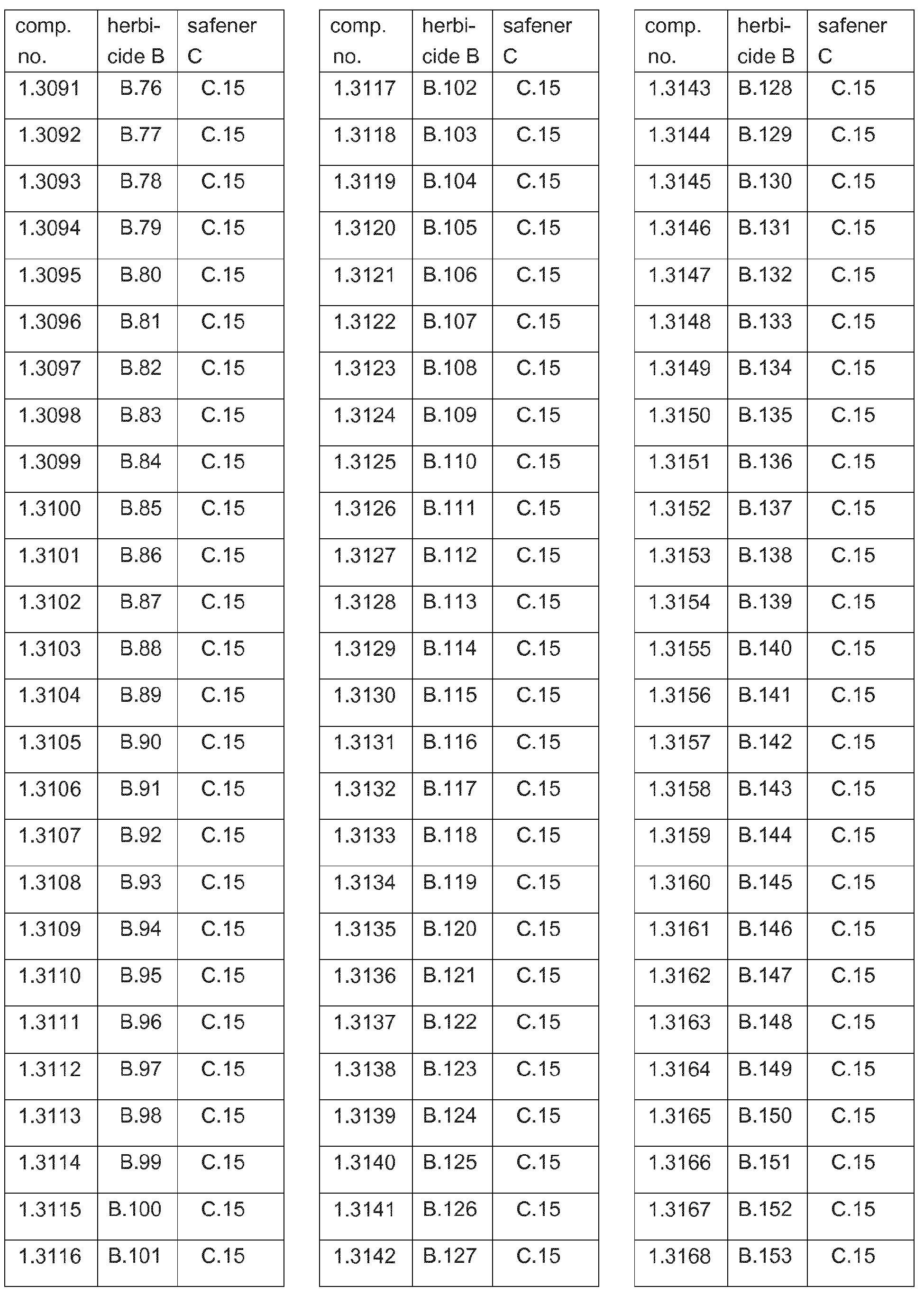

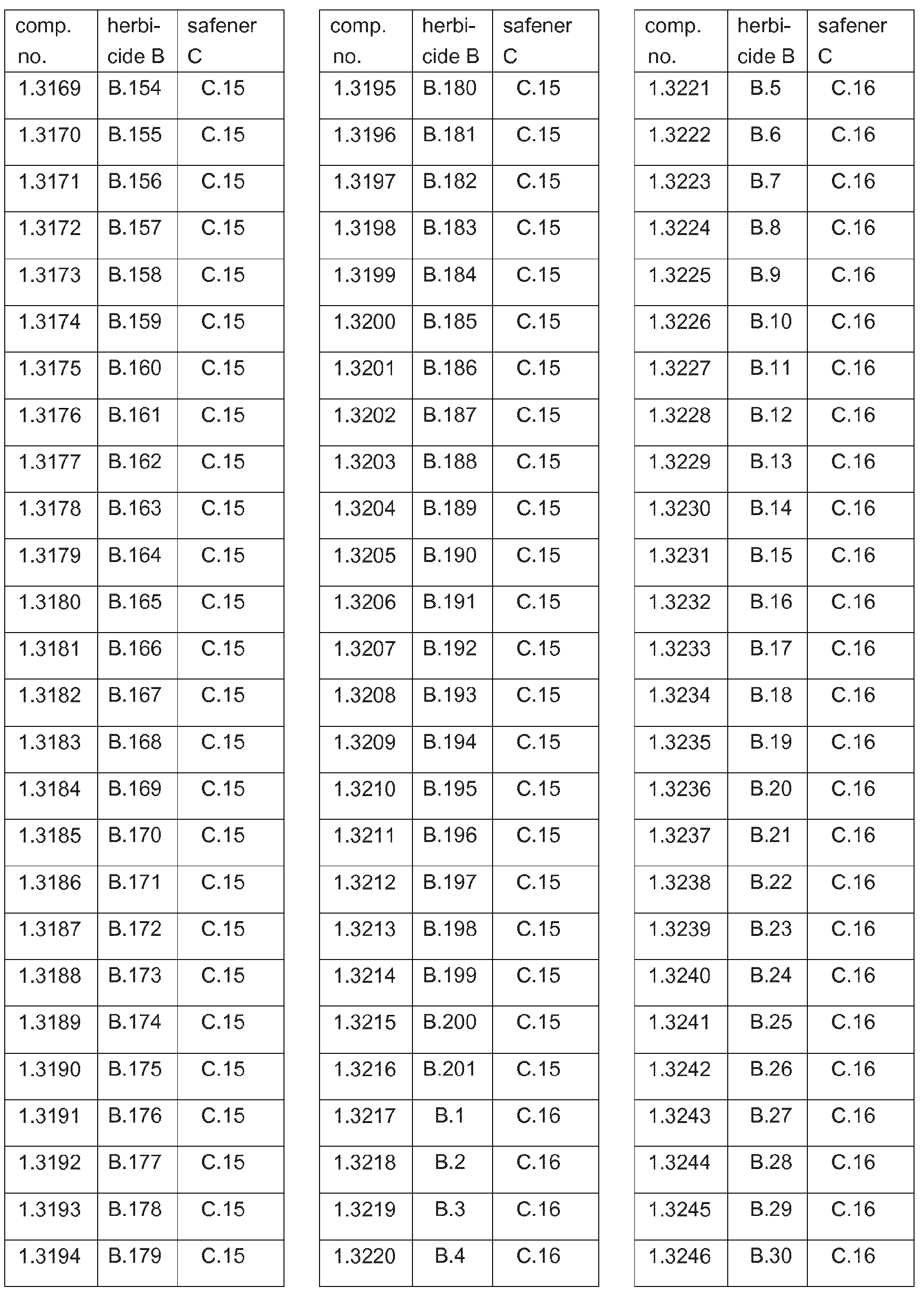

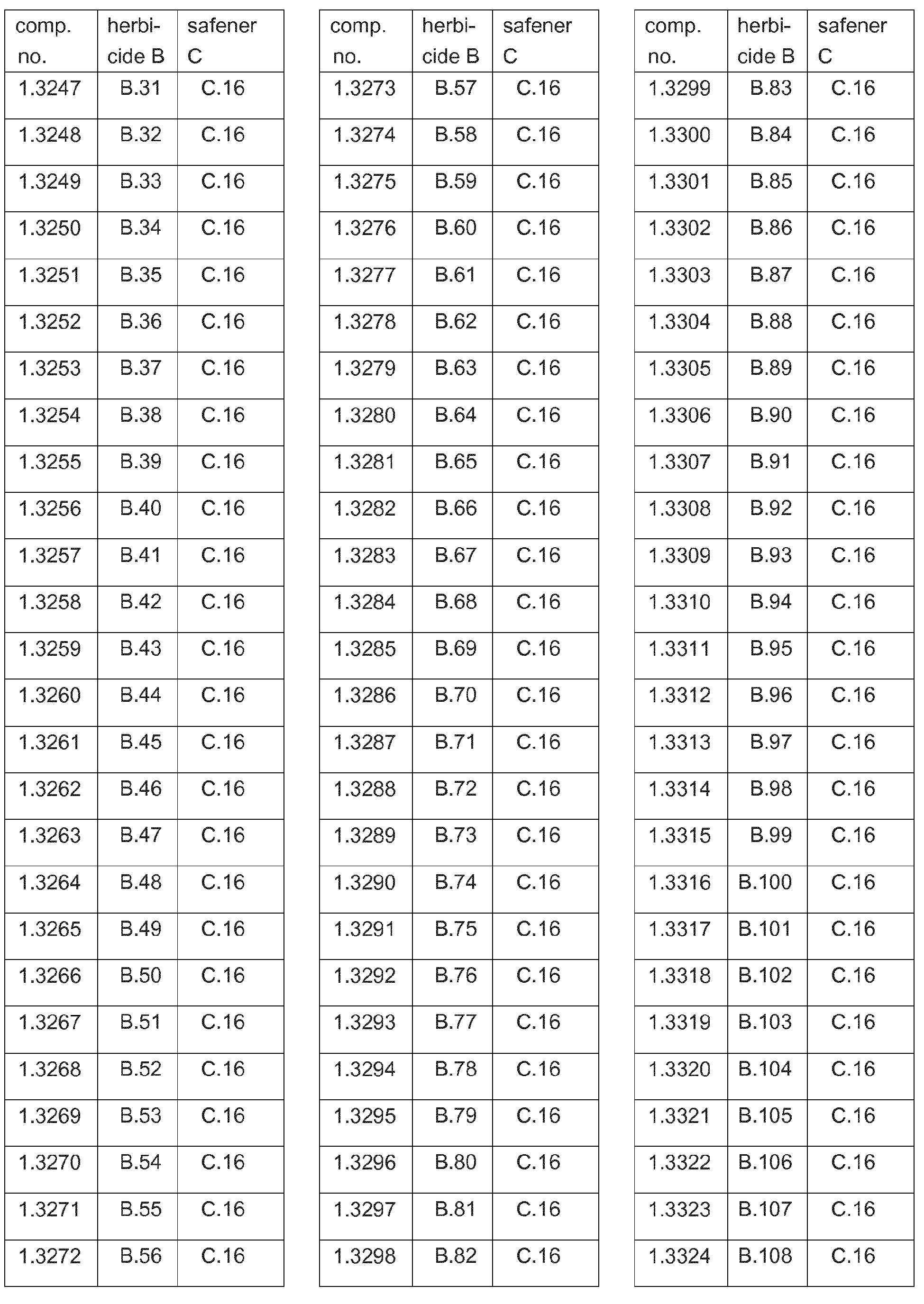

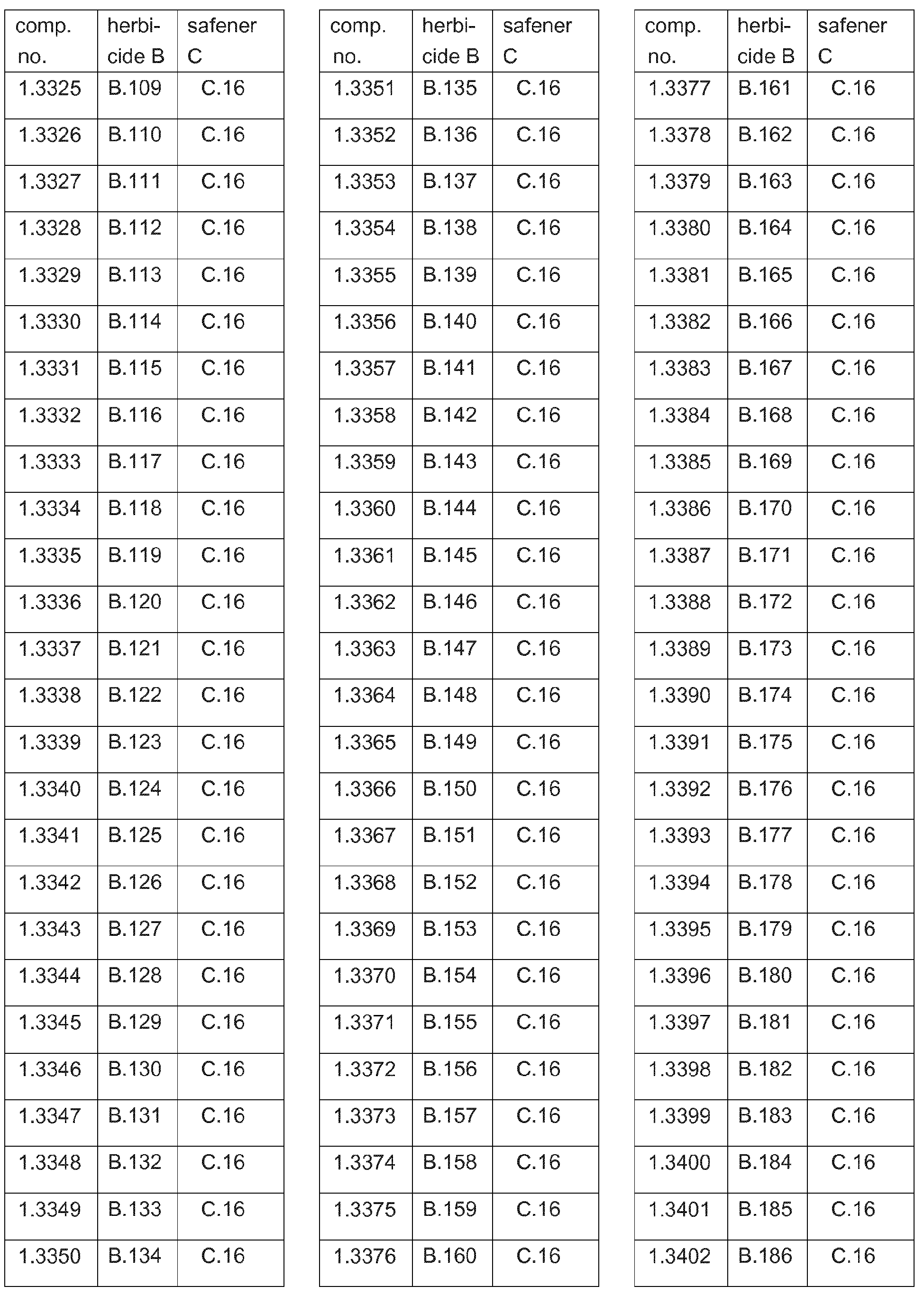

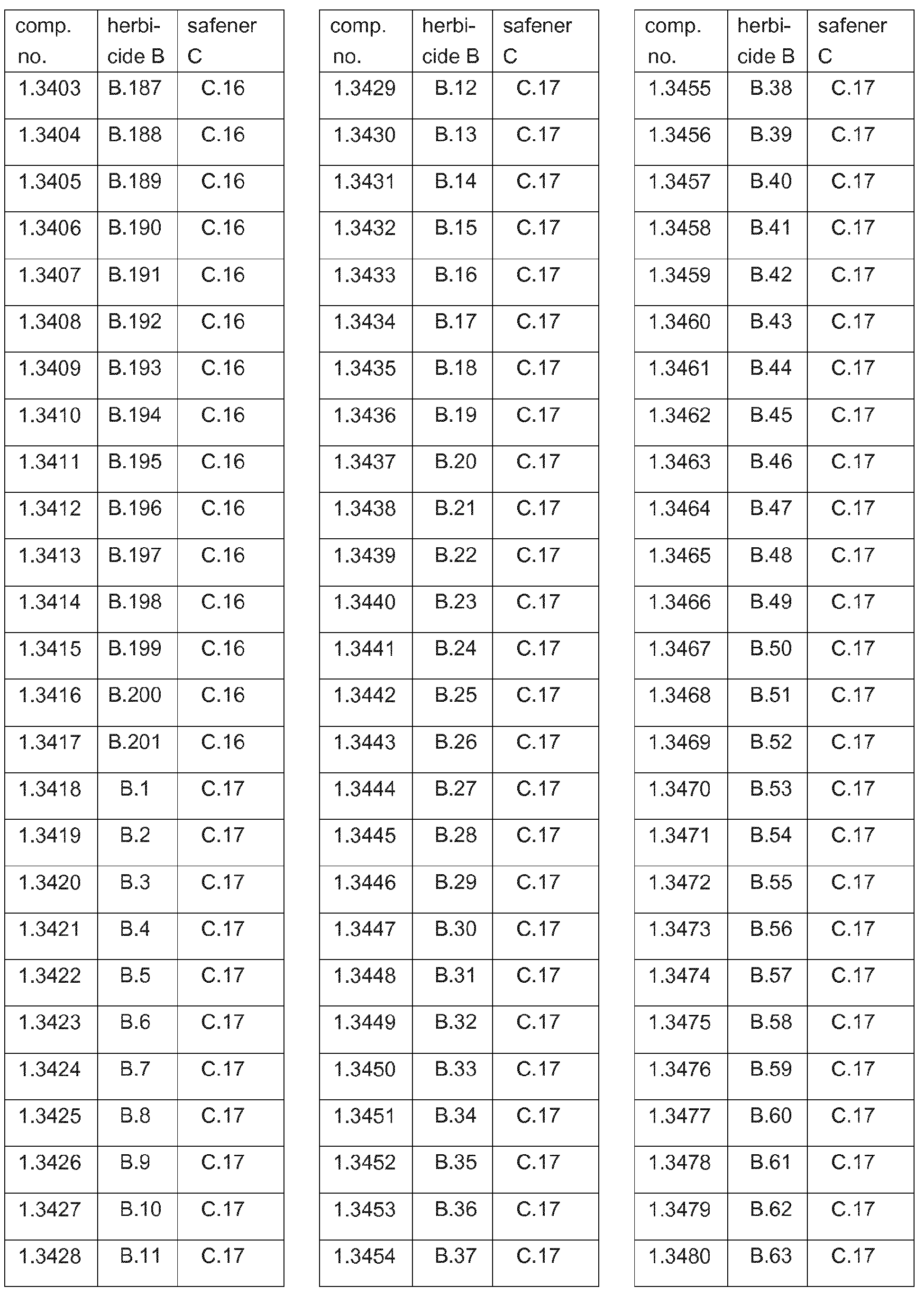

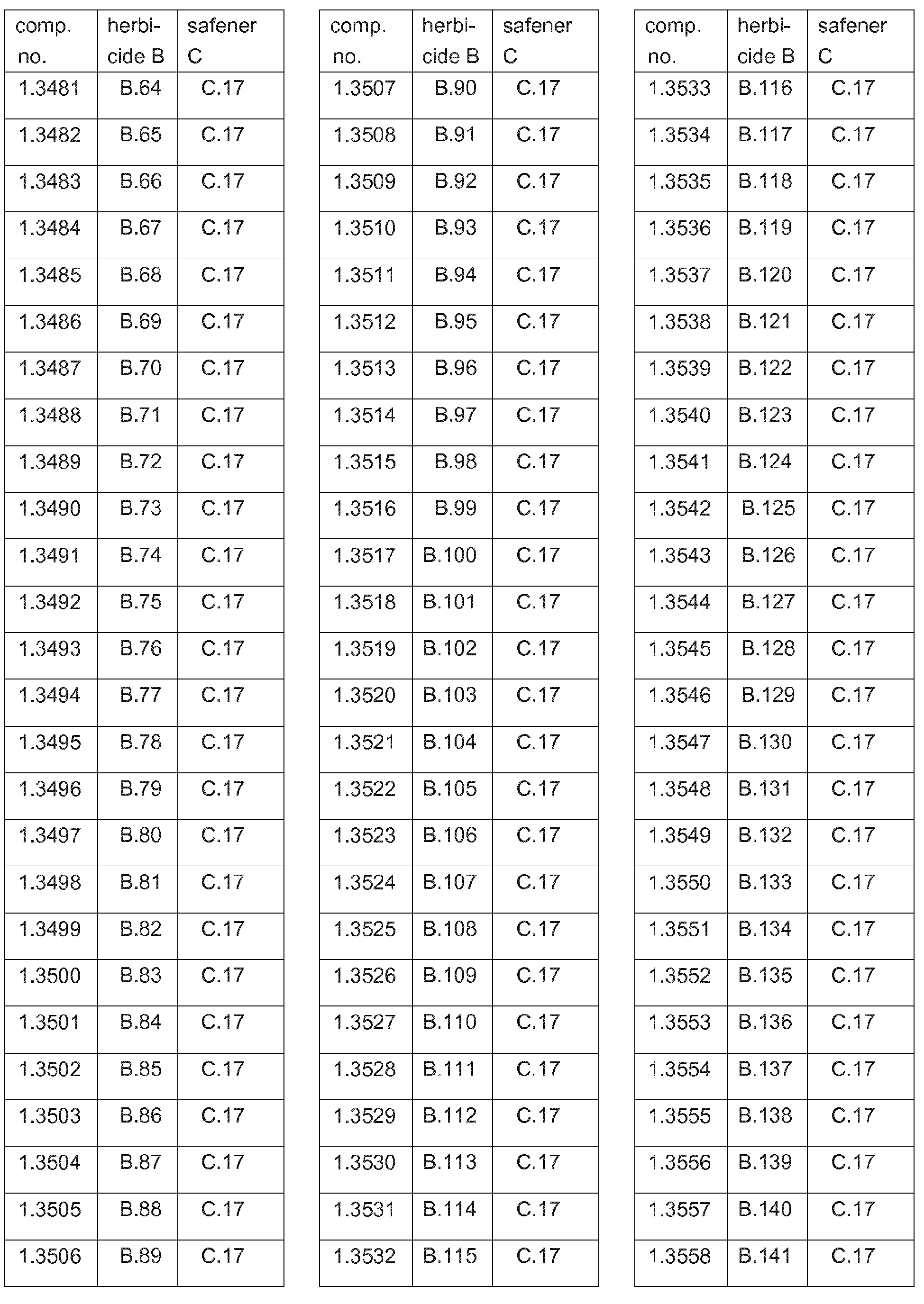

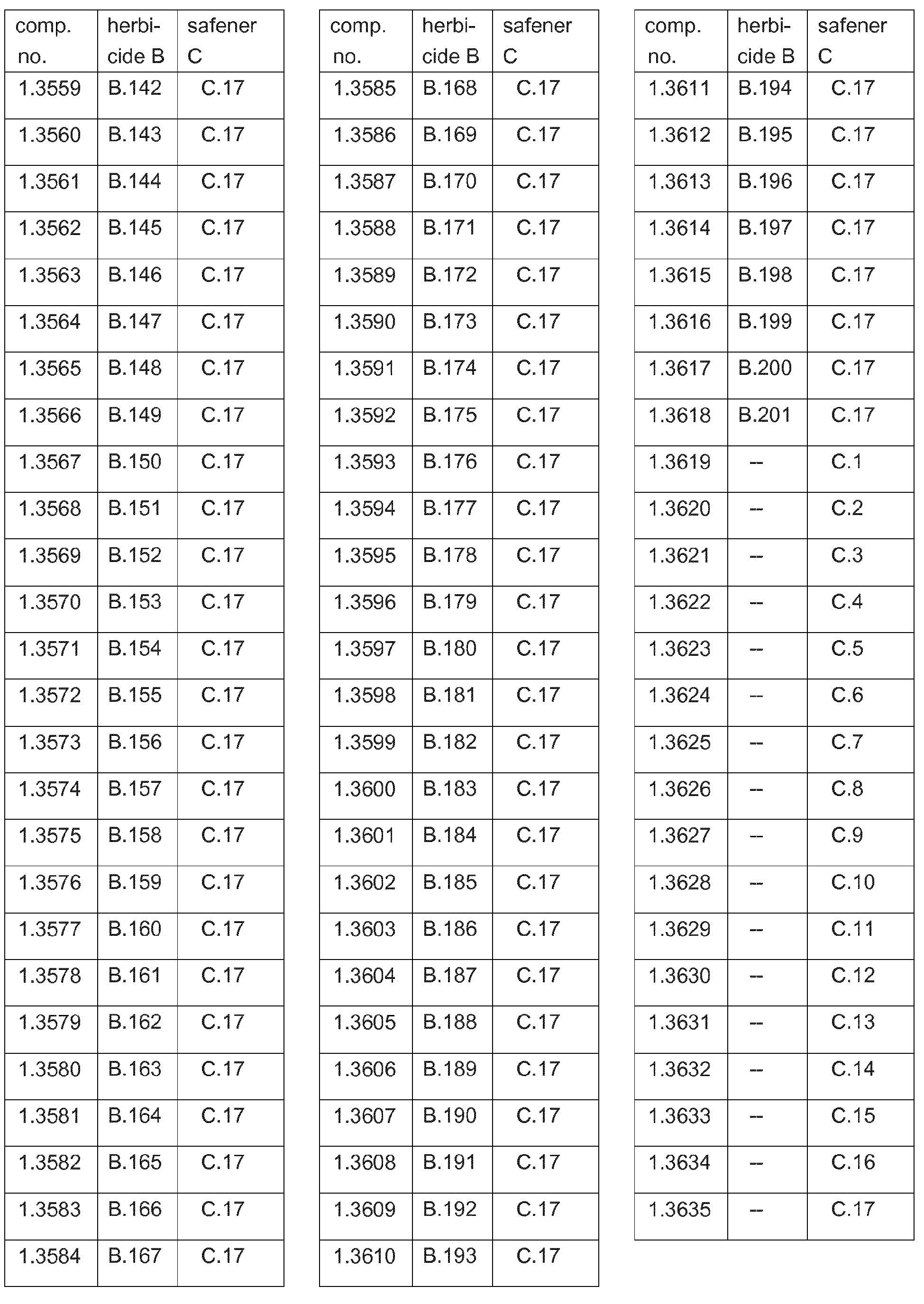

The specific number for each single composition is deductible as follows:
Composition 1.202 for example comprises the compound (1.3.1-3), clethodim (B.1) and benoxacor (C.1) (see table B, entry B.4 and table C, entry C.1).

Also especially preferred are compositions 2.1 to 2.3635 which differ from the corresponding compositions 1.1 to 1.3635 only in that they comprise the compound (1.3.1-4) in place of the compound (1.3.1-3).

Also especially preferred are compositions 3.1 to 3.3635 which differ from the corresponding compositions 1.1 to 1.3635 only in that they additionally comprise B.2 as further herbicide B.

Also especially preferred are compositions 4.1 to 4.3635 which differ from the corresponding compositions 1.1 to 1.3635 only in that they additionally comprise B.8 as further herbicide B.

Also especially preferred are compositions 5.1 to 5.3635 which differ from the corresponding compositions 1.1 to 1.3635 only in that they additionally comprise B.30 as further herbicide B.

Also especially preferred are compositions 6.1 to 6.3635 which differ from the corresponding compositions 1.1 to 1.3635 only in that they additionally comprise B.32 as further herbicide B.

Also especially preferred are compositions 7.1 to 7.3635 which differ from the corresponding compositions 1.1 to 1.3635 only in that they additionally comprise B.35 as further herbicide B.

Also especially preferred are compositions 8.1 to 8.3635 which differ from the corresponding compositions 1.1 to 1.3635 only in that they additionally comprise B.38 as further herbicide B.

Also especially preferred are compositions 9.1 to 9.3635 which differ from the corresponding compositions 1.1 to 1.3635 only in that they additionally comprise B.40 as further herbicide B.

Also especially preferred are compositions 10.1 to 10.3635 which differ from the corresponding compositions 1.1 to 1.3635 only in that they additionally comprise B.51 as further herbicide B.

Also especially preferred are compositions 11.1 to 11.3635 which differ from the corresponding compositions 1.1 to 1.3635 only in that they additionally comprise B.55 as further herbicide B.

Also especially preferred are compositions 12.1 to 12.3635 which differ from the corresponding compositions 1.1 to 1.3635 only in that they additionally comprise B.56 as further herbicide B.

Also especially preferred are compositions 13.1 to 13.3635 which differ from the corresponding compositions 1.1 to 1.3635 only in that they additionally comprise B.64 as further herbicide B.

Also especially preferred are compositions 14.1 to 14.3635 which differ from the corresponding compositions 1.1 to 1.3635 only in that they additionally comprise B.66 as further herbicide B.

Also especially preferred are compositions 15.1 to 15.3635 which differ from the corresponding compositions 1.1 to 1.3635 only in that they additionally comprise B.67 as further herbicide B.

Also especially preferred are compositions 16.1 to 16.3635 which differ from the corresponding compositions 1.1 to 1.3635 only in that they additionally comprise B.68 as further herbicide B.

Also especially preferred are compositions 17.1 to 17.3635 which differ from the corresponding compositions 1.1 to 1.3635 only in that they additionally comprise B.69 as further herbicide B.

Also especially preferred are compositions 18.1 to 18.3635 which differ from the corresponding compositions 1.1 to 1.3635 only in that they additionally comprise B.73 as further herbicide B.

Also especially preferred are compositions 19.1 to 19.3635 which differ from the corresponding compositions 1.1 to 1.3635 only in that they additionally comprise B.76 as further herbicide B.

Also especially preferred are compositions 20.1 to 20.3635 which differ from the corresponding compositions 1.1 to 1.3635 only in that they additionally comprise B.81 as further herbicide B.

Also especially preferred are compositions 21.1 to 21.3635 which differ from the corresponding compositions 1.1 to 1.3635 only in that they additionally comprise B.82 as further herbicide B.

Also especially preferred are compositions 22.1 to 22.3635 which differ from the corresponding compositions 1.1 to 1.3635 only in that they additionally comprise B.85 as further herbicide B.

Also especially preferred are compositions 23.1 to 23.3635 which differ from the corresponding compositions 1.1 to 1.3635 only in that they additionally comprise B.88 as further herbicide B.

Also especially preferred are compositions 24.1 to 24.3635 which differ from the corresponding compositions 1.1 to 1.3635 only in that they additionally comprise B.89 as further herbicide B.

Also especially preferred are compositions 25.1 to 25.3635 which differ from the corresponding compositions 1.1 to 1.3635 only in that they additionally comprise B.94 as further herbicide B.

Also especially preferred are compositions 26.1 to 26.3635 which differ from the corresponding compositions 1.1 to 1.3635 only in that they additionally comprise B.95 as further herbicide B.

Also especially preferred are compositions 27.1 to 27.3635 which differ from the corresponding compositions 1.1 to 1.3635 only in that they additionally comprise B.98 as further herbicide B.

Also especially preferred are compositions 28.1 to 28.3635 which differ from the corresponding compositions 1.1 to 1.3635 only in that they additionally comprise B.100 as further herbicide B.

Also especially preferred are compositions 29.1 to 29.3635 which differ from the corresponding compositions 1.1 to 1.3635 only in that they additionally comprise B.103 as further herbicide B.

Also especially preferred are compositions 30.1 to 30.3635 which differ from the corresponding compositions 1.1 to 1.3635 only in that they additionally comprise B.103 and B.67 as further herbicides B.

Also especially preferred are compositions 31.1 to 31.3635 which differ from the corresponding compositions 1.1 to 1.3635 only in that they additionally comprise B.103 and B.76 as further herbicides B.

Also especially preferred are compositions 32.1 to 32.3635 which differ from the corresponding compositions 1.1 to 1.3635 only in that they additionally comprise B.103 and B.82 as further herbicides B.

Also especially preferred are compositions 33.1 to 33.3635 which differ from the corresponding compositions 1.1 to 1.3635 only in that they additionally comprise B.104 as further herbicide B.

Also especially preferred are compositions 34.1 to 34.3635 which differ from the corresponding compositions 1.1 to 1.3635 only in that they additionally comprise B.104 and B.67 as further herbicides B.

Also especially preferred are compositions 35.1 to 35.3635 which differ from the corresponding compositions 1.1 to 1.3635 only in that they additionally comprise B.104 and B.76 as further herbicides B.

Also especially preferred are compositions 36.1 to 36.3635 which differ from the corresponding compositions 1.1 to 1.3635 only in that they additionally comprise B.104 and B.82 as further herbicides B.

Also especially preferred are compositions 37.1 to 37.3635 which differ from the corresponding compositions 1.1 to 1.3635 only in that they additionally comprise B.106 as further herbicide B.

Also especially preferred are compositions 38.1 to 38.3635 which differ from the corresponding compositions 1.1 to 1.3635 only in that they additionally comprise B.107 as further herbicide B.

Also especially preferred are compositions 39.1 to 39.3635 which differ from the corresponding compositions 1.1 to 1.3635 only in that they additionally comprise B. 107 and B.67 as further herbicides B.

Also especially preferred are compositions 40.1 to 40.3635 which differ from the corresponding compositions 1.1 to 1.3635 only in that they additionally comprise B. 107 and B.76 as further herbicides B.

Also especially preferred are compositions 41.1 to 41.3635 which differ from the corresponding compositions 1.1 to 1.3635 only in that they additionally comprise B. 107 and B.82 as further herbicides B.

Also especially preferred are compositions 42.1 to 42.3635 which differ from the corresponding compositions 1.1 to 1.3635 only in that they additionally comprise B.109 as further herbicide B.

Also especially preferred are compositions 43.1 to 43.3635 which differ from the corresponding compositions 1.1 to 1.3635 only in that they additionally comprise B.111 as further herbicide B.

Also especially preferred are compositions 44.1 to 44.3635 which differ from the corresponding compositions 1.1 to 1.3635 only in that they additionally comprise B.111 and B.67 as further herbicides B.

Also especially preferred are compositions 45.1 to 45.3635 which differ from the corresponding compositions 1.1 to 1.3635 only in that they additionally comprise B.111 and B.76 as further herbicides B.

Also especially preferred are compositions 46.1 to 46.3635 which differ from the corresponding compositions 1.1 to 1.3635 only in that they additionally comprise B.111 and B.82 as further herbicides B.

Also especially preferred are compositions 47.1 to 47.3635 which differ from the corresponding compositions 1.1 to 1.3635 only in that they additionally comprise B. 116 as further herbicide B.

Also especially preferred are compositions 48.1 to 48.3635 which differ from the corresponding compositions 1.1 to 1.3635 only in that they additionally comprise B.116 and B.67 as further herbicides B.

Also especially preferred are compositions 49.1 to 49.3635 which differ from the corresponding compositions 1.1 to 1.3635 only in that they additionally comprise B.116 and B.94 as further herbicides B.

Also especially preferred are compositions 50.1 to 50.3635 which differ from the corresponding compositions 1.1 to 1.3635 only in that they additionally comprise B.116 and B.103 as further herbicides B.

Also especially preferred are compositions 51.1 to 51.3635 which differ from the corresponding compositions 1.1 to 1.3635 only in that they additionally comprise B.116 and B.128 as further herbicides B.

Also especially preferred are compositions 52.1 to 52.3635 which differ from the corresponding compositions 1.1 to 1.3635 only in that they additionally comprise B.116 and B.104 as further herbicides B.

Also especially preferred are compositions 53.1 to 53.3635 which differ from the corresponding compositions 1.1 to 1.3635 only in that they additionally comprise B.116 and B.107 as further herbicides B.

Also especially preferred are compositions 54.1 to 54.3635 which differ from the corresponding compositions 1.1 to 1.3635 only in that they additionally comprise B.116 and B.111 as further herbicides B.

Also especially preferred are compositions 55.1 to 55.3635 which differ from the corresponding compositions 1.1 to 1.3635 only in that they additionally comprise B.122 as further herbicide B.

Also especially preferred are compositions 56.1 to 56.3635 which differ from the corresponding compositions 1.1 to 1.3635 only in that they additionally comprise B.126 as further herbicide B.

Also especially preferred are compositions 57.1 to 57.3635 which differ from the corresponding compositions 1.1 to 1.3635 only in that they additionally comprise B.128 as further herbicide B.

Also especially preferred are compositions 58.1 to 58.3635 which differ from the corresponding compositions 1.1 to 1.3635 only in that they additionally comprise B.131 as further herbicide B.

Also especially preferred are compositions 59.1 to 59.3635 which differ from the corresponding compositions 1.1 to 1.3635 only in that they additionally comprise B.132 as further herbicide B.

Also especially preferred are compositions 60.1 to 60.3635 which differ from the corresponding compositions 1.1 to 1.3635 only in that they additionally comprise B.133 as further herbicide B.

Also especially preferred are compositions 61.1 to 61.3635 which differ from the corresponding compositions 1.1 to 1.3635 only in that they additionally comprise B.135 as further herbicide B.

Also especially preferred are compositions 62.1 to 62.3635 which differ from the corresponding compositions 1.1 to 1.3635 only in that they additionally comprise B.137 as further herbicide B.

Also especially preferred are compositions 63.1 to 63.3635 which differ from the corresponding compositions 11.1 to 1.3635 only in that they additionally comprise B.138 as further herbicide B.

Also especially preferred are compositions 64.1 to 64.3635 which differ from the corresponding compositions 1.1 to 1.3635 only in that they additionally comprise B.140 as further herbicide B.

Also especially preferred are compositions 65.1 to 65.3635 which differ from the corresponding compositions 1.1 to 1.3635 only in that they additionally comprise B.145 as further herbicide B.

Also especially preferred are compositions 66.1 to 66.3635 which differ from the corresponding compositions 1.1 to 1.3635 only in that they additionally comprise B.153 as further herbicide B.

Also especially preferred are compositions 67.1 to 67.3635 which differ from the corresponding compositions 1.1 to 1.3635 only in that they additionally comprise B.156 as further herbicide B.

Also especially preferred are compositions 68.1 to 68.3635 which differ from the corresponding compositions 1.1 to 1.3635 only in that they additionally comprise B.171 as further herbicide B.

Also especially preferred are compositions 69.1 to 69.3635 which differ from the corresponding compositions 1.1 to 1.3635 only in that they additionally comprise B.174 as further herbicide B.

Also especially preferred are compositions 70.1 to 70.3635 which differ from the corresponding compositions 1.1 to 1.3635 only in that they comprise the compound (1.3.I-10) in place of the compound (1.3.I-3).

Also especially preferred are compositions 71.1 to 71.3635 which differ from the corresponding compositions 1.1 to 1.3635 only in that they comprise the compound (1.3.I-11) in place of the compound (1.3.I-3).

Also especially preferred are compositions 72.1 to 72.3635 which differ from the corresponding compositions 1.1 to 1.3635 only in that they comprise the compound (1.3.I-17) in place of the compound (1.3.I-3).

Also especially preferred are compositions 73.1 to 73.3635 which differ from the corresponding compositions 1.1 to 1.3635 only in that they comprise the compound (1.3.I-18) in place of the compound (1.3.I-3).

Also especially preferred are compositions 74.1 to 74.3635 which differ from the corresponding compositions 1.1 to 1.3635 only in that they comprise the compound (1.3.I-24) in place of the compound (1.3.I-3).

Also especially preferred are compositions 75.1 to 75.3635 which differ from the corresponding compositions 1.1 to 1.3635 only in that they comprise the compound (1.3.I-25) in place of the compound (1.3.I-3).

Also especially preferred are compositions 76.1 to 76.3635 which differ from the corresponding compositions 1.1 to 1.3635 only in that they comprise the compound (1.3.I-31) in place of the compound (1.3.I-3).

Also especially preferred are compositions 77.1 to 77.3635 which differ from the corresponding compositions 1.1 to 1.3635 only in that they comprise the compound (1.3.I-32) in place of the compound (1.3.I-3).

Also especially preferred are compositions 78.1 to 78.3635 which differ from the corresponding compositions 1.1 to 1.3635 only in that they comprise the compound (1.3.I-38) in place of the compound (1.3.I-3).

Also especially preferred are compositions 79.1 to 79.3635 which differ from the corresponding compositions 1.1 to 1.3635 only in that they comprise the compound (1.3.I-39) in place of the compound (1.3.I-3).

Also especially preferred are compositions 80.1 to 80.3635 which differ from the corresponding compositions 1.1 to 1.3635 only in that they comprise the compound (1.3.I-45) in place of the compound (1.3.I-3).

Also especially preferred are compositions 81.1 to 81.3635 which differ from the corresponding compositions 1.1 to 1.3635 only in that they comprise the compound (1.3.I-46) in place of the compound (1.3.I-3).

Also especially preferred are compositions 82.1 to 82.3635 which differ from the corresponding compositions 1.1 to 1.3635 only in that they comprise the compound (1.15.I-3) in place of the compound (1.3.I-3).

Also especially preferred are compositions 83.1 to 83.3635 which differ from the corresponding compositions 1.1 to 1.3635 only in that they comprise the compound (1.15.I-4) in place of the compound (1.3.I-3).

Also especially preferred are compositions 84.1 to 84.3635 which differ from the corresponding compositions 1.1 to 1.3635 only in that they comprise the compound (1.15.I-10) in place of the compound (1.3.I-3).

Also especially preferred are compositions 85.1 to 85.3635 which differ from the corresponding compositions 1.1 to 1.3635 only in that they comprise the compound (1.15.I-11) in place of the compound (1.3.I-3).

Also especially preferred are compositions 86.1 to 86.3635 which differ from the corresponding compositions 1.1 to 1.3635 only in that they comprise the compound (1.15.I-17) in place of the compound (1.3.I-3).

Also especially preferred are compositions 87.1 to 87.3635 which differ from the corresponding compositions 1.1 to 1.3635 only in that they comprise the compound (1.15.1-18) in place of the compound (1.3.1-3).

Also especially preferred are compositions 88.1 to 88.3635 which differ from the corresponding compositions 1.1 to 1.3635 only in that they comprise the compound (1.15.1-24) in place of the compound (1.3.1-3).

Also especially preferred are compositions 89.1 to 89.3635 which differ from the corresponding compositions 1.1 to 1.3635 only in that they comprise the compound (1.15.1-25) in place of the compound (1.3.1-3).

Also especially preferred are compositions 90.1 to 90.3635 which differ from the corresponding compositions 1.1 to 1.3635 only in that they comprise the compound (1.15.I-31) in place of the compound (1.3.I-3).

Also especially preferred are compositions 91.1 to 91.3635 which differ from the corresponding compositions 1.1 to 1.3635 only in that they comprise the compound (1.15.I-32) in place of the compound (1.3.I-3).

Also especially preferred are compositions 92.1 to 92.3635 which differ from the corresponding compositions 1.1 to 1.3635 only in that they comprise the compound (1.15.I-38) in place of the compound (1.3.I-3).

Also especially preferred are compositions 93.1 to 93.3635 which differ from the corresponding compositions 1.1 to 1.3635 only in that they comprise the compound (1.15.I-39) in place of the compound (1.3.I-3).

Also especially preferred are compositions 94.1 to 94.3635 which differ from the corresponding compositions 1.1 to 1.3635 only in that they comprise the compound (1.15.I-45) in place of the compound (1.3.I-3).

Also especially preferred are compositions 95.1 to 95.3635 which differ from the corresponding compositions 1.1 to 1.3635 only in that they comprise the compound (1.15.I-46) in place of the compound (1.3.I-3).

Also especially preferred are compositions 96.1 to 96.3635 which differ from the corresponding compositions 1.1 to 1.3635 only in that they comprise the compound (1.16.I-3) in place of the compound (1.3.I-3).

Also especially preferred are compositions 97.1 to 97.3635 which differ from the corresponding compositions 1.1 to 1.3635 only in that they comprise the compound (1.16.I-4) in place of the compound (1.3.I-3).

Also especially preferred are compositions 98.1 to 98.3635 which differ from the corresponding compositions 1.1 to 1.3635 only in that they comprise the compound (1.16.I-10) in place of the compound (1.3.I-3).

Also especially preferred are compositions 99.1 to 99.3635 which differ from the corresponding compositions 1.1 to 1.3635 only in that they comprise the compound (1.16.I-11) in place of the compound (1.3.1-3).

Also especially preferred are compositions 100.1 to 100.3635 which differ from the corresponding compositions 1.1 to 1.3635 only in that they comprise the compound (1.16.I-17) in place of the compound (1.3.I-3).

Also especially preferred are compositions 101.1 to 101.3635 which differ from the corresponding compositions 1.1 to 1.3635 only in that they comprise the compound (1.16.I-18) in place of the compound (1.3.I-3).

Also especially preferred are compositions 102.1 to 102.3635 which differ from the corresponding compositions 1.1 to 1.3635 only in that they comprise the compound (1.16.I-24) in place of the compound (1.3.I-3).

Also especially preferred are compositions 103.1 to 103.3635 which differ from the corresponding compositions 1.1 to 1.3635 only in that they comprise the compound (1.16.I-25) in place of the compound (1.3.I-3).

Also especially preferred are compositions 104.1 to 104.3635 which differ from the corresponding compositions 1.1 to 1.3635 only in that they comprise the compound (1.16.I-31) in place of the compound (1.3.I-3).

Also especially preferred are compositions 105.1 to 105.3635 which differ from the corresponding compositions 1.1 to 1.3635 only in that they comprise the compound (1.16.I-32) in place of the compound (1.3.I-3).

Also especially preferred are compositions 106.1 to 106.3635 which differ from the corresponding compositions 1.1 to 1.3635 only in that they comprise the compound (1.16.I-38) in place of the compound (1.3.I-3).

Also especially preferred are compositions 107.1 to 107.3635 which differ from the corresponding compositions 1.1 to 1.3635 only in that they comprise the compound (1.16.I-39) in place of the compound (1.3.I-3).

Also especially preferred are compositions 108.1 to 108.3635 which differ from the corresponding compositions 1.1 to 1.3635 only in that they comprise the compound (1.16.I-45) in place of the compound (1.3.I-3).

Also especially preferred are compositions 109.1 to 109.3635 which differ from the corresponding compositions 1.1 to 1.3635 only in that they comprise the compound (1.16.I-46) in place of the compound (1.3.I-3).

The invention also relates to agrochemical compositions comprising at least an auxiliary and at least one pyridine compound of formula (I) according to the invention.

An agrochemical composition comprises a pesticidal effective amount of a pyridine compound of formula (I). The term "effective amount" denotes an amount of the composition or of the compounds I, which is sufficient for controlling unwanted plants, especially for controlling unwanted plants in cultivated plants and which does not result in a substantial damage to the treated plants. Such an amount can vary in a broad range and is dependent on various factors, such as the plants to be controlled, the treated cultivated plant or material, the climatic conditions and the specific pyridine compound of formula (I) used.

The pyridine compounds of formula (I), their N-oxides, salts or derivatives can be converted into customary types of agrochemical compositions, e. g. solutions, emulsions, suspensions, dusts, powders, pastes, granules, pressings, capsules, and mixtures thereof. Examples for agrochemical composition types are suspensions (e.g. SC, OD, FS), emulsifiable concentrates (e.g. EC), emulsions (e.g. EW, EO, ES, ME), capsules (e.g. CS, ZC), pastes, pastilles, wettable powders or dusts (e.g. WP, SP, WS, DP, DS), pressings (e.g. BR, TB, DT), granules (e.g. WG, SG, GR, FG, GG, MG), insecticidal articles (e.g. LN), as well as gel formulations for the treatment of plant propagation materials such as seeds (e.g. GF). These and further agrochemical compositions types are defined in the "Catalogue of pesticide formulation types and international coding system", Technical Monograph No. 2, 6th Ed. May 2008, CropLife International.

The agrochemical compositions are prepared in a known manner, such as described by Mollet and Grubemann, Formulation technology, Wiley VCH, Weinheim, 2001; or Knowles, New developments in crop protection product formulation, Agrow Reports DS243, T&F Informa, London, 2005.

Suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable solvents and liquid carriers are water and organic solvents, such as mineral oil fractions of medium to high boiling point, e.g. kerosene, diesel oil; oils of vegetable or animal origin; aliphatic, cyclic, and aromatic hydrocarbons, e. g. toluene, paraffin, tetrahydronaphthalene, alkylated naphthalenes; alcohols, e.g. ethanol, propanol, butanol, benzylalcohol, cyclohexanol; glycols; DMSO; ketones, e.g. cyclohexanone; esters, e.g. lactates, carbonates, fatty acid esters, gamma-butyrolactone; fatty acids; phosphonates; amines; amides, e.g. N-methylpyrrolidone, fatty acid dimethylamides; and mixtures thereof.

Suitable solid carriers or fillers are mineral earths, e.g. silicates, silica gels, talc, kaolins, limestone, lime, chalk, clays, dolomite, diatomaceous earth, bentonite, calcium sulfate, magnesium sulfate, magnesium oxide; polysaccharides, e.g. cellulose, starch; fertilizers, e.g. ammonium sulfate, ammonium phosphate, ammonium nitrate, ureas; products of vegetable origin, e.g. cereal meal, tree bark meal, wood meal, nutshell meal, and mixtures thereof.

Suitable surfactants are surface-active compounds, such as anionic, cationic, non-ionic, and amphoteric surfactants, block polymers, polyelectrolytes, and mixtures thereof. Such surfactants can be used as emulsifier, dispersant, solubilizer, wetter, penetration enhancer, protective colloid, or adjuvant. Examples of surfactants are listed in McCutcheon's, Vol.1: Emulsifiers & Detergents, McCutcheon's Directories, Glen Rock, USA, 2008 (International Ed. or North American Ed.).

Suitable anionic surfactants are alkali, alkaline earth or ammonium salts of sulfonates, sulfates, phosphates, carboxylates, and mixtures thereof. Examples of sulfonates are alkylarylsulfonates, diphenylsulfonates, alpha-olefin sulfonates, lignine sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and alkylnaphthalenes, sulfosuccinates or sulfosuccinamates. Examples of sulfates are sulfates of fatty acids and oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates.

Suitable nonionic surfactants are alkoxylates, N-substituted fatty acid amides, amine oxides, esters, sugar-based surfactants, polymeric surfactants, and mixtures thereof. Examples of alkoxylates are compounds such as alcohols, alkylphenols, amines, amides, arylphenols, fatty acids, or fatty acid esters which have been alkoxylated with 1 to 50 equivalents. Ethylene oxide and/or propylene oxide may be employed for the alkoxylation, preferably ethylene oxide. Examples of N-substituted fatty acid amides are fatty acid glucamides or fatty acid alkanolamides. Examples of esters are fatty acid esters, glycerol esters, or monoglycerides. Examples of sugar-based surfactants are sorbitans, ethoxylated sorbitans, sucrose, and glucose esters, or alkylpoly-glucosides. Examples of polymeric surfactants are home- or copolymers of vinylpyrrolidone, vinylalcohols, or vinylacetate.

Suitable cationic surfactants are quaternary surfactants, for example quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide and polypropylene oxide. Suitable polyelectrolytes are polyacids or polybases. Examples of polyacids are alkali salts of polyacrylic acid or polyacid comb polymers. Examples of polybases are polyvinylamines or polyethyleneamines.

Suitable adjuvants are compounds, which have a neglectable or even no pesticidal activity themselves, and which improve the biological performance of the pyridine compounds of formula (I) on the target. Examples are surfactants, mineral or vegetable oils, and other auxiliaries. Further examples are listed by Knowles, Adjuvants, and additives, Agrow Reports DS256, T&F Informa UK, 2006, chapter 5.

Suitable thickeners are polysaccharides (e.g. xanthan gum, carboxymethylcellulose), inorganic clays (organically modified or unmodified), polycarboxylates, and silicates.

Suitable bactericides are bronopol and isothiazolinone derivatives such as alkylisothiazolinones and benzisothiazolinones.

Suitable anti-freezing agents are ethylene glycol, propylene glycol, urea, and glycerin. Suitable anti-foaming agents are silicones, long chain alcohols, and salts of fatty acids. Suitable colorants (e.g. in red, blue, or green) are pigments of low water solubility and water-soluble dyes. Examples are inorganic colorants (e.g. iron oxide, titan oxide, iron hexacyanoferrate) and organic colorants (e.g. alizarin-, azo-, and phthalocyanine colorants).

Suitable tackifiers or binders are polyvinylpyrrolidons, polyvinylacetates, polyvinyl alcohols, polyacrylates, biological or synthetic waxes, and cellulose ethers.

### Examples for agrochemical composition types and their preparation are:

### i) Water-soluble concentrates (SL, LS)

10-60 wt% of a pyridine compound of formula (I) according to the invention and 5-15 wt% wetting agent (e.g. alcohol alkoxylates) are dissolved in water and/or in a water-soluble solvent (e.g. alcohols) ad 100 wt%. The active substance dissolves upon dilution with water.

### ii) Dispersible concentrates (DC)

5-25 wt% of a pyridine compound of formula (I) according to the invention and 1-10 wt% dispersant (e. g. polyvinylpyrrolidone) are dissolved in organic solvent (e.g. cyclohexanone) ad 100 wt%. Dilution with water gives a dispersion.

### iii) Emulsifiable concentrates (EC)

15-70 wt% of a pyridine compound of formula (I) according to the invention and 5-10 wt% emulsifiers (e.g. calcium dodecylbenzenesulfonate and castor oil ethoxylate) are dissolved in water-insoluble organic solvent (e.g. aromatic hydrocarbon) ad 100 wt%. Dilution with water gives an emulsion.

### iv) Emulsions (EW, EO, ES)

5-40 wt% of a pyridine compound of formula (I) according to the invention and 1-10 wt% emulsifiers (e.g. calcium dodecylbenzenesulfonate and castor oil ethoxylate) are dissolved in 20-40 wt% water-insoluble organic solvent (e.g. aromatic hydrocarbon). This mixture is introduced into water ad 100 wt% by means of an emulsifying machine and made into a homogeneous emulsion. Dilution with water gives an emulsion.

### v) Suspensions (SC, OD, FS)

In an agitated ball mill, 20-60 wt% of a pyridine compound of formula (I) according to the invention are comminuted with addition of 2-10 wt% dispersants and wetting agents (e.g. sodium lignosulfonate and alcohol ethoxylate), 0,1-2 wt% thickener (e.g. xanthan gum) and water ad 100 wt% to give a fine active substance suspension. Dilution with water gives a stable suspension of the active substance. For FS type composition up to 40 wt% binder (e.g. polyvinylalcohol) is added.

### vi) Water-dispersible granules and water-soluble granules (WG, SG)

50-80 wt% of a pyridine compound of formula (I) according to the invention are ground finely with addition of dispersants and wetting agents (e.g. sodium lignosulfonate and alcohol ethoxylate) ad 100 wt% and prepared as water-dispersible or water-soluble granules by means of technical appliances (e. g. extrusion, spray tower, fluidized bed). Dilution with water gives a stable dispersion or solution of the active substance.

### vii) Water-dispersible powders and water-soluble powders (WP, SP, WS)

50-80 wt% of a pyridine compound of formula (I) according to the invention are ground in a rotor-stator mill with addition of 1-5 wt% dispersants (e.g. sodium lignosulfonate), 1-3 wt% wetting agents (e.g. alcohol ethoxylate) and solid carrier (e.g. silica gel) ad 100 wt%. Dilution with water gives a stable dispersion or solution of the active substance.

### viii) Gel (GW, GF)

In an agitated ball mill, 5-25 wt% of a pyridine compound of formula (I) according to the invention are comminuted with addition of 3-10 wt% dispersants (e.g. sodium lignosulfonate), 1-5 wt% thickener (e.g. carboxymethylcellulose) and water ad 100 wt% to give a fine suspension of the active substance. Dilution with water gives a stable suspension of the active substance.

### iv) Microemulsion (ME)

5-20 wt% of a pyridine compound of formula (I) according to the invention are added to 5-30 wt% organic solvent blend (e.g. fatty acid dimethylamide and cyclohexanone), 10-25 wt% surfactant blend (e.g. alcohol ethoxylate and arylphenol ethoxylate), and water ad 100 %. This mixture is stirred for 1 h to produce spontaneously a thermodynamically stable microemulsion.

### iv) Microcapsules (CS)

An oil phase comprising 5-50 wt% of a pyridine compound of formula (I) according to the invention, 0-40 wt% water insoluble organic solvent (e.g. aromatic hydrocarbon), 2-15 wt% acrylic monomers (e.g. methylmethacrylate, methacrylic acid, and a di- or triacrylate) are dispersed into an aqueous solution of a protective colloid (e.g. polyvinyl alcohol). Radical polymerization initiated by a radical initiator results in the formation of poly(meth)acrylate microcapsules. Alternatively, an oil phase comprising 5-50 wt% of a pyridine compound of formula (I) according to the invention, 0-40 wt% water insoluble organic solvent (e.g. aromatic hydrocarbon), and an isocyanate monomer (e.g. diphenylmethene-4,4'-diisocyanate) are dispersed into an aqueous solution of a protective colloid (e.g. polyvinyl alcohol). The addition of a polyamine (e.g. hexamethylenediamine) results in the formation of polyurea microcapsules. The monomers amount to 1-10 wt%. The wt% relate to the total CS composition.

### ix) Dustable powders (DP, DS)

1-10 wt% of a pyridine compound of formula (I) according to the invention are ground finely and mixed intimately with solid carrier (e.g. finely divided kaolin) ad 100 wt%.

### x) Granules (GR, FG)

0.5-30 wt% of a pyridine compound of formula (I) according to the invention is ground finely and associated with solid carrier (e.g. silicate) ad 100 wt%. Granulation is achieved by extrusion, spray-drying or the fluidized bed.

### xi) Ultra-low volume liquids (UL)

1-50 wt% of a pyridine compound of formula (I) according to the invention are dissolved in organic solvent (e.g. aromatic hydrocarbon) ad 100 wt%.

The agrochemical compositions types i) to xi) may optionally comprise further auxiliaries, such as 0,1-1 wt% bactericides, 5-15 wt% anti-freezing agents, 0,1-1 wt% anti-foaming agents, and 0,1-1 wt% colorants.

The agrochemical compositions comprising generally comprise between 0.01 and 95%, preferably between 0.1 and 90%, and in particular between 0.5 and 75%, by weight of the pyridine compound of formula (I). The pyridine compounds of formula (I) are employed in a purity of from 90% to 100%, preferably from 95% to 100% (according to NMR spectrum).

Solutions for seed treatment (LS), suspoemulsions (SE), flowable concentrates (FS), powders for dry treatment (DS), water-dispersible powders for slurry treatment (WS), water-soluble powders (SS), emulsions (ES), emulsifiable concentrates (EC) and gels (GF) are usually employed for the purposes of treatment of plant propagation materials, particularly seeds. The agrochemical compositions in question give, after two-to-tenfold dilution, active substance concentrations of from 0.01 to 60% by weight, preferably from 0.1 to 40% by weight, in the ready-to-use preparations. Application can be carried out before or during sowing.

Methods for applying pyridine compounds of formula (I) and agrochemical compositions thereof, on to plant propagation material, especially seeds, include dressing, coating, pelleting, dusting, soaking and in-furrow application methods of the propagation material. Preferably, pyridine compounds of formula (I) and agrochemical compositions thereof, are applied on to the plant propagation material by a method such that germination is not induced, e. g. by seed dressing, pelleting, coating and dusting.

Various types of oils, wetters, adjuvants, fertilizer, or micronutrients, and further pesticides (e.g. herbicides, insecticides, fungicides, growth regulators, safeners) may be added to the pyridine compounds of formula (I) and the agrochemical compositions comprising them as premix or, if appropriate not until immediately prior to use (tank mix). These agents can be admixed with the agrochemical compositions according to the invention in a weight ratio of 1:100 to 100:1, preferably 1:10 to 10:1.

The user applies the pyridine compound of formula (I) according to the invention and the agrochemical compositions comprising them usually from a pre-dosage device, a knapsack sprayer, a spray tank, a spray plane, or an irrigation system. Usually, the agrochemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

According to one embodiment, either individual components of the agrochemical composition according to the invention or partially premixed components, e. g. components comprising pyridine compounds of formula (I) may be mixed by the user in a spray tank and further auxiliaries and additives may be added, if appropriate.

In a further embodiment, individual components of the agrochemical composition according to the invention such as parts of a kit or parts of a binary or ternary mixture may be mixed by the user himself in a spray tank and further auxiliaries may be added, if appropriate.

In a further embodiment, either individual components of the agrochemical composition according to the invention or partially premixed components, e. g components comprising pyridine compounds of formula (I) can be applied jointly (e.g. after tank mix) or consecutively.

The pyridine compounds of formula (I), are suitable as herbicides. They are suitable as such or as an appropriately formulated composition (agrochemical composition).

The pyridine compounds of formula (I), or the agrochemical compositions comprising the pyridine compounds of formula (I), control vegetation on non-crop areas very efficiently, especially at high rates of application. They act against broad-leaved weeds and grass weeds in crops such as wheat, rice, maize, soya, and cotton without causing any significant damage to the crop plants. This effect is mainly observed at low rates of application.

The pyridine compounds of formula (I), or the agrochemical compositions comprising them, are applied to the plants mainly by spraying the leaves. Here, the application can be carried out using, for example, water as carrier by customary spraying techniques using spray liquor amounts of from about 100 to 1000 l/ha (for example from 300 to 400 l/ha). The pyridine compounds of formula (I), or the agrochemical compositions comprising them, may also be applied by the low-volume or the ultra-low-volume method, or in the form of micro granules. Application of the pyridine compounds of formula (I), or the agrochemical compositions comprising them, can be done before, during, and/or after, preferably during and/or after, the emergence of the undesirable plants.

The pyridine compounds of formula (I), or the agrochemical compositions comprising them, can be applied pre-, post-emergence or pre-plant, or together with the seed of a crop plant. It is also possible to apply the pyridine compounds of formula (I), or the agrochemical compositions comprising them, by applying seed, pretreated with the pyridine compounds of formula (I), or the agrochemical compositions comprising them, of a crop plant. If the active ingredients are less well tolerated by certain crop plants, application techniques may be used in which the herbicidal compositions are sprayed, with the aid of the spraying equipment, in such a way that as far as possible they do not come into contact with the leaves of the sensitive crop plants, while the active ingredients reach the leaves of undesirable plants growing underneath, or the bare soil surface (post-directed, lay-by).

In a further embodiment, the pyridine compounds of formula (I), or the agrochemical compositions comprising them, can be applied by treating seed. The treatment of seeds comprises essentially all procedures familiar to the person skilled in the art (seed dressing, seed coating, seed dusting, seed soaking, seed film coating, seed multilayer coating, seed encrusting, seed dripping and seed pelleting) based on the pyridine compounds of formula (I), or the agrochemical compositions prepared therefrom. Here, the herbicidal compositions can be applied diluted or undiluted.

The term "seed" comprises seed of all types, such as, for example, corns, seeds, fruits, tubers, seedlings and similar forms. Here, preferably, the term seed describes corns and seeds. The seed used can be seed of the useful plants mentioned above, but also the seed of transgenic plants or plants obtained by customary breeding methods.

When employed in plant protection, the amounts of active substances applied, i.e. the pyridine compounds of formula (I) without formulation auxiliaries, are, depending on the kind of effect desired, from 0.001 to 2 kg per ha, preferably from 0.005 to 2 kg per ha, more preferably from 0.05 to 0.9 kg per ha and in particular from 0.1 to 0.75 kg per ha.

In another embodiment of the invention, the application rate of the pyridine compounds of formula (I) is from 0.001 to 3 kg/ha, preferably from 0.005 to 2.5 kg/ha and in particular from 0.01 to 2 kg/ha of active substance (a.s.).

In another preferred embodiment of the invention, the rates of application of the pyridine compounds of formula (I) according to the present invention (total amount of pyridine compounds of formula (I)) are from 0.1 g/ha to 3000 g/ha, preferably 10 g/ha to 1000 g/ha, depending on the control target, the season, the target plants and the growth stage.

In another preferred embodiment of the invention, the application rates of the pyridine compounds of formula (I) are in the range from 0.1 g/ha to 5000 g/ha and preferably in the range from 1 g/ha to 2500 g/ha or from 5 g/ha to 2000 g/ha.

In another preferred embodiment of the invention, the application rate of the pyridine compounds of formula (I) is 0.1 to 1000 g/ha, preferably1 to 750 g/ha, more preferably 5 to 500 g/ha.

In treatment of plant propagation materials such as seeds, e. g. by dusting, coating or drenching seed, amounts of active substance of from 0.1 to 1000 g, preferably from 1 to 1000 g, more preferably from 1 to 100 g, and most preferably from 5 to 100 g, per 100 kilogram of plant propagation material (preferably seeds) are generally required.

In another embodiment of the invention, to treat the seed, the amounts of active substances applied, i.e. the pyridine compounds of formula (I) are generally employed in amounts of from 0.001 to 10 kg per 100 kg of seed.

When used in the protection of materials or stored products, the amount of active substance applied depends on the kind of application area and on the desired effect. Amounts customarily applied in the protection of materials are 0.001 g to 2 kg, preferably 0.005 g to 1 kg, of active substance per cubic meter of treated material.

Depending on the application method in question, the pyridine compounds of formula (I), or the agrochemical compositions comprising them, can additionally be employed in a further number of crop plants for eliminating undesirable plants. Examples of suitable crops are the following:
Allium cepa, Ananas comosus, Arachis hypogaea, Asparagus officinalis, Avena sativa, Beta vulgaris spec. altissima, Beta vulgaris spec. rapa, Brassica napus var. napus, Brassica napus var. napobrassica, Brassica rapa var. silvestris, Brassica oleracea, Brassica nigra, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pistacia vera, Pisum sativum, Prunus avium, Prunus persica, Pyrus communis, Prunus armeniaca, Prunus cerasus, Prunus dulcis and Prunus domestica, Ribes sylvestre, Ricinus communis, Saccharum officinarum, Secale cereale, Sinapis alba, Solanum tuberosum, Sorghum bicolor (s. vulgare), Theobroma cacao, Trifolium pratense, Triticum aestivum, Triticale, Triticum durum, Vicia faba, Vitis vinifera, and Zea mays.

Preferred crops are Arachis hypogaea, Beta vulgaris spec. altissima, Brassica napus var. napus, Brassica oleracea, Citrus limon, Citrus sinensis, Coffea arabica (Coffea canephora, Coffea liberica), Cynodon dactylon, Glycine max, Gossypium hirsutum, (Gossypium arboreum, Gossypium herbaceum, Gossypium vitifolium), Helianthus annuus, Hordeum vulgare, Juglans regia, Lens culinaris, Linum usitatissimum, Lycopersicon lycopersicum, Malus spec., Medicago sativa, Nicotiana tabacum (N.rustica), Olea europaea, Oryza sativa , Phaseolus lunatus, Phaseolus vulgaris, Pistacia vera, Pisum sativum, Prunus dulcis, Saccharum officinarum, Secale cereale, Solanum tuberosum, Sorghum bicolor (s. vulgare), Triticale, Triticum aestivum, Triticum durum, Vicia faba, Vitis vinifera, and Zea mays.

Especially preferred crops are crops of cereals, corn, soybeans, rice, oilseed rape, cotton, potatoes, peanuts, or permanent crops.

The pyridine compounds of formula (I) according to the invention, or the agrochemical compositions comprising them, can also be used in genetically modified plants. The term "genetically modified plants" is to be understood as plants whose genetic material has been modified by the use of recombinant DNA techniques to include an inserted sequence of DNA that is not native to that plant species' genome or to exhibit a deletion of DNA that was native to that species' genome, wherein the modification(s) cannot readily be obtained by cross breeding, mutagenesis or natural recombination alone. Often, a particular genetically modified plant will be one that has obtained its genetic modification(s) by inheritance through a natural breeding or propagation process from an ancestral plant whose genome was the one directly treated by use of a recombinant DNA technique. Typically, one or more genes have been integrated into the genetic material of a genetically modified plant in order to improve certain properties of the plant. Such genetic modifications also include but are not limited to targeted post-translational modification of protein(s), oligo- or polypeptides. e. g., by inclusion therein of amino acid mutation(s) that permit, decrease, or promote glycosylation or polymer additions such as prenylation, acetylation farnesylation, or PEG moiety attachment.

Plants that have been modified by breeding, mutagenesis or genetic engineering, e.g. have been rendered tolerant to applications of specific classes of herbicides, such as auxin herbicides such as dicamba or 2,4-D; bleacher herbicides such as hydroxyphenylpyruvate dioxygenase (HPPD) inhibitors or phytoene desaturase (PDS) inhibitors; acetolactate synthase (ALS) inhibitors such as sulfonyl ureas or imidazolinones; enolpyruvyl shikimate 3-phosphate synthase (EPSP) inhibitors such as glyphosate; glutamine synthetase (GS) inhibitors such as glufosinate; protoporphyrinogen-IX oxidase inhibitors; lipid biosynthesis inhibitors such as acetyl CoA carboxylase (ACCase) inhibitors; or oxynil (i. e. bromoxynil or ioxynil) herbicides as a result of conventional methods of breeding or genetic engineering; furthermore, plants have been made resistant to multiple classes of herbicides through multiple genetic modifications, such as resistance to both glyphosate and glufosinate or to both glyphosate and a herbicide from another class such as ALS inhibitors, HPPD inhibitors, auxin herbicides, or ACCase inhibitors. These herbicide resistance technologies are, for example, described in Pest Management Science 61, 2005, 246; 61, 2005, 258; 61, 2005, 277; 61, 2005, 269; 61, 2005, 286; 64, 2008, 326; 64, 2008, 332; Weed Science 57, 2009, 108; Australian Journal of Agricultural Research 58, 2007, 708; Science 316, 2007, 1185; and references quoted therein. Several cultivated plants have been rendered tolerant to herbicides by mutagenesis and conventional methods of breeding, e. g., Clearfield® summer rape (Canola, BASF SE, Germany) being tolerant to imidazolinones, e. g., imazamox, or ExpressSun® sunflowers (DuPont, USA) being tolerant to sulfonyl ureas, e. g., tribenuron. Genetic engineering methods have been used to render cultivated plants such as soybean, cotton, corn, beets and rape, tolerant to herbicides such as glyphosate, imidazolinones, and glufosinate, some of which are under development or commercially available under the brands or trade names RoundupReady® (glyphosate tolerant, Monsanto, USA), Cultivance® (imidazolinone tolerant, BASF SE, Germany), and LibertyLink® (glufosinate tolerant, Bayer CropScience, Germany).

Furthermore, plants are also covered that are by the use of recombinant DNA techniques capable to synthesize one or more insecticidal proteins, especially those known from the bacterial genus Bacillus, particularly from Bacillus thuringiensis, such as delta-endotoxins, e. g., CryIA(b), CryIA(c), CryIF, CryIF(a2), CryIIA(b), CryIIIA, CryIIIB(b1) or Cry9c; vegetative insecticidal proteins (VIP), e. g., VIP1, VIP2, VIP3, or VIP3A; insecticidal proteins of bacteria colonizing nematodes, e. g., Photorhabdus spp. or Xenorhabdus spp.; toxins produced by animals, such as scorpion toxins, arachnid toxins, wasp toxins, or other insect-specific neurotoxins; toxins produced by fungi, such as Streptomycetes toxins, plant lectins, such as pea or barley lectins; agglutinins; proteinase inhibitors, such as trypsin inhibitors, serine protease inhibitors, patatin, cystatin or papain inhibitors; ribosome-inactivating proteins (RIP), such as ricin, maize-RIP, abrin, luffin, saporin or bryodin; steroid metabolism enzymes, such as 3-hydroxy-steroid oxidase, ecdysteroid-IDP-glycosyl-transferase, cholesterol oxidases, ecdysone inhibitors or HMG-CoA-reductase; ion channel blockers, such as blockers of sodium or calcium channels; juvenile hormone esterase; diuretic hormone receptors (helicokinin receptors); stilbene synthase, bibenzyl synthase, chitinases or glucanases. In the context of the present invention these insecticidal proteins or toxins are to be understood expressly also as including pre-toxins, hybrid proteins, truncated or otherwise modified proteins. Hybrid proteins are characterized by a new combination of protein domains, (see, e. g., WO 02/015701). Further examples of such toxins or genetically modified plants capable of synthesizing such toxins are disclosed, e. g., in EP-A 374 753, WO 93/007278, WO 95/34656, EP-A 427 529, EP-A 451 878, WO 03/18810, and WO 03/52073. The methods for producing such genetically modified plants are generally known to the person skilled in the art and are described, e. g., in the publications mentioned above. These insecticidal proteins contained in the genetically modified plants impart to the plants producing these proteins tolerance to harmful pests from all taxonomic groups of arthropods, especially to beetles (Coeloptera), two-winged insects (Diptera), and moths (Lepidoptera) and to nematodes (Nematoda). Genetically modified plants capable to synthesize one or more insecticidal proteins are, e. g., described in the publications mentioned above, and some of which are commercially available such as YieldGard® (corn cultivars producing the Cry1Ab toxin), YieldGard@ Plus (corn cultivars producing Cry1Ab and Cry3Bb1 toxins), Starlink® (corn cultivars producing the Cry9c toxin), Herculex® RW (corn cultivars producing Cry34Ab1, Cry35Ab1 and the enzyme Phosphinothricin-N-Acetyltransferase [PAT]); NuCOTN® 33B (cotton cultivars producing the Cry1Ac toxin), Bollgard® I (cotton cultivars producing the Cry1Ac toxin), Bollgard® II (cotton cultivars producing Cry1Ac and Cry2Ab2 toxins); VIPCOT® (cotton cultivars producing a VIP-toxin); NewLeaf® (potato cultivars producing the Cry3A toxin); Bt-Xtra®, NatureGard®, KnockOut®, BiteGard®, Protecta®, Bt11 (e. g., Agrisure® CB) and Bt176 from Syngenta Seeds SAS, France, (corn cultivars producing the Cry1Ab toxin and PAT enzyme), MIR604 from Syngenta Seeds SAS, France (corn cultivars producing a modified version of the Cry3A toxin, c.f. WO 03/018810), MON 863 from Monsanto Europe S.A., Belgium (corn cultivars producing the Cry3Bb1 toxin), IPC 531 from Monsanto Europe S.A., Belgium (cotton cultivars producing a modified version of the Cry1Ac toxin) and 1507 from Pioneer Overseas Corporation, Belgium (corn cultivars producing the Cry1 F toxin and PAT enzyme).

Furthermore, plants are also covered that are by the use of recombinant DNA techniques capable to synthesize one or more proteins to increase the resistance or tolerance of those plants to bacterial, viral or fungal pathogens. Examples of such proteins are the so-called "pathogenesis-related proteins" (PR proteins, see, e.g., EP-A 392 225), plant disease resistance genes (e. g., potato culti-vars, which express resistance genes acting against Phytophthora infestans derived from the Mexican wild potato, Solanum bulbocastanum) or T4-lyso-zym (e.g., potato cultivars capable of synthesizing these proteins with increased resistance against bacteria such as Erwinia amylovora). The methods for producing such genetically modi-fied plants are generally known to the person skilled in the art and are described, e.g., in the publications mentioned above.

Furthermore, plants are also covered that are by the use of recombinant DNA techniques capable to synthesize one or more proteins to increase the productivity (e.g., bio-mass production, grain yield, starch content, oil content or protein content), tolerance to drought, salinity or other growth-limiting environmental factors or tolerance to pests and fungal, bacterial or viral pathogens of those plants.

Furthermore, plants are also covered that contain by the use of recombinant DNA techniques a modified amount of ingredients or new ingredients, specifically to improve human or animal nutrition, e. g., oil crops that produce health-promoting long-chain omega-3 fatty acids or unsaturated omega-9 fatty acids (e. g., Nexera® rape, Dow AgroSciences, Canada).

Furthermore, plants are also covered that contain by the use of recombinant DNA techniques a modified amount of ingredients or new ingredients, specifically to improve raw material production, e.g., potatoes that produce increased amounts of amylopectin (e.g. Amflora® potato, BASF SE, Germany).

## Claims

1. The pyridine compounds of formula (I) 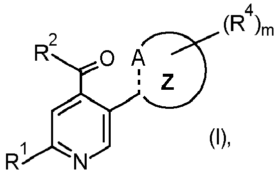 including their agriculturally acceptable salts or derivatives, provided the pyridine compounds of formula (I) have an acidic functionality,
wherein in formula (I) the variables have the following meanings:
the dotted line (------) is a single bond or a double bond;;
R¹ is C₁-C₆-alkyl, C₁-C₆-haloalkyl, hydroxy-C₁-C₆-alkyl, C₂-C₆-alkenyl, C₂-C₆-haloalkenyl, C₂-C₆-alkynyl, C₃-C₆-haloalkynyl, C₁-C₆-alkoxy-C₁-C₆-alkyl, C₁-C₆-alkoxy, C₃-C₆-alkenyloxy, C₃-C₆-haloalkenyloxy, C₃-C₆-alkynyloxy, C₃-C₆-haloalkynyloxy, C₁-C₆-haloalkoxy, C₃-C₆-cycloalkoxy, C₃-C₆-halocycloalkoxy, C₃-C₆-cycloalkenyloxy, C₃-C₆-halocycloalkenyloxy, C₁-C₆-alkylthio, C₁-C₆-haloalkylthio, (C₁-C₆-alkyl)amino, di(C₁-C₆-alkyl)amino, C₁-C₆-alkylsulfinyl, C₁-C₆-alkylsulfonyl, C₃-C₆-cycloalkyl, C₃-C₆-cycloalkenyl, C₃-C₆-halocycloalkyl, C₃-C₆-halocycloalkenyl, [1-(C₁-C₆-alkyl)]-C₃-C₆-cycloalkyl, [1-(C₂-C₆-alkenyl)]-C₃-C₆-cycloalkyl, [1-(C₂-C₆-alkynyl)]-C₃-C₆-cycloalkyl, [1-(C₁-C₆-haloalkyl)]-C₃-C₆-cycloalkyl, [1-(C₂-C₆-haloalkenyl)]-C₃-C₆-cycloalkyl, [1-(C₃-C₆-haloalkynyl)]-C₃-C₆-cycloalkyl, C₃-C₆-cycloalkyl-C₁-C₆-alkyl, C₃-C₆-cycloalkyl-C₁-C₆-haloalkyl, C₃-C₆-cycloalkyl-C₁-C₆-alkoxy, C₃-C₆-cycloalkyl-C₁-C₆-haloalkoxy, phenyl, 5- or 6-membered heteroaryl, or 3- to 6-membered heterocyclyl;
wherein the cyclic groups of R¹ are unsubstituted or substituted by R^{a};
R² is H, halogen, C₁-C₆-alkyl, C₁-C₆-haloalkyl, C₁-C₆-alkylcarbonyl-C₁-C₆-alkyl, C₁-C₆-alkoxycarbonyl-C₁-C₆-alkyl, C₁-C₆-haloalkylcarbonyl-C₁-C₆-alkyl, C₁-C₆-haloalkoxy-carbonyl-C₁-C₆-alkyl, C₁-C₆-alkylcarbonyl-C₁-C₆-haloalkyl, C₁-C₆-alkoxycarbonyl-C₁-C₆-haloalkyl, C₁-C₆-haloalkylcarbonyl-C₁-C₆-haloalkyl, C₁-C₆-haloalkoxycarbonyl-C₁-C₆-haloalkyl, OH, C₁-C₆-alkoxy, C₁-C₆-alkoxy-C₁-C₆-alkoxy, C₁-C₆-haloalkoxy-C₁-C₆-alkoxy, C₁-C₆-alkoxy-C₁-C₆-haloalkoxy, C₁-C₆-haloalkoxy-C₁-C₆-haloalkoxy, C₁-C₆-alkoxy-C₁-C₆-alkoxy-C₁-C₆-alkoxy, C₁-C₆-haloalkoxy, C₁-C₆-cyanoalkoxy, C₁-C₆-hydr-oxyalkoxy, C₃-C₆-alkenyloxy, C₃-C₆-alkenyloxy-C₁-C₆-alkoxy, C₃-C₆-haloalkenyloxy-C₁-C₆-haloalkoxy, C₃-C₆-alkenyloxy- C₁-C₆-haloalkoxy, C₃-C₆-haloalkenyloxy, C₃-C₆-alkynyloxy, C₃-C₆-haloalkynyloxy, C₃-C₆-alkynyloxy-C₁-C₆-alkoxy, C₃-C₆-haloal-kynyloxy-C₁-C₆-haloalkoxy, C₃-C₆-alkynyloxy- C₁-C₆-haloalkoxy, C₃-C₆-alkyn-yloxy- C₃-C₆-alkenyloxy, C₃-C₆-haloalkynyloxy- C₃-C₆-alkenyloxy, C₃-C₆-alkynyloxy- C₃-C₆-haloalkenyloxy, C₃-C₆-haloalkynyloxy- C₃-C₆-haloalkenyloxy, C₃-C₆-alkynyloxy- C₃-C₆-alkynyloxy, C₃-C₆-haloalkynyloxy- C₃-C₆-alkynyloxy, C₃-C₆-alkynyloxy- C₃-C₆-haloalkynyloxy, C₃-C₆-haloalkynyloxy- C₃-C₆-haloalkynyloxy, (C₁-C₆-alkyl)carbonyl-C₁-C₆-alkoxy, (C₁-C₆-haloalkyl)carbonyl-C₁-C₆-haloalkoxy, (C₁-C₆-haloalkyl)carbonyl-C₁-C₆-alkoxy, (C₁-C₆-alkyl)carbonyl-C₁-C₆-haloalkoxy, (C₁-C₆-alkoxy)carbonyl-C₁-C₆-alkoxy, (C₁-C₆-haloalkoxy)carbonyl-C₁-C₆-alkoxy, (C₁-C₆-alkoxy)carbonyl-C₁-C₆-haloalkoxy, (C₁-C₆-haloalkoxy)carbonyl-C₁-C₆-haloalkoxy, (C₁-C₆-alkoxy-C₁-C₆-alkyl)carbonyl-C₁-C₆-alkoxy, (C₁-C₆-haloalkoxy-C₁-C₆-alkyl)carbonyl-C₁-C₆-alkoxy, (C₁-C₆-alkoxy-C₁-C₆-haloalkyl)carbonyl-C₁-C₆-alkoxy, (C₁-C₆-alkoxy-C₁-C₆-alkyl)carbonyl-C₁-C₆-haloalkoxy, (C₁-C₆-haloalkoxy-C₁-C₆-haloalkyl)-carbonyl-C₁-C₆-alkoxy, (C₁-C₆-haloalkoxy-C₁-C₆-alkyl)carbonyl-C₁-C₆-haloalkoxy, (C₁-C₆-alkoxy-C₁-C₆-haloalkyl)carbonyl-C₁-C₆-haloalkoxy, (C₁-C₆-haloalkoxy-C₁-C₆-haloalkyl)carbonyl-C₁-C₆-haloalkoxy, (C₁-C₆-alkylthio)carbonyl-C₁-C₆-alkoxy, (C₁-C₆-haloalkylthio)carbonyl-C₁-C₆-alkoxy, (C₁-C₆-alkylthio)carbonyl-C₁-C₆-haloalkoxy, (C₁-C₆-haloalkylthio)carbonyl-C₁-C₆-haloalkoxy, (C₁-C₆-alkylthio-C₁-C₆-alkyl)carbonyl-C₁-C₆-alkoxy, (C₁-C₆-haloalkylthio-C₁-C₆-alkyl)carbonyl-C₁-C₆-alkoxy, (C₁-C₆-alkylthio-C₁-C₆-haloalkyl)carbonyl-C₁-C₆-alkoxy, (C₁-C₆-alkylthio-C₁-C₆-alkyl)car-bonyl-C₁-C₆-haloalkoxy, (C₁-C₆-haloalkylthio-C₁-C₆-haloalkyl)carbonyl-C₁-C₆-alkoxy, (C₁-C₆-haloalkylthio-C₁-C₆-alkyl)carbonyl-C₁-C₆-haloalkoxy, (C₁-C₆-alkylthio-C₁-C₆-haloalkyl)carbonyl-C₁-C₆-haloalkoxy, (C₁-C₆-haloalkylthio-C₁-C₆-haloalkyl)carbonyl-C₁-C₆-haloalkoxy, C₃-C₆-cycloalkoxy, C₃-C₆-halocycloalkoxy, (C₃-C₆-cycloalkyl)C₁-C₆-alkoxy, (C₃-C₆-halocycloalkyl)C₁-C₆-alkoxy, (C₃-C₆-cycloalkyl)C₁-C₆-haloalkoxy, aminocarbonyl- C₁-C₆-alkoxy, (C₃-C₆-halocycloalkyl)C₁-C₆-haloalkoxy, aminocar-bonyl-C₁-C₆-haloalkoxy, N-(C₁-C₆-alkyl)-aminocarbonyl-C₁-C₆-alkoxy, N-(C₁-C₆-alkyl)-aminocarbonyl- C₁-C₆-haloalkoxy, N,N-di(C₁-C₆-alkyl)-aminocarbonyl- C₁-C₆-alkoxy, N,N-di(C₁-C₆-alkyl)-aminocarbonyl- C₁-C₆-haloalkoxy, O-N=C(di(phenyl), O-N=C(phenyl)(C₁-C₆-alkyl), O-N=C[di(C₁-C₆-alkyl)], (C₁-C₆-alkyl)₃-silyl-C₁-C₆-alkoxy, C₁-C₆-alkylthio, C₁-C₆-haloalkylthio, C₁-C₆-alkoxy-C₁-C₆-alkylthio, C₁-C₆-haloalkoxy-C₁-C₆-alkylthio, C₁-C₆-alkoxy-C₁-C₆-haloalkylthio, C₁-C₆-haloalkoxy-C₁-C₆-haloalkylthio, C₁-C₆-alkoxy-C₁-C₆-alkoxy- C₁-C₆-alkylthio, C₁-C₆-cyanoalkylthio, C₃-C₆-alkenylthio, C₂-C₆-haloalke-nylthio, C₃-C₆-alkenyloxy-C₁-C₆-alkylthio, C₃-C₆-haloalkenyloxy-C₁-C₆-alkylthio, C₃-C₆-alkenyloxy-C₁-C₆-haloalkylthio, C₃-C₆-haloalkenyloxy-C₁-C₆-haloalkylthio, C₃-C₆-alkynylthio, C₃-C₆-haloalkynylthio, C₃-C₆-alkynyloxy-C₁-C₆-alkylthio, C₃-C₆-haloalk-ynyloxy-C₁-C₆-haloalkylthio, C₃-C₆-alkynyloxy-C₁-C₆-haloalkylthio, C₃-C₆-alkynyl-oxy- C₃-C₆-alkenylthio, C₃-C₆-haloalkynyloxy- C₃-C₆-alkenylthio, C₃-C₆-alkynyloxy-C₃-C₆-haloalkenylthio, C₃-C₆-haloalkynyloxy-C₃-C₆-haloalkenylthio, C₃-C₆-alkynyl-oxy- C₃-C₆-alkynylthio, C₃-C₆-haloalkynyloxy- C₃-C₆-alkynylthio, C₃-C₆-alkynyloxy-C₃-C₆-haloalkynylthio, C₃-C₆-haloalkynyloxy-C₃-C₆-haloalkynylthio, (C₁-C₆-alkyl)-carbonyl-C₁-C₆-alkylthio, (C₁-C₆-haloalkyl)carbonyl-C₁-C₆-alkylthio, (C₁-C₆-alkyl)car-bonyl-C₁-C₆-haloalkylthio, (C₁-C₆-haloalkyl)carbonyl-C₁-C₆-haloalkylthio, (C₁-C₆-alkoxy)carbonyl-C₁-C₆-alkylthio, (C₁-C₆-haloalkoxy)carbonyl-C₁-C₆-alkylthio, (C₁-C₆-alkoxy)carbonyl-C₁-C₆-haloalkylthio, (C₁-C₆-haloalkoxy)carbonyl-C₁-C₆-haloalkylthio, (C₁-C₆-alkoxy-C₁-C₆-alkyl)carbonyl-C₁-C₆-alkylthio, (C₁-C₆-haloalkoxy-C₁-C₆-alkyl)-carbonyl-C₁-C₆-alkylthio, (C₁-C₆-alkoxy-C₁-C₆-haloalkyl)carbonyl-C₁-C₆-alkylthio, (C₁-C₆-alkoxy-C₁-C₆-alkyl)carbonyl-C₁-C₆-haloalkylthio, (C₁-C₆-haloalkoxy-C₁-C₆-haloal-kyl)carbonyl-C₁-C₆-alkylthio, (C₁-C₆-haloalkoxy-C₁-C₆-alkyl)carbonyl-C₁-C₆-haloalkyl-thio, (C₁-C₆-alkoxy-C₁-C₆-haloalkyl)carbonyl-C₁-C₆-haloalkylthio, (C₁-C₆-haloalkoxy-C₁-C₆-haloalkyl)carbonyl-C₁-C₆-haloalkylthio, (C₁-C₆-alkylthio)carbonyl-C₁-C₆-alkylthio, (C₁-C₆-haloalkylthio)carbonyl-C₁-C₆-alkylthio, (C₁-C₆-alkylthio)carbonyl-C₁-C₆-haloalkylthio, (C₁-C₆-haloalkylthio)carbonyl-C₁-C₆-haloalkylthio, (C₁-C₆-alkylthio-C₁-C₆-alkyl)carbonyl-C₁-C₆-alkylthio, (C₁-C₆-haloalkylthio-C₁-C₆-alkyl)carbonyl-C₁-C₆-alkylthio, (C₁-C₆-alkylthio-C₁-C₆-haloalkyl)carbonyl-C₁-C₆-alkylthio, (C₁-C₆-alkylthio-C₁-C₆-alkyl)carbonyl-C₁-C₆-haloalkylthio, (C₁-C₆-haloalkylthio-C₁-C₆-haloalkyl)carbonyl-C₁-C₆-alkylthio, (C₁-C₆-haloalkylthio-C₁-C₆-alkyl)carbonyl-C₁-C₆-haloalkylthio, (C₁-C₆-alkylthio-C₁-C₆-haloalkyl)carbonyl-C₁-C₆-haloalkylthio, (C₁-C₆-haloalkylthio-C₁-C₆-haloalkyl)carbonyl-C₁-C₆-haloalkylthio, C₃-C₆-cycloalkylthio, C₃-C₆-halocycloalkylthio, (C₃-C₆-cycloalkyl)C₁-C₆-alkylthio, (C₃-C₆-cycloalkyl)C₁-C₆-haloalkylthio, (C₃-C₆-halocycloalkyl)C₁-C₆-alkylthio, (C₃-C₆-halocycloalkyl)C₁-C₆-haloalkylthio, aminocarbonyl- C₁-C₆-alkylthio, aminocarbonyl- C₁-C₆-haloalkylthio, N-(C₁-C₆-alkyl)-aminocarbonyl- C₁-C₆-alkylthio, N-(C₁-C₆-haloalkyl)-aminocarbonyl- C₁-C₆-alkylthio, N-(C₁-C₆-alkyl)-aminocarbonyl-C₁-C₆-haloalkylthio, N-(C₁-C₆-haloalkyl)-aminocarbonyl- C₁-C₆-haloalkylthio, N,N-di(C₁-C₆-alkyl)-aminocarbonyl- C₁-C₆-alkylthio, N,N-di(C₁-C₆-haloalkyl)-aminocarbonyl-C₁-C₆-alkylthio, N,N-di(C₁-C₆-alkyl)-aminocarbonyl- C₁-C₆-haloalkylthio, N,N-di(C₁-C₆-haloalkyl)-aminocarbonyl-C₁-C₆-haloalkylthio, NH₂, (C₁-C₆-alkyl)amino, hydroxyamino, (C₁-C₆-alkoxy)amino, (C₃-C₆-cycloalkoxy)amino, (C₁-C₆-alkyl)sulfinylamino, (C₁-C₆-alkyl)sulfonylamino, (amino)sulfinylamino, [(C₁-C₆-alkyl)amino]sulfinylamino, (amino)sulfonylamino, [(C₁-C₆-alkyl)amino]sulfonylamino, [di(C₁-C₆-alkyl)amino]sulfonylamino, di(C₁-C₆-alkyl)amino, (hydroxy)(C₁-C₆-alkyl)amino, (hydroxy)(C₁-C₆-cycloalkyl)amino, (C₁-C₆-alkoxy)(C₁-C₆-alkyl)amino, (C₁-C₆-alkoxy)(C₃-C₆-cycloalkyl)amino, (C₃-C₆-cycloalkoxy)(C₁-C₆-alkyl)amino, (C₃-C₆-cycloalkoxy)(C₃-C₆-cycloalkyl)amino, [(C₁-C₆-alkyl)sulfinyl](C₁-C₆-alkyl)amino, [(C₁-C₆-alkyl)sulfonyl](C₁-C₆-alkyl)amino, [di(C₁-C₆-alkyl)amino]sulfinylamino, [di(C₁-C₆-alkyl)amino]sulfonylamino, phenyloxy, phenyl-C₁-C₆-alkoxy, phenyl-thio, phenyl-C₁-C₆-alkylthio, phenylamino, (C₁-C₆-alkyl)(phenyl)amino, C₁-C₆-alkylcarbonylamino, [(C₁-C₆-alkyl)carbonyl](C₁-C₆-alkyl)amino, C₁-C₆-haloalkylcarbonylamino, [(C₁-C₆-haloalkyl)carbonyl](C₁-C₆-alkyl)amino, C₃-C₆-cycloalkylcarbonylamino, [(C₃-C₆-cycloalkyl)carbonyl](C₁-C₆-alkyl)amino, phenylcarbonylamino, (phenylcarbonyl)(C₁-C₆-alkyl)amino, heterocyclylcarbonylamino, (heterocyclylcarbonyl)(C₁-C₆-alkyl)amino, heteroarylcarbonylamino, (heteroarylcarbonyl)(C₁-C₆-alkyl)amino, [(C₁-C₆-alkyl)carbonyl](C₁-C₆-alkoxy)amino, [(C₁-C₆-haloalkyl)carbonyl](C₁-C₆-alkoxy)amino, [(C₃-C₆-cycloalkyl)carbonyl](C₁-C₆-alkyloxy)amino, (phenylcarbonyl)(C₁-C₆-alkoxy)amino, (heterocyclylcarbonyl)(C₁-C₆-alkoxy)amino, (heteroarylcarbonyl)(C₁-C₆-alkoxy)amino, [(C₁-C₆-alkyl)carbonyl](C₂-C₆-alkenyl)amino, [(C₁-C₆-haloalkyl)carbonyl](C₂-C₆- alkenyl)amino, [(C₃-C₆-cycloalkyl)carbonyl](C₂-C₆-alkenyl)amino, (phenylcarbonyl)(C₂-C₆-alkenyl)amino, (heterocyclylcarbonyl)(C₂-C₆-alkenyl)amino, (heteroarylcarbonyl)(C₂-C₆-alkenyl)amino, [(C₁-C₆-alkyl)carbonyl](C₃-C₆-alkynyl)amino, [(C₁-C₆-haloalkyl)carbonyl](C₃-C₆-alkynyl)amino, [(C₃-C₆-cycloalkyl)carbonyl](C₃-C₆-alkynyl)amino, (phenylcarbonyl)(C₃-C₆-alkynyl)amino, (heterocyclylcarbonyl)(C₃-C₆-alkynyl)amino, (heteroarylcarbonyl)(C₃-C₆-alkynyl)amino, [(C₂-C₆-alkenyl)carbonyl]amino, [(C₂-C₆-alkenyl)carbonyl](C₁-C₆-alkyl)amino, [(C₂-C₆-alkenyl)carbonyl](C₁-C₆-alkoxy)amino, [(C₃-C₆-alkynyl)carbonyl]amino, [(C₃-C₆-alkynyl)carbonyl](C₁-C₆-alkyl)amino, [(C₃-C₆-alkynyl)carbonyl](C₁-C₆-alkoxy)amino, [di(C₁-C₆-alkyl)amino]carbonylaminocarbonyl, [di(C₁-C₆-alkyl)aminocarbonyl](C₁-C₆-alkyl)amino, [di(C₁-C₆-alkyl)aminocarbonyl](C₁-C₆-alkoxy)amino, (heteroaryl)oxy, heteroaryl-C₁-C₆-alkoxy, (heterocyclyl)oxy, or heterocyclyl-C₁-C₆-alkoxy;
wherein the cyclic groups of R² are unsubstituted or substituted by R^{a};
Z is a 9 or 10 membered bicyclic ring comprising A;
A is C*, CR³, NR^{3A}, N, O, or S;
C* is a bridge carbon of the bicyclic ring Z;
R³ is halogen, CN, CHO, NO₂, C₁-C₆-alkyl, C₁-C₆-haloalkyl, C₁-C₆-alkylcarbonyl, C₂-C₆-alkenyl, C₂-C₆-haloalkenyl, C₂-C₆-alkenyl, C₂-C₆-haloalkenyl, C₁-C₆-alkoxy, C₁-C₆-haloalkoxy, C₃-C₆-alkenyloxy, C₃-C₆-haloalkenyloxy, C₃-C₆-alkenyloxy, C₃-C₆-haloalkenyloxy, C₁-C₆-alkoxy-C₁-C₆-alkoxy, hydroxycarbonyl, C₁-C₆-alkoxycarbonyl, C₁-C₆-alkylthio, C₁-C₆-haloalkylthio, NH₂, (C₁-C₆-alkyl)amino, di(C₁-C₆-alkyl)amino, (C₁C₆-alkyl)sulfinyl, (C₁-C₆-alkyl)sulfonyl, C₃-C₆-cycloalkyl, (C₃-C₆-cycloalkyl)oxy, or phenyl;
wherein the cyclic groups of R³ are unsubstituted or substituted by substituents R^{a};
R^{3A} is H, C₁-C₆-alkyl, C₁-C₆-haloalkyl, C₁-C₆-alkylcarbonyl, C₂-C₆-alkenyl, C₂-C₆-haloalkenyl, C₂-C₆-alkenyl, C₂-C₆-haloalkenyl, C₁-C₆-alkoxy, C₁-C₆-haloalkoxy, C₃-C₆-alkenyloxy, C₃-C₆-haloalkenyloxy, C₃-C₆-alkenyloxy, C₃-C₆-haloalkenyloxy, C₁-C₆-alkoxy-C₁-C₆-alkoxy, C₁-C₆-alkoxycarbonyl, C₁-C₆-alkylthio, C₁-C₆-haloalkylthio, NH₂, (C₁-C₆-alkyl)amino, di(C₁-C₆-alkyl)amino, (C₁-C₆-alkyl)sulfinyl, (C₁-C₆-alkyl)sulfonyl, C₃-C₆-cycloalkyl, (C₃-C₆-cycloalkyl)oxy, or phenyl;
wherein the cyclic groups of R^{3A} are unsubstituted or substituted by R^{a};
R⁴ is halogen, CN, CHO, NO₂, C₁-C₆-alkyl, C₁-C₆-haloalkyl, C₁-C₆-alkylcarbonyl, C₂-C₆-alkenyl, C₂-C₆-haloalkenyl, C₂-C₆-alkenyl, C₂-C₆-haloalkenyl, C₁-C₆-alkoxy, C₁-C₆-haloalkoxy, C₃-C₆-alkenyloxy, C₃-C₆-haloalkenyloxy, C₃-C₆-alkenyloxy, C₃-C₆-haloalkenyloxy, C₁-C₆-alkoxy-C₁-C₆-alkoxy, hydroxycarbonyl, C₁-C₆-alkoxycarbonyl, C₁-C₆-alkylthio, C₁-C₆-haloalkylthio, NH₂, (C₁-C₆-alkyl)amino, di(C₁-C₆-alkyl)amino, (C₁-C₆-alkyl)sulfinyl, (C₁-C₆-alkyl)sulfonyl, C₃-C₆-cycloalkyl, (C₃-C₆-cycloalkyl)oxy, or phenyl;
wherein the cyclic groups of R⁴ are unsubstituted or substituted by R^{a};
R^{a} is halogen, CN, NO₂, C₁-C₆-alkyl, C₁-C₆-haloalkyl, C₁-C₆-alkoxy, or C₁-C₆-haloalkoxy;
m is 0, 1, 2, or 3.

2. The pyridine compounds of formula (I) according to claim 1, wherein the bicyclic ring Z is selected from rings A to O, 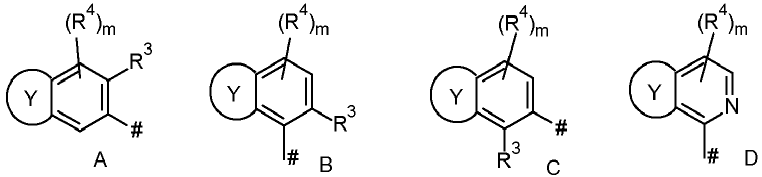 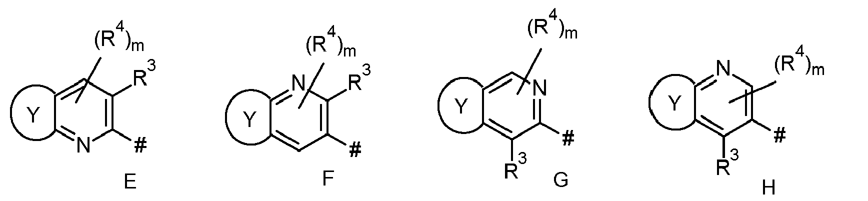 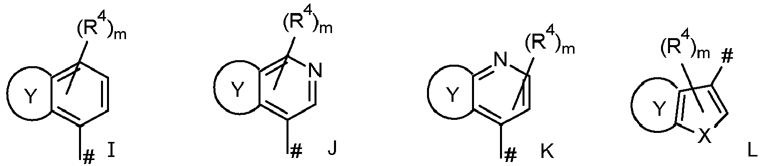 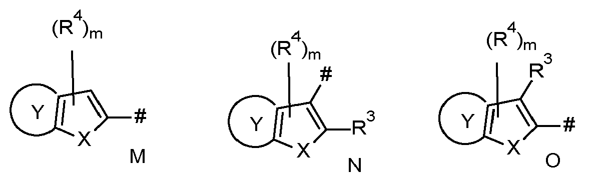 wherein
Y is 5- or 6-membered partially or fully unsaturated carbocycle comprising 0, 1, 2, or 3 heteroatoms selected from O, N, and S;
R³ is halogen, CN, C₁-C₆-alkyl, C₁-C₆-haloalkyl, or C₁-C₆-alkoxy;
m is 0, 1 or 2;
R⁴ is halogen, CN, C₁-C₆-alkyl, C₁-C₆-haloalkyl, or C₁-C₆-alkoxy;
X is O, S, or NR^{3A};
R^{3A} is H, C₁-C₆-alkyl, C₁-C₆-haloalkyl, C₁-C₆-alkylcarbonyl, C₃-C₆-alkenyl, C₃-C₆-haloalkenyl, C₃-C₆-alkenyl, C₃-C₆-haloalkenyl, or C₃-C₆-cycloalkyl; and
# denotes the point of attachment to the pyridine ring.

3. The pyridine compounds of formula (I) according to claim 1 to 2, wherein
R¹ is C₁-C₆-alkyl, C₁-C₆-alkoxy, C₁-C₆-haloalkoxy, C₃-C₆-alkenyloxy, C₃-C₆-haloalkenyloxy, C₃-C₆-alkynyloxy, C₃-C₆-haloalkynyloxy, C₁-C₆-alkylthio, or C₃-C₆-cycloalkyl, wherein the cycloalkyl substituent is unsubstituted.

4. The pyridine compounds of formula (I) according to any of claims 1 to 3, wherein
R² is OH, C₁-C₆-alkoxy, C₁-C₆-haloalkoxy, C₃-C₆-alkynyloxy, C₁-C₆-alkylthio, phenyloxy, C₁-C₆-alkyl-carbonylamino, C₁-C₆-haloalkylcarbonylamino, phenylcarbonylamino, heterocyclyl-carbonylamino, heteroarylcarbonylamino, (C₁-C₆-alkoxy)amino , (C₁-C₆-alkoxy)(C₁-C₆-alkyl)amino, hydroxy(C₁-C₆-alkyl)amino, hydroxyamino, or phenyl-C₁-C₆-alkoxy;
wherein the phenyl substituent is unsubstituted or substituted by R^{a};
R^{a} is halogen, CN, NO₂, C₁-C₆-alkyl, C₁-C₆-haloalkyl, C₁-C₆-alkoxy, or C₁-C₆-haloalkoxy.

5. The pyridine compounds of formula (I) according to any of claims 1 to 4, wherein
R¹ is C₁-C₆-alkyl, C₁-C₆-alkoxy, C₁-C₆-haloalkoxy, C₃-C₆-alkenyloxy, C₃-C₆-haloalkenyloxy, C₃-C₆-alkynyloxy, C₃-C₆-haloalkynyloxy, C₁-C₆-alkylthio, or C₃-C₆-cycloalkyl, wherein the cycloalkyl substituent is unsubstituted;
R² is OH, C₁-C₆-alkoxy, C₁-C₆-haloalkoxy, C₃-C₆-alkynyloxy, C₁-C₆-alkylthio, phenyloxy, C₁-C₆-alkyl-carbonylamino, C₁-C₆-haloalkylcarbonylamino, phenylcarbonylamino, heterocyclyl-carbonylamino, heteroarylcarbonylamino, (C₁-C₆-alkoxy)amino , (C₁-C₆-alkoxy)(C₁-C₆-alkyl)amino, hydroxy(C₁-C₆-alkyl)amino, hydroxyamino, or phenyl-C₁-C₆-alkoxy, wherein the phenyl substituent is unsubstituted or substituted by R^{a};
R^{a} is halogen, CN, NO₂, C₁-C₆-alkyl, C₁-C₆-haloalkyl, C₁-C₆-alkoxy, or C₁-C₆-haloalkoxy;
Z is selected from rings A to O 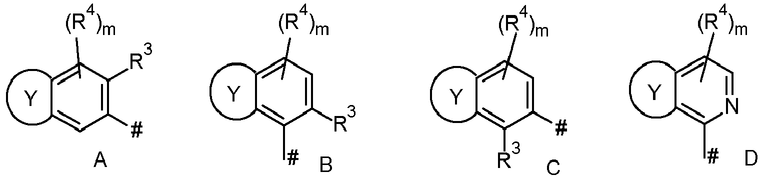 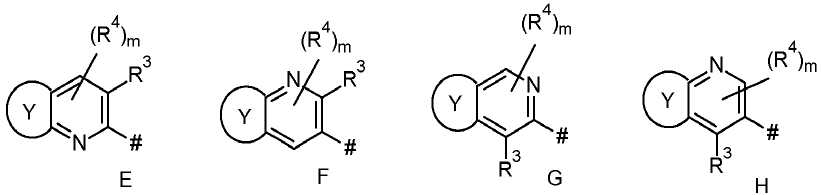 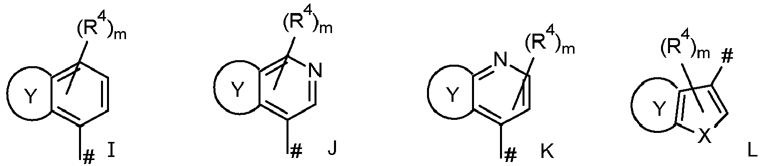 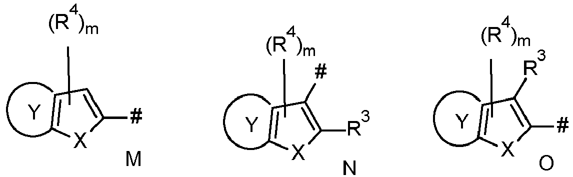
wherein
Y is 5- or 6-membered partially or fully unsaturated carbocycle comprising 0, 1, 2, or 3 heteroatoms selected from O, N, and S;
R³ is halogen, CN, C₁-C₆-alkyl, C₁-C₆-haloalkyl, or C₁-C₆-alkoxy;
m is 0, 1 or 2;
R⁴ is halogen, CN, C₁-C₆-alkyl, C₁-C₆-haloalkyl, or C₁-C₆-alkoxy;
X is O, S, or NR^{3A};
R^{3A} is H, C₁-C₆-alkyl, C₁-C₆-haloalkyl, C₁-C₆-alkylcarbonyl, C₃-C₆-alkenyl, C₃-C₆-haloalkenyl, C₃-C₆-alkenyl, C₃-C₆-haloalkenyl, or C₃-C₆-cycloalkyl;
# denotes the point of attachment to the pyridine ring.

6. A use of pyridine compounds of formula (I), including their agriculturally acceptable salts or derivatives, provided the compounds of formula (I) have an acidic functionality, according to any of claims 1 to 5, as herbicide.

7. A herbicidal composition comprising:
A) at least one pyridine compound of formula I, including their agriculturally acceptable salts or derivatives, provided the compounds of formula (I) have an acidic functionality, according to any of claims 1 to 5;
and
B) herbicides of class b1) to b15):
b1) lipid biosynthesis inhibitors;
b2) acetolactate synthase inhibitors (ALS inhibitors);
b3) photosynthesis inhibitors;
b4) protoporphyrinogen-IX oxidase inhibitors,
b5) bleacher herbicides;
b6) enolpyruvyl shikimate 3-phosphate synthase inhibitors (EPSP inhibitors);
b7) glutamine synthetase inhibitors;
b8) 7,8-dihydropteroate synthase inhibitors (DHP inhibitors);
b9) mitosis inhibitors;
b10) inhibitors of the synthesis of very long chain fatty acids (VLCFA inhibitors);
b11) cellulose biosynthesis inhibitors;
b12) decoupler herbicides;
b13) auxinic herbicides;
b14) auxin transport inhibitors; and
b15) other herbicides selected from the group consisting of bromobutide, chlorflurenol, chlorflurenol-methyl, cinmethylin, cumyluron, dalapon, dazomet, difenzoquat, difenzoquat-metilsulfate, dimethipin, DSMA, dymron, endothal and its salts, etobenzanid, flamprop, flamprop-isopropyl, flamprop-methyl, flamprop-M-isopropyl, flamprop-M-methyl, flurenol, flurenol-butyl, flurprimidol, fosamine, fosamine-ammonium, indanofan, indaziflam, maleic hydrazide, mefluidide, metam, methiozolin (CAS 403640-27-7), methyl azide, methyl bromide, methyl-dymron, methyl iodide, MSMA, oleic acid, oxaziclomefone, pelargonic acid, pyributicarb, quinoclamine, triaziflam, tridiphane and 6-chloro-3-(2-cyclopropyl-6-methylphenoxy)-4-pyridazinol, and its salts and esters;
including their agriculturally acceptable salts or derivatives.

8. A herbicidal composition comprising the herbicidal composition according to claim 7, and safeners.

9. The herbicidal composition according to claim 7 or 8, wherein the herbicidal composition comprises at least one herbicide B selected from herbicides of class b1, b2, b3, b4, b5, b6, b9, b10, b13, and b14.

10. The herbicidal composition according to any of claims 7 to 9, wherein the herbicidal composition comprises at least one herbicide B selected from herbicides of class b1, b2, b4, b5, b9, b10, b13, and b14.

11. The herbicidal composition according to any of claims 7 to 10, wherein the weight ratio of component A to component B is in the range of from 1:500 to 500:1.

12. A herbicidal composition comprising a herbicidal active amount of at least one pyridine compound of formula (I), including their agriculturally acceptable salts or derivatives, provided the compounds of formula (I) have an acidic functionality, according to any of claims 1 to 5, and at least one inert liquid and/or solid carrier and, if appropriate, at least one surface-active substance.

13. A herbicidal composition comprising a herbicidal composition according to any of claims 7 to 11, and at least one inert liquid and/or solid carrier and, if appropriate, at least one surface-active substance.

14. A method of controlling undesired vegetation, which comprises allowing a herbicidal active amount of at least one pyridine compound of formula (I), including their agriculturally acceptable salts or derivatives, provided the compounds of formula (I) have an acidic functionality, according to any of claims 1 to 5 or a herbicidal composition according to any of claims 7 to 13 to act on plants, their environment or on seed.

15. A use of the herbicidal compositions according to any of claims 7 to 13 as herbicides.
